# EUROPÄISCHE PATENTANMELDUNG

(11) **EP 2 246 328 A1**
(43) Veröffentlichungstag der Anmeldung: **03.11.2010**
(21) Anmeldenummer: 10162708.1
(22) Anmeldetag: 18.06.2005
(51) Int. Cl.: C07D 209/54, C07D 307/94, A61K 31/34, A61K 31/40, A01N 43/00

(54) **3'-Alkoxy spirocyclische Tetronsäuren**

(30) Priorität: 25.06.2004 DE 102004030753
(62) Teilanmeldung aus: 05754755.6
(71) Anmelder: Bayer CropScience AG, 40789 Monheim (DE)
(72) Erfinder: Fischer, Reiner, 40789 Monheim (DE); Gaertzen, Oliver, 50931 Köln (DE); Lehr, Stefan, 65835 Liederbach (DE); Feucht, Dieter, 65760 Eschborn (DE); Maslam, Olga, 51503 Rösrath (DE); Auler, Thomas, 42799 Leichlingen (DE); Hills, Martin Jeffrey, 65510 Idstein (DE); Bretschneider, Thomas, 53797 Lohmar (DE); Arnold, Christian, 40764 Langefeld (DE); Kehne, Heinz, 65719 Hofheim (DE); Rosinger, Christopher Hugh, 65719 Hofheim (DE)

(57) **Zusammenfassung**

Die Erfindung betrifft neue 3'-Alkoxy spirocyclische Tetronsäuren der Formel (I), 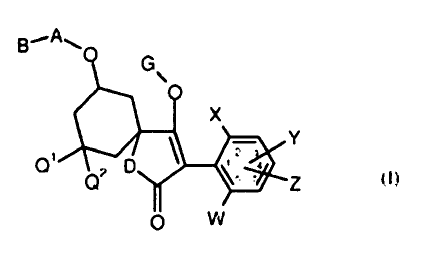 in welcher
A, B, D, Q¹, Q², G, W, X, Y und Z
die oben angegebenen Bedeutungen haben,

mehrere Verfahren und Zwischenprodukte zu ihrer Herstellung und ihre Verwendung als Schädlingsbekämpfungsmittel und/oder Herbizide und/oder Mikrobizide, sowie selektiv herbizide Mittel, die 3'-Alkoxy spirocyclische Tetram- und Tetronsäuren einerseits und zumindest eine die Kulturpflanzenverträglichkeit verbesserte Verbindung andererseits enthalten.

## Beschreibung

Die vorliegende Erfindung betrifft neue 3'-Alkoxy substituierte spirocyclische Ketoenole, mehrere Verfahren zu ihrer Herstellung und ihre Verwendung als Schädlingsbekämpfungsmittel, Mikrobizide und/oder Herbizide. Gegenstand der Erfindung sind auch selektiv herbizide Mittel, die 3'-Alkoxy substituierte spirocyclische Ketoenole einerseits und eine die Kulturpflanzenverträglichkeit verbessernde Verbindung andererseits enthalten.

1-H-Arylpyrrolidin-dion Derivate mit herbizider, insektizider oder akarizider Wirkung sind bekannt: EP-A-456 063, EP-A-521 334, EP-A-613 884, EP-A-613 885, WO 95/01 358, WO 98/06 721, WO 98/25 928, WO 99/16 748, WO 99/24 437 oder WO 01/17 972.

Weiterhin bekannt sind alkoxysubstituierte spirocyclische 1H-Arylpyrrolidin-dion-Derivate: EP-A-596 298, WO 95/26 954, WO 95/20 572, EP-A-0 668 267, WO 96/25 395, WO 96/35 664, WO 97/01 535, WO 97/02 243, WO 97/36 868, WO 98/05 638, WO 99/43 649, WO 99/48 869, WO 99/55 673, WO 01/23 354, WO 01/74 770, WO 01/17 972, WO 03/013 249, WO 2004/02 4688, WO 2004/065 366, WO 2004/08 0962, WO 2004/00 7448, WO 2004/111042, DE-A-1035 1646, DE-A-1035 4628, DE-A-1035 4629, DE-A-1035 1647.

Es ist bekannt, dass bestimmte Δ³-Dihydrofuran-2-on Derivate herbizide, insektizide oder akarizide Eigenschaften aufweisen: EP-A-528 156, EP-A-647637, WO 95/26 954, WO 96/20 196, WO 96/25 395, WO 96/35 664, WO 97/01 535, WO 97/02 243, WO 97/36 868, WO 98/05 638, WO 98/06 721, WO 99/16 748, WO 98/25 928, WO 99/43 649, WO 99/48 869, WO 99/55 673, WO 01/23354, WO 01/74 770, WO 01/17 972, WO 2004/024 688, WO 2004/080 962.

Die herbizide und/oder akarizide und/oder insektizide Wirksamkeit und/oder Wirkungsbreite und/oder die Pflanzenverträglichkeit der bekannten Verbindungen, insbesondere gegenüber Kulturpflanzen, ist jedoch nicht immer ausreichend.

Es wurden nun neue Verbindungen der Formel (I) gefunden,
in welcher
- W: 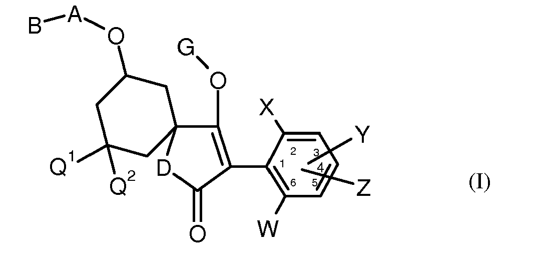 für Wasserstoff, Alkyl, Alkenyl, Alkinyl, Halogen, Alkoxy, Halogenalkyl, Halogenalkoxy oder Cyano steht,
- X: für Halogen, Alkyl, Alkenyl, Alkinyl, Alkoxy, Alkoxy-alkoxy, Halogenalkyl, Halogenalkoxy oder Cyano steht,
- Y: in der 4-Position für Wasserstoff, Halogen, Alkoxy, Cyano, Halogenalkyl oder Halogenalkoxy steht,
- Z: für Wasserstoff steht.
- W: auch für Wasserstoff, Halogen oder Alkyl steht,
- X: auch für Halogen, Alkyl, Alkoxy, Halogenalkyl, Halogenalkoxy oder Cyano steht,
- Y: in der 4-Position auch für gegebenenfalls substituiertes Phenyl oder Hetaryl steht,
- Z: auch für Wasserstoff steht.
- W: ebenfalls für Wasserstoff, Halogen oder Alkyl steht,
- X: ebenfalls für Halogen, Alkyl, Alkoxy, Halogenalkyl, Halogenalkoxy oder Cyano steht,
- Y: ebenfalls in der 5-Position für gegebenenfalls substituiertes Phenyl oder Hetaryl steht,
- Z: in der 4-Position ebenfalls für Wasserstoff, Alkyl oder Halogen steht.
- W: außerdem für Wasserstoff, Alkyl, Alkenyl, Alkinyl, Alkoxy, Halogen, Cyano oder Trifluormethyl steht,
- X: außerdem für Halogen, Alkyl, Alkenyl, Alkinyl, Alkoxy, Alkoxy-alkoxy, Halogenalkyl, Halogenalkoxy oder Cyano steht,
- Y: in der 4-Position außerdem für Alkyl steht,
- Z: außerdem für Wasserstoff steht.
- W: weiterhin für Wasserstoff, Halogen, Alkyl oder Alkoxy steht,
- X: weiterhin für Halogen, Alkyl, Alkoxy, Halogenalkyl, Halogenalkoxy oder Cyano steht,
- Y: in der 4-Position weiterhin für Wasserstoff, Halogen, Alkyl, Halogenalkyl oder Halogenalkoxy steht,
- Z: in der 3- oder 5-Position weiterhin für Halogen, Alkyl, Halogenalkyl, Cyano, Alkoxy oder Halogenalkoxy steht.
- A: für eine gegebenenfalls substituierte Alkandiylgruppe oder für gegebenenfalls substituiertes und/oder gegebenenfalls durch ein Heteroatom unterbrochenes Cycloalkyl steht,
- B: für Wasserstoff oder jeweils gegebenenfalls substituiertes Alkyl, Alkenyl, Alkoxy, Alkoxy-alkoxy, Phenyl, Hetaryl oder für gegebenenfalls substituiertes und/oder gegebenenfalls durch Heteroatome und/oder C=O unterbrochenes Cycloalkyl steht,
- D: für NH oder Sauerstoff steht,
- Q¹: für Wasserstoff, für jeweils gegebenenfalls substituiertes Alkyl, Alkoxy, Alkoxyalkyl oder Alkylthioalkyl, für gegebenenfalls substituiertes Cycloalkyl, worin gegebenenfalls eine Methylengruppe durch Heteroatome ersetzt ist oder für gegebenenfalls substituiertes Phenyl, Hetaryl, Phenylalkyl oder Hetarylalkyl steht,
- Q²: für Wasserstoff oder Alkyl steht,
- Q¹ und Q²: gemeinsam mit dem Kohlenstoff, an das sie gebunden sind für einen gegebenenfalls substituierten C₃-C₆-Ring stehen, der gegebenenfalls durch ein Heteroatom unterbrochen sein kann,
- G: für Wasserstoff (a) oder für eine der Gruppen 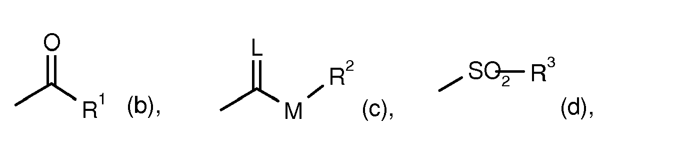 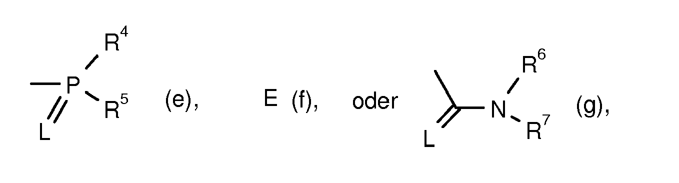 steht,
worin
E für ein Metallion oder ein Ammoniumion steht,
L für Sauerstoff oder Schwefel steht,
M für Sauerstoff oder Schwefel steht,
- R¹: für jeweils gegebenenfalls durch Halogen oder Cyano substituiertes Alkyl, Alkenyl, Alkoxyalkyl, Alkylthioalkyl oder Polyalkoxyalkyl oder für jeweils gegebenenfalls durch Halogen, Alkyl oder Alkoxy substituiertes Cycloalkyl oder Heterocyclyl oder für jeweils gegebenenfalls substituiertes Phenyl, Phenylalkyl, Hetaryl, Phenoxyalkyl oder Hetaryloxyalkyl steht,
- R²: für jeweils gegebenenfalls durch Halogen oder Cyano substituiertes Alkyl, Alkenyl, Alkoxyalkyl oder Polyalkoxyalkyl oder für jeweils gegebenenfalls substituiertes Cycloalkyl, Phenyl oder Benzyl steht,
- R³, R⁴ und R⁵: unabhängig voneinander für jeweils gegebenenfalls durch Halogen substituiertes Alkyl, Alkoxy, Alkylamino, Dialkylamino, Alkylthio, Alkenylthio oder Cycloalkylthio oder für jeweils gegebenenfalls substituiertes Phenyl, Benzyl, Phenoxy oder Phenylthio stehen,
- R⁶ und R⁷: unabhängig voneinander für Wasserstoff, für jeweils gegebenenfalls durch Halogen oder Cyano substituiertes Alkyl, Cycloalkyl, Alkenyl, Alkoxy, Alkoxyalkyl, für jeweils gegebenenfalls substituiertes Phenyl oder Benzyl stehen, oder gemeinsam mit dem N-Atom, an das sie gebunden sind, einen gegebenenfalls Sauerstoff oder Schwefel enthaltenden und gegebenenfalls substituierten Cyclus bilden.

Die Verbindungen der Formel (I) können, auch in Abhängigkeit von der Art der Substituenten, als optische Isomere oder Isomerengemische, in unterschiedlicher Zusammensetzung vorliegen, die gegebenenfalls in üblicher Art und Weise getrennt werden können. Sowohl die reinen Isomeren als auch die Isomerengemische, deren Herstellung und Verwendung sowie diese enthaltende Mittel sind Gegenstand der vorliegenden Erfindung. Im folgenden wird der Einfachheit halber jedoch stets von Verbindungen der Formel (I) gesprochen, obwohl sowohl die reinen Verbindungen als gegebenenfalls auch Gemische mit unterschiedlichen Anteilen an isomeren Verbindungen gemeint sind.

Unter Einbeziehung von D für NH (1) und D für O (2) ergeben sich folgende hauptsächliche Strukturen (I-1) bis (I-2): 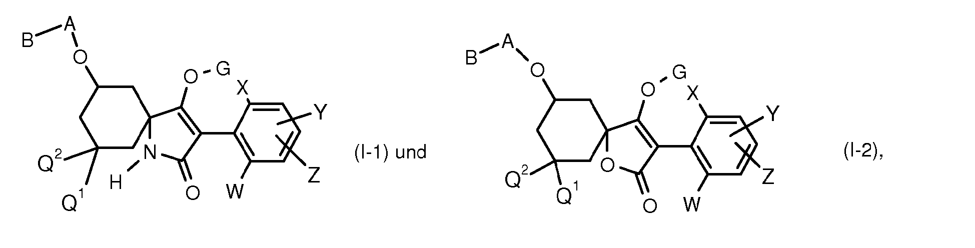 worin

A, B, G, Q¹, Q², W, X, Y und Z die oben angegebene Bedeutung haben.

Unter Einbeziehung der verschiedenen Bedeutungen (a), (b), (c), (d), (e), (f) und (g) der Gruppe G ergeben sich folgende hauptsächliche Strukturen (I-1-a) bis (I-1-g), wenn D für NH (1) steht, 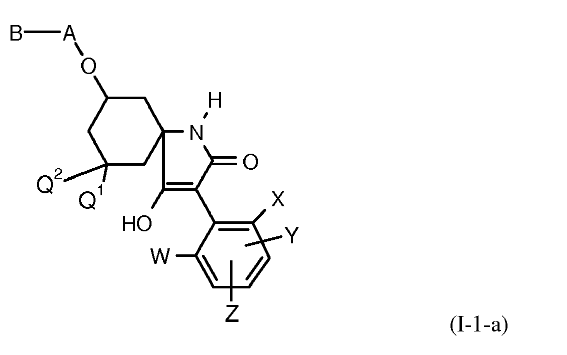 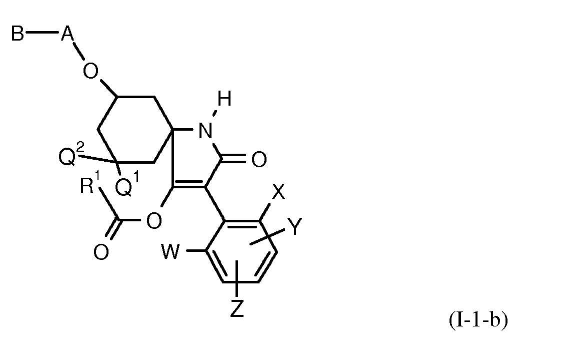 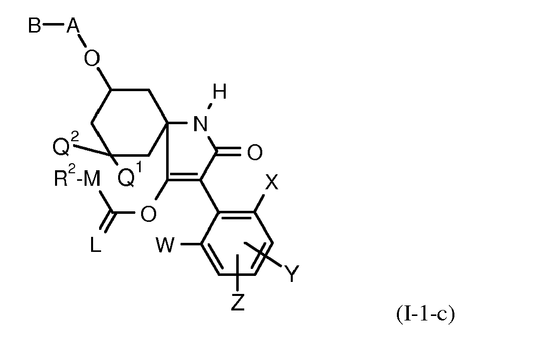 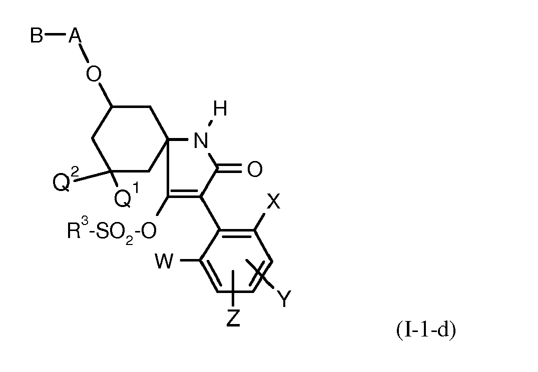 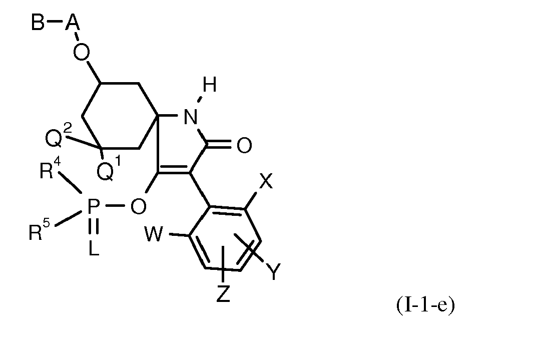 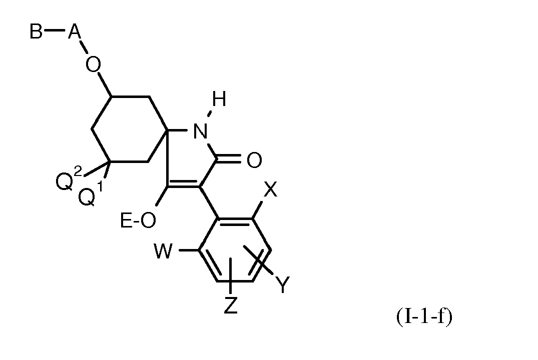 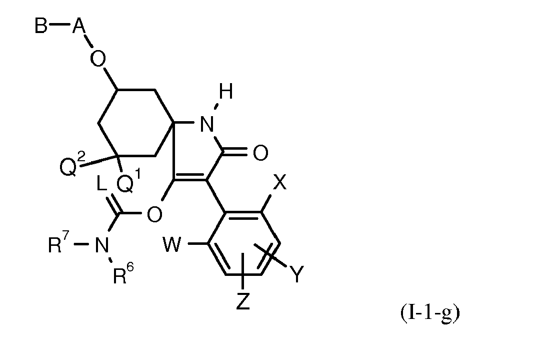 worin
A, B, E, L, M, Q¹, Q², W, X, Y, Z, R¹, R², R³, R⁴, R⁵, R⁶ und R⁷ die oben angegebenen Bedeutungen besitzen.

Unter Einbeziehung der verschiedenen Bedeutungen (a), (b), (c), (d), (e), (f) und (g) der Gruppe G ergeben sich folgende hauptsächliche Strukturen (I-2-a) bis (I-2-g), wenn D für O (2) steht, 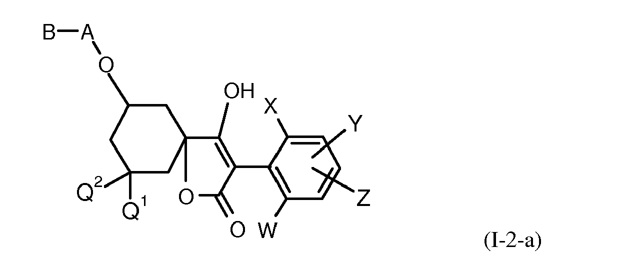 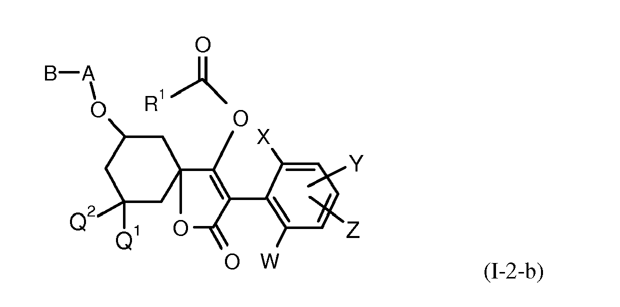 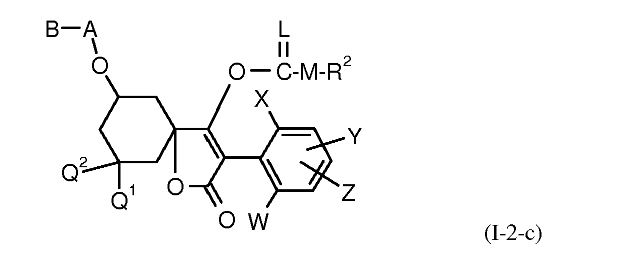 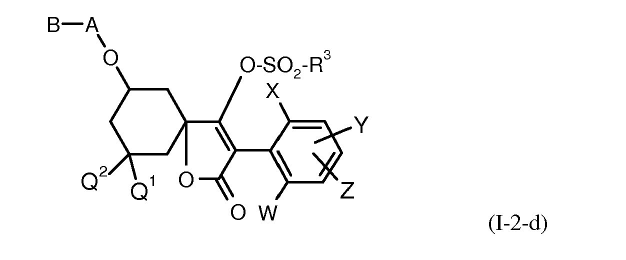 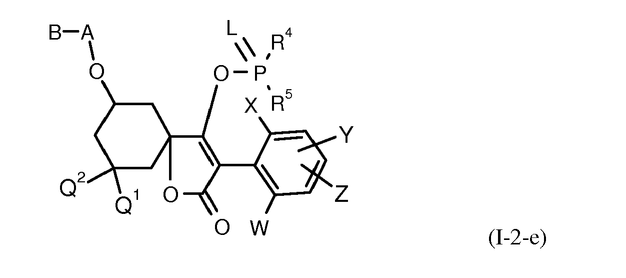 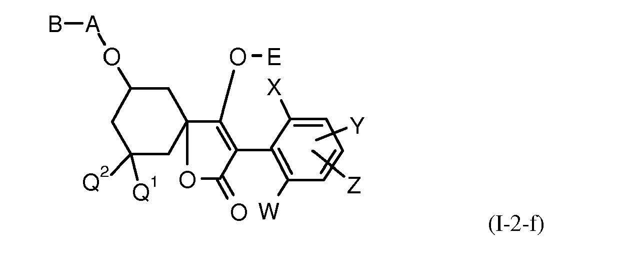 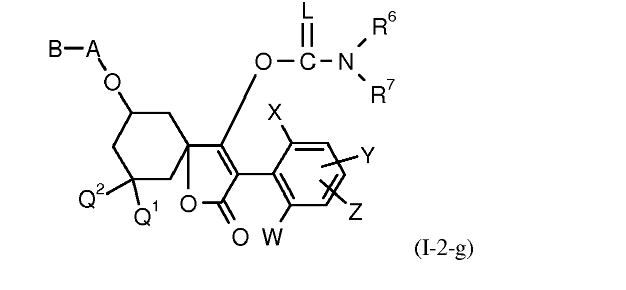 worin
A, B, E, L, M, Q¹, Q², W, X, Y, Z, R¹, R², R³, R⁴, R⁵, R⁶ und R⁷ die oben angegebene Bedeutung haben.

Weiterhin wurde gefunden, dass man die neuen Verbindungen der Formel (I) nach den im Folgenden beschriebenen Verfahren erhält:
(A) Man erhält Verbindungen der Formel (I-1-a) 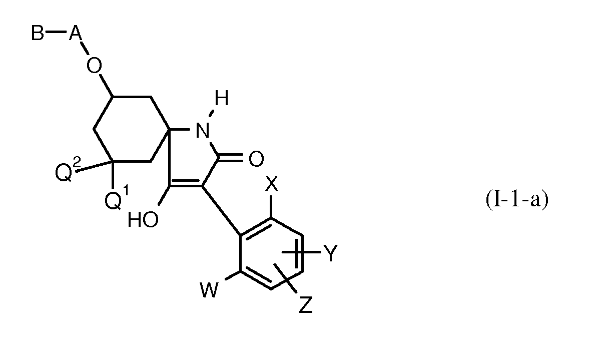 in welcher
   A, B, Q¹, Q², W, X, Y und Z die oben angegebenen Bedeutungen haben,
   wenn man
   Verbindungen der Formel (II) 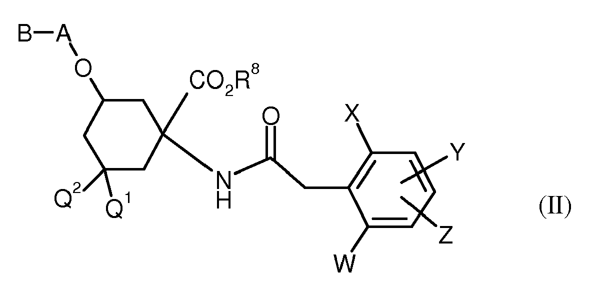 in welcher
   A, B, Q¹, Q², W, X, Y und Z die oben angegebenen Bedeutungen haben,
   und
   R⁸ für Alkyl (bevorzugt C₁-C₆-Alkyl) steht,
   in Gegenwart eines Verdünnungsmittels und in Gegenwart einer Base intramolekular kondensiert.
(B) Außerdem wurde gefunden, dass man Verbindungen der Formel (I-2-a) 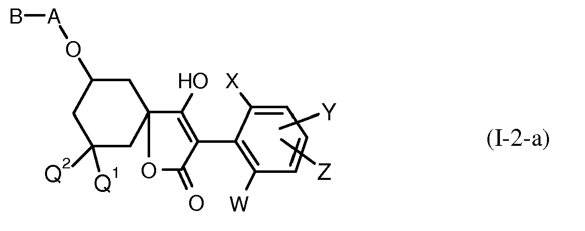 in welcher
   A, B, Q¹, Q², W, X, Y und Z die oben angegebenen Bedeutungen haben,
   erhält, wenn man
   Verbindungen der Formel (III) 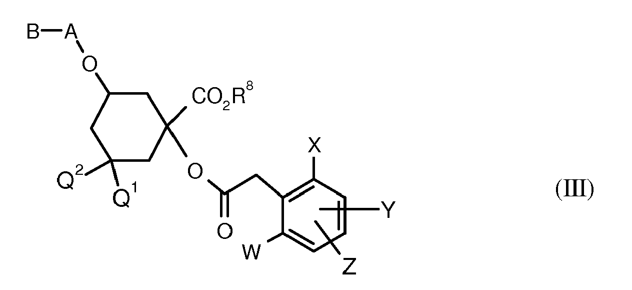 in welcher
   A, B, Q¹, Q², W, X, Y, Z und R⁸ die oben angegebenen Bedeutungen haben,
   in Gegenwart eines Verdünnungsmittels und in Gegenwart einer Base intramolekular kondensiert.
   Außerdem wurde gefunden
(C) dass man die Verbindungen der oben gezeigten Formeln (I-1-b) bis (I-2-b), in welchen R¹, A, B, Q¹, Q²,W, X, Y und Z die oben angegebenen Bedeutungen haben, erhält, wenn man Verbindungen der oben gezeigten Formeln (I-1-a) bis (I-2-a), in welchen A, B, Q¹, Q², W, X, Y und Z die oben angegebenen Bedeutungen haben, jeweils
   α) mit Verbindungen der Formel (IV) 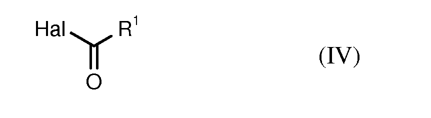 in welcher
      R¹ die oben angegebene Bedeutung hat und
      Hal für Halogen (insbesondere Chlor oder Brom) steht
      oder
   β) mit Carbonsäureanhydriden der Formel (V)

      R¹-CO-O-CO-R¹ (V)

      in welcher
      R¹ die oben angegebene Bedeutung hat,
   gegebenenfalls in Gegenwart eines Verdünnungsmittels und gegebenenfalls in Gegenwart eines Säurebindemittels umsetzt;
(D) dass man die Verbindungen der oben gezeigten Formeln (I-1-c) bis (I-2-c), in welchen R², A, B, Q¹, Q², W, M, X, Y und Z die oben angegebenen Bedeutungen haben und L für Sauerstoff steht, erhält, wenn man Verbindungen der oben gezeigten Formeln (I-1-a) bis (I-2-a), in welchen A, B, Q¹, Q², W, X, Y und Z die oben angegebenen Bedeutungen haben, jeweils
   mit Chlorameisensäureestern oder Chlorameisensäurethioestern der Formel (VI)

   R²-M-CO-Cl (VI)

   in welcher
   R² und M die oben angegebenen Bedeutungen haben,
   gegebenenfalls in Gegenwart eines Verdünnungsmittels und gegebenenfalls in Gegenwart eines Säurebindemittels umsetzt;
(E) dass man Verbindungen der oben gezeigten Formeln (I-1-c) bis (I-2-c), in welchen R², A, B, Q¹, Q², W, M, X, Y und Z die oben angegebenen Bedeutungen haben und L für Schwefel steht, erhält, wenn man Verbindungen der oben gezeigten Formeln (I-1-a) bis (I-2-a), in welchen A, B, Q¹, Q², W, X, Y und Z die oben angegebenen Bedeutungen haben, jeweils
   mit Chlormonothioameisensäureestern oder Chlordithioameisensäureestern der Formel (VII) 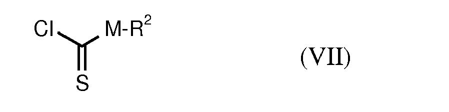 in welcher
   M und R² die oben angegebenen Bedeutungen haben,
   gegebenenfalls in Gegenwart eines Verdünnungsmittels und gegebenenfalls in Gegenwart eines Säurebindemittels umsetzt,
(F) dass man Verbindungen der oben gezeigten Formeln (I-1-d) bis (I-2-d), in welchen R³, A, B, W, Q¹, Q² X, Y und Z die oben angegebenen Bedeutungen haben, erhält, wenn man Verbindungen der oben gezeigten Formeln (I-1-a) bis (I-2-a), in welchen A, B, Q¹, Q², W, X, Y und Z die oben angegebenen Bedeutungen haben, jeweils
   mit Sulfonsäurechloriden der Formel (VIII)

   R³-SO₂-Cl (VIII)

   in welcher
   R³ die oben angegebene Bedeutung hat,
   gegebenenfalls in Gegenwart eines Verdünnungsmittels und gegebenenfalls in Gegenwart eines Säurebindemittels umsetzt,
(G) dass man Verbindungen der oben gezeigten Formeln (I-1-e) bis (I-2-e), in welchen L, R⁴, R⁵, A, B, Q¹, Q², W, X, Y und Z die oben angegebenen Bedeutungen haben, erhält, wenn man Verbindungen der oben gezeigten Formeln (I-1-a) bis (I-2-a), in welchen A, B, Q¹, Q², W, X, Y und Z die oben angegebenen Bedeutungen haben, jeweils
   mit Phosphorverbindungen der Formel (IX) 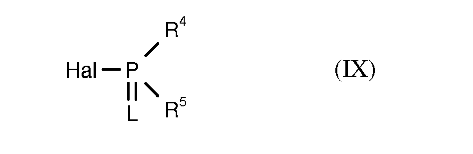 in welcher
   L, R⁴ und R⁵ die oben angegebenen Bedeutungen haben und
   Hal für Halogen (insbesondere Chlor oder Brom) steht,
   gegebenenfalls in Gegenwart eines Verdünnungsmittels und gegebenenfalls in Gegenwart eines Säurebindemittels umsetzt,
(H) dass man Verbindungen der oben gezeigten Formeln (I-1-f) bis (I-2-f), in welchen E, A, B, Q¹ Q² W, X, Y und Z die oben angegebenen Bedeutungen haben, erhält, wenn man Verbindungen der oben gezeigten Formeln (I-1-a) bis (I-2-a), in welchen A, B, Q¹, Q², W, X, Y und Z die oben angegebenen Bedeutungen haben, jeweils
   mit Metallverbindungen oder Aminen der Formeln (X) oder (XI)

   Me(OR¹⁰)ₜ (X)

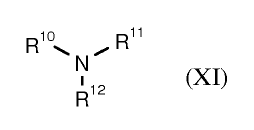

   in welchen
   - Me: für ein ein- oder zweiwertiges Metall (bevorzugt ein Alkali- oder Erdalkalimetall wie Lithium, Natrium, Kalium, Magnesium oder Calcium),
   - t: für die Zahl 1 oder 2 und
   - R¹⁰, R¹¹, R¹²: unabhängig voneinander für Wasserstoff oder Alkyl (bevorzugt C₁-C₈-Alkyl) stehen,
   gegebenenfalls in Gegenwart eines Verdünnungsmittels umsetzt,
(I) dass man Verbindungen der oben gezeigten Formeln (I-1-g) bis (I-2-g), in welchen L, R⁶, R⁷, A, B, Q¹, Q², W, X, Y und Z die oben angegebenen Bedeutungen haben, erhält, wenn man Verbindungen der oben gezeigten Formeln (I-1-a) bis (I-2-a), in welchen A, B, Q¹, Q², W, X, Y und Z die oben angegebenen Bedeutungen haben, jeweils
   α) mit Isocyanaten oder Isothiocyanaten der Formel (XII)

      R⁶-N=C=L (XII)

      in welcher
      R⁶ und L die oben angegebenen Bedeutungen haben,
      gegebenenfalls in Gegenwart eines Verdünnungsmittels und gegebenenfalls in Gegenwart eines Katalysators umsetzt oder
   β) mit Carbamidsäurechloriden oder Thiocarbamidsäurechloriden der Formel (XIII) 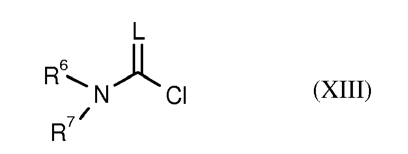 in welcher
      L, R⁶ und R⁷ die oben angegebenen Bedeutungen haben,
      gegebenenfalls in Gegenwart eines Verdünnungsmittels und gegebenenfalls in Gegenwart eines Säurebindemittels, umsetzt.

Weiterhin wurde gefunden, dass die neuen Verbindungen der Formel (I) eine gute Wirksamkeit als Schädlingsbekämpfungsmittel, vorzugsweise als Insektizide, Akarizide und/oder Fungizide und/oder Herbizide aufweisen und darüber hinaus häufig sehr gut pflanzenverträglich, insbesondere gegenüber Kulturpflanzen, sind.

Überraschenderweise wurde nun auch gefunden, dass bestimmte substituierte, cyclische Ketoenole bei gemeinsamer Anwendung mit den im weiteren beschriebenen, die Kulturpflanzen-Verträglichkeit verbessernden Verbindungen (Safenern/Antidots) ausgesprochen gut die Schädigung der Kulturpflanzen verhindern und besonders vorteilhaft als breit wirksame Kombinationspräparate zur selektiven Bekämpfung von unerwünschten Pflanzen in Nutzpflanzenkulturen, wie z.B. in Getreide aber auch Mais, Soja und Reis, verwendet werden können.

Gegenstand der Erfindung sind auch selektiv-herbizide Mittel enthaltend einen wirksamen Gehalt an einer Wirkstoffkombination umfassend als Komponenten
(a') mindestens ein substituiertes, cyclisches Ketoenol der Formel (I), in welcher A, B, D, G, Q¹, Q² W, X, Y und Z die oben angegebene Bedeutung haben
   und
(b') zumindest eine die Kulturpflanzen-Verträglichkeit verbessernde Verbindung aus der folgenden Gruppe von Verbindungen:

4-Dichloracetyl-1-oxa-4-aza-spiro[4.5]-decan (AD-67, MON-4660), 1-Dichloracetyl-hexahydro-3,3,8a-trimethylpyrrolo[1,2-a]-pyrimidin-6(2H)-on (Dicyclonon, BAS-145138), 4-Dichloracetyl-3,4-dihydro-3-methyl-2H-1,4-benzoxazin (Benoxacor), 5-Chlor-chinolin-8-oxy-essigsäure-(1-methyl-hexylester) (Cloquintocet-mexyl - vgl. auch verwandte Verbindungen in EP-A-86750, EP-A-94349, EP-A-191736, EP-A-492366), 3-(2-Chlor-benzyl)-1-(1-methyl-1-phenyl-ethyl)-harnstoff (Cumyluron), a-(Cyanomethoximino)-phenylacetonitril (Cyometrinil), 2,4-Dichlor-phenoxyessigsäure (2,4-D), 4-(2,4-Dichlor-phenoxy)-buttersäure (2,4-DB), 1-(1-Methyl-1-phenyl-ethyl)-3-(4-methyl-phenyl)-harnstoff (Daimuron, Dymron), 3,6-Dichlor-2-methoxy-benzoesäure (Dicamba), Piperidin-1-thiocarbonsäure-S-1-methyl-1-phenyl-ethylester (Dimepiperate), 2,2-Dichlor-N-(2-oxo-2-(2-propenylamino)-ethyl)-N-(2-propenyl)-acetamid (DKA-24), 2,2-Dichlor-N,N-di-2-propenyl-acetamid (Dichlormid), 4,6-Dichlor-2-phenyl-pyrimidin (Fenclorim), 1-(2,4-Dichlorphenyl)-5-trichlormethyl-1H-1,2,4-triazol-3-carbonsäure-ethylester (Fenchlorazole-ethyl - vgl. auch verwandte Verbindungen in EP-A-174562 und EP-A-346620), 2-Chlor-4-trifluormethyl-thiazol-5-carbonsäure-phenylmethylester (Flurazole), 4-Chlor-N-(1,3-dioxolan-2-yl-methoxy)-α-trifluor-acetophenonoxim (Fluxofenim), 3-Dichloracetyl-5-(2-furanyl)-2,2-dimethyl-oxazolidin (Furilazole, MON-13900), Ethyl-4,5-dihydro-5,5-diphenyl-3-isoxazolcarboxylat (Isoxadifen-ethyl - vgl. auch verwandte Verbindungen in WO-A-95/07897), 1-(Ethoxycarbonyl)-ethyl-3,6-dichlor-2-methoxybenzoat (Lactidichlor), (4-Chlor-o-tolyloxy)-essigsäure (MCPA), 2-(4-Chlor-o-tolyloxy)-propionsäure (Mecoprop), Diethyl-1-(2,4-dichlor-phenyl)-4,5-dihydro-5-methyl-1H-pyrazol-3,5-dicarboxylat (Mefenpyr-diethyl - vgl. auch verwandte Verbindungen in WO-A-91/07874) 2-Dichlormethyl-2-methyl-1,3-dioxolan (MG-191), 2-Propenyl-1-oxa-4-azaspiro[4.5]decane-4-carbodithioate (MG-838), 1,8-Naphthalsäureanhydrid, α-(1,3-Dioxolan-2-yl-methoximino)-phenylacetonitril (Oxabetrinil), 2,2-Dichlor-N-(1,3-dioxolan-2-yl-methyl)-N-(2-propenyl)-acetamid (PPG-1292), 3-Dichloracetyl-2,2-dimethyl-oxazolidin (R-28725), 3-Dichloracetyl-2,2,5-trimethyloxazolidin (R-29148), 4-(4-Chlor-o-tolyl)-buttersäure, 4-(4-Chlor-phenoxy)-buttersäure, Diphenylmethoxyessigsäure, Diphenylmethoxyessigsäure-methylester, Diphenylmethoxyessigsäure-ethylester, 1-(2-Chlor-phenyl)-5-phenyl-1H-pyrazol-3-carbonsäure-methylester, 1-(2,4-Dichlor-phenyl)-5-methyl-1H-pyrazol-3-carbonsäure-ethylester, 1-(2,4-Dichlor-phenyl)-5-isopropyl-1H-pyrazol-3-carbonsäure-ethylester, 1-(2,4-Dichlor-phenyl)-5-(1,1-dimethyl-ethyl)-1H-pyrazol-3-carbonsäure-ethylester, 1-(2,4-Dichlor-phenyl)-5-phenyl-1H-pyrazol-3-carbonsäure-ethylester (vgl. auch verwandte Verbindungen in EP-A-269806 und EP-A-333131), 5-(2,4-Dichlor-benzyl)-2-isoxazolin-3-carbonsäure-ethylester, 5-Phenyl-2-isoxazolin-3-carbonsäure-ethylester, 5-(4-Fluor-phenyl)-5-phenyl-2-isoxazolin-3-carbonsäure-ethylester (vgl. auch verwandte Verbindungen in WO-A-91/08202), 5-Chlor-chinolin-8-oxy-essigsäure-(1,3-dimethyl-but-1-yl)-ester, 5-Chlor-chinolin-8-oxy-essigsäure-4-allyloxy-butylester, 5-Chlor-chinolin-8-oxy-essigsäure-1-allyloxy-prop-2-yl-ester, 5-Chlor-chinoxalin-8-oxy-essigsäure-methylester, 5-Chlor-chinolin-8-oxy-essigsäure-ethylester, 5-Chlor-chinoxalin-8-oxy-essigsäure-allylester, 5-Chlor-chinolin-8-oxy-essigsäure-2-oxo-prop-1-yl-ester, 5-Chlor-chinolin-8-oxy-malonsäure-diethylester, 5-Chlor-chinoxalin-8-oxy-malonsäure-di-allylester, 5-Chlor-chinolin-8-oxy-malonsäure-diethylester (vgl. auch verwandte Verbindungen in EP-A-582198), 4-Carboxy-chroman-4-yl-essigsäure (AC-304415, vgl. EP-A-613618), 4-Chlor-phenoxy-essigsäure, 3,3'-Dimethyl-4-methoxy-benzophenon, 1-Brom-4-chlormethylsulfonylbenzol, 1-[4-(N-2-Methoxybenzoylsulfamoyl)-phenyl]-3-methyl-harnstoff (alias N-(2-Methoxybenzoyl)-4-[(methylamino-carbonyl)-amino]-benzolsulfonamid), 1-[4-(N-2-Methoxybenzoylsulfamoyl)-phenyl]-3,3-dimethyl-harnstoff, 1-[4-(N-4,5-Dimethylbenzoylsulfamoyl)-phenyl]-3-methyl-harnstoff, 1-[4-(N-Naphthylsulfamoyl)-phenyl]-3,3-dimethyl-harnstoff, N-(2-Methoxy-5-methyl-benzoyl)-4-(cyclopropylaminocarbonyl)-benzolsulfonamid,
und/oder eine der folgenden durch allgemeine Formeln definierten Verbindungen
der allgemeinen Formel (IIa) 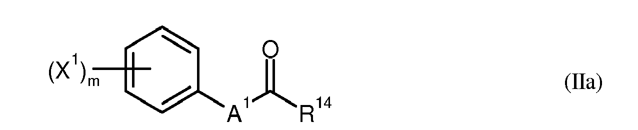 oder der allgemeinen Formel (IIb) 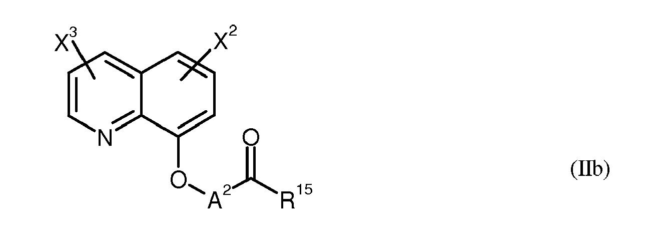 oder der Formel (IIc) 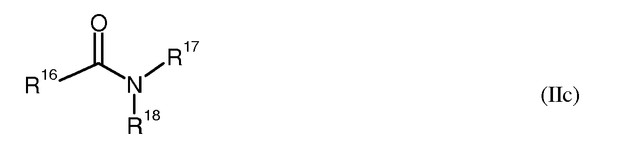 wobei
- m: für eine Zahl 0, 1, 2, 3, 4 oder 5 steht,
- A¹: für eine der nachstehend skizzierten divalenten heterocyclischen Gruppierungen steht, 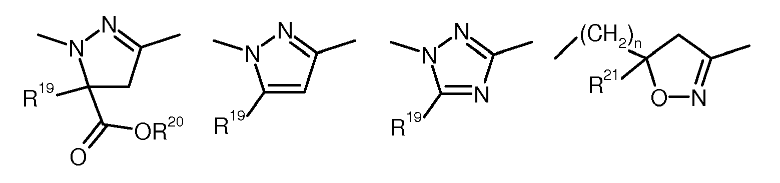
- n: für eine Zahl 0, 1, 2, 3, 4 oder 5 steht,
- A²: für gegebenenfalls durch C₁-C₄-Alkyl und/oder C₁-C₄-Alkoxy-carbonyl und/oder C₁-C₄-Alkenyloxy-carbonyl substituiertes Alkandiyl mit 1 oder 2 Kohlenstoffatomen steht,
- R¹⁴: für Hydroxy, Mercapto, Amino, C₁-C₆-Alkoxy, C₁-C₆-Alkylthio, C₁-C₆-Alkylamino oder Di-(C₁-C₄-alkyl)-amino steht,
- R¹⁵: für Hydroxy, Mercapto, Amino, C₁-C₇-Alkoxy, C₁-C₆-Alkenyloxy, C₁-C₆-Alkenyloxy-C₁-C₆-alkoxy, C₁-C₆-Alkylthio, C₁-C₆-Alkylamino oder Di-(C₁-C₄-alkyl)-amino steht,
- R¹⁶: für gegebenenfalls durch Fluor, Chlor und/oder Brom substituiertes C₁-C₄-Alkyl steht,
- R¹⁷: für Wasserstoff, jeweils gegebenenfalls durch Fluor, Chlor und/oder Brom substituiertes C₁-C₆-Alkyl, C₂-C₆-Alkenyl oder C₂-C₆-Alkinyl, C₁-C₄-Alkoxy-C₁-C₄-alkyl, Dioxolanyl-C₁-C₄-alkyl, Furyl, Furyl-C₁-C₄-alkyl, Thienyl, Thiazolyl, Piperidinyl, oder gegebenenfalls durch Fluor, Chlor und/oder Brom oder C₁-C₄-Alkyl substituiertes Phenyl steht,
- R¹⁸: für Wasserstoff, jeweils gegebenenfalls durch Fluor, Chlor und/oder Brom substituiertes C₁-C₆-Alkyl, C₂-C₆-Alkenyl oder C₂-C₆-Alkinyl, C₁-C₄-Alkoxy-C₁-C₄-alkyl, Dioxolanyl-C₁-C₄-alkyl, Furyl, Furyl-C₁-C₄-alkyl, Thienyl, Thiazolyl, Piperidinyl, oder gegebenenfalls durch Fluor, Chlor und/oder Brom oder C₁-C₄-Alkyl substituiertes Phenyl steht, R¹⁷ und R¹⁸ auch gemeinsam für jeweils gegebenenfalls durch C₁-C₄-Alkyl, Phenyl, Furyl, einen annellierten Benzolring oder durch zwei Substituenten, die gemeinsam mit dem C-Atom, an das sie gebunden sind, einen 5- oder 6-gliedrigen Carboxyclus bilden, substituiertes C₃-C₆-Alkandiyl oder C₂-C₅-Oxaalkandiyl steht,
- R¹⁹: für Wasserstoff, Cyano, Halogen, oder für jeweils gegebenenfalls durch Fluor, Chlor und/oder Brom substituiertes C₁-C₄-Alkyl, C₃-C₆-Cycloalkyl oder Phenyl steht,
- R²⁰: für Wasserstoff, jeweils gegebenenfalls durch Hydroxy, Cyano, Halogen oder C₁-C₄-Alkoxy substituiertes C₁-C₆-Alkyl, C₃-C₆-Cycloalkyl oder Tri-(C₁-C₄-alkyl)-silyl steht,
- R²¹: für Wasserstoff, Cyano, Halogen, oder für jeweils gegebenenfalls durch Fluor, Chlor und/oder Brom substituiertes C₁-C₄-Alkyl, C₃-C₆-Cycloalkyl oder Phenyl steht,
- X¹: für Nitro, Cyano, Halogen, C₁-C₄-Alkyl, C₁-C₄-Halogenalkyl, C₁-C₄-Alkoxy oder C₁-C₄-Halogenalkoxy steht,
- X²: für Wasserstoff, Cyano, Nitro, Halogen, C₁-C₄-Alkyl, C₁-C₄-Halogenalkyl, C₁-C₄-Alkoxy oder C₁-C₄-Halogenalkoxy steht,
- X³: für Wasserstoff, Cyano, Nitro, Halogen, C₁-C₄-Alkyl, C₁-C₄-Halogenalkyl, C₁-C₄-Alkoxy oder C₁-C₄-Halogenalkoxy steht,
und/oder die folgenden durch allgemeine Formeln definierten Verbindungen
der allgemeinen Formel (IId) 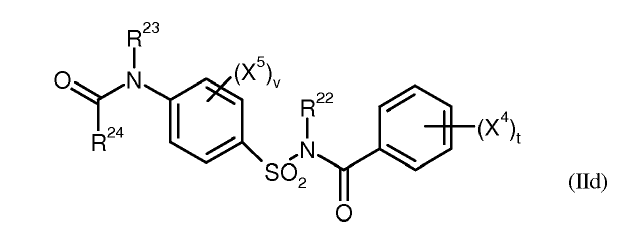 oder der allgemeinen Formel (IIe) 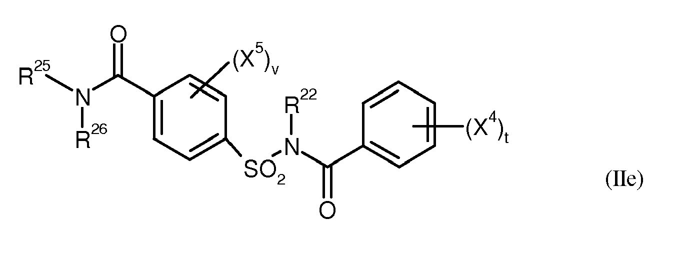 wobei
- t: für eine Zahl 0, 1, 2, 3, 4 oder 5 steht,
- v: für eine Zahl 0, 1, 2, 3, 4 oder 5 steht,
- R²²: für Wasserstoff oder C₁-C₄-Alkyl steht,
- R²³: für Wasserstoff oder C₁-C₄-Alkyl steht,
- R²⁴: für Wasserstoff, jeweils gegebenenfalls durch Cyano, Halogen oder C₁-C₄-Alkoxy substituiertes C₁-C₆-Alkyl, C₁-C₆-Alkoxy, C₁-C₆-Alkylthio, C₁-C₆-Alkylamino oder Di-(C₁-C₄-alkyl)-amino, oder jeweils gegebenenfalls durch Cyano, Halogen oder C₁-C₄-Alkyl substituiertes C₃-C₆-Cycloalkyl, C₃-C₆-Cycloalkyloxy, C₃-C₆-Cycloalkylthio oder C₃-C₆-Cycloalkylamino steht,
- R²⁵: für Wasserstoff, gegebenenfalls durch Cyano, Hydroxy, Halogen oder C₁-C₄-Alkoxy substituiertes C₁-C₆-Alkyl, jeweils gegebenenfalls durch Cyano oder Halogen substituiertes C₃-C₆-Alkenyl oder C₃-C₆-Alkinyl, oder gegebenenfalls durch Cyano, Halogen oder C₁-C₄-Alkyl substituiertes C₃-C₆-Cycloalkyl steht,
- R²⁶: für Wasserstoff, gegebenenfalls durch Cyano, Hydroxy, Halogen oder C₁-C₄-Alkoxy substituiertes C₁-C₆-Alkyl, jeweils gegebenenfalls durch Cyano oder Halogen substituiertes C₃-C₆-Alkenyl oder C₃-C₆-Alkinyl, gegebenenfalls durch Cyano, Halogen oder C₁-C₄-Alkyl substituiertes C₃-C₆-Cycloalkyl, oder gegebenenfalls durch Nitro, Cyano, Halogen, C₁-C₄-Alkyl, C₁-C₄-Halogenalkyl, C₁-C₄-Alkoxy oder C₁-C₄-Halogenalkoxy substituiertes Phenyl steht, oder zusammen mit R²⁵ für jeweils gegebenenfalls durch C₁-C₄-Alkyl substituiertes C₂-C₆-Alkandiyl oder C₂-C₅-Oxaalkandiyl steht,
- X⁴: für Nitro, Cyano, Carboxy, Carbamoyl, Formyl, Sulfamoyl, Hydroxy, Amino, Halogen, C₁-C₄-Alkyl, C₁-C₄-Halogenalkyl, C₁-C₄-Alkoxy oder C₁-C₄-Halogenalkoxy steht, und
- X⁵: für Nitro, Cyano, Carboxy, Carbamoyl, Formyl, Sulfamoyl, Hydroxy, Amino, Halogen, C₁-C₄-Alkyl, C₁-C₄-Halogenalkyl, C₁-C₄-Alkoxy oder C₁-C₄-Halogenalkoxy steht.

Die erfindungsgemäßen Verbindungen sind durch die Formel (I) allgemein definiert. Bevorzugte Substituenten bzw. Bereiche der in der oben und nachstehend erwähnten Formeln aufgeführten Reste werden im Folgenden erläutert:
- W: steht bevorzugt für Wasserstoff, C₁-C₆-Alkyl, C₂-C₆-Alkenyl, C₂-C₆-Alkinyl, Halogen, C₁-C₆-Alkoxy, C₁-C₄-Halogenalkyl, C₁-C₄-Halogenalkoxy oder Cyano,
- X: steht bevorzugt für Halogen, C₁-C₆-Alkyl, C₂-C₆-Alkenyl, C₂-C₆-Alkinyl, C₁-C₆-Alkoxy, C₁-C₆-Alkoxy-C₁-C₄-alkoxy, C₁-C₄-Halogenalkyl, C₁-C₄-Halogenalkoxy oder Cyano,
- Y: steht bevorzugt in der 4-Position für Wasserstoff, Halogen, C₁-C₆-Alkoxy, Cyano, C₁-C₄-Halogenalkyl oder C₁-C₄-Halogenalkoxy,
- Z: steht bevorzugt für Wasserstoff.
- W: steht auch bevorzugt für Wasserstoff, Halogen oder C₁-C₆-Alkyl,
- X: steht auch bevorzugt für Halogen, C₁-C₆-Alkyl, C₁-C₆-Alkoxy, C₁-C₄-Halogenalkyl, C₁-C₄-Halogenalkoxy oder Cyano,
- Y: steht auch bevorzugt in der 4-Position für durch V¹ und V² substituiertes Phenyl oder Pyridyl,
- Z: steht auch bevorzugt für Wasserstoff,
- V¹: steht auch bevorzugt für Halogen, C₁-C₁₂-Alkyl, C₁-C₆-Alkoxy, C₁-C₄-Halogenalkyl oder C₁-C₄-Halogenalkoxy,
- V²: steht auch bevorzugt für Wasserstoff, Halogen, C₁-C₆-Alkyl, C₁-C₆-Alkoxy oder C₁-C₄-Halogenalkyl,
- V¹ und V²: stehen gemeinsam auch bevorzugt für C₃-C₄-Alkandiyl, welches gegebenenfalls durch Halogen und/oder C₁-C₂-Alkyl substituiert sein kann und welches gegebenenfalls durch ein oder zwei Sauerstoffatome unterbrochen sein kann.
- W: steht ebenfalls bevorzugt für Wasserstoff, Halogen oder C₁-C₆-Alkyl,
- X: steht ebenfalls bevorzugt für Halogen C₁-C₆-Alkyl, C₁-C₆-Alkoxy, C₁-C₄-Halogenalkyl, C₁-C₄-Halogenalkoxy oder Cyano,
- Y: steht ebenfalls bevorzugt in der 5-Position für durch V¹ und V² substituiertes Phenyl oder Pyridyl,
- Z: steht ebenfalls bevorzugt in der 4-Position für Wasserstoff, C₁-C₆-Alkyl oder Halogen,
- V¹: steht ebenfalls bevorzugt für Halogen, C₁-C₁₂-Alkyl, C₁-C₆-Alkoxy, C₁-C₄-Halogenalkyl oder C₁-C₄-Halogenalkoxy,
- V²: steht ebenfalls bevorzugt für Wasserstoff, Halogen, C₁-C₆-Alkyl, C₁-C₆-Alkoxy oder C₁-C₄-Halogenalkyl,
- V¹ und V²: stehen gemeinsam ebenfalls bevorzugt für C₃-C₄-Alkandiyl, welches gegebenenfalls durch Halogen und/oder C₁-C₂-Alkyl substituiert sein kann und welches gegebenenfalls durch ein oder zwei Sauerstoffatome unterbrochen sein kann.
- W: steht außerdem bevorzugt für Wasserstoff, C₁-C₆-Alkyl, C₂-C₆-Alkenyl, C₂-C₆-Alkinyl, C₁-C₆-Alkoxy, Halogen, Trifluormethyl oder Cyano,
- X: steht außerdem bevorzugt für Halogen, C₁-C₆-Alkyl, C₂-C₆-Alkenyl, C₂-C₆-Alkinyl, C₁-C₆-Alkoxy, C₁-C₆-Alkoxy-C₁-C₄-alkoxy, C₁-C₄-Halogenalkyl, C₁-C₄-Halogenalkoxy oder Cyano,
- Y: steht außerdem bevorzugt in der 4-Position für C₁-C₆-Alkyl,
- Z: steht außerdem bevorzugt für Wasserstoff.
- W: steht weiterhin bevorzugt für Wasserstoff, Halogen, C₁-C₆-Alkyl oder C₁-C₆-Alkoxy,
- X: steht weiterhin bevorzugt für Halogen, C₁-C₆-Alkyl, C₁-C₆-Alkoxy, C₁-C₄-Halogenalkyl, C₁-C₄-Halogenalkoxy oder Cyano,
- Y: steht weiterhin bevorzugt in der 4-Position für Wasserstoff, Halogen oder C₁-C₆-Alkyl, C₁-C₄-Halogenalkyl oder C₁-C₄-Halogenalkoxy,
- Z: steht weiterhin bevorzugt in der 3- oder 5-Position für Halogen, C₁-C₆-Alkyl, C₁-C₄-Halogenalkyl, Cyano, C₁-C₆-Alkoxy oder C₁-C₄-Halogenalkoxy.
- A: steht bevorzugt für eine gegebenenfalls durch C₁-C₄-Alkyl substituierte C₁-C₄-Alkandiyl-gruppe oder für gegebenenfalls durch C₁-C₄-Alkyl substituiertes C₅-C₈-Cycloalkyl, in welchem gegebenenfalls eine Methylengruppe durch Sauerstoff ersetzt ist.
- B: steht bevorzugt für Wasserstoff oder für jeweils gegebenenfalls durch Halogen substituiertes C₁-C₈-Alkyl, C₂-C₈-Alkenyl, C₁-C₆-Alkoxy, C₁-C₆-Alkoxy-C₁-C₄-alkoxy, C₁-C₄-Alkoxy-bis-C₁-C₄-alkoxy, für gegebenenfalls durch Halogen, C₁-C₆-Alkyl, C₁-C₆-Alkoxy, C₁-C₄-Halogenalkyl, C₁-C₄-Halogenalkoxy, Cyano oder Nitro substituiertes Phenyl, für gegebenenfalls durch Halogen, C₁-C₄-Alkyl oder C₁-C₂-Halogenalkyl substituiertes Pyridyl, Pyrimidyl, Thiazolyl oder Thienyl oder für gegebenenfalls durch Halogen, C₁-C₄-Alkyl, C₁-C₄-Alkoxy oder C₁-C₂-Halogenalkyl substituiertes C₃-C₈-Cyclo-alkyl, in welchem gegebenenfalls eine oder zwei nicht direkt benachbarte Methylengruppen durch Sauerstoff ersetzt, zwei Methylengruppen durch den Rest -O-CO- oder drei Methylengruppen durch den Rest -O-CO-O- ersetzt sind.
- D: steht bevorzugt für NH oder Sauerstoff.
- Q¹: steht bevorzugt für Wasserstoff oder für jeweils gegebenenfalls durch Halogen substituiertes C₁-C₆-Alkyl, C₁-C₆-Alkoxy, C₁-C₄-Alkoxy-C₁-C₄-alkyl, C₁-C₄-Alkylthio-C₁-C₄-alkyl oder für gegebenenfalls durch Halogen, C₁-C₄-Alkyl oder C₁-C₄-Alkoxy substituiertes C₃-C₆-Cycloalkyl worin gegebenenfalls eine Methylengruppe durch Sauerstoff ersetzt ist oder für jeweils gegebenenfalls einfach bis zweifach durch Halogen, C₁-C₄-Alkyl, C₁-C₄-Alkoxy, C₁-C₄-Halogenalkyl oder C₁-C₄-Halogenalkoxy substituiertes Phenyl, Phenyl-C₁-C₂-alkyl oder Hetaryl,
- Q²: steht bevorzugt für Wasserstoff oder C₁-C₆-Alkyl, oder
- Q¹ und Q²: stehen gemeinsam mit dem Kohlenstoff, an das sie gebunden sind für einen gegebenenfalls einfach bis zweifach durch Fluor, Chlor, C₁-C₄-Alkyl, C₁-C₄-Alkoxy oder Trifluormethyl substituierten C₃-C₆-Ring, worin gegebenenfalls eine Methylengruppe durch Sauerstoff ersetzt sein kann.
- G: steht bevorzugt für Wasserstoff (a) oder für eine der Gruppen 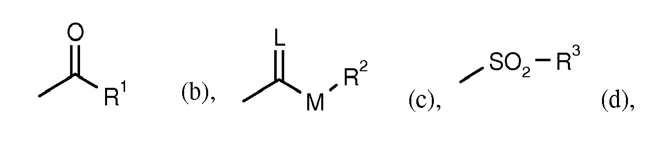 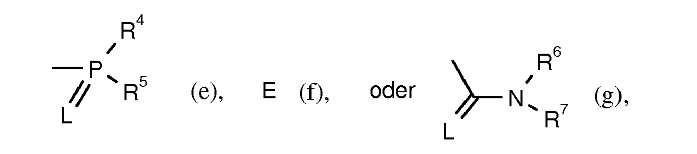 in welchen
- E: für ein Metallion oder ein Ammoniumion steht,
- L: für Sauerstoff oder Schwefel steht und
- M: für Sauerstoff oder Schwefel steht.
- R¹: steht bevorzugt für jeweils gegebenenfalls durch Halogen oder Cyano substituiertes C₁-C₂₀-Alkyl, C₂-C₂₀-Alkenyl, C₁-C₈-Alkoxy-C₁-C₈-alkyl, C₁-C₈-Alkylthio-C₁-C₈-alkyl oder Poly-C₁-C₈-alkoxy-C₁-C₈-alkyl oder für gegebenenfalls durch Halogen, C₁-C₆-Alkyl oder C₁-C₆-Alkoxy substituiertes C₃-C₈-Cycloalkyl, in welchem gegebenenfalls eine oder zwei nicht direkt benachbarte Methylengruppen durch Sauerstoff und/oder Schwefel ersetzt sind,
für gegebenenfalls durch Halogen, Cyano, Nitro, C₁-C₆-Alkyl, C₁-C₆-Alkoxy, C₁-C₆-Halogenalkyl, C₁-C₆-Halogenalkoxy, C₁-C₆-Alkylthio oder C₁-C₆-Alkylsulfonyl substituiertes Phenyl,
für gegebenenfalls durch Halogen, Nitro, Cyano, C₁-C₆-Alkyl, C₁-C₆-Alkoxy, C₁-C₆-Halogenalkyl oder C₁-C₆-Halogenalkoxy substituiertes Phenyl-C₁-C₆-alkyl,
für gegebenenfalls durch Halogen oder C₁-C₆-Alkyl substituiertes 5- oder 6-gliedriges Hetaryl mit ein oder zwei Heteroatomen aus der Reihe Sauerstoff, Schwefel und Stickstoff,
für gegebenenfalls durch Halogen oder C₁-C₆-Alkyl substituiertes Phenoxy-C₁-C₆-alkyl oder
für gegebenenfalls durch Halogen, Amino oder C₁-C₆-Alkyl substituiertes 5- oder 6-gliedriges Hetaryloxy-C₁-C₆-alkyl mit ein oder zwei Heteroatomen aus der Reihe Sauerstoff, Schwefel und Stickstoff.
- R²: steht bevorzugt für jeweils gegebenenfalls durch Halogen oder Cyano substituiertes C₁-C₂₀-Alkyl, C₂-C₂₀-Alkenyl, C₁-C₈-Alkoxy-C₂-C₈-alkyl oder Poly-C₁-C₈-alkoxy-C₂-C₈-alkyl,
für gegebenenfalls durch Halogen, C₁-C₆-Alkyl oder C₁-C₆-Alkoxy substituiertes C₃-C₈-Cycloalkyl oder
für jeweils gegebenenfalls durch Halogen, Cyano, Nitro, C₁-C₆-Alkyl, C₁-C₆-Alkoxy, C₁-C₆-Halogenalkyl oder C₁-C₆-Halogenalkoxy substituiertes Phenyl oder Benzyl.
- R³: steht bevorzugt für gegebenenfalls durch Halogen substituiertes C₁-C₈-Alkyl oder jeweils gegebenenfalls durch Halogen, C₁-C₆-Alkyl, C₁-C₆-Alkoxy, C₁-C₄-Halogenalkyl, C₁-C₄-Halogenalkoxy, Cyano oder Nitro substituiertes Phenyl oder Benzyl.
- R⁴ und R⁵: stehen unabhängig voneinander bevorzugt für jeweils gegebenenfalls durch Halogen substituiertes C₁-C₈-Alkyl, C₁-C₈-Alkoxy, C₁-C₈-Alkylamino, Di-(C₁-C₈-alkyl)amino, C₁-C₈-Alkylthio oder C₃-C₈-Alkenylthio oder für jeweils gegebenenfalls durch Halogen, Nitro, Cyano, C₁-C₄-Alkoxy, C₁-C₄-Halogenalkoxy, C₁-C₄-Alkylthio, C₁-C₄-Halogenalkylthio, C₁-C₄-Alkyl oder C₁-C₄-Halogenalkyl substituiertes Phenyl, Phenoxy oder Phenylthio.
- R⁶ und R⁷: stehen unabhängig voneinander bevorzugt für Wasserstoff, für jeweils gegebenenfalls durch Halogen oder Cyano substituiertes C₁-C₈-Alkyl, C₃-C₈-Cycloalkyl, C₁-C₈-Alkoxy, C₃-C₈-Alkenyl oder C₁-C₈-Alkoxy-C₂-C₈-alkyl, für jeweils gegebenenfalls durch Halogen, C₁-C₈-Alkyl, C₁-C₈-Halogenalkyl oder C₁-C₈-Alkoxy substituiertes Phenyl oder Benzyl oder zusammen für einen gegebenenfalls durch C₁-C₆-Alkyl substituierten C₃-C₆-Alkylenrest, in welchem gegebenenfalls eine Methylengruppe durch Sauerstoff oder Schwefel ersetzt ist.

In den als bevorzugt genannten Restedefinitionen steht Halogen für Fluor, Chlor, Brom und Iod, insbesondere für Fluor, Chlor und Brom.
- W: steht besonders bevorzugt für Wasserstoff, Chlor, Brom, C₁-C₄-Alkyl, C₂-C₄-Alkenyl, C₂-C₄-Alkinyl, C₁-C₄-Alkoxy, C₁-C₂-Halogenalkyl oder C₁-C₂-Halogenalkoxy,
- X: steht besonders bevorzugt für Chlor, Brom, C₁-C₄-Alkyl, C₂-C₄-Alkenyl, C₂-C₄-Alkinyl, C₁-C₄-Alkoxy, C₁-C₄-Alkoxy-C₁-C₃-alkoxy, C₁-C₂-Halogenalkyl, C₁-C₂-Halogenalkoxy oder Cyano,
- Y: steht besonders bevorzugt in der 4-Position für Wasserstoff, Fluor, Chlor, Brom, Methoxy, Ethoxy, Cyano, Trifluormethyl, Difluormethoxy oder Trifluormethoxy,
- Z: steht besonders bevorzugt für Wasserstoff.
- W: steht auch besonders bevorzugt für Wasserstoff, Chlor, Brom oder C₁-C₄-Alkyl,
- X: steht auch besonders bevorzugt für Chlor, Brom, C₁-C₄-Alkyl, C₁-C₄-Alkoxy, C₁-C₂-Halogenalkyl, C₁-C₂-Halogenalkoxy oder Cyano,
- Y: steht auch besonders bevorzugt in der 4-Position für den Rest 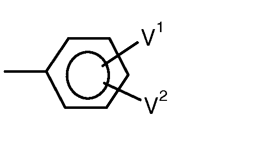
- Z: steht auch besonders bevorzugt für Wasserstoff,
- V¹: steht auch besonders bevorzugt für Fluor, Chlor, C₁-C₄-Alkyl, C₁-C₄-Alkoxy, C₁-C₂-Halogenalkyl oder C₁-C₂-Halogenalkoxy,
- V²: steht auch besonders bevorzugt für Wasserstoff, Fluor, Chlor, C₁-C₄-Alkyl, C₁-C₄-Alkoxy oder C₁-C₂-Halogenalkyl,
- V¹ und V²: stehen gemeinsam auch besonders bevorzugt für -O-CH₂-O- und -O-CF₂-O-.
- W: steht ebenfalls besonders bevorzugt für Wasserstoff, Chlor, Brom oder C₁-C₄-Alkyl,
- X: steht ebenfalls besonders bevorzugt für Chlor, Brom, C₁-C₄-Alkyl oder C₁-C₂-Halogenalkyl,
- Y: steht ebenfalls besonders bevorzugt in der 5-Position für den Rest 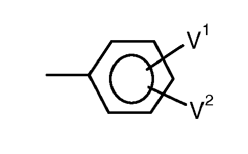
- Z: steht ebenfalls besonders bevorzugt in der 4-Position für Wasserstoff, C₁-C₄-Alkyl oder Chlor,
- V¹: steht ebenfalls besonders bevorzugt für Fluor, Chlor, C₁-C₄-Alkyl, C₁-C₄-Alkoxy, C₁-C₂-Halogenalkyl oder C₁-C₂-Halogenalkoxy,
- V²: steht ebenfalls besonders bevorzugt für Wasserstoff, Fluor, Chlor, C₁-C₄-Alkyl, C₁-C₄-Alkoxy oder C₁-C₂-Halogenalkyl,
- V¹ und V²: stehen gemeinsam ebenfalls besonders bevorzugt für -O-CH₂-O- und -O-CF₂-O-.
- W: steht außerdem besonders bevorzugt für Wasserstoff, C₁-C₄-Alkyl, C₂-C₄-Alkenyl, C₂-C₄-Alkinyl, C₁-C₄-Alkoxy, Chlor, Brom oder Trifluormethyl,
- X: steht außerdem besonders bevorzugt für Chlor, Brom, C₁-C₄-Alkyl, C₂-C₄-Alkenyl, C₂-C₄-Alkinyl, C₁-C₄-Alkoxy, C₁-C₄-Alkoxy-C₁-C₃-alkoxy, C₁-C₂-Halogenalkyl, C₁-C₂-Halogenalkoxy oder Cyano,
- Y: steht außerdem besonders bevorzugt in der 4-Position für C₁-C₄-Alkyl,
- Z: steht außerdem besonders bevorzugt für Wasserstoff.
- W: steht weiterhin besonders bevorzugt für Wasserstoff, Chlor, Brom, C₁-C₄-Alkyl oder C₁-C₄-Alkoxy,
- X: steht weiterhin besonders bevorzugt für Chlor, Brom, C₁-C₄-Alkyl, C₁-C₄-Alkoxy, C₁-C₂-Halogenalkyl, C₁-C₂-Halogenalkoxy oder Cyano,
- Y: steht weiterhin besonders bevorzugt in der 4-Position für Wasserstoff, Chlor, Brom, C₁-C₄-Alkyl, C₁-C₂-Halogenalkyl oder C₁-C₂-Halogenalkoxy,
- Z: steht weiterhin besonders bevorzugt in der 3- oder 5-Position für Fluor, Chlor, Brom, C₁-C₄-Alkyl, C₁-C₂-Halogenalkyl, C₁-C₄-Alkoxy oder C₁-C₂-Halogenalkoxy.
- A: steht besonders bevorzugt für eine gegebenenfalls durch C₁-C₂-Alkyl substituierte C₁-C₃-Alkandiylgruppe oder für C₅-C₆-Cycloalkyl in welchen gegebenenfalls eine Methylengruppe durch Sauerstoff ersetzt ist.
- B: steht besonders bevorzugt für Wasserstoff oder jeweils gegebenenfalls einfach bis dreifach durch Fluor oder Chlor substituiertes C₁-C₆-Alkyl, C₂-C₆-Alkenyl, C₁-C₄-Alkoxy, C₁-C₄-Alkoxy-C₁-C₃-alkoxy, C₁-C₄-Alkoxy-bis-C₁-C₃-alkoxy, für gegebenenfalls einfach bis dreifach durch Fluor, Chlor, Brom, C₁-C₄-Alkyl, C₁-C₄-Alkoxy, C₁-C₂-Halogenalkyl, C₁-C₂-Halogenalkoxy, Cyano oder Nitro substituiertes Phenyl, für gegebenenfalls einfach bis zweifach durch Fluor, Chlor, Brom, Methyl, Ethyl oder Trifluormethyl substituiertes Pyridyl, Pyrimidyl, Thiazolyl oder Thienyl oder für gegebenenfalls einfach bis zweifach durch Fluor, Chlor, Methyl, Methoxy oder Trifluormethyl substituiertes C₃-C₆-Cycloalkyl, in welchem gegebenenfalls zwei nicht direkt benachbarte Methylengruppen durch Sauerstoff ersetzt sind.
- D: steht für besonders bevorzugt für NH oder Sauerstoff.
- Q¹: steht besonders bevorzugt für Wasserstoff, für gegebenenfalls einfach bis dreifach durch Fluor substituiertes C₁-C₄-Alkyl, C₁-C₄-Alkoxy, C₁-C₂-Alkoxy-C₁-C₂-alkyl, oder für gegebenenfalls einfach bis zweifach durch Fluor, Chlor, Methyl, Ethyl oder Methoxy substituiertes C₃-C₆-Cycloalkyl worin gegebenenfalls eine Methylengruppe durch Sauerstoff ersetzt sein kann.
- Q²: steht besonders bevorzugt für Wasserstoff oder C₁-C₄-Alkyl.
- Q¹ und Q²: stehen gemeinsam mit dem Kohlenstoffatom, an das sie gebunden sind, besonders bevorzugt für einen gegebenenfalls einfach durch Fluor, Methyl, Methoxy oder Trifluormethyl substituierten C₃-C₆-Ring, worin gegebenenfalls eine Methylengruppe durch Sauerstoff ersetzt sein kann.
- G: steht besonders bevorzugt für Wasserstoff (a) oder für eine der Gruppen 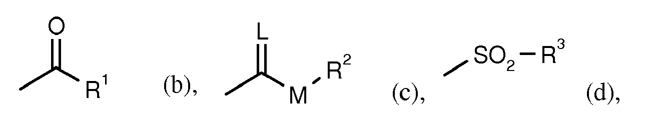 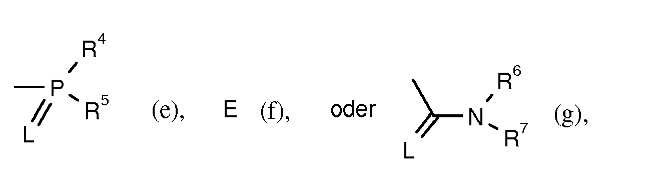 in welchen
E für ein Metallion oder ein Ammoniumion steht,
L für Sauerstoff oder Schwefel steht und
M für Sauerstoff oder Schwefel steht.
- R¹: steht besonders bevorzugt für jeweils gegebenenfalls einfach bis dreifach durch Fluor oder Chlor substituiertes C₁-C₁₆-Alkyl, C₂-C₁₆-Alkenyl, C₁-C₆-Alkoxy-C₁-C₄-alkyl, C₁-C₆-Alkylthio-C₁-C₄-alkyl oder Poly-C₁-C₆-alkoxy-C₁-C₄-alkyl oder für gegebenenfalls einfach bis zweifach durch Fluor, Chlor, C₁-C₅-Alkyl oder C₁-C₅-Alkoxy substituiertes C₃-C₇-Cycloalkyl, in welchem gegebenenfalls eine oder zwei nicht direkt benachbarte Methylengruppen durch Sauerstoff und/oder Schwefel ersetzt sind,
für gegebenenfalls einfach bis dreifach durch Fluor, Chlor, Brom, Cyano, Nitro, C₁-C₄-Alkyl, C₁-C₄-Alkoxy, C₁-C₃-Halogenalkyl, C₁-C₃-Halogenalkoxy, C₁-C₄-Alkylthio oder C₁-C₄-Alkylsulfonyl substituiertes Phenyl,
für gegebenenfalls einfach bis zweifach durch Fluor, Chlor, Brom, C₁-C₄-Alkyl, C₁-C₄-Alkoxy, C₁-C₃-Halogenalkyl oder C₁-C₃-Halogenalkoxy substituiertes Phenyl-C₁-C₄-alkyl,
für jeweils gegebenenfalls einfach bis zweifach durch Fluor, Chlor, Brom oder C₁-C₄-Alkyl substituiertes Pyrazolyl, Thiazolyl, Pyridyl, Pyrimidyl, Furanyl oder Thienyl,
für gegebenenfalls einfach bis zweifach durch Fluor, Chlor, Brom oder C₁-C₄-Alkyl substituiertes Phenoxy-C₁-C₅-alkyl oder
für jeweils gegebenenfalls einfach bis zweifach durch Fluor, Chlor, Brom, Amino oder C₁-C₄-Alkyl substituiertes Pyridyloxy-C₁-C₅-alkyl, Pyrimidyloxy-C₁-C₅-alkyl oder Thiazolyloxy-C₁-C₅-alkyl.
- R²: steht besonders bevorzugt für jeweils gegebenenfalls einfach bis dreifach durch Fluor oder Chlor substituiertes C₁-C₁₆-Alkyl, C₂-C₁₆-Alkenyl, C₁-C₆-Alkoxy-C₂-C₆-alkyl oder Poly-C₁-C₆-alkoxy-C₂-C₆-alkyl,
für gegebenenfalls einfach bis zweifach durch Fluor, Chlor, C₁-C₄-Alkyl oder C₁-C₄-Alkoxy substituiertes C₃-C₇-Cycloalkyl oder
für jeweils gegebenenfalls einfach bis dreifach durch Fluor, Chlor, Brom, Cyano, Nitro, C₁-C₄-Alkyl, C₁-C₃-Alkoxy, C₁-C₃-Halogenalkyl oder C₁-C₃-Halogenalkoxy substituiertes Phenyl oder Benzyl.
- R³: steht besonders bevorzugt für gegebenenfalls einfach bis dreifach durch Fluor oder Chlor substituiertes C₁-C₆-Alkyl oder jeweils gegebenenfalls einfach bis zweifach durch Fluor, Chlor, Brom, C₁-C₄-Alkyl, C₁-C₄-Alkoxy, C₁-C₂-Halogenalkoxy, C₁-C₂-Halogenalkyl, Cyano oder Nitro substituiertes Phenyl oder Benzyl.
- R⁴ und R⁵: stehen unabhängig voneinander besonders bevorzugt für jeweils gegebenenfalls einfach bis dreifach durch Fluor oder Chlor substituiertes C₁-C₆-Alkyl, C₁-C₆-Alkoxy, C₁-C₆-Alkylamino, Di-(C₁-C₆-alkyl)anüno, C₁-C₆-Alkylthio oder C₃-C₄-Alkenylthio oder für jeweils gegebenenfalls einfach bis zweifach durch Fluor, Chlor, Brom, Nitro, Cyano, C₁-C₃-Alkoxy, C₁-C₃-Halogenalkoxy, C₁-C₃-Alkylthio, C₁-C₃-Halogenalkylthio, C₁-C₃-Alkyl oder C₁-C₃-Halogenalkyl substituiertes Phenyl, Phenoxy oder Phenylthio.
- R⁶ und R⁷: stehen unabhängig voneinander besonders bevorzugt für Wasserstoff, für jeweils gegebenenfalls einfach bis dreifach durch Fluor oder Chlor substituiertes C₁-C₆-Alkyl, C₃-C₆-Cycloalkyl, C₁-C₆-Alkoxy, C₃-C₆-Alkenyl oder C₁-C₆-Alkoxy-C₂-C₆-alkyl, für jeweils gegebenenfalls einfach bis dreifach durch Fluor, Chlor, Brom, C₁-C₅-Halogenalkyl, C₁-C₅-Alkyl oder C₁-C₅-Alkoxy substituiertes Phenyl oder Benzyl, oder zusammen für einen gegebenenfalls durch C₁-C₄-Alkyl substituierten C₃-C₆-Alkylenrest, in welchem gegebenenfalls eine Methylengruppe durch Sauerstoff oder Schwefel ersetzt ist.

In den als besonders bevorzugt genannten Restedefinitionen steht Halogen für Fluor, Chlor und Brom, insbesondere für Fluor und Chlor.
- W: steht ganz besonders bevorzugt für Wasserstoff, Methyl, Chlor, Brom, Ethyl, Methoxy, Ethoxy oder Trifluormethyl,
- X: steht ganz besonders bevorzugt für Chlor, Brom, Methyl, Ethyl, Propyl, Methoxy, Ethoxy, Propoxy, Methoxy-ethoxy, Ethoxy-ethoxy, Trifluormethyl, Difluormethoxy, Trifluormethoxy oder Cyano,
- Y: steht ganz besonders bevorzugt in der 4-Position für Wasserstoff, Chlor, Brom, Trifluormethyl oder Trifluormethoxy,
- Z: steht ganz besonders bevorzugt für Wasserstoff.
- W: steht auch ganz besonders bevorzugt für Wasserstoff, Chlor, Brom, Methyl oder Ethyl,
- X: steht auch ganz besonders bevorzugt für Chlor, Brom, Methyl, Ethyl, Propyl, Methoxy, Trifluormethyl, Difluormethoxy oder Cyano,
- Y: steht auch ganz besonders bevorzugt in der 4-Position für den Rest 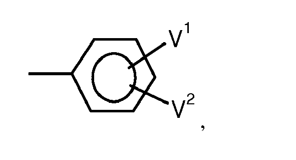
- Z: steht auch ganz besonders bevorzugt für Wasserstoff,
- V¹: steht auch ganz besonders bevorzugt für Fluor, Chlor, Methyl, Methoxy, Trifluormethyl oder Trifluormethoxy,
- V²: steht auch ganz besonders bevorzugt für Wasserstoff, Fluor, Chlor, Methyl, Methoxy oder Trifluormethyl.
- W: steht ebenfalls ganz besonders bevorzugt für Wasserstoff, Chlor oder Methyl,
- X: steht ebenfalls ganz besonders bevorzugt für Chlor, Methyl oder Trifluormethyl,
- Y: steht ebenfalls ganz besonders bevorzugt in der 5-Position für den Rest 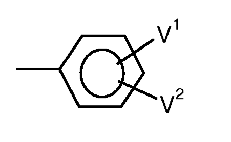
- Z: steht ebenfalls ganz besonders bevorzugt in der 4-Position für Wasserstoff oder Methyl,
- V¹: steht ebenfalls ganz besonders bevorzugt für Fluor, Chlor, Methyl, Methoxy, Trifluormethyl oder Trifluormethoxy,
- V²: steht ebenfalls ganz besonders bevorzugt für Wasserstoff, Fluor, Chlor, Methyl, Methoxy oder Trifluormethyl.
- W: steht außerdem ganz besonders bevorzugt für Wasserstoff, Methyl, Ethyl, Methoxy, Ethoxy, Chlor oder Brom,
- X: steht außerdem ganz besonders bevorzugt für Chlor, Brom, Methyl, Ethyl, Propyl, Methoxy, Ethoxy, Propoxy, Methoxy-ethoxy, Ethoxy-ethoxy, Trifluormethyl, Difluormethoxy, Trifluormethoxy oder Cyano,
- Y: steht außerdem ganz besonders bevorzugt in der 4-Position für Methyl oder Ethyl,
- Z: steht außerdem ganz besonders bevorzugt für Wasserstoff.
- W: steht weiterhin ganz besonders bevorzugt für Wasserstoff, Chlor, Brom, Methyl oder Ethyl,
- X: steht weiterhin ganz besonders bevorzugt für Chlor, Brom, Methyl, Ethyl, Methoxy, Trifluormethyl, Difluormethoxy oder Trifluormethoxy,
- Y: steht weiterhin ganz besonders bevorzugt in der 4-Position für Wasserstoff, Chlor, Brom, Methyl oder Ethyl,
- Z: steht weiterhin ganz besonders bevorzugt in der 3- oder 5-Position für Fluor, Chlor, Brom, Methyl, Ethyl, Trifluormethyl oder Trifluormethoxy.
- A: steht ganz besonders bevorzugt für -CH₂-, -CHCH₃-, -CH₂-CH₂-, -CH₂-CHCH₃-, -CH₂-CH₂-CH₂-.
- B: steht ganz besonders bevorzugt für Wasserstoff, Methyl, Ethyl, Propyl, iso-Propyl, Butyl, iso-Butyl, C₂-C₄-Alkenyl, Methoxy, Ethoxy, Propoxy, Isopropoxy, Butoxy, Isobutoxy, Methoxy-ethoxy, Ethoxy-ethoxy, für gegebenenfalls einfach bis zweifach durch Fluor, Chlor, Brom, Methyl, Methoxy, Trifluormethyl, Trifluormethoxy, Cyano oder Nitro substituiertes Phenyl, für Cyclopropyl, für Cyclopentyl oder Cyclohexyl, in welchem gegebenenfalls eine Methylengruppe durch Sauerstoff ersetzt ist.
- D: steht ganz besonders bevorzugt für NH oder Sauerstoff.
- Q¹: steht ganz besonders bevorzugt für Wasserstoff, Methyl, Ethyl, Propyl, iso-Propyl, Cyclopropyl, Cyclopentyl oder Cyclohexyl.
- Q²: steht ganz besonders bevorzugt für Wasserstoff, Methyl oder Ethyl.
- Q¹ und Q²: stehen ganz besonders bevorzugt gemeinsam mit dem Kohlenstoffatom an das sie gebunden sind für Cyclopropyl, Cyclopentyl oder Cyclohexyl.
- G: steht ganz besonders bevorzugt für Wasserstoff (a) oder für eine der Gruppen 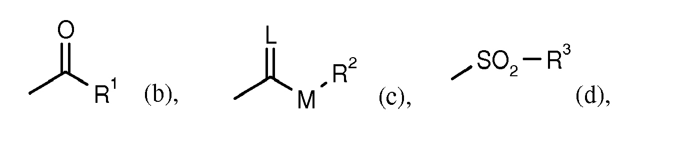 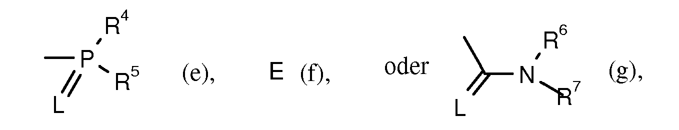 in welchen
E für ein Metallion oder ein Ammoniumion steht,
L für Sauerstoff oder Schwefel steht und
M für Sauerstoff oder Schwefel steht.
- R¹: steht ganz besonders bevorzugt für jeweils gegebenenfalls einfach bis dreifach durch Fluor oder Chlor substituiertes C₁-C₁₀-Alkyl, C₂-C₁₀-Alkenyl, C₁-C₄-Alkoxy-C₁-C₂-alkyl, C₁-C₄-Alkylthio-C₁-C₂-alkyl oder für gegebenenfalls einfach durch Fluor, Chlor, Methyl, Ethyl oder Methoxy substituiertes C₃-C₆-Cycloalkyl,
für gegebenenfalls einfach bis zweifach durch Fluor, Chlor, Brom, Cyano, Nitro, Methyl, Ethyl, n-Propyl, i-Propyl, Methoxy, Ethoxy, Trifluormethyl oder Trifluormethoxy substituiertes Phenyl,
für jeweils gegebenenfalls einfach durch Chlor, Brom oder Methyl substituiertes Furanyl, Thienyl oder Pyridyl.
- R²: steht ganz besonders bevorzugt für jeweils gegebenenfalls einfach bis dreifach durch Fluor oder Chlor substituiertes C₁-C₁₀-Alkyl, C₂-C₁₀-Alkenyl oder C₁-C₄-Alkoxy-C₂-C₄-alkyl,
für Cyclopentyl oder Cyclohexyl
oder für jeweils gegebenenfalls einfach bis zweifach durch Fluor, Chlor, Cyano, Nitro, Methyl, Ethyl, Methoxy, Trifluormethyl oder Trifluormethoxy substituiertes Phenyl oder Benzyl.
- R³: steht ganz besonders bevorzugt für jeweils gegebenenfalls einfach bis dreifach durch Fluor oder Chlor substituiertes Methyl, Ethyl, Propyl oder iso-Propyl, oder gegebenenfalls einfach durch Fluor, Chlor, Brom, Methyl, Ethyl, iso-Propyl, tert.-Butyl, Methoxy, Ethoxy, iso-Propoxy, Trifluormethyl, Trifluormethoxy, Cyano oder Nitro substituiertes Phenyl.
- R⁴ und R⁵: stehen unabhängig voneinander ganz besonders bevorzugt für C₁-C₄-Alkoxy oder C₁-C₄-Alkylthio oder für jeweils gegebenenfalls einfach durch Fluor, Chlor, Brom, Nitro, Cyano, Methyl, Methoxy, Trifluormethyl oder Trifluormethoxy substituiertes Phenyl, Phenoxy oder Phenylthio.
- R⁶ und R⁷: stehen unabhängig voneinander ganz besonders bevorzugt für Wasserstoff, für C₁-C₄-Alkyl, C₃-C₆-Cycloalkyl, C₁-C₄-Alkoxy, C₃-C₄-Alkenyl oder C₁-C₄-Alkoxy-C₂-C₄-alkyl, für gegebenenfalls einfach bis zweifach durch Fluor, Chlor, Brom, Methyl, Methoxy oder Trifluormethyl substituiertes Phenyl, oder zusammen für einen C₅-C₆-Alkylenrest, in welchem gegebenenfalls eine Methylengruppe durch Sauerstoff oder Schwefel ersetzt ist.
- W: steht insbesondere bevorzugt für Wasserstoff, Methyl, Ethyl, Chlor oder Brom (hervorgehoben für Wasserstoff, Methyl, Ethyl oder Chlor),
- X: steht insbesondere bevorzugt für Chlor, Brom, Methyl, Ethyl, Propyl, Methoxy, Ethoxy, Propoxy, Methoxy-ethoxy, Cyclopropyl-methyloxy, Trifluormethyl oder Difluormethoxy (hervorgehoben für Chlor, Brom, Ethyl, Methoxy oder Ethoxy),
- Y: steht insbesondere bevorzugt in der 4-Position für Wasserstoff, Chlor, Brom, Trifluormethyl oder Trifluormethoxy (hervorgehoben für Chlor oder Brom),
- Z: steht insbesondere bevorzugt für Wasserstoff,
- A: steht insbesondere bevorzugt für -CH₂-, -CHCH₃- oder -CH₂-CH₂- (hervorgehoben für -CH₂- oder -CH₂-CH₂-),
- B: steht insbesondere bevorzugt für Wasserstoff, Methyl, Ethyl, Propyl, iso-Propyl, C₂-C₄-Alkenyl, Methoxy, Ethoxy, Propoxy, Isopropoxy, Butoxy, Isobutoxy, für gegebenenfalls einfach bis zweifach durch Fluor, Chlor, Brom, Methyl, Methoxy, Trifluormethyl, Trifluormethoxy, Cyano oder Nitro substituiertes Phenyl, für Cyclopropyl, Cyclopentyl oder Cyclohexyl (hervorgehoben für Wasserstoff, Methyl, Ethyl, Propyl oder Methoxy),
- D: steht insbesondere bevorzugt für NH,
- Q¹: steht insbesondere besonders bevorzugt für Wasserstoff oder Methyl (hervorgehoben für Wasserstoff),
- Q²: steht insbesondere besonders bevorzugt für Wasserstoff oder Methyl (hervorgehoben für Wasserstoff),
- G: steht insbesonders bevorzugt für Wasserstoff (a) oder für eine der Gruppen 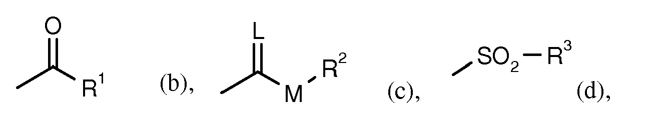 in welchen L für Sauerstoff steht und
M für Sauerstoff oder Schwefel steht (hervorgehoben für Sauerstoff),
- R¹: steht insbesondere bevorzugt für jeweils gegebenenfalls einfach bis dreifach durch Fluor oder Chlor substituiertes C₁-C₆-Alkyl, C₂-C₆-Alkenyl, C₁-C₂-Alkoxy-C₁-C₂-alkyl, C₁-C₂-Alkylthio-C₁-C₂-alkyl oder für Cyclopropyl, Cyclopentyl oder Cyclohexyl,
für gegebenenfalls einfach durch Fluor, Chlor, Brom, Cyano, Nitro, Methyl, Methoxy, Trifluormethyl oder Trifluormethoxy substituiertes Phenyl,
für jeweils gegebenenfalls einfach durch Chlor oder Methyl substituiertes Furanyl, Thienyl oder Pyridyl (hervorgehoben für C₁-C₆-Alkyl, C₁-C₂-Alkoxy-C₁-C₂-alkyl, Cyclopropyl, für jeweils durch Chlor substituiertes Phenyl oder Thienyl),
- R²: steht insbesondere bevorzugt für C₁-C₈-Alkyl, C₂-C₆-Alkenyl oder C₁-C₂-Alkoxy-C₂-C₃-alkyl,
für Cyclopentyl oder Cyclohexyl,
oder für jeweils gegebenenfalls einfach durch Fluor, Chlor, Cyano, Nitro, Methyl, Methoxy, Trifluormethyl oder Trifluormethoxy substituiertes Phenyl oder Benzyl (hervorgehoben für C₁-C₈-Alkyl, C₂-C₆-Alkenyl oder Benzyl).
- R³: steht insbesondere bevorzugt für durch Methyl substituiertes Phenyl.
- W: steht auch insbesondere bevorzugt für Wasserstoff, Chlor, Brom, Methyl oder Ethyl,
- X: steht auch insbesondere bevorzugt für Chlor, Brom, Methyl, Ethyl, Propyl, Methoxy, Trifluormethyl, Difluormethoxy oder Cyano,
- Y: steht auch insbesondere bevorzugt in der 4-Position für den Rest 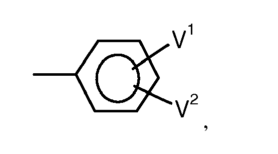
- Z: steht auch insbesondere bevorzugt für Wasserstoff,
- V¹: steht auch insbesondere bevorzugt für Fluor, Chlor, Methyl, Methoxy, Trifluormethyl oder Trifluormethoxy,
- V²: steht auch insbesondere bevorzugt für Wasserstoff, Fluor, Chlor, Methyl, Methoxy oder Trifluormethyl,
- A: steht auch insbesondere bevorzugt für -CH₂-, -CHCH₃- oder -CH₂-CH₂-,
- B: steht auch insbesondere bevorzugt für Wasserstoff, Methyl, Ethyl, Propyl, iso-Propyl, C₂-C₄-Alkenyl, Methoxy, Ethoxy, Propoxy, Isopropoxy, Butoxy, Isobutoxy, für gegebenenfalls einfach bis zweifach durch Fluor, Chlor, Brom, Methyl, Methoxy, Trifluormethyl, Trifluormethoxy, Cyano oder Nitro substituiertes Phenyl,
- D: steht auch insbesondere bevorzugt für NH,
- Q¹: steht auch insbesondere bevorzugt für Wasserstoff oder Methyl,
- Q²: steht auch insbesondere bevorzugt für Wasserstoff oder Methyl,
- G: steht auch insbesondere bevorzugt für Wasserstoff (a) oder für eine der Gruppen 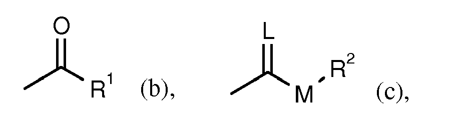 in welchen
L für Sauerstoff steht und
M für Sauerstoff oder Schwefel steht,
- R¹: steht auch insbesondere bevorzugt für jeweils gegebenenfalls einfach bis dreifach durch Fluor oder Chlor substituiertes C₁-C₆-Alkyl, C₂-C₆-Alkenyl, C₁-C₂-Alkoxy-C₁-C₂-alkyl, C₁-C₂-Alkylthio-C₁-C₂-alkyl oder für Cyclopropyl, Cyclopentyl oder Cyclohexyl,
für gegebenenfalls einfach durch Fluor, Chlor, Brom, Cyano, Nitro, Methyl, Methoxy, Trifluormethyl oder Trifluormethoxy substituiertes Phenyl,
für jeweils gegebenenfalls einfach durch Chlor oder Methyl substituiertes Furanyl, Thienyl oder Pyridyl,
- R²: steht auch insbesondere bevorzugt für C₁-C₈-Alkyl, C₂-C₆-Alkenyl oder C₁-C₂-Alkoxy-C₂-C₃-alkyl,
für Cyclopentyl oder Cyclohexyl,
oder für jeweils gegebenenfalls einfach durch Fluor, Chlor, Cyano, Nitro, Methyl, Methoxy, Trifluormethyl oder Trifluormethoxy substituiertes Phenyl oder Benzyl.
- W: steht ebenfalls insbesondere bevorzugt für Wasserstoff oder Methyl (hervorgehoben für Wasserstoff),
- X: steht ebenfalls insbesondere bevorzugt für Chlor oder Methyl (hervorgehoben für Methyl),
- Y: steht ebenfalls insbesondere bevorzugt in der 5-Position für den Rest 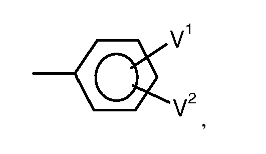
- Z: steht ebenfalls insbesondere bevorzugt in der 4-Position für Wasserstoff oder Methyl (hervorgehoben für Wasserstoff),
- V¹: steht ebenfalls insbesondere bevorzugt für Fluor, Chlor, Methyl, Methoxy, Trifluormethyl oder Trifluormethoxy,
- V²: steht ebenfalls insbesondere bevorzugt für Wasserstoff, Fluor, Chlor, Methyl, Methoxy oder Trifluormethyl (Y steht hervorgehoben für durch Chlor substituiertes Phenyl),
- A: steht ebenfalls insbesondere bevorzugt für -CH₂-, -CHCH₃- oder -CH₂-CH₂-(hervorgehoben für -CH₂-),
- B: steht ebenfalls insbesondere bevorzugt für Wasserstoff, Methyl, Ethyl, Propyl, iso-Propyl, C₂-C₄-Alkenyl, Methoxy, Ethoxy, Propoxy, Isopropoxy, Butoxy, Isobutoxy, für gegebenenfalls einfach bis zweifach durch Fluor, Chlor, Brom, Methyl, Methoxy, Trifluormethyl, Trifluormethoxy, Cyano oder Nitro substituiertes Phenyl (hervorgehoben für Propyl),
- D: steht ebenfalls insbesondere bevorzugt für NH,
- Q¹: steht ebenfalls insbesondere bevorzugt für Wasserstoff oder Methyl (hervorgehoben für Wasserstoff),
- Q²: steht ebenfalls insbesondere bevorzugt für Wasserstoff oder Methyl (hervorgehoben für Wasserstoff),
- G: steht ebenfalls insbesondere bevorzugt für Wasserstoff (a) oder für eine der Gruppen 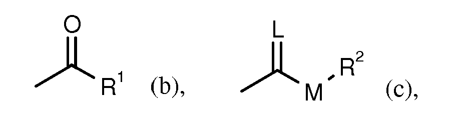 (hervorgehoben für Wasserstoff),
in welchen
L für Sauerstoff steht und
M für Sauerstoff oder Schwefel steht,
- R¹: steht ebenfalls insbesondere bevorzugt für jeweils gegebenenfalls einfach bis dreifach durch Fluor oder Chlor substituiertes C₁-C₆-Alkyl, C₂-C₆-Alkenyl, C₁-C₂-Alkoxy-C₁-C₂-alkyl, C₁-C₂-Alkylthio-C₁-C₂-alkyl oder für Cyclopropyl, Cyclopentyl oder Cyclohexyl,
für gegebenenfalls einfach durch Fluor, Chlor, Brom, Cyano, Nitro, Methyl, Methoxy, Trifluormethyl oder Trifluormethoxy substituiertes Phenyl,
für jeweils gegebenenfalls einfach durch Chlor oder Methyl substituiertes Furanyl, Thienyl oder Pyridyl,
- R²: steht ebenfalls insbesondere bevorzugt für C₁-C₈-Alkyl, C₂-C₆-Alkenyl oder C₁-C₂-Alkoxy-C₂-C₃-alkyl,
für Cyclopentyl oder Cyclohexyl,
oder für jeweils gegebenenfalls einfach durch Fluor, Chlor, Cyano, Nitro, Methyl, Methoxy, Trifluormethyl oder Trifluormethoxy substituiertes Phenyl oder Benzyl.
- W: steht außerdem insbesondere bevorzugt für Wasserstoff, Methyl, Ethyl, Chlor oder Brom (hervorgehoben für Methyl, Ethyl, Chlor oder Brom),
- X: steht außerdem insbesondere bevorzugt für Chlor, Brom, Methyl, Ethyl, Propyl, Methoxy, Ethoxy, Propoxy, H₃CO-(CH₂)₂-O, Cyclopropylmethoxy, Trifluormethyl oder Difluormethoxy (hervorgehoben für Chlor, Brom, Methyl, Ethyl, Methoxy oder H₃CO-(CH₂)₂-O)),
- Y: steht außerdem insbesondere bevorzugt in der 4-Position für Methyl,
- Z: steht außerdem insbesondere bevorzugt für Wasserstoff,
- A: steht außerdem insbesondere bevorzugt für -CH₂-, -CHCH₃- oder -CH₂-CH₂-(hervorgehoben für -CH₂- oder -CH₂-CH₂-),
- B: steht außerdem insbesondere bevorzugt für Wasserstoff, Methyl, Ethyl, Propyl, iso-Propyl, C₂-C₄-Alkenyl, Methoxy, Ethoxy, Propoxy, Isopropoxy, Butoxy, Isobutoxy, für gegebenenfalls einfach bis zweifach durch Fluor, Chlor, Brom, Methyl, Methoxy, Trifluormethyl, Trifluormethoxy, Cyano oder Nitro substituiertes Phenyl, für Cyclopropyl, für Cyclopentyl oder Cyclohexyl, in welchem gegebenenfalls eine Methylengruppe durch Sauerstoff ersetzt ist (hervorgehoben für Wasserstoff, Methyl, Ethyl, Propyl, iso-Propyl, Methoxy oder Cyclopropyl),
- Q¹: steht außerdem insbesondere bevorzugt für Wasserstoff oder Methyl,
- Q²: steht außerdem insbesondere bevorzugt für Wasserstoff oder Methyl,
- D: steht außerdem insbesondere bevorzugt für NH,
- G: steht außerdem insbesondere bevorzugt für Wasserstoff (a) oder für eine der Gruppen 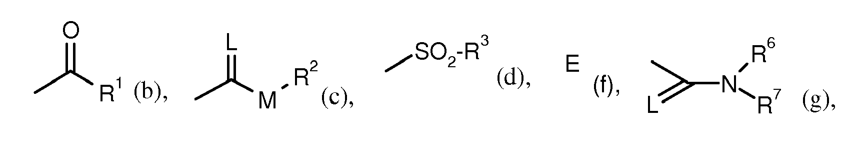 in welchen
E für ein Ammoniumion steht,
L für Sauerstoff oder Schwefel steht und
M für Sauerstoff oder Schwefel steht,
- R¹: steht außerdem insbesondere bevorzugt für jeweils gegebenenfalls einfach bis dreifach durch Fluor oder Chlor substituiertes C₁-C₆-Alkyl, C₂-C₆-Alkenyl, C₁-C₂-Alkoxy-C₁-C₂-alkyl, C₁-C₂-Alkylthio-C₁-C₂-alkyl oder für Cyclopropyl, Cyclopentyl oder Cyclohexyl,
für gegebenenfalls einfach durch Fluor, Chlor, Brom, Cyano, Nitro, Methyl, Methoxy, Trifluormethyl oder Trifluormethoxy substituiertes Phenyl, für 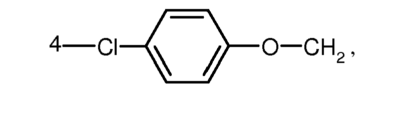 für jeweils gegebenenfalls einfach durch Chlor oder Methyl substituiertes Furanyl, Thienyl oder Pyridyl (hervorgehoben für gegebenenfalls einfach durch Chlor substituiertes C₁-C₆-Alkyl, C₁-C₂-Alkoxy-C₁-C₂-alkyl, Cyclopropyl, für durch Chlor substituiertes Phenyl, Pyridyl, Thienyl oder 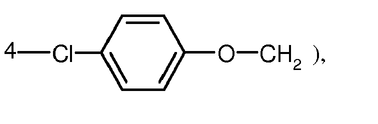
- R²: steht außerdem insbesondere bevorzugt für C₁-C₁₀-Alkyl, C₂-C₁₀-Alkenyl oder C₁-C₂-Alkoxy-C₂-C₄-alkyl,
für Cyclopentyl oder Cyclohexyl,
oder für jeweils gegebenenfalls einfach durch Fluor, Chlor, Cyano, Nitro, Methyl, Methoxy, Trifluormethyl oder Trifluormethoxy substituiertes Phenyl oder Benzyl (hervorgehoben für C₁-C₁₀-Alkyl, C₂-C₁₀-Alkenyl oder Benzyl),
- R³: steht außerdem insbesondere bevorzugt für Methyl oder für durch Methyl substituiertes Phenyl,
- R⁶ und R⁷: stehen außerdem insbesondere bevorzugt zusammen für einen C₅-C₆-Alkylenrest, in welchem eine Methylengruppe für Sauerstoff steht.
- W: steht weiterhin insbesondere bevorzugt für Wasserstoff, Methyl, Chlor oder Brom (hervorgehoben für Wasserstoff oder Methyl),
- X: steht weiterhin insbesondere bevorzugt für Chlor, Brom, Methyl, Methoxy oder Trifluormethyl (hervorgehoben für Methyl),
- Y: steht weiterhin insbesondere bevorzugt in der 4-Position für Wasserstoff, Chlor, Brom oder Methyl (hervorgehoben für Wasserstoff oder Methyl),
- Z: steht weiterhin insbesondere bevorzugt in der 3- oder 5-Position für Chlor, Brom, Methyl, Ethyl, Trifluormethyl oder Trifluormethoxy (hervorgehoben für Methyl),
- A: steht weiterhin insbesondere bevorzugt für -CH₂-, -CHCH₃- oder -CH₂-CH₂-(hervorgehoben für -CH₂-),
- B: steht weiterhin insbesondere bevorzugt für Wasserstoff, Methyl, Ethyl, Propyl, iso-Propyl, C₂-C₄-Alkenyl, Methoxy, Ethoxy, Propoxy, Isopropoxy, Butoxy, Isobutoxy, Cyclopropyl, für gegebenenfalls einfach bis zweifach durch Fluor, Chlor, Brom, Methyl, Methoxy, Trifluormethyl, Trifluormethoxy, Cyano oder Nitro substituiertes Phenyl (hervorgehoben für Wasserstoff, Methyl, Ethyl oder Cyclopropyl),
- D: steht weiterhin insbesondere bevorzugt für NH,
- Q¹: steht weiterhin insbesondere bevorzugt für Wasserstoff oder Methyl (hervorgehoben für Wasserstoff),
- Q²: steht weiterhin insbesondere bevorzugt für Wasserstoff oder Methyl (hervorgehoben für Wasserstoff),
- G: steht weiterhin insbesondere bevorzugt für Wasserstoff (a) oder für eine der Gruppen 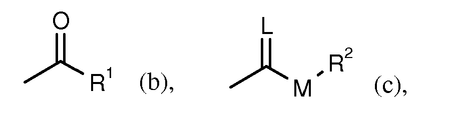 (hervorgehoben für Wasserstoff oder die Gruppe (c)),
in welchen
L für Sauerstoff steht und
M für Sauerstoff oder Schwefel steht (hervorgehoben für Sauerstoff),
- R¹: steht weiterhin insbesondere bevorzugt für jeweils gegebenenfalls einfach bis dreifach durch Fluor oder Chlor substituiertes C₁-C₆-Alkyl, C₂-C₆-Alkenyl, C₁-C₂-Alkoxy-C₁-C₂-alkyl, C₁-C₂-Alkylthio-C₁-C₂-alkyl oder für Cyclopropyl, Cyclopentyl oder Cyclohexyl,
für gegebenenfalls einfach durch Fluor, Chlor, Brom, Cyano, Nitro, Methyl, Methoxy, Trifluormethyl oder Trifluormethoxy substituiertes Phenyl,
für jeweils gegebenenfalls einfach durch Chlor oder Methyl substituiertes Furanyl, Thienyl oder Pyridyl,
- R²: steht weiterhin insbesondere bevorzugt für C₁-C₈-Alkyl, C₂-C₆-Alkenyl oder C₁-C₂-Alkoxy-C₂-C₃-alkyl,
für Cyclopentyl oder Cyclohexyl,
oder für jeweils gegebenenfalls einfach durch Fluor, Chlor, Cyano, Nitro, Methyl, Methoxy, Trifluormethyl oder Trifluormethoxy substituiertes Phenyl oder Benzyl (hervorgehoben für C₁-C₈-Alkyl).
- W: steht darüber hinaus insbesondere bevorzugt für Wasserstoff, Methyl, Ethyl, Chlor oder Brom (hervorgehoben für Wasserstoff, Chlor, Methyl oder Ethyl),
- X: steht darüber hinaus insbesondere bevorzugt für Chlor, Brom, Methyl, Ethyl, Propyl, Methoxy, Ethoxy, Propoxy, Trifluormethyl, Difluormethoxy oder Cyano (hervorgehoben für Chlor, Brom, Methyl, Ethyl oder Trifluormethyl),
- Y: steht darüber hinaus insbesondere bevorzugt in der 4-Position für Wasserstoff, Chlor, Brom, Methoxy, Trifluormethyl oder Trifluormethoxy (hervorgehoben für Wasserstoff, Chlor, Brom oder Methoxy),
- Z: steht darüber hinaus insbesondere bevorzugt für Wasserstoff,
- A: steht darüber hinaus insbesondere bevorzugt für -CH₂-,
- B: steht darüber hinaus insbesondere bevorzugt für Wasserstoff, Methyl, Ethyl, Propyl, iso-Propyl, C₂-C₄-Alkenyl, Methoxy, Ethoxy, Propoxy, Isopropoxy, Butoxy, Isobutoxy, für gegebenenfalls einfach bis zweifach durch Fluor, Chlor, Brom, Methyl, Methoxy, Trifluormethyl, Trifluormethoxy, Cyano oder Nitro substituiertes Phenyl, für Cyclopropyl, Cyclopentyl oder Cyclohexyl (hervorgehoben für Wasserstoff oder Propyl),
- D: steht darüber hinaus insbesondere bevorzugt für Sauerstoff,
- Q¹: steht darüber hinaus insbesondere bevorzugt für Wasserstoff oder Methyl (hervorgehoben für Wasserstoff),
- Q²: steht darüber hinaus insbesondere bevorzugt für Wasserstoff oder Methyl (hervorgehoben für Wasserstoff),
- G: steht insbesondere bevorzugt für Wasserstoff (a) oder für eine der Gruppen 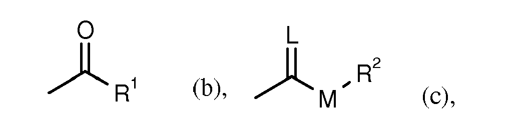 (hervorgehoben für Wasserstoff oder die Gruppe (b)),
in welchen
L für Sauerstoff steht und
M für Sauerstoff oder Schwefel steht,
- R¹: steht darüber hinaus insbesondere bevorzugt für jeweils gegebenenfalls einfach bis dreifach durch Fluor oder Chlor substituiertes C₁-C₆-Alkyl, C₂-C₆-Alkenyl, C₁-C₂-Alkoxy-C₁-C₂-alkyl, C₁-C₂-Alkylthio-C₁-C₂-alkyl oder für Cyclopropyl, Cyclopentyl oder Cyclohexyl,
für gegebenenfalls einfach durch Fluor, Chlor, Brom, Cyano, Nitro, Methyl, Methoxy, Trifluormethyl oder Trifluormethoxy substituiertes Phenyl,
für jeweils gegebenenfalls einfach durch Chlor oder Methyl substituiertes Furanyl, Thienyl oder Pyridyl (hervorgehoben für C₁-C₆-Alkyl),
- R²: steht darüber hinaus insbesondere bevorzugt für C₁-C₈-Alkyl, C₂-C₆-Alkenyl oder C₁-C₂-Alkoxy-C₂-C₃-alkyl,
für Cyclopentyl oder Cyclohexyl,
oder für jeweils gegebenenfalls einfach durch Fluor, Chlor, Cyano, Nitro, Methyl, Methoxy, Trifluormethyl oder Trifluormethoxy substituiertes Phenyl oder Benzyl.
- W: steht darüber hinaus auch insbesondere bevorzugt für Wasserstoff, Chlor, Brom, Methyl oder Ethyl (hervorgehoben für Methyl),
- X: steht darüber hinaus auch insbesondere bevorzugt für Chlor, Brom, Methyl, Ethyl, Propyl, Methoxy, Trifluormethyl, Difluormethoxy oder Cyano (hervorgehoben für Methyl oder Ethyl),
- Y: steht darüber hinaus auch insbesondere bevorzugt in der 4-Position für den Rest 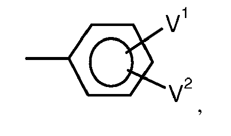
- Z: steht darüber hinaus auch insbesondere bevorzugt für Wasserstoff,
- V¹: steht darüber hinaus auch insbesondere bevorzugt für Fluor, Chlor, Methyl, Methoxy, Trifluormethyl oder Trifluormethoxy,
- V²: steht darüber hinaus auch insbesondere bevorzugt für Wasserstoff, Fluor, Chlor, Methyl, Methoxy oder Trifluormethyl,
- Y: steht darüber hinaus auch insbesondere bevorzugt für 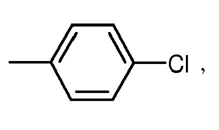
- A: steht darüber hinaus auch insbesondere bevorzugt für -CH₂-,
- B: steht darüber hinaus auch insbesondere bevorzugt für Wasserstoff, Methyl, Ethyl, Propyl, iso-Propyl, C₂-C₄-Alkenyl, Methoxy, Ethoxy, Propoxy, Isopropoxy, Butoxy, Isobutoxy, für gegebenenfalls einfach bis zweifach durch Fluor, Chlor, Brom, Methyl, Methoxy, Trifluormethyl, Trifluormethoxy, Cyano oder Nitro substituiertes Phenyl (hervorgehoben für Wasserstoff oder Methyl),
- D: steht darüber hinaus auch insbesondere bevorzugt für Sauerstoff,
- Q¹: steht darüber hinaus auch insbesondere bevorzugt für Wasserstoff oder Methyl (hervorgehoben für Wasserstoff),
- Q²: steht darüber hinaus auch insbesondere bevorzugt für Wasserstoff oder Methyl (hervorgehoben für Wasserstoff),
- G: steht darüber hinaus auch insbesondere bevorzugt für Wasserstoff (a) oder für eine der Gruppen 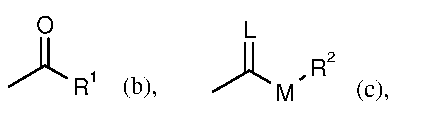 (hervorgehoben für Wasserstoff),
in welchen
L für Sauerstoff steht und
M für Sauerstoff oder Schwefel steht,
- R¹: steht darüber hinaus auch insbesondere bevorzugt für jeweils gegebenenfalls einfach bis dreifach durch Fluor oder Chlor substituiertes C₁-C₆-Alkyl, C₂-C₆-Alkenyl, C₁-C₂-Alkoxy-C₁-C₂-alkyl, C₁-C₂-Alkylthio-C₁-C₂-alkyl oder für Cyclpropyl, Cyclopentyl oder Cyclohexyl,
für gegebenenfalls einfach durch Fluor, Chlor, Brom, Cyano, Nitro, Methyl, Methoxy, Trifluormethyl oder Trifluormethoxy substituiertes Phenyl,
für jeweils gegebenenfalls einfach durch Chlor oder Methyl substituiertes Furanyl, Thienyl oder Pyridyl,
- R²: steht darüber hinaus auch insbesondere bevorzugt für C₁-C₈-Alkyl, C₂-C₆-Alkenyl oder C₁-C₂-Alkoxy-C₂-C₃-alkyl,
für Cyclopentyl oder Cyclohexyl,
oder für jeweils gegebenenfalls einfach durch Fluor, Chlor, Cyano, Nitro, Methyl, Methoxy, Trifluormethyl oder Trifluormethoxy substituiertes Phenyl oder Benzyl (hervorgehoben für C₁-C₈-Alkyl).
- W: steht darüber hinaus ebenfalls insbesondere bevorzugt für Wasserstoff oder Methyl,
- X: steht darüber hinaus ebenfalls insbesondere bevorzugt für Chlor oder Methyl,
- Y: steht darüber hinaus ebenfalls insbesondere bevorzugt in der 5-Position für den Rest 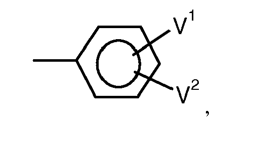
- Z: steht darüber hinaus ebenfalls insbesondere bevorzugt in der 4-Position für Wasserstoff oder Methyl,
- V¹: steht darüber hinaus ebenfalls insbesondere bevorzugt für Fluor, Chlor, Methyl, Methoxy, Trifluormethyl oder Trifluormethoxy,
- V²: steht darüber hinaus ebenfalls insbesondere bevorzugt für Wasserstoff, Fluor, Chlor, Methyl, Methoxy oder Trifluormethyl,
- Y: steht darüber hinaus ebenfalls insbesondere bevorzugt für 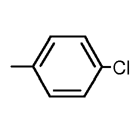 oder 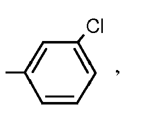
- A: steht darüber hinaus ebenfalls insbesondere bevorzugt für -CH₂-,
- B: steht darüber hinaus ebenfalls insbesondere bevorzugt für Wasserstoff, Methyl, Ethyl, Propyl, iso-Propyl, C₂-C₄-Alkenyl, Methoxy, Ethoxy, Propoxy, Isopropoxy, Butoxy, Isobutoxy, für gegebenenfalls einfach bis zweifach durch Fluor, Chlor, Brom, Methyl, Methoxy, Trifluormethyl, Trifluormethoxy, Cyano oder Nitro substituiertes Phenyl (hervorgehoben für Wasserstoff),
- D: steht darüber hinaus ebenfalls insbesondere bevorzugt für Sauerstoff,
- Q¹: steht darüber hinaus ebenfalls insbesondere bevorzugt für Wasserstoff oder Methyl (hervorgehoben für Wasserstoff),
- Q²: steht darüber hinaus ebenfalls insbesondere bevorzugt für Wasserstoff oder Methyl (hervorgehoben für Wasserstoff),
- G: steht darüber hinaus ebenfalls insbesondere bevorzugt für Wasserstoff (a) oder für eine der Gruppen 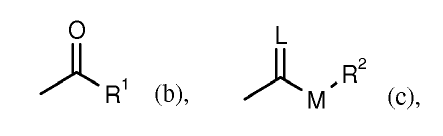 in welchen
L für Sauerstoff steht und
M für Sauerstoff oder Schwefel steht (hervorgehoben für Sauerstoff),
- R¹: steht darüber hinaus ebenfalls insbesondere bevorzugt für jeweils gegebenenfalls einfach bis dreifach durch Fluor oder Chlor substituiertes C₁-C₆-Alkyl, C₂-C₆-Alkenyl, C₁-C₂-Alkoxy-C₁-C₂-alkyl, C₁-C₂-Alkylthio-C₁-C₂-alkyl oder für Cyclopropyl, Cyclopentyl oder Cyclohexyl,
für gegebenenfalls einfach durch Fluor, Chlor, Brom, Cyano, Nitro, Methyl, Methoxy, Trifluormethyl oder Trifluormethoxy substituiertes Phenyl,
für jeweils gegebenenfalls einfach durch Chlor oder Methyl substituiertes Furanyl, Thienyl oder Pyridyl (hervorgehoben für C₁-C₆-Alkyl),
- R²: steht darüber hinaus ebenfalls insbesondere bevorzugt für C₁-C₈-Alkyl, C₂-C₆-Alkenyl oder Cₗ-C₂-Alkoxy-C₂-C₃-alkyl,
für Cyclopentyl oder Cyclohexyl,
oder für jeweils gegebenenfalls einfach durch Fluor, Chlor, Cyano, Nitro, Methyl, Methoxy, Trifluormethyl oder Trifluormethoxy substituiertes Phenyl oder Benzyl (hervorgehoben für C₁-C₈-Alkyl).
- W: steht darüber hinaus außerdem insbesondere bevorzugt für Wasserstoff, Methyl, Chlor oder Brom,
- X: steht darüber hinaus außerdem insbesondere bevorzugt für Chlor, Brom, Methyl, Ethyl, Propyl, Methoxy, Ethoxy, Propoxy, Trifluormethyl, Difluormethoxy oder Cyano (hervorgehoben für Chlor, Brom, Methyl oder Methoxy),
- Y: steht darüber hinaus außerdem insbesondere bevorzugt in der 4-Position für Methyl oder Ethyl,
- Z: steht darüber hinaus außerdem insbesondere bevorzugt für Wasserstoff,
- A: steht darüber hinaus außerdem insbesondere bevorzugt für -CH₂-,
- B: steht darüber hinaus außerdem insbesondere bevorzugt für Wasserstoff, Methyl, Ethyl, Propyl, iso-Propyl, C₂-C₄-Alkenyl, Methoxy, Ethoxy, Propoxy, Isopropoxy, Butoxy, Isobutoxy, für gegebenenfalls einfach bis zweifach durch Fluor, Chlor, Brom, Methyl, Methoxy, Trifluormethyl, Trifluormethoxy, Cyano oder Nitro substituiertes Phenyl, für Cyclopropyl, für Cyclopentyl oder Cyclohexyl, in welchem gegebenenfalls eine Methylengruppe durch Sauerstoff ersetzt ist (hervorgehoben für Wasserstoff oder Propyl),
- Q¹: steht darüber hinaus außerdem insbesondere bevorzugt für Wasserstoff oder Methyl (hervorgehoben für Wasserstoff),
- Q²: steht darüber hinaus außerdem insbesondere bevorzugt für Wasserstoff oder Methyl (hervorgehoben für Wasserstoff),
- D: steht darüber hinaus außerdem insbesondere bevorzugt für Sauerstoff,
- G: steht darüber hinaus außerdem insbesondere bevorzugt für Wasserstoff (a) oder für eine der Gruppen 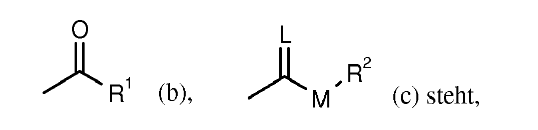 in welchen
L für Sauerstoff steht und
M für Sauerstoff oder Schwefel steht (hervorgehoben für Sauerstoff),
- R¹: steht darüber hinaus außerdem insbesondere bevorzugt für jeweils gegebenenfalls einfach bis dreifach durch Fluor oder Chlor substituiertes C₁-C₆-Alkyl, C₂-C₆-Alkenyl, C₁-C₂-Alkoxy-C₁-C₂-alkyl, C₁-C₂-Alkylthio-C₁-C₂-alkyl oder für Cyclopropyl, Cyclopentyl oder Cyclohexyl,
für gegebenenfalls einfach durch Fluor, Chlor, Brom, Cyano, Nitro, Methyl, Methoxy, Trifluormethyl oder Trifluormethoxy substituiertes Phenyl,
für jeweils gegebenenfalls einfach durch Chlor oder Methyl substituiertes Furanyl, Thienyl oder Pyridyl (hervorgehoben für C₁-C₆-Alkyl),
- R²: steht darüber hinaus außerdem insbesondere bevorzugt für C₁-C₁₀-Alkyl, C₂-C₁₀-Alkenyl oder C₁-C₂-Alkoxy-C₂-C₄-alkyl,
für Cyclopentyl oder Cyclohexyl,
oder für jeweils gegebenenfalls einfach durch Fluor, Chlor, Cyano, Nitro, Methyl, Methoxy, Trifluormethyl oder Trifluormethoxy substituiertes Phenyl oder Benzyl (hervorgehoben für C₁-C₁₀-Alkyl).
- W: steht darüber hinaus weiterhin insbesondere bevorzugt für Wasserstoff, Methyl, Chlor oder Brom (hervorgehoben für Wasserstoff, Chlor oder Methyl),
- X: steht darüber hinaus weiterhin insbesondere bevorzugt für Chlor, Brom, Methyl, Methoxy oder Trifluormethyl (hervorgehoben für Chlor, Brom oder Methyl),
- Y: steht darüber hinaus weiterhin insbesondere bevorzugt in der 4-Position für Wasserstoff, Chlor, Brom oder Methyl,
- Z: steht darüber hinaus weiterhin insbesondere bevorzugt in der 3- oder 5-Position für Chlor, Fluor, Brom, Methyl, Ethyl, Trifluormethyl oder Trifluormethoxy (hervorgehoben für Fluor, Chlor, Brom oder Methyl),
- A: steht darüber hinaus weiterhin insbesondere bevorzugt für -CH₂-,
- B: steht darüber hinaus weiterhin insbesondere bevorzugt für Wasserstoff, Methyl, Ethyl, Propyl, iso-Propyl, C₂-C₄-Alkenyl, Methoxy, Ethoxy, Propoxy, Isopropoxy, Butoxy, Isobutoxy, für gegebenenfalls einfach bis zweifach durch Fluor, Chlor, Brom, Methyl, Methoxy, Trifluormethyl, Trifluormethoxy, Cyano oder Nitro substituiertes Phenyl, für Cyclopropyl, für Cyclopentyl oder Cyclohexyl, in welchem gegebenenfalls eine Methylengruppe durch Sauerstoff ersetzt ist (hervorgehoben für Wasserstoff oder Propyl),
- Q¹: steht darüber hinaus weiterhin insbesondere bevorzugt für Wasserstoff oder Methyl (hervorgehoben für Wasserstoff),
- Q²: steht darüber hinaus weiterhin insbesondere bevorzugt für Wasserstoff oder Methyl (hervorgehoben für Wasserstoff),
- D: steht darüber hinaus weiterhin insbesondere bevorzugt für Sauerstoff,
- G: steht darüber hinaus weiterhin insbesondere bevorzugt für Wasserstoff (a) oder für eine der Gruppen 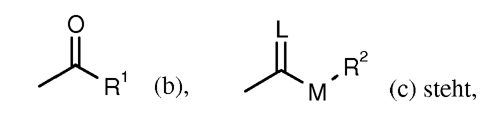 (hervorgehoben für Wasserstoff),
in welchen
L für Sauerstoff steht und
M für Sauerstoff oder Schwefel steht,
- R¹: steht darüber hinaus weiterhin insbesondere bevorzugt für jeweils gegebenenfalls einfach bis dreifach durch Fluor oder Chlor substituiertes C₁-C₆-Alkyl, C₂-C₆-Alkenyl, C₁-C₂-Alkoxy-C₁-C₂-alkyl, C₁-C₂-Alkylthio-C₁-C₂-alkyl oder für Cyclopropyl, Cyclopentyl oder Cyclohexyl,
für gegebenenfalls einfach durch Fluor, Chlor, Brom, Cyano, Nitro, Methyl, Methoxy, Trifluormethyl oder Trifluormethoxy substituiertes Phenyl,
für jeweils gegebenenfalls einfach durch Chlor oder Methyl substituiertes Furanyl, Thienyl oder Pyridyl,
- R²: steht darüber hinaus weiterhin insbesondere bevorzugt für C₁-C₁₀-Alkyl, C₂-C₁₀-Alkenyl oder C₁-C₂-Alkoxy-C₂-C₄-alkyl,
für Cyclopentyl oder Cyclohexyl,
oder für jeweils gegebenenfalls einfach durch Fluor, Chlor, Cyano, Nitro, Methyl, Methoxy, Trifluormethyl oder Trifluormethoxy substituiertes Phenyl oder Benzyl.

Die oben aufgeführten allgemeinen oder in Vorzugsbereichen aufgeführten Restedefinitionen bzw. Erläuterungen können untereinander, also auch zwischen den jeweiligen Bereichen und Vorzugsbereichen beliebig kombiniert werden. Sie gelten für die Endprodukte sowie für die Vor- und Zwischenprodukte entsprechend.

Erfindungsgemäß bevorzugt werden die Verbindungen der Formel (I), in welchen eine Kombination der vorstehend als bevorzugt (vorzugsweise) aufgeführten Bedeutungen vorliegt.

Erfindungsgemäß besonders bevorzugt werden die Verbindungen der Formel (I), in welchen eine Kombination der vorstehend als besonders bevorzugt aufgeführten Bedeutungen vorliegt.

Erfindungsgemäß ganz besonders bevorzugt werden die Verbindungen der Formel (I), in welchen eine Kombination der vorstehend als ganz besonders bevorzugt aufgeführten Bedeutungen vorliegt.

Erfindungsgemäß insbesondere bevorzugt werden die Verbindungen der Formel (I), in welchen eine Kombination der vorstehend als insbesondere bevorzugt aufgeführten Bedeutungen vorliegt.

Gesättigte oder ungesättigte Kohlenwasserstoffreste wie Alkyl, Alkandiyl oder Alkenyl können, auch in Verbindung mit Heteroatomen, wie z.B. in Alkoxy, soweit möglich, jeweils geradkettig oder verzweigt sein.

Gegebenenfalls substituierte Reste können, sofern nicht anderes angegeben ist, einfach oder mehrfach substituiert sein, wobei bei Mehrfachsubstitutionen die Substituenten gleich oder verschieden sein können.

Im einzelnen seien außer den bei den Herstellungsbeispielen genannten Verbindungen die folgenden Verbindungen der Formel (I-1-a) genannt:

**Tabelle 1**

| | | | | | |
|---|---|---|---|---|---|
| 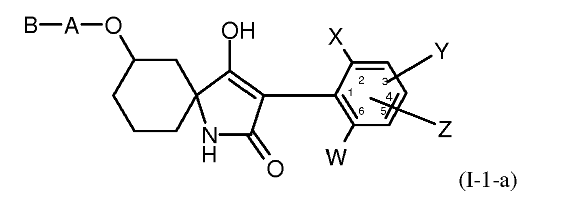 | | | | | |

| **A** | **B** | **X** | **W** | **Y** | Z |
|---|---|---|---|---|---|
| CH₂ | H | CH₃ | H | H | H |
| CH₂ | H | Br | H | H | H |
| CH₂ | H | Cl | H | H | H |
| CH₂ | H | CF₃ | H | H | H |
| CH₂ | H | OCH₃ | H | H | H |
| CH₂ | H | Br | H | 4-Cl | H |
| CH₂ | H | Cl | H | 4-Br | H |
| CH₂ | H | Cl | H | 4-Cl | H |
| CH₂ | H | Cl | H | 4-CH₃ | H |
| CH₂ | H | CH₃ | H | 4-Cl | H |
| CH₂ | H | CH₃ | H | 4-CH₃ | H |
| CH₂ | H | Cl | Cl | H | H |
| CH₂ | H | Cl | OCH₃ | H | H |
| CH₂ | H | Cl | CH₃ | H | H |
| CH₂ | H | Cl | OC₂H₅ | H | H |
| CH₂ | H | OCH₃ | OCH₃ | H | H |
| CH₂ | H | CH₃ | CH₃ | H | H |
| CH₂ | H | Br | CH₃ | 4-Br | H |
| CH₂ | H | Cl | Cl | 4-CH₃ | H |
| CH₂ | H | CH₃ | Br | 4-CH₃ | H |
| CH₂ | H | CH₃ | Cl | 4-CH₃ | H |
| CH₂ | H | OCH₃ | CH₃ | 4-CH₃ | H |
| CH₂ | H | OC₂H₅ | CH₃ | 4-CH₃ | H |
| CH₂ | H | OC₃H₇ | CH₃ | 4-CH₃ | H |
| CH₂ | H | CH₃ | CH₃ | 4-CH₃ | H |
| CH₂ | H | Br | Br | 4-CH₃ | H |
| CH₂ | H | CH₃ | CH₃ | 4-Br | H |
| CH₂ | H | C₂H₅ | CH₃ | H | H |
| CH₂ | H | C₂H₅ | C₂H₅ | H | H |
| CH₂ | H | OCH₃ | C₂H₅ | 4-CH₃ | H |
| CH₂ | H | CH₃ | CH₃ | 4-OCH₃ | H |
| CH₂ | H | Br | Cl | 4-CH₃ | H |
| CH₂ | H | Br | CH₃ | 4-Cl | H |
| CH₂ | H | Cl | CH₃ | 4-Br | H |
| CH₂ | H | CH₃ | CH₃ | 4-Cl | H |
| CH₂ | H | C₂H₅ | CH₃ | 4-CH₃ | H |
| CH₂ | H | C₂H₅ | CH₃ | 4-C₂H₅ | H |
| CH₂ | H | C₂H₅ | C₂H₅ | 4-CH₃ | H |
| CH₂ | H | C₂H₅ | C₂H₅ | 4-C₂H₅ | H |
| CH₂ | H | C₂H₅ | CH₃ | 4-Cl | H |
| CH₂ | H | C₂H₅ | C₂H₅ | 4-Cl | H |
| CH₂ | H | C₂H₅ | CH₃ | 4-Br | H |
| CH₂ | H | C₂H₅ | C₂H₅ | 4-Br | H |
| CH₂ | H | C₂H₅ | Cl | 4-CH₃ | H |
| CH₂ | H | C₂H₅ | Br | 4-CH₃ | H |
| CH₂ | H | C₂H₅ | Cl | 4-Cl | H |
| CH₂ | H | C₂H₅ | Br | 4-Br | H |
| CH₂ | H | C₂H₅ | Cl | 4-Br | H |
| CH₂ | H | C₂H₅ | Br | 4-Cl | H |
| CH₂ | H | OCH₃ | CH₃ | 4-Cl | H |
| CH₂ | H | OCH₃ | C₂H₅ | 4-Cl | H |
| CH₂ | H | OC₂H₅ | CH₃ | 4-Cl | H |
| CH₂ | H | OC₂H₅ | C₂H₅ | 4-Cl | H |
| CH₂ | H | Cl | OCH₃ | 4-CH₃ | H |
| CH₂ | H | Cl | OC₂H₅ | 4-CH₃ | H |
| CH₂ | H | Cl | Cl | 4-Cl | H |
| CH₂ | H | Cl | H | 4-Cl | 5-Cl |
| CH₂ | H | CH₃ | H | 4-CH₃ | 5-CH₃ |
| CH₂ | H | CH₃ | H | 4-Cl | 5-CH₃ |
| CH₂ | H | Br | H | 4-Cl | 5-CH₃ |
| CH₂ | H | Br | H | 4-CH₃ | 5-CH₃ |
| CH₂ | H | Cl | H | 4-Br | 5-CH₃ |
| CH₂ | H | Cl | H | 4-Cl | 5-CH₃ |
| CH₂ | H | CH₃ | H | 4-Br | 5-CH₃ |
| CH₂ | H | Cl | H | 4-CH₃ | 5-Cl |
| CH₂ | H | CH₃ | H | H | 5-CH₃ |
| CH₂ | H | Cl | H | H | 5-CH₃ |
| CH₂ | H | Br | H | H | 5-CH₃ |
| CH₂ | H | CH₃ | H | H | 5-Cl |
| CH₂ | H | CH₃ | H | H | 5-Br |
| CH₂ | H | CH₃ | CH₃ | 4-CH₃ | 5-CH₃ |
| CH₂ | H | CH₃ | CH₃ | 4-CH₃ | 5-Cl |
| CH₂ | H | CH₃ | CH₃ | 4-CH₃ | 5-Br |
| CH₂ | H | CH₃ | CH₃ | H | 3-Cl |
| CH₂ | H | CH₃ | CH₃ | H | 3-Br |
| CH₂ | H | Cl | Cl | H | 3-Br |
| CH₂ | H | CH₃ | CH₃ | 4-(4-Cl-C₆H₄) | H |
| CH₂ | H | C₂H₅ | CH₃ | 4-(4-Cl-C₆H₄) | H |
| CH₂ | H | C₂H₅ | C₂H₅ | 4-(4-Cl-C₆H₄) | H |
| CH₂ | H | Cl | CH₃ | 4-(4-Cl-C₆H₄) | H |
| CH₂ | H | Cl | C₂H₅ | 4-(4-Cl-C₆H₄) | H |
| CH₂ | H | CH₃ | H | 5-(4-Cl-C₆H₄) | H |
| CH₂ | H | CH₃ | CH₃ | 5-(4-Cl-C₆H₄) | H |
| CH₂ | H | CH₃ | H | 5-(4-Cl-C₆H₄) | 4-CH₃ |
| CH₂ | H | CH₃ | CH₃ | 5-(4-Cl-C₆H₄) | 4-CH₃ |
| CH₂ | H | Cl | H | 5-(4-Cl-C₆H₄) | H |
| CH₂ | H | O-(CH₂)₂-OCH₃ | CH₃ | 4-Cl | H |
| CH₂ | H | O-(CH₂)₂-OCH₃ | C₂H₅ | 4-Cl | H |
| CH₂ | H | O-CH₃ | CH₃ | 4-Br | H |
| CH₂ | H | O-CH₃ | C₂H₅ | 4-Br | H |
| CH₂ | H | O-C₂H₅ | CH₃ | 4-Br | H |
| CH₂ | H | O-C₂H₅ | C₂H₅ | 4-Br | H |

- **Tabelle 2**:: A, W, X, Y und Z wie in Tabelle 1 angegeben B = CH₃
- **Tabelle 3:**: A, W, X, Y und Z wie in Tabelle 1 angegeben B = C₂H₅
- **Tabelle 4:**: A, W, X, Y und Z wie in Tabelle 1 angegeben B = C₃H₇
- **Tabelle 5:**: A, W, X, Y und Z wie in Tabelle 1 angegeben B = i-C₃H₇
- **Tabelle 6:**: A, W, X, Y und Z wie in Tabelle 1 angegeben B = 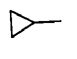
- **Tabelle 7:**: A, W, X, Y und Z wie in Tabelle 1 angegeben B = 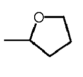
- **Tabelle 8:**: A, W, X, Y und Z wie in Tabelle 1 angegeben A = -CH₂-CH₂-; B = OCH₃
- **Tabelle 9:**: A, W, X, Y und Z wie in Tabelle 1 angegeben A = -CH₂-CH₂-; B = OC₂H₅

Im einzelnen seien außer den bei den Herstellungsbeispielen genannten Verbindungen die folgenden Verbindungen der Formel (I-2-a) genannt:

**Tabelle 10**

| | | | | | |
|---|---|---|---|---|---|
| 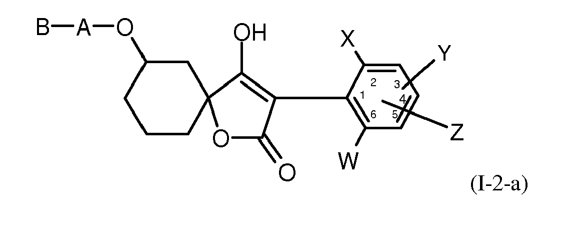 | | | | | |

| **A** | **B** | **X** | **W** | **Y** | **Z** |
|---|---|---|---|---|---|
| CH₂ | H | CH₃ | H | H | H |
| CH₂ | H | Br | H | H | H |
| CH₂ | H | Cl | H | H | H |
| CH₂ | H | CF₃ | H | H | H |
| CH₂ | H | OCH₃ | H | H | H |
| CH₂ | H | Br | H | 4-Cl | H |
| CH₂ | H | Cl | H | 4-Br | H |
| CH₂ | H | Cl | H | 4-Cl | H |
| CH₂ | H | Cl | H | 4-CH₃ | H |
| CH₂ | H | CH₃ | H | 4-Cl | H |
| CH₂ | H | CH₃ | H | 4-CH₃ | H |
| CH₂ | H | Cl | Cl | H | H |
| CH₂ | H | Cl | OCH₃ | H | H |
| CH₂ | H | Cl | CH₃ | H | H |
| CH₂ | H | Cl | OC₂H₅ | H | H |
| CH₂ | H | OCH₃ | OCH₃ | H | H |
| CH₂ | H | CH₃ | CH₃ | H | H |
| CH₂ | H | C₂H₅ | CH₃ | H | H |
| CH₂ | H | C₂H₅ | C₂H₅ | H | H |
| CH₂ | H | Br | CH₃ | 4-Br | H |
| CH₂ | H | Cl | Cl | 4-CH₃ | H |
| CH₂ | H | CH₃ | Br | 4-CH₃ | H |
| CH₂ | H | CH₃ | Cl | 4-CH₃ | H |
| CH₂ | H | OCH₃ | CH₃ | 4-CH₃ | H |
| CH₂ | H | OCH₃ | C₂H₅ | 4-CH₃ | H |
| CH₂ | H | OC₂H₅ | CH₃ | 4-CH₃ | H |
| CH₂ | H | OC₃H₇ | CH₃ | 4-CH₃ | H |
| CH₂ | H | CH₃ | CH₃ | 4-CH₃ | H |
| CH₂ | H | Br | Br | 4-CH₃ | H |
| CH₂ | H | Cl | Cl | 4-CH₃ | H |
| CH₂ | H | CH₃ | CH₃ | 4-Br | H |
| CH₂ | H | CH₃ | CH₃ | 4-OCH₃ | H |
| CH₂ | H | Br | Cl | 4-CH₃ | H |
| CH₂ | H | Br | CH₃ | 4-Cl | H |
| CH₂ | H | Cl | CH₃ | 4-Br | H |
| CH₂ | H | CH₃ | CH₃ | 4-Cl | H |
| CH₂ | H | C₂H₅ | CH₃ | 4-CH₃ | H |
| CH₂ | H | C₂H₅ | C₂H₅ | 4-CH₃ | H |
| CH₂ | H | C₂H₅ | CH₃ | 4-C₂H₅ | H |
| CH₂ | H | C₂H₅ | C₂H₅ | 4-C₂H₅ | H |
| CH₂ | H | C₂H₅ | CH₃ | 4-Cl | H |
| CH₂ | H | C₂H₅ | C₂H₅ | 4-Cl | H |
| CH₂ | H | C₂H₅ | CH₃ | 4-Br | H |
| CH₂ | H | C₂H₅ | C₂H₅ | 4-Br | H |
| CH₂ | H | C₂H₅ | Cl | 4-CH₃ | H |
| CH₂ | H | C₂H₅ | Br | 4-CH₃ | H |
| CH₂ | H | C₂H₅ | Cl | 4-Cl | H |
| CH₂ | H | C₂H₅ | Br | 4-Br | H |
| CH₂ | H | C₂H₅ | Cl | 4-Br | H |
| CH₂ | H | C₂H₅ | Br | 4-Cl | H |
| CH₂ | H | OCH₃ | CH₃ | 4-Cl | H |
| CH₂ | H | OCH₃ | C₂H₅ | 4-Cl | H |
| CH₂ | H | OC₂H₅ | CH₃ | 4-Cl | H |
| CH₂ | H | OC₂H₅ | C₂H₅ | 4-Cl | H |
| CH₂ | H | Cl | OCH₃ | 4-CH₃ | H |
| CH₂ | H | Cl | OC₂H₅ | 4-CH₃ | H |
| CH₂ | H | CH₃ | CH₃ | 4-Cl | H |
| CH₂ | H | Cl | H | 4-Cl | 5-Cl |
| CH₂ | H | CH₃ | H | 4-CH₃ | 5-CH₃ |
| CH₂ | H | CH₃ | H | 4-Cl | 5-CH₃ |
| CH₂ | H | Br | H | 4-Cl | 5-CH₃ |
| CH₂ | H | Br | H | 4-CH₃ | 5-CH₃ |
| CH₂ | H | Cl | H | 4-Br | 5-CH₃ |
| CH₂ | H | Cl | H | 4-Cl | 5-CH₃ |
| CH₂ | H | CH₃ | H | 4-Br | 5-CH₃ |
| CH₂ | H | Cl | H | 4-CH₃ | 5-Cl |
| CH₂ | H | CH₃ | H | H | 5-CH₃ |
| CH₂ | H | Cl | H | H | 5-CH₃ |
| CH₂ | H | Br | H | H | 5-CH₃ |
| CH₂ | H | CH₃ | H | H | 5-Cl |
| CH₂ | H | CH₃ | H | H | 5-Br |
| CH₂ | H | CH₃ | CH₃ | 4-CH₃ | 5-CH₃ |
| CH₂ | H | CH₃ | CH₃ | 4-CH₃ | 5-Cl |
| CH₂ | H | CH₃ | CH₃ | 4-CH₃ | 5-Br |
| CH₂ | H | CH₃ | CH₃ | H | 3-Cl |
| CH₂ | H | CH₃ | CH₃ | H | 3-Br |
| CH₂ | H | Cl | Cl | H | 3-Br |
| CH₂ | H | CH₃ | CH₃ | 4-(4-Cl-C₆H₄) | H |
| CH₂ | H | C₂H₅ | CH₃ | 4-(4-Cl-C₆H₄) | H |
| CH₂ | H | C₂H₅ | C₂H₅ | 4-(4-Cl-C₆H₄) | H |
| CH₂ | H | Cl | CH₃ | 4-(4-Cl-C₆H₄) | H |
| CH₂ | H | Cl | C₂H₅ | 4-(4-Cl-C₆H₄) | H |
| CH₂ | H | CH₃ | H | 5-(4-Cl-C₆H₄) | H |
| CH₂ | H | CH₃ | CH₃ | 5-(4-Cl-C₆H₄) | H |
| CH₂ | H | CH₃ | H | 5-(4-Cl-C₆H₄) | 4-CH₃ |
| CH₂ | H | CH₃ | CH₃ | 5-(4-Cl-C₆H₄) | 4-CH₃ |
| CH₂ | H | Cl | H | 5-(4-Cl-C₆H₄) | H |

- **Tabelle 11:**: A, W, X, Y und Z wie in Tabelle 10 angegeben B = CH3
- **Tabelle 12**:: A, W, X, Y und Z wie in Tabelle 10 angegeben B = C₂H₅
- **Tabelle 13:**: A, W, X, Y und Z wie in Tabelle 10 angegeben B = C₃H₇
- **Tabelle 14:**: A, W, X, Y und Z wie in Tabelle 10 angegeben B = i-C₃H₇
- **Tabelle 15**:: A, W, X, Y und Z wie in Tabelle 10 angegeben B = 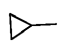
- **Tabelle 16:**: A, W, X, Y und Z wie in Tabelle 10 angegeben B = 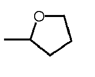
- **Tabelle 17:**: W, X, Y und Z wie in Tabelle 10 angegeben A = -CH₂-CH₂-; B = OCH₃
- **Tabelle 18:**: W, X, Y und Z wie in Tabelle 10 angegeben A = -CH₂-CH₂-; B = OC₂H₅

Bevorzugte Bedeutungen der oben in Zusammenhang mit den die Kulturpflanzen-Verträglichkeit verbessernden Verbindungen ("Herbizid-Safenern") der Formeln (IIa), (IIb), (IIc), (IId) und (IIe) aufgeführten Gruppen werden im Folgenden definiert.
- m: steht bevorzugt für die Zahlen 0, 1, 2, 3 oder 4.
- A¹: steht bevorzugt für eine der nachstehend skizzierten divalenten heterocyclischen Gruppierungen 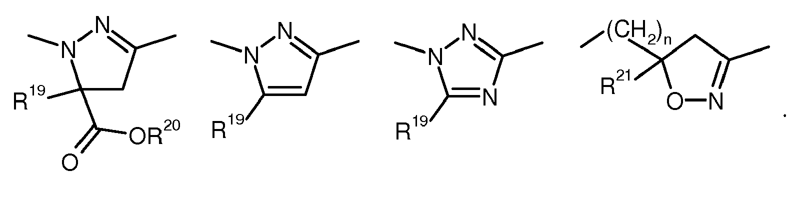
- n: steht bevorzugt für die Zahlen 0, 1, 2, 3 oder 4.
- A²: steht bevorzugt für jeweils gegebenenfalls durch Methyl, Ethyl, Methoxycarbonyl, Ethoxycarbonyl oder Alkyloxycarbonyl substituiertes Methylen oder Ethylen.
- R¹⁴: steht bevorzugt für Hydroxy, Mercapto, Amino, Methoxy, Ethoxy, n- oder i-Propoxy, n-, i-, s- oder t-Butoxy, Methylthio, Ethylthio, n- oder i-Propylthio, n-, i-, s- oder t-Butylthio, Methylamino, Ethylamino, n- oder i-Propylamino, n-, i-, s- oder t-Butylamino, Dimethylamino oder Diethylamino.
- R¹⁵: steht bevorzugt für Hydroxy, Mercapto, Amino, Methoxy, Ethoxy, n- oder i-Propoxy, n-, i-, s- oder t-Butoxy, 1-Methyl-hexyloxy, Allyloxy, 1-Allyloxymethyl-ethoxy, Methylthio, Ethylthio, n- oder i-Propylthio, n-, i-, s- oder t-Butylthio, Methylamino, Ethylamino, n- oder i-Propylamino, n-, i-, s- oder t-Butylamino, Dimethylamino oder Diethylamino.
- R¹⁶: steht bevorzugt für jeweils gegebenenfalls durch Fluor, Chlor und/oder Brom substituiertes Methyl, Ethyl, n- oder i-Propyl.
- R¹⁷: steht bevorzugt für Wasserstoff, jeweils gegebenenfalls durch Fluor und/oder Chlor substituiertes Methyl, Ethyl, n- oder i-Propyl, n-, i-, s- oder t-Butyl, Propenyl, Butenyl, Propinyl oder Butinyl, Methoxymethyl, Ethoxymethyl, Methoxyethyl, Ethoxyethyl, Dioxolanylmethyl, Furyl, Furylmethyl, Thienyl, Thiazolyl, Piperidinyl, oder gegebenenfalls durch Fluor, Chlor, Methyl, Ethyl, n- oder i-Propyl, n-, i-, s- oder t-Butyl substituiertes Phenyl.
- R¹⁸: steht bevorzugt für Wasserstoff, jeweils gegebenenfalls durch Fluor und/oder Chlor substituiertes Methyl, Ethyl, n- oder i-Propyl, n-, i-, s- oder t-Butyl, Propenyl, Butenyl, Propinyl oder Butinyl, Methoxymethyl, Ethoxymethyl, Methoxyethyl, Ethoxyethyl, Dioxolanylmethyl, Furyl, Furylmethyl, Thienyl, Thiazolyl, Piperidinyl, oder gegebenenfalls durch Fluor, Chlor, Methyl, Ethyl, n- oder i-Propyl, n-, i-, s- oder t-Butyl substituiertes Phenyl, oder zusammen mit R¹⁷ für einen der Reste -CH₂-O-CH₂-CH₂- und -CH₂-CH₂-O-CH₂-CH₂-, die gegebenenfalls substituiert sind durch Methyl, Ethyl, Furyl, Phenyl, einen annellierten Benzolring oder durch zwei Substituenten, die gemeinsam mit dem C-Atom, an das sie gebunden sind, einen 5- oder 6-gliedrigen Carbocyclus bilden.
- R¹⁹: steht bevorzugt für Wasserstoff, Cyano, Fluor, Chlor, Brom, oder für jeweils gegebenenfalls durch Fluor, Chlor und/oder Brom substituiertes Methyl, Ethyl, n- oder i-Propyl, Cyclopropyl, Cyclobutyl, Cyclopentyl, Cyclohexyl oder Phenyl.
- R²⁰: steht bevorzugt für Wasserstoff, jeweils gegebenenfalls durch Hydroxy, Cyano, Fluor, Chlor, Methoxy, Ethoxy, n- oder i-Propoxy substituiertes Methyl, Ethyl, n- oder i-Propyl, n-, i-, s- oder t-Butyl.
- R²¹: steht bevorzugt für Wasserstoff, Cyano, Fluor, Chlor, Brom, oder für jeweils gegebenenfalls durch Fluor, Chlor und/oder Brom substituiertes Methyl, Ethyl, n- oder i-Propyl, n-, i-, s- oder t-Butyl, Cyclopropyl, Cyclobutyl, Cyclopentyl, Cyclohexyl oder Phenyl.
- X¹: steht bevorzugt für Nitro, Cyano, Fluor, Chlor, Brom, Methyl, Ethyl, n- oder i-Propyl, n-, i-, s- oder t-Butyl, Difluormethyl, Dichlormethyl, Trifluormethyl, Trichlormethyl, Chlordifluormethyl, Fluordichlormethyl, Methoxy, Ethoxy, n- oder i-Propoxy, Difluormethoxy oder Trifluormethoxy.
- X²: steht bevorzugt für Wasserstoff, Nitro, Cyano, Fluor, Chlor, Brom, Methyl, Ethyl, n- oder i-Propyl, n-, i-, s- oder t-Butyl, Difluormethyl, Dichlormethyl, Trifluormethyl, Trichlormethyl, Chlordifluormethyl, Fluordichlormethyl, Methoxy, Ethoxy, n- oder i-Propoxy, Difluormethoxy oder Trifluormethoxy.
- X³: steht bevorzugt für Wasserstoff, Nitro, Cyano, Fluor, Chlor, Brom, Methyl, Ethyl, n- oder i-Propyl, n-, i-, s- oder t-Butyl, Difluormethyl, Dichlormethyl, Trifluormethyl, Trichlormethyl, Chlordifluormethyl, Fluordichlormethyl, Methoxy, Ethoxy, n- oder i-Propoxy, Difluormethoxy oder Trifluormethoxy.
- t: steht bevorzugt für die Zahlen 0, 1, 2, 3 oder 4.
- v: steht bevorzugt für die Zahlen 0, 1, 2, 3 oder 4.
- R²²: steht bevorzugt für Wasserstoff, Methyl, Ethyl, n- oder i-Propyl.
- R²³: steht bevorzugt für Wasserstoff, Methyl, Ethyl, n- oder i-Propyl.
- R²⁴: steht bevorzugt für Wasserstoff, jeweils gegebenenfalls durch Cyano, Fluor, Chlor, Methoxy, Ethoxy, n- oder i-Propoxy substituiertes Methyl, Ethyl, n- oder i-Propyl, n-, i-, s- oder t-Butyl, Methoxy, Ethoxy, n- oder i-Propoxy, n-, i-, s- oder t-Butoxy, Methylthio, Ethylthio, n- oder i-Propylthio, n-, i-, s- oder t-Butylthio, , Methylamino, Ethylamino, n- oder i-Propylamino, n-, i-, s- oder t-Butylamino, Dimethylamino oder Diethylamino, oder jeweils gegebenenfalls durch Cyano, Fluor, Chlor, Brom, Methyl, Ethyl, n- oder i-Propyl substituiertes Cyclopropyl, Cyclobutyl, Cyclopentyl, Cyclohexyl, Cyclopropyloxy, Cyclobutyloxy, Cyclopentyloxy, Cyclohexyloxy, Cyclopropylthio, Cyclobutylthio, Cyclopentylthio, Cyclohexylthio, Cyclopropylamino, Cyclobutylamino, Cyclopentylamino oder Cyclohexylamino.
- R²⁵: steht bevorzugt für Wasserstoff, jeweils gegebenenfalls durch Cyano, Hydroxy, Fluor, Chlor, Methoxy, Ethoxy, n- oder i-Propoxy substituiertes Methyl, Ethyl, n- oder i-Propyl, n-, i- oder s-Butyl, jeweils gegebenenfalls durch Cyano, Fluor, Chlor oder Brom substituiertes Propenyl, Butenyl, Propinyl oder Butinyl, oder jeweils gegebenenfalls durch Cyano, Fluor, Chlor, Brom, Methyl, Ethyl, n- oder i-Propyl substituiertes Cyclopropyl, Cyclobutyl, Cyclopentyl oder Cyclohexyl.
- R²⁶: steht bevorzugt für Wasserstoff, jeweils gegebenenfalls durch Cyano, Hydroxy, Fluor, Chlor, Methoxy, Ethoxy, n- oder i-Propoxy substituiertes Methyl, Ethyl, n- oder i-Propyl, n-, i- oder s-Butyl, jeweils gegebenenfalls durch Cyano, Fluor, Chlor oder Brom substituiertes Propenyl, Butenyl, Propinyl oder Butinyl, jeweils gegebenenfalls durch Cyano, Fluor, Chlor, Brom, Methyl, Ethyl, n- oder i-Propyl substituiertes Cyclopropyl, Cyclobutyl, Cyclopentyl oder Cyclohexyl, oder gegebenenfalls durch Nitro, Cyano, Fluor, Chlor, Brom, Methyl, Ethyl, n-oder i-Propyl, n-, i-, s- oder t-Butyl, Trifluormethyl, Methoxy, Ethoxy, n- oder i-Propoxy, Difluormethoxy oder Trifluormethoxy substituiertes Phenyl, oder zusammen mit R²⁵ für jeweils gegebenenfalls durch Methyl oder Ethyl substituiertes Butan-1,4-diyl (Trimethylen), Pentan-1,5-diyl, 1-Oxa-butan-1,4-diyl oder 3-Oxa-pentan-1,5-diyl.
- X⁴: steht bevorzugt für Nitro, Cyano, Carboxy, Carbamoyl, Formyl, Sulfamoyl, Hydroxy, Amino, Fluor, Chlor, Brom, Methyl, Ethyl, n- oder i-Propyl, n-, i-, s- oder t-Butyl, Trifluormethyl, Methoxy, Ethoxy, n- oder i-Propoxy, Difluormethoxy oder Trifluormethoxy.
- X⁵: steht bevorzugt für Nitro, Cyano, Carboxy, Carbamoyl, Formyl, Sulfamoyl, Hydroxy, Amino, Fluor, Chlor, Brom, Methyl, Ethyl, n- oder i-Propyl, n-, i-, s- oder t-Butyl, Trifluormethyl, Methoxy, Ethoxy, n- oder i-Propoxy, Difluormethoxy oder Trifluormethoxy.

Beispiele für die als erfindungsgemäße Herbizid-Safener ganz besonders bevorzugten Verbindungen der Formel (IIa) sind in der nachstehenden Tabelle 19 aufgeführt.

**Tabelle 19: Beispiele für die Verbindungen der Formel (IIa)**

| | | | |
|---|---|---|---|
| 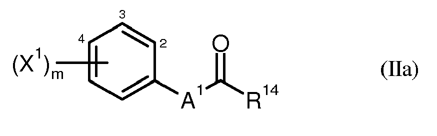 | | | |

| **Beispiel-Nr.** | **(Positionen)** **(X¹)ₘ** | **A¹** | **R¹⁴** |
|---|---|---|---|
| IIa-1 | (2) Cl, (4) Cl | 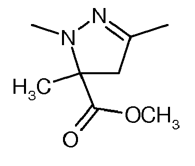 | OCH₃ |
| IIa-2 | (2) Cl, (4) Cl | 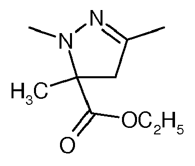 | OCH₃ |
| IIa-3 | (2) Cl, (4) Cl | 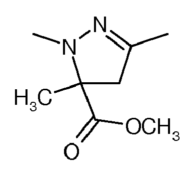 | OC₂H₅ |
| IIa-4 | (2) Cl, (4) Cl | 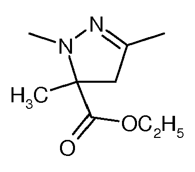 | OC₂H₅ |
| IIa-5 | (2) Cl | 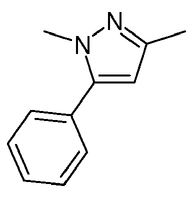 | OCH₃ |
| IIa-6 | (2) Cl, (4) Cl | 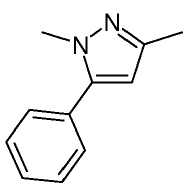 | OCH₃ |
| IIa-7 | (2) F | 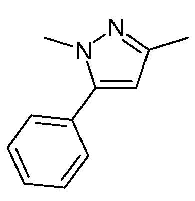 | OCH₃ |
| IIa-8 | (2) F | 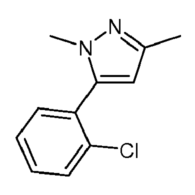 | OCH₃ |
| IIa-9 | (2) Cl, (4) Cl | 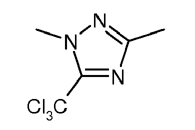 | OC₂H₅ |
| IIa-10 | (2) Cl, (4) CF₃ | 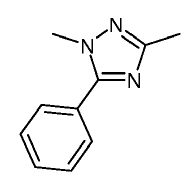 | OCH₃ |
| IIa-11 | (2) Cl | 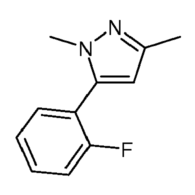 | OCH₃ |
| IIa-12 | - | 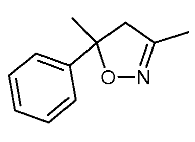 | OC₂H₅ |
| IIa-13 | (2) Cl, (4) Cl | 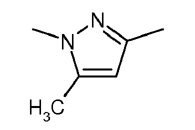 | OC₂H₅ |
| IIa-14 | (2) Cl, (4) Cl | 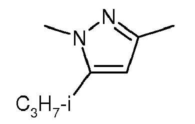 | OC₂H₅ |
| IIa-15 | (2) Cl, (4) Cl | 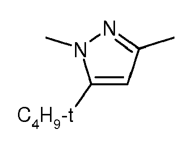 | OC₂H₅ |
| IIa-16 | (2) Cl, (4) Cl | 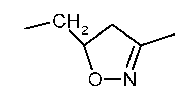 | OC₂H₅ |
| IIa-17 | (2) Cl, (4) Cl | 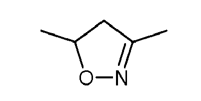 | OC₂H₅ |
| IIa-18 | - | 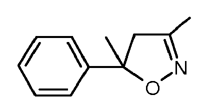 | OH |

Beispiele für die als erfindungsgemäße Herbizid-Safener ganz besonders bevorzugten Verbindungen der Formel (IIb) sind in der nachstehenden Tabelle 20 aufgeführt.

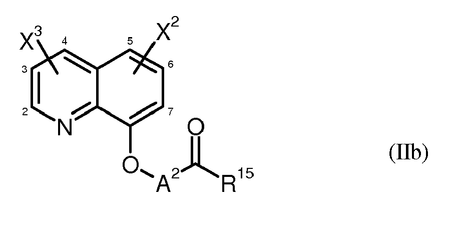

**Tabelle 20: Beispiele für die Verbindungen der Formel (IIb)**

| **Beispiel-Nr.** | **(Position)** **X²** | **(Position)** **X³** | **A²** | **R¹⁵** |
|---|---|---|---|---|
| IIb-1 | (5) | - | CH₂ | OH |
| | Cl | | | |
| IIb-2 | (5) | - | CH₂ | OCH₃ |
| | Cl | | | |
| IIb-3 | (5) | - | CH₂ | OC₂H₅ |
| | Cl | | | |
| IIb-4 | (5) Cl | - | CH₂ | OC₃H₇-n |
| IIb-5 | (5) Cl | - | CH₂ | OC₃H₇-i |
| IIb-6 | (5) Cl | - | CH₂ | OC₄H₉-n |
| IIb-7 | (5) Cl | - | CH₂ | OCH(CH₃)C₅H₁₁-n |
| IIb-8 | (5) Cl | (2) F | CH₂ | OH |
| IIb-9 | (5) Cl | (2) Cl | CH₂ | OH |
| IIb-10 | (5) Cl | - | CH₂ | OCH₂CH=CH₂ |
| IIb-11 | (5) Cl | - | CH₂ | OC₄H₉-i |
| IIb-12 | (5) Cl | - | CH₂ | 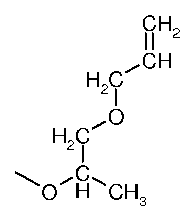 |
| IIb-13 | (5) Cl | - | 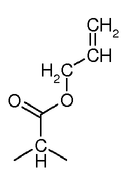 | OCH₂CH=CH₂ |
| IIb-14 | (5) Cl | - | 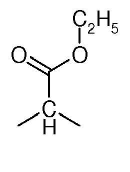 | OC₂H₅ |
| IIb-15 | (5) Cl | - | 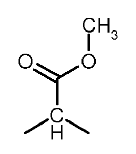 | OCH₃ |

Beispiele für die als erfindungsgemäße Herbizid-Safener ganz besonders bevorzugten Verbindungen der Formel (IIc) sind in der nachstehenden Tabelle 21 aufgeführt.

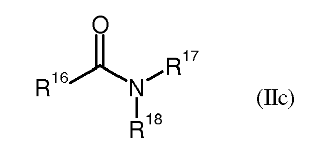

**Tabelle 21: Beispiele für die Verbindungen der Formel (IIc)**

| **Beispiel-Nr.** | **R¹⁶** | **N(R¹⁷,R¹⁸)** |
|---|---|---|
| IIc-1 | CHCl₂ | N(CH₂CH=CH₂)₂ |
| IIc-2 | CHCl₂ | 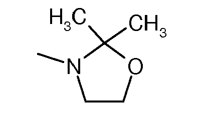 |
| IIc-3 | CHCl₂ | 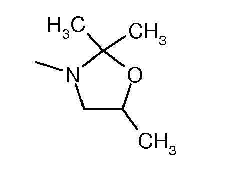 |
| IIc-4 | CHCl₂ | 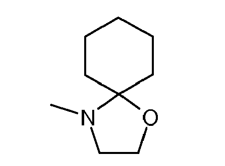 |
| IIc-5 | CHCl₂ | 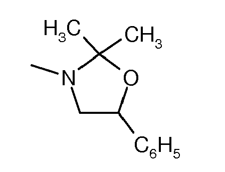 |
| IIc-6 | CHCl₂ | 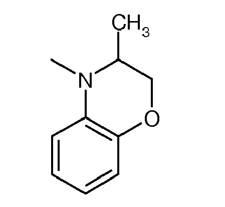 |
| IIc-7 | CHCl₂ | 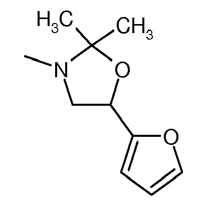 |

Beispiele für die als erfindungsgemäße Herbizid-Safener ganz besonders bevorzugten Verbindungen der Formel (IId) sind in der nachstehenden Tabelle 22 aufgeführt.

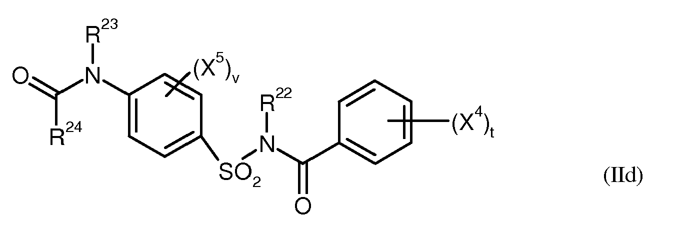

**Tabelle 22: Beispiele für die Verbindungen der Formel (IId)**

| **Beispiel-Nr.** | **R²²** | **R²³** | **R²⁴** | **(Positionen)** **(X⁴)ₜ** | **(Positionen)** **(X⁵)ᵥ** |
|---|---|---|---|---|---|
| IId-1 | H | H | CH₃ | (2) OCH₃ | - |
| IId-2 | H | H | C₂H₅ | (2) OCH₃ | - |
| IId-3 | H | H | C₃H₇-n | (2) OCH₃ | - |
| IId-4 | H | H | C₃H₇-i | (2) OCH₃ | - |
| IId-5 | H | H | 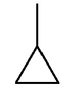 | (2) OCH₃ | - |
| IId-6 | H | H | CH₃ | (2) OCH₃ (5) CH₃ | - |
| IId-7 | H | H | C₂H₅ | (2) OCH₃ (5) CH₃ | - |
| IId-8 | H | H | C₃H₇-n | (2) OCH₃ (5) CH₃ | - |
| IId-9 | H | H | C₃H₇-i | (2) OCH₃ (5) CH₃ | - |
| IId-10 | H | H | 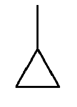 | (2) OCH₃ (5) CH₃ | (X⁵)ᵥ |
| IId-11 | H | H | OCH₃ | (2) OCH₃ (5) CH₃ | - |
| IId-12 | H | H | OC₂H₅ | (2) OCH₃ (5) CH₃ | - |
| IId-13 | H | H | OC₃H₇-i | (2) OCH₃ (5) CH₃ | - |
| IId-14 | H | H | SCH₃ | (2) OCH₃ (5) CH₃ | - |
| IId-15 | H | H | SC₂H₅ | (2) OCH₃ (5) CH₃ | - |
| IId-16 | H | H | SC₃H₇-i | (2) OCH₃ (5) CH₃ | - |
| IId-17 | H | H | NHCH₃ | (2) OCH₃ (5) CH₃ | - |
| IId-18 | H | H | NHC₂H₅ | (2) OCH₃ (5) CH₃ | - |
| IId-19 | H | H | NHC₃H₇-i | (2) OCH₃ (5) CH₃ | - |
| IId-20 | H | H | 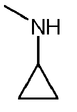 | (2) OCH₃ (5) CH₃ | - |
| IId-21 | H | H | NHCH₃ | (2) OCH₃ | - |
| IId-22 | H | H | NHC₃H₇-i | (2) OCH₃ | - |
| IId-23 | H | H | N(CH₃)₂ | (2) OCH₃ | - |
| IId-24 | H | H | N(CH₃)₂ | (3) CH₃ (4) CH₃ | - |
| IId-25 | H | H | CH₂-O-CH₃ | (2) OCH₃ | - |

Beispiele für die als erfindungsgemäße Herbizid-Safener ganz besonders bevorzugten Verbindungen der Formel (IIe) sind in der nachstehenden Tabelle 23 aufgeführt.

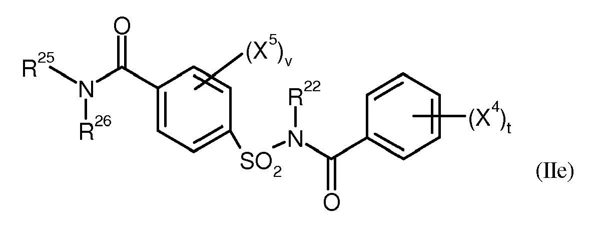

**Tabelle 23: Beispiele für die Verbindungen der Formel (IIe)**

| **Beispiel-Nr.** | **R²²** | **R²⁵** | **R²⁶** | **(Positionen)** **(X⁴)ₜ** | **(Positionen)** **(X⁵)ᵥ** |
|---|---|---|---|---|---|
| IIe-1 | H | H | CH₃ | (2) OCH₃ | - |
| IIe-2 | H | H | C₂H₅ | (2) OCH₃ | - |
| IIe-3 | H | H | C₃H₇-n | (2) OCH₃ | - |
| IIe-4 | H | H | C₃H₇-i | (2) OCH₃ | - |
| IIe-5 | H | H | 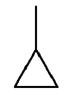 | (2) OCH₃ | - |
| IIe-6 | H | CH₃ | CH₃ | (2) OCH₃ | - |
| IIe-7 | H | H | CH₃ | (2) OCH₃ (5) CH₃ | - |
| IIe-8 | H | H | C₂H₅ | (2) OCH₃ (5) CH₃ | - |
| IIe-9 | H | H | C₃H₇-n | (2) OCH₃ (5) CH₃ | - |
| IIe-10 | H | H | C₃H₇-i | (2) OCH₃ (5) CH₃ | - |
| IIe-11 | H | H | 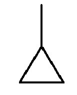 | (2) OCH₃ (5) CH₃ | - |
| IIe-12 | H | CH₃ | CH₃ | (2) OCH₃ (5) CH₃ | - |

Als die die Kulturpflanzen-Verträglichkeit verbessernde Verbindung [Komponente (b')] sind Cloquintocet-mexyl, Fenchlorazol-ethyl, Isoxadifen-ethyl, Mefenpyr-diethyl, Furilazole, Fenclorim, Cumyluron, Dymron, Dimepiperate und die Verbindungen IIe-5 und IIe-11 am meisten bevorzugt, wobei Cloquintocet-mexyl und Mefenpyr-diethyl, aber auch Isoxadifen-ethyl besonders hervorgehoben seien.

Die als Safener erfindungsgemäß zu verwendenden Verbindungen der allgemeinen Formel (IIa) sind bekannt und/oder können nach an sich bekannten Verfahren hergestellt werden (vgl. WO-A-91/07874, WO-A-95/07897).

Die als Safener erfindungsgemäß zu verwendenden Verbindungen der allgemeinen Formel (IIb) sind bekannt und/oder können nach an sich bekannten Verfahren hergestellt werden (vgl. EP-A-191736).

Die als Safener erfindungsgemäß zu verwendenden Verbindungen der allgemeinen Formel (IIc) sind bekannt und/oder können nach an sich bekannten Verfahren hergestellt werden (vgl. DE-A-2218097, DE-A-2350547).

Die als Safener erfindungsgemäß zu verwendenden Verbindungen der allgemeinen Formel (IId) sind bekannt und/oder können nach an sich bekannten Verfahren hergestellt werden (vgl. DE-A-19621522/US-A-6235680).

Die als Safener erfindungsgemäß zu verwendenden Verbindungen der allgemeinen Formel (IIe) sind bekannt und können nach an sich bekannten Verfahren hergestellt werden (vgl. WO-A-99/66795/US-A-6251827).

Beispiele für die erfindungsgemäßen selektiv herbiziden Kombinationen aus jeweils einem Wirkstoff der Formel (I) und jeweils einem der oben definierten Safener sind in der nachstehenden Tabelle 24 aufgeführt.

**Tabelle 24: Beispiele für die erfindungsgemäßen Kombinationen**

| **Wirkstoffe der Formel (I)** | **Safener** |
|---|---|
| I-1-a | Cloquintocet-mexyl |
| I-1-a | Fenchlorazole-ethyl |
| I-1-a | Isoxadifen-ethyl |
| I-1-a | Mefenpyr-diethyl |
| I-1-a | Furilazole |
| I-1-a | Fenclorim |
| I-1-a | Cumyluron |
| I-1-a | Daimuron /Dymron |
| I-1-a | Dimepiperate |
| I-1-a | IIe-11 |
| I-1-a | IIe-5 |
| I-1-b | Cloquintocet-mexyl |
| I-1-b | Fenchlorazole-ethyl |
| I-1-b | Isoxadifen-ethyl |
| I-1-b | Mefenpyr-diethyl |
| I-1-b | Furilazole |
| I-1-b | Fenclorim |
| I-1-b | Cumyluron |
| I-1-b | Daimuron /Dymron |
| I-1-b | Dimepiperate |
| I-1-b | IIe-11 |
| I-1-b | IIe-5 |
| I-1-c | Cloquintocet-mexyl |
| I-1-c | Fenchlorazole-ethyl |
| I-1-c | Isoxadifen-ethyl |
| I-1-c | Mefenpyr-diethyl |
| I-1-c | Furilazole |
| I-1-c | Fenclorim |
| I-1-c | Cumyluron |
| I-1-c | Daimuron /Dymron |
| I-1-c | Dimepiperate |
| I-1-c | IIe-5 |
| I-1-c | IIe-11 |
| I-1-d | Cloquintocet-mexyl |
| I-1-d | Fenchlorazole-ethyl |
| I-1-d | Isoxadifen-ethyl |
| I-1-d | Mefenpyr-diethyl |
| I-1-d | Furilazole |
| I-1-d | Fenclorim |
| I-1-d | Cumyluron |
| I-1-d | Daimuron /Dymron |
| I-1-d | Dimepiperate |
| I-1-d | IIe-11 |
| I-1-d | IIe-5 |
| I-1-e | Cloquintocet-mexyl |
| I-1-e | Fenchlorazole-ethyl |
| I-1-e | Isoxadifen-ethyl |
| I-1-e | Mefenpyr-diethyl |
| I-1-e | Furilazole |
| I-1-e | Fenclorim |
| I-1-e | Cumyluron |
| I-1-e | Daimuron /Dymron |
| I-1-e | Dimepiperate |
| I-1-e | IIe-5 |
| I-1-e | IIe-11 |
| I-1-f | Cloquintocet-mexyl |
| I-1-f | Fenchlorazole-ethyl |
| I-1-f | Isoxadifen-ethyl |
| I-1-f | Mefenpyr-diethyl |
| I-1-f | Furilazole |
| I-1-f | Fenclorim |
| I-1-f | Cumyluron |
| I-1-f | Daimuron /Dymron |
| I-1-f | Dimepiperate |
| I-1-f | IIe-5 |
| I-1-f | IIe-11 |
| I-1-g | Cloquintocet-mexyl |
| I-1-g | Fenchlorazole-ethyl |
| I-1-g | Isoxadifen-ethyl |
| I-1-g | Mefenpyr-diethyl |
| I-1-g | Furilazole |
| I-1-g | Fenclorim |
| I-1-g | Cumyluron |
| I-1-g | Daimuron /Dymron |
| I-1-g | Dimepiperate |
| I-1-g | IIe-5 |
| I-1-g | IIe-11 |

**Tabelle 25: Beispiele für die erfindungsgemäßen Kombinationen**

| **Wirkstoffe der Formel (I)** | **Safener** |
|---|---|
| I-2-a | Cloquintocet-mexyl |
| I-2-a | Fenchlorazole-ethyl |
| I-2-a | Isoxadifen-ethyl |
| I-2-a | Mefenpyr-diethyl |
| I-2-a | Furilazole |
| I-2-a | Fenclorim |
| I-2-a | Cumyluron |
| I-2-a | Daimuron /Dymron |
| I-2-a | Dimepiperate |
| I-2-a | IIe-11 |
| I-2-a | IIe-5 |
| I-2-b | Cloquintocet-mexyl |
| I-2-b | Fenchlorazole-ethyl |
| I-2-b | Isoxadifen-ethyl |
| I-2-b | Mefenpyr-diethyl |
| I-2-b | Furilazole |
| I-2-b | Fenclorim |
| I-2-b | Cumyluron |
| I-2-b | Daimuron /Dymron |
| I-2-b | Dimepiperate |
| I-2-b | IIe-11 |
| I-2-b | IIe-5 |
| I-2-c | Cloquintocet-mexyl |
| I-2-c | Fenchlorazole-ethyl |
| I-2-c | Isoxadifen-ethyl |
| I-2-c | Mefenpyr-diethyl |
| I-2-c | Furilazole |
| I-2-c | Fenclorim |
| I-2-c | Cumyluron |
| I-2-c | Daimuron /Dymron |
| I-2-c | Dimepiperate |
| I-2-c | IIe-5 |
| I-2-c | IIe-11 |
| I-2-d | Cloquintocet-mexyl |
| I-2-d | Fenchlorazole-ethyl |
| I-2-d | Isoxadifen-ethyl |
| I-2-d | Mefenpyr-diethyl |
| I-2-d | Furilazole |
| I-2-d | Fenclorim |
| I-2-d | Cumyluron |
| I-2-d | Daimuron /Dymron |
| I-2-d | Dimepiperate |
| I-2-d | IIe-11 |
| I-2-d | IIe-5 |
| I-2-e | Cloquintocet-mexyl |
| I-2-e | Fenchlorazole-ethyl |
| I-2-e | Isoxadifen-ethyl |
| I-2-e | Mefenpyr-diethyl |
| I-2-e | Furilazole |
| I-2-e | Fenclorim |
| I-2-e | Cumyluron |
| I-2-e | Daimuron /Dymron |
| I-2-e | Dimepiperate |
| I-2-e | IIe-5 |
| I-2-e | IIe-11 |
| I-2-f | Cloquintocet-mexyl |
| I-2-f | Fenchlorazole-ethyl |
| I-2-f | Isoxadifen-ethyl |
| I-2-f | Mefenpyr-diethyl |
| I-2-f | Furilazole |
| I-2-f | Fenclorim |
| I-2-f | Cumyluron |
| I-2-f | Daimuron /Dymron |
| I-2-f | Dimepiperate |
| I-2-f | IIe-5 |
| I-2-f | IIe-11 |
| I-2-g | Cloquintocet-mexyl |
| I-2-g | Fenchlorazole-ethyl |
| I-2-g | Isoxadifen-ethyl |
| I-2-g | Mefenpyr-diethyl |
| I-2-g | Furilazole |
| I-2-g | Fenclorim |
| I-2-g | Cumyluron |
| I-2-g | Daimuron /Dymron |
| I-2-g | Dimepiperate |
| I-2-g | IIe-5 |
| I-2-g | IIe-11 |

Es wurde nun überraschend gefunden, dass die oben definierten Wirkstoffkombinationen aus substituierten cyclischen Ketoenole der allgemeinen Formel (I) und Safenern (Antidots) aus der oben aufgeführten Gruppe (b') bei sehr guter Nutzpflanzen-Verträglichkeit eine besonders hohe herbizide Wirksamkeit aufweisen und in verschiedenen Kulturen, insbesondere in Getreide (vor allem Weizen), aber auch in Soja, Kartoffeln, Mais und Reis zur selektiven Unkrautbekämpfung verwendet werden können.

Dabei ist es als überraschend anzusehen, dass aus einer Vielzahl von bekannten Safenern oder Antidots, die befähigt sind, die schädigende Wirkung eines Herbizids auf die Kulturpflanzen zu antagonisieren, gerade die oben aufgeführten Verbindungen der Gruppe (b') geeignet sind, die schädigende Wirkung von substituierten cyclischen Ketoenolen auf die Kulturpflanzen annähernd vollständig aufzuheben, ohne dabei die herbizide Wirksamkeit gegenüber den Unkräutern maßgeblich zu beeinträchtigen.

Hervorgehoben sei hierbei die besonders vorteilhafte Wirkung der besonders und am meisten bevorzugten Kombinationspartner aus der Gruppe (b'), insbesondere hinsichtlich der Schonung von Getreidepflanzen, wie z.B. Weizen, Gerste und Roggen, aber auch Mais und Reis, als Kulturpflanzen.

Verwendet man beispielsweise gemäß Verfahren (A) N-[(4-Chlor-2,6-dimethyl)-phenylacetyl]-1-amino-3-methoxy-cyclohexancarbonsäureethylester als Ausgangsstoff, so kann der Verlauf des erfindungsgemäßen Verfahrens durch folgendes Reaktionsschema wiedergegeben werden:

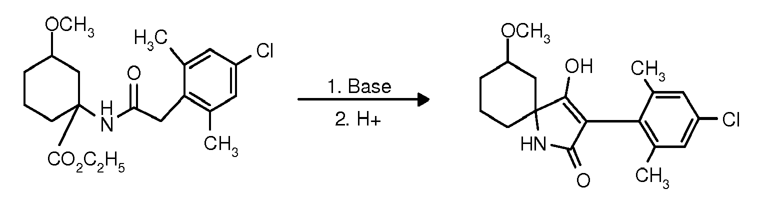

Verwendet man beispielsweise gemäß Verfahren (B) O-[(2-Chlor-6-methyl)-phenylacetyl]-1-hydroxy-3-ethoxy-cyclohexancarbonsäureethylester, so kann der Verlauf des erfindungsgemäßen Verfahrens durch folgendes Reaktionsschema wiedergegeben werden:

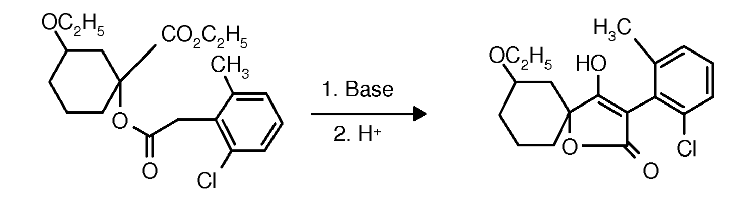

Verwendet man beispielsweise gemäß Verfahren (Cα) 7-Butoxy-3-[(4-chlor-2,6-dimethyl)-phenyl]-1-azaspiro[4,5]decan-2,4-dion und Pivaloylchlorid als Ausgangsstoffe, so kann der Verlauf des erfindungsgemäßen Verfahrens durch folgendes Reaktionsschema wiedergegeben werden:

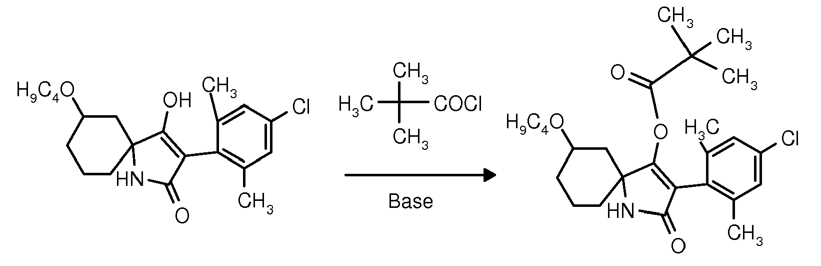

Verwendet man beispielsweise gemäß Verfahren (C) (Variante β) 7-Ethoxy-3-[(2,4-dichlor)-phenyl]-l-oxaspiro-[4,5]-decan-2,4-dion und Acetanhydrid als Ausgangsverbindungen, so kann der Verlauf des erfindungsgemäßen Verfahrens durch folgendes Reaktionsschema wiedergegeben werden:

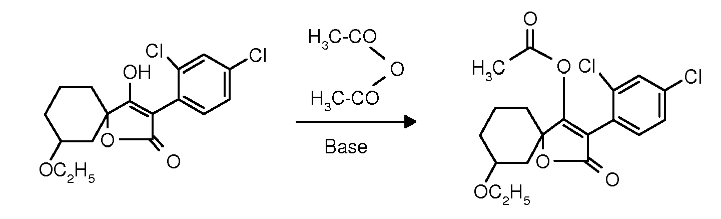

Verwendet man beispielsweise gemäß Verfahren (D) 7-Methoxy-3-[(2,4-dichlor-6-methyl)-phenyl]-1-azaspiro[4,5]decan-2,4-dion und Chlorameisensäureethylester als Ausgangsverbindungen, so kann der Verlauf des erfindungsgemäßen Verfahrens durch folgendes Reaktionsschema wiedergegeben werden:

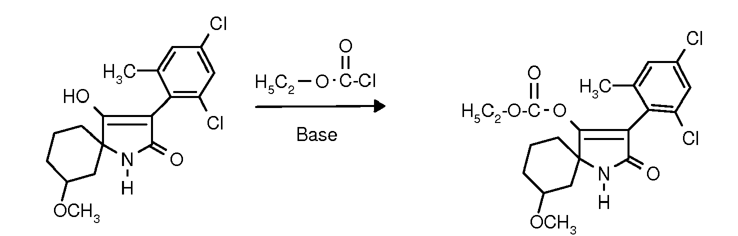

Verwendet man beispielsweise gemäß Verfahren (E) 7-Ethoxy-3-[(2,4,6-trimethyl)-phenyl]-1-oxaspiro[4,5]decan-2,4-dion und Chlormonothioameisensäuremethylester als Ausgangsprodukte, so kann der Reaktionsverlauf folgendermaßen wiedergegeben werden:

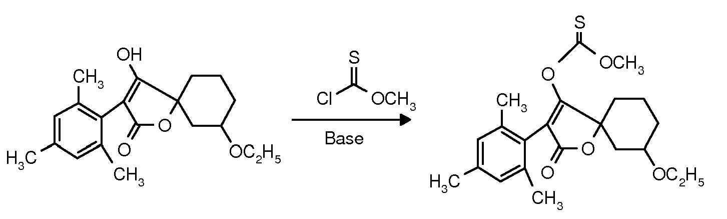

Verwendet man beispielsweise gemäß Verfahren (F) 7-Butoxy-3-[(2,4,6-trimethyl)-phenyl]-1-azaspiro[4,5]decan-2,4-dion und Methansulfonsäurechlorid als Ausgangsprodukte, so kann der Reaktionsverlauf durch folgendes Reaktionsschema wiedergegeben werden:

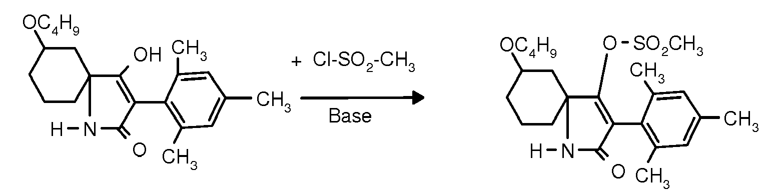

Verwendet man beispielsweise gemäß Verfahren (G) 7-Methoxy-3-[(2,4-dichlor-6-methyl)-phenyl]-1-oxaspiro[4,5]decan-2,4-dion und Methanthio-phosphonsäurechlorid-(2,2,2-trifluorethylester) als Ausgangsprodukte, so kann der Reaktionsverlauf durch folgendes Reaktionsschema wiedergegeben werden:

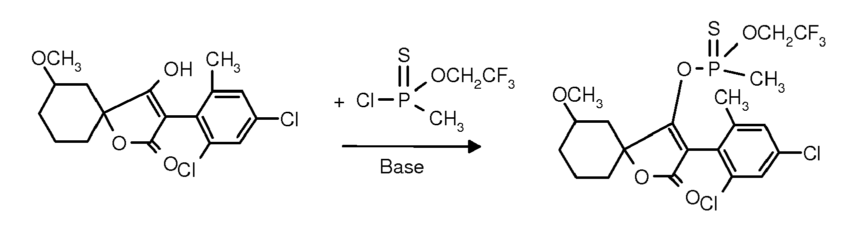

Verwendet man beispielsweise gemäß Verfahren (H) 7-Methoxy-3-[(2,3,4,6-tetramethylphenyl]-1-azaspiro[4,5]decan-2,4-dion und NaOH als Komponenten, so kann der Verlauf des erfindungsgemäßen Verfahrens durch folgendes Reaktionsschema wiedergegeben werden:

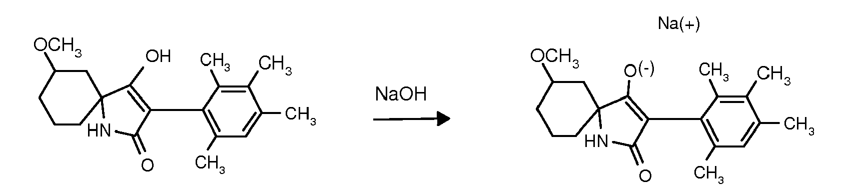

Verwendet man beispielsweise gemäß Verfahren (I) (Variante α) 7-Ethoxy-3-[(2,4,5-trimethyl)-phenyll-l-oxaspiro[4,5]decan-2,4-dion und Ethylisocyanat als Ausgangsprodukte, so kann der Reaktionsverlauf durch folgendes Reaktionsschema wiedergegeben werden:

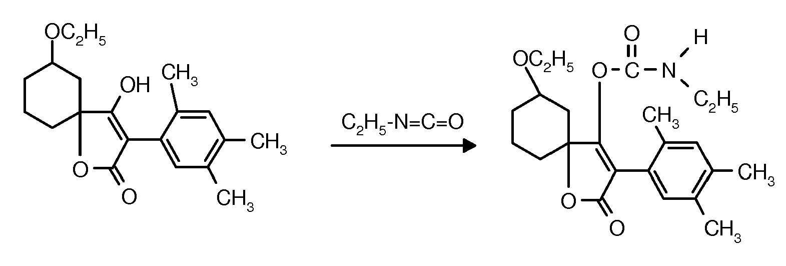

Verwendet man beispielsweise gemäß Verfahren (I) (Variante β) 7-Butoxy-3-[(2,4,6-trimethyl)-phenyl]-1-azaspiro[4,5]decan-2,4-dion und Dimethylcarbamidsäurechlorid als Ausgangsprodukte, so kann der Reaktionsverlauf durch folgendes Schema wiedergegeben werden:

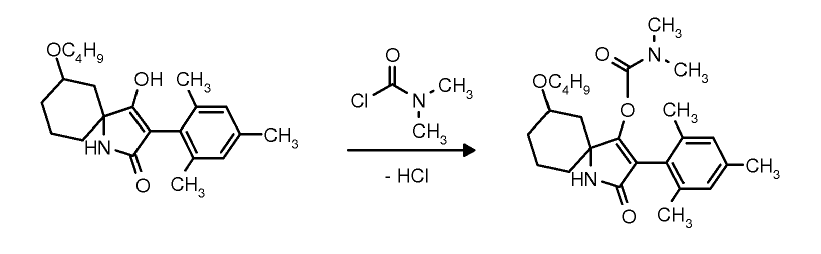

Die beim erfindungsgemäßen Verfahren (A) als Ausgangsstoffe benötigten Verbindungen der Formel (II) 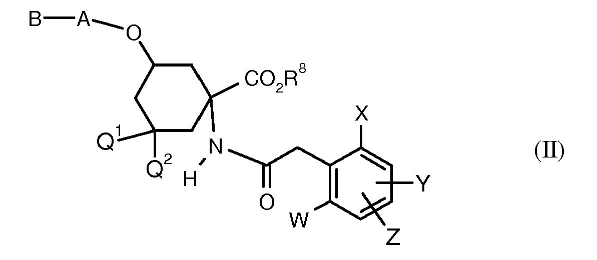 in welcher
- A, B, Q¹, Q², W, X, Y, Z und R⁸: die oben angegebenen Bedeutungen haben,
sind neu.

Man erhält die Acylaminosäureester der Formel (II) beispielsweise, wenn man Aminosäurederivate der Formel (XIV) 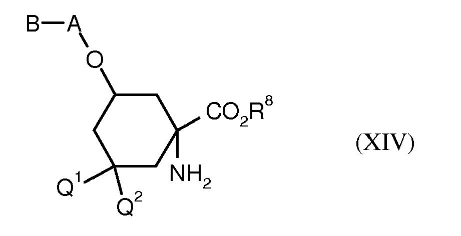 in welcher
A, B, Q¹ und Q² und R⁸ die oben angegebene Bedeutung haben,
mit substituierten Phenylessigsäurederivaten der Formel (XV) 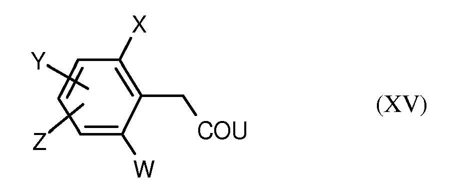 in welcher
- W, X, Y und Z: die oben angegebenen Bedeutungen haben und
- U: für eine durch Carbonsäureaktivierungsreagenzien wie Carbonyldiimidazol, Carbonyldiimide (wie z.B. Dicyclohexylcarbondiimid), Phosphorylierungsreagenzien (wie z.B. POCl₃, BOP-Cl), Halogenierungnsmittel, wie z.B. Thionylchlorid, Oxalylchlorid, Phosgen oder Chlorameisensäureester eingeführte Abgangsgruppe steht,
acyliert (Chem. Reviews 52, 237-416 (1953); Bhattacharya, Indian J. Chem. 6, 341-5, 1968)
oder wenn man Acylaminosäuren der Formel (XVI) 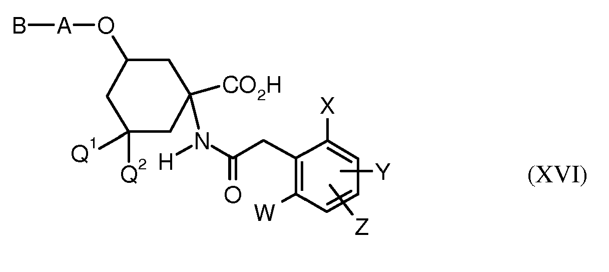 in welcher
- A, B, Q¹, Q², W, X, Y und Z: die oben angegebenen Bedeutungen haben,
verestert (Chem. Ind. (London) 1568 (1968)).

Die Verbindungen der Formel (XVI) 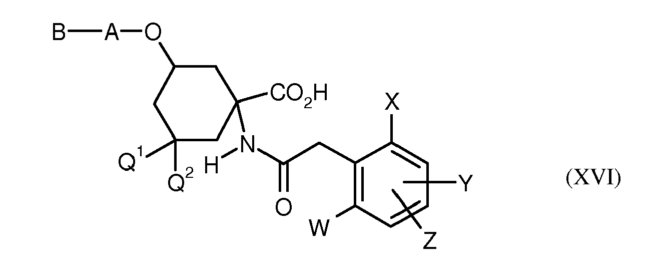 in welcher
- A, B, Q¹, Q², W, X, Y und Z: die oben angegebenen Bedeutungen haben,
sind neu.

Man erhält die Verbindungen der Formel (XVI) beispielsweise, wenn man 1-Amino-cyclohexan-carbonsäuren der Formel (XVII) 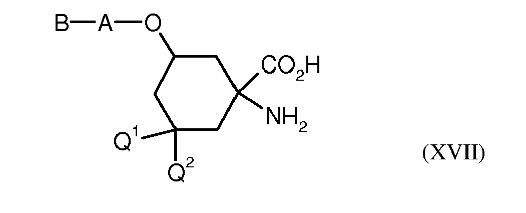 in welcher
- A, B, Q¹ und Q²: die oben angegebenen Bedeutungen haben
mit substituierten Phenylessigsäurederivaten der Formel (XV) 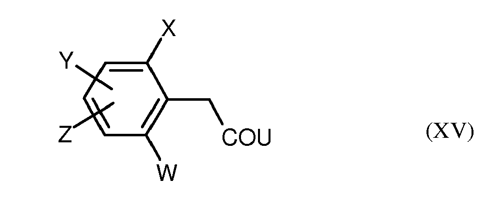 in welcher
- U, W, X, Y und Z: die oben angegebenen Bedeutungen haben
nach Schotten-Baumann acyliert (Organikum, VEB Deutscher Verlag der Wissenschaften, Berlin 1977, S. 505).

Die Verbindungen der Formel (XV) sind teilweise bekannt und/oder lassen sich nach den bekannten Verfahren in den eingangs zitierten Offenlegungsschriften herstellen.

Die Verbindungen der Formel (XIV) und (XVII) sind neu und lassen sich nach bekannten Verfahren darstellen (siehe z.B. Compagnon, Ann. Chim. (Paris) [14] 5, S. 11-22, 23-27 (1970), L. Munday, J. Chem. Soc. 4372 (1961); J.T. Eward, C. Jitrangeri, Can. J. Chem. 53, 3339 (1975)).

Die neuen 1-Amino-cyclohexan-carbonsäuren (XVII) sind im Allgemeinen nach der Bucherer-Bergs-Synthese oder nach der Strecker-Synthese erhältlich und fallen dabei jeweils in unterschiedlichen Isomerenformen an. Im Folgenden werden der Einfachheit halber die Isomeren als β bezeichnet, in welchem der 3-Substituent (O-A-B) und die Aminogruppe äquatorial/axial oder axial/äquatorial stehen. Im Folgenden wurden der Einfachheit halber die Isomeren als α bezeichnet, in welchen die Aminogruppe und der 3-Substituent (O-A-B) äquatorial/äquatorial oder axial/axial stehen.

| | |
|---|---|
| 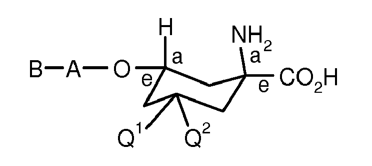 | 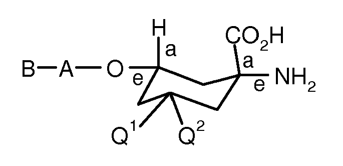 |
| Beispiel: β-Isomeres | Beispiel: α-Isomeres |

(L. Munday, J. Chem. Soc. 4372 (1961).

Weiterhin lassen sich die bei dem obigen Verfahren (A) verwendeten Ausgangsstoffe der Formel (II) 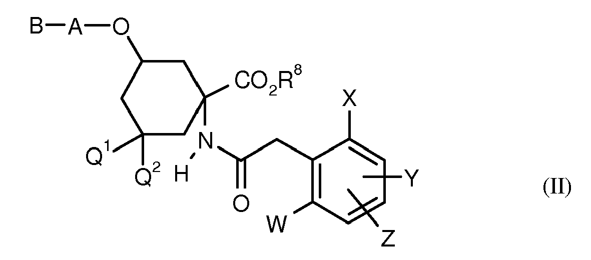 in welcher
- A, B, Q¹, Q², W, X, Y, Z und R⁸: die oben angegebenen Bedeutungen haben,
herstellen, wenn man 1-Amino-cyclohexan-carbonsäurenitrile der Formel (XVIII) 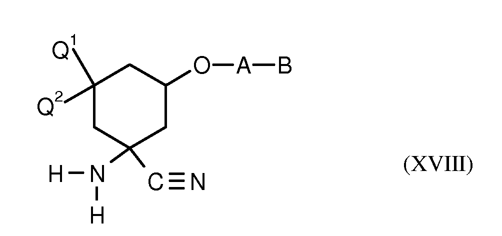 in welcher
A, B, Q¹ und Q² die oben angegebenen Bedeutungen haben,
mit substituierten Phenylessigsäurederivaten der Formel (XV) 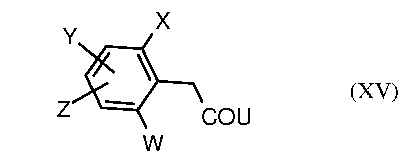 in welcher
U, W, X, Y und Z die oben angegebenen Bedeutungen haben,
zu Verbindungen der Formel (XIX) 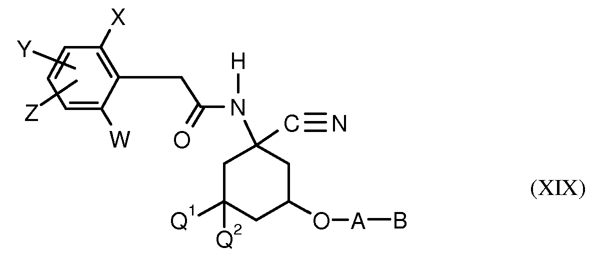 in welcher
A, B, Q¹, Q², W, X, Y und Z die oben angegebenen Bedeutungen haben,
umsetzt,
und diese anschließend einer sauren Alkoholyse unterwirft.

Die Verbindungen der Formel (XIX) sind ebenfalls neu. Die Verbindungen der Formel (XVIII) sind ebenfalls neu und lassen sich z.B. wie in EP 595 130 beschrieben herstellen.

Die bei dem erfindungsgemäßen Verfahren (B) als Ausgangstoffe benötigten Verbindungen der Formel (III) 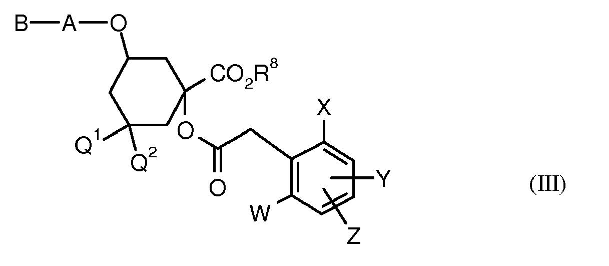 in welcher
A, B, Q¹, Q², W, X, Y, Z und R⁸ die oben angegebenen Bedeutungen haben, sind neu.

Sie lassen sich nach im Prinzip bekannten Methoden in einfacher Weise herstellen.

Man erhält die Verbindungen der Formel (III) beispielsweise, wenn man
1-Hydroxy-cyclohexan-carbonsäureester der Formel (XX) 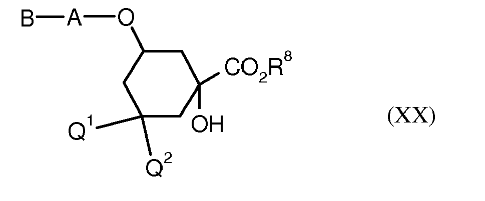 in welcher
- A, B, Q¹, Q² und R⁸: die oben angegebenen Bedeutungen haben,
mit substituierten Phenylessigsäurederivaten der Formel (XV) 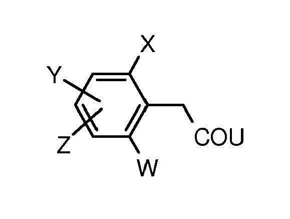 in welcher
U, W, X, Y und Z die oben angegebenen Bedeutungen haben, acyliert (Chem. Reviews 52, 237-416 (1953)).

Die 1-Hydroxy-3-alkoxy-cyclohexyl-carbonsäureester der Formel (XX) sind neu. Man erhält sie beispielsweise, indem man substituierte 1-Hydroxy-3-alkoxy-cyclohexan-carbonsäurenitrile in Gegenwart von Säuren, z.B. nach Pinner mit Alkoholen umsetzt. Das Cyanhydrin erhält man beispielsweise durch Umsetzung von substituierten 3-Alkoxy-cyclohexan-1-onen mit Blausäure.

Die zur Durchführung der erfindungsgemäßen Verfahren (C), (D), (E), (F), (G), (H) und (I) außerdem als Ausgangsstoffe benötigten Säurehalogenide der Formel (IV), Carbonsäureanhydride der Formel (V), Chlorameisensäureester oder Chlorameisensäurethioester der Formel (VI), Chlormonothioameisensäureester oder Chlordithioameisensäureester der Formel (VII), Sulfonsäurechloride der Formel (VIII), Phosphorverbindungen der Formel (IX) und Metallhydroxide, Metallalkoxide oder Amine der Formel (X) und (XI) und Isocyanate der Formel (XII) und Carbamidsäurechloride der Formel (XIII) sind allgemein bekannte Verbindungen der organischen bzw. anorganischen Chemie.

Die Verbindungen der Formel (XV) sind darüber hinaus aus den eingangs zitierten Patentanmeldungen bekannt und/oder lassen sich nach den dort angegebenen Methoden herstellen.

Das Verfahren (A) ist dadurch gekennzeichnet, dass man Verbindungen der Formel (II), in welcher A, B, Q¹, Q², W, X, Y, Z und R⁸ die oben angegebenen Bedeutungen haben, in Gegenwart eines Verdünnungsmittels und in Gegenwart einer Base einer intramolekularen Kondensation unterwirft.

Als Verdünnungsmittel können bei dem erfindungsgemäßen Verfahren (A) alle gegenüber den Reaktionsteilnehmern inerten organischen Solventien eingesetzt werden. Vorzugsweise verwendbar sind Kohlenwasserstoffe, wie Toluol und Xylol, ferner Ether, wie Dibutylether, Tetrahydrofuran, Dioxan, Glykoldimethylether und Diglykoldimethylether, außerdem polare Lösungsmittel, wie Dimethylsulfoxid, Sulfolan, Dimethylformamid und N-Methyl-pyrrolidon, sowie Alkohole wie Methanol, Ethanol, Propanol, Iso-Propanol, Butanol, Iso-Butanol und tert.-Butanol.

Als Base (Deprotonierungsmittel) können bei der Durchführung des erfindungsgemäßen Verfahrens (A) alle üblichen Protonenakzeptoren eingesetzt werden. Vorzugsweise verwendbar sind Alkalimetall- und Erdalkalimetalloxide, -hydroxide und -carbonate, wie Natriumhydroxid, Kaliumhydroxid, Magnesiumoxid, Calciumoxid, Natriumcarbonat, Kaliumcarbonat und Calciumcarbonat, die auch in Gegenwart von Phasentransferkatalysatoren wie z.B. Triethylbenzylammoniumchlorid, Tetrabutylammoniumbromid, Adogen 464 (=Methyltrialkyl(C₈-C₁₀)ammoniumchlorid) oder TDA 1 (=Tris-(methoxyethoxyethyl)-amin) eingesetzt werden können. Weiterhin können Alkalimetalle wie Natrium oder Kalium verwendet werden. Ferner sind Alkalimetall- und Erdalkalimetallamide und -hydride, wie Natriumamid, Natriumhydrid und Calciumhydrid, und außerdem auch Alkalimetallalkoholate, wie Natriummethylat, Natriumethylat und Kalium-tert.-butylat einsetzbar.

Die Reaktionstemperatur kann bei der Durchführung des erfindungsgemäßen Verfahrens (A) innerhalb eines größeren Bereiches variiert werden. Im allgemeinen arbeitet man bei Temperaturen zwischen -75°C und 200°C, vorzugsweise zwischen -50°C und 150°C.

Das erfindungsgemäße Verfahren (A) wird im allgemeinen unter Normaldruck durchgeführt.

Bei der Durchführung des erfindungsgemäßen Verfahrens (A) setzt man die Reaktionskomponente der Formel (II) und die deprotonierende Base im allgemeinen in äquimolaren bis etwa doppeltäquimolaren Mengen ein. Es ist jedoch auch möglich, die eine oder andere Komponente in einem größeren Überschuss (bis zu 3 Mol) zu verwenden.

Das Verfahren (B) ist dadurch gekennzeichnet, dass man Verbindungen der Formel (III), in welcher A, B, Q¹, Q², W, X, Y, Z und R⁸ die oben angegebenen Bedeutungen haben, in Gegenwart eines Verdünnungsmittels und in Gegenwart einer Base intramolekular kondensiert.

Als Verdünnungsmittel können bei dem erfindungsgemäßen Verfahren (B) alle gegenüber den Reaktionsteilnehmern inerten organischen Solventien eingesetzt werden. Vorzugsweise verwendbar sind Kohlenwasserstoffe, wie Toluol und Xylol, ferner Ether, wie Dibutylether, Tetrahydrofuran, Dioxan, Glykoldimethylether und Diglykoldimethylether, außerdem polare Lösungsmittel, wie Dimethylsulfoxid, Sulfolan, Dimethylformamid und N-Methyl-pyrrolidon. Weiterhin können Alkohole wie Methanol, Ethanol, Propanol, Iso-Propanol, Butanol, Iso-Butanol und tert.-Butanol eingesetzt werden.

Als Base (Deprotonierungsmittel) können bei der Durchführung des erfindungsgemäßen Verfahrens (B) alle üblichen Protonenakzeptoren eingesetzt werden. Vorzugsweise verwendbar sind Alkalimetall- und Erdalkalimetalloxide, -hydroxide und -carbonate, wie Natriumhydroxid, Kaliumhydroxid, Magnesiumoxid, Calciumoxid, Natriumcarbonat, Kaliumcarbonat und Calciumcarbonat, die auch in Gegenwart von Phasentransferkatalysatoren wie z.B. Triethylbenzylammoniumchlorid, Tetrabutylammoniumbromid, Adogen 464 (= Methyltrialkyl(C₈-C₁₀)ammoniumchlorid) oder TDA 1 (= Tris-(methoxyethoxyethyl)-amin) eingesetzt werden können. Weiterhin können Alkalimetalle wie Natrium oder Kalium verwendet werden. Ferner sind Alkalimetall- und Erdalkalimetallamide und -hydride, wie Natriumamid, Natriumhydrid und Calciumhydrid, und außerdem auch Alkalimetallalkoholate, wie Natriummethylat, Natriumethylat und Kalium-tert.-butylat einsetzbar.

Die Reaktionstemperatur kann bei der Durchführung des erfindungsgemäßen Verfahrens (B) innerhalb eines größeren Bereiches variiert werden. Im allgemeinen arbeitet man bei Temperaturen zwischen -75°C und 200°C, vorzugsweise zwischen -50°C und 150°C.

Das erfindungsgemäße Verfahren (B) wird im allgemeinen unter Normaldruck durchgeführt.

Bei der Durchführung des erfindungsgemäßen Verfahrens (B) setzt man die Reaktionskomponenten der Formel (III) und die deprotonierenden Basen im allgemeinen in etwa äquimolaren Mengen ein. Es ist jedoch auch möglich, die eine oder andere Komponente in einem größeren Überschuss (bis zu 3 Mol) zu verwenden.

Das Verfahren (C_{α}) ist dadurch gekennzeichnet, dass man Verbindungen der Formeln (I-1-a) bis (1-2-a) jeweils mit Carbonsäurehalogeniden der Formel (IV) gegebenenfalls in Gegenwart eines Verdünnungsmittels und gegebenenfalls in Gegenwart eines Säurebindemittels umsetzt.

Als Verdünnungsmittel können bei dem erfindungsgemäßen Verfahren (C_{α}) alle gegenüber den Säurehalogeniden inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenstoff, Chlorbenzol und o-Dichlorbenzol, außerdem Ketone, wie Aceton und Methylisopropylketon, weiterhin Ether, wie Diethylether, Tetrahydrofuran und Dioxan, darüber hinaus Carbonsäureester, wie Ethylacetat, und auch stark polare Solventien, wie Dimethylformamid, Dimethylsulfoxid und Sulfolan. Wenn die Hydrolysestabilität des Säurehalogenids es zulässt, kann die Umsetzung auch in Gegenwart von Wasser durchgeführt werden.

Als Säurebindemittel kommen bei der Umsetzung nach dem erfindungsgemäßen Verfahren (C_{α}) alle üblichen Säureakzeptoren in Betracht. Vorzugsweise verwendbar sind tertiäre Amine, wie Triethylamin, Pyridin, Diazabicyclooctan (DABCO), Diazabicycloundecen (DBU), Diazabicyclononen (DBN), Hünig-Base und N,N-Dimethyl-anilin, ferner Erdalkalimetalloxide, wie Magnesium- und Calciumoxid, außerdem Alkali- und Erdalkali-metall-carbonate, wie Natriumcarbonat, Kaliumcarbonat und Calciumcarbonat sowie Alkalihydroxide wie Natriumhydroxid und Kaliumhydroxid.

Die Reaktionstemperatur kann bei dem erfindungsgemäßen Verfahren (C_{α}) innerhalb eines größeren Bereiches variiert werden. Im allgemeinen arbeitet man bei Temperaturen zwischen -20°C und +150°C, vorzugsweise zwischen 0°C und 100°C.

Bei der Durchführung des erfindungsgemäßen Verfahrens (C_{α}) werden die Ausgangsstoffe der Formeln (I-1-a) bis (I-2-a) und das Carbonsäurehalogenid der Formel (IV) im allgemeinen jeweils in angenähert äquivalenten Mengen verwendet. Es ist jedoch auch möglich, das Carbonsäurehalogenid in einem größeren Überschuss (bis zu 5 Mol) einzusetzen. Die Aufarbeitung erfolgt nach üblichen Methoden.

Das Verfahren (C_{β}) ist dadurch gekennzeichnet, dass man Verbindungen der Formeln (I-1-a) bis (I-2-a) jeweils mit Carbonsäureanhydriden der Formel (V) gegebenenfalls in Gegenwart eines Verdünnungsmittels und gegebenenfalls in Gegenwart eines Säurebindemittels umsetzt.

Als Verdünnungsmittel können bei dem erfindungsgemäßen Verfahren (C_{β}) vorzugsweise diejenigen Verdünnungsmittel verwendet werden, die auch bei der Verwendung von Säurehalogeniden vorzugsweise in Betracht kommen. Im übrigen kann auch ein im Überschuss eingesetztes Carbonsäureanhydrid gleichzeitig als Verdünnungsmittel fungieren.

Als gegebenenfalls zugesetzte Säurebindemittel kommen beim Verfahren (C_{β}) vorzugsweise diejenigen Säurebindemittel in Frage, die auch bei der Verwendung von Säurehalogeniden vorzugsweise in Betracht kommen.

Die Reaktionstemperatur kann bei dem erfindungsgemäßen Verfahren (C_{β}) innerhalb eines größeren Bereiches variiert werden. Im allgemeinen arbeitet man bei Temperaturen zwischen -20°C und +150°C, vorzugsweise zwischen 0°C und 100°C.

Bei der Durchführung des erfindungsgemäßen Verfahrens (C_{β}) werden die Ausgangsstoffe der Formeln (I-1-a) bis (1-2-a) und das Carbonsäureanhydrid der Formel (V) im allgemeinen in jeweils angenähert äquivalenten Mengen verwendet. Es ist jedoch auch möglich, das Carbonsäureanhydrid in einem größeren Überschuss (bis zu 5 Mol) einzusetzen. Die Aufarbeitung erfolgt nach üblichen Methoden.

Im allgemeinen geht man so vor, dass man Verdünnungsmittel und im Überschuss vorhandenes Carbonsäureanhydrid sowie die entstehende Carbonsäure durch Destillation oder durch Waschen mit einem organischen Lösungsmittel oder mit Wasser entfernt.

Das Verfahren (D) ist dadurch gekennzeichnet, dass man Verbindungen der Formeln (I-1-a) bis (I-2-a) jeweils mit Chlorameisensäureestern oder Chlorameisensäurethiolestern der Formel (VI) gegebenenfalls in Gegenwart eines Verdünnungsmittels und gegebenenfalls in Gegenwart eines Säurebindemittels umsetzt.

Als Säurebindemittel kommen bei dem erfindungsgemäßen Verfahren (D) alle üblichen Säureakzeptoren in Betracht. Vorzugsweise verwendbar sind tertiäre Amine, wie Triethylamin, Pyridin, DABCO, DBU, DBN, Hünig-Base und N,N-Dimethyl-anilin, ferner Erdalkalimetalloxide, wie Magnesium- und Calciumoxid, außerdem Alkali- und Erdalkalimetallcarbonate, wie Natriumcarbonat, Kaliumcarbonat und Calciumcarbonat sowie Alkalihydroxide wie Natriumhydroxid und Kaliumhydroxid.

Als Verdünnungsmittel können bei dem erfindungsgemäßen Verfahren (D) alle gegenüber den Chlorameisensäureestern bzw. Chlorameisensäurethiolestern inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenwasserstoff, Chlorbenzol und o-Dichlorbenzol, außerdem Ketone, wie Aceton und Methylisopropylketon, weiterhin Ether, wie Diethylether, Tetrahydrofuran und Dioxan, darüber hinaus Carbonsäureester, wie Ethylacetat, außerdem Nitrile wie Acetonitril und auch stark polare Solventien, wie Dimethylformamid, Dimethylsulfoxid und Sulfolan.

Die Reaktionstemperatur kann bei der Durchführung des erfindungsgemäßen Verfahrens (D) innerhalb eines größeren Bereiches variiert werden. Die Reaktionstemperatur liegt im allgemeinen zwischen -20°C und +100°C, vorzugsweise zwischen 0°C und 50°C.

Das erfindungsgemäße Verfahren (D) wird im allgemeinen unter Normaldruck durchgeführt.

Bei der Durchführung des erfindungsgemäßen Verfahrens (D) werden die Ausgangsstoffe der Formeln (I-1-a) bis (I-2-a) und der entsprechende Chlorameisensäureester bzw. Chlorameisensäurethiolester der Formel (VI) im Allgemeinen jeweils in angenähert äquivalenten Mengen verwendet. Es ist jedoch auch möglich, die eine oder andere Komponente in einem größeren Überschuss (bis zu 2 Mol) einzusetzen. Die Aufarbeitung erfolgt nach üblichen Methoden. Im allgemeinen geht man so vor, dass man ausgefallene Salze entfernt und das verbleibende Reaktionsgemisch durch Abziehen des Verdünnungsmittels einengt.

Das erfindungsgemäße Verfahren (E) ist dadurch gekennzeichnet, dass man Verbindungen der Formeln (I-1-a) bis (I-2-a) jeweils mit Verbindungen der Formel (VII) in Gegenwart eines Verdünnungsmittels und gegebenenfalls in Gegenwart eines Säurebindemittels umsetzt.

Beim Herstellungsverfahren (E) setzt man pro Mol Ausgangsverbindung der Formeln (I-1-a) bis (I-2-a) ca. 1 Mol Chlormonothioameisensäureester bzw. Chlordithioameisensäureester der Formel (VII) bei 0 bis 120°C, vorzugsweise bei 20 bis 60°C um.

Als gegebenenfalls zugesetzte Verdünnungsmittel kommen alle inerten polaren organischen Lösungsmittel in Frage, wie Ether, Amide, Sulfone, Sulfoxide, aber auch Halogenalkane.

Vorzugsweise werden Dimethylsulfoxid, Tetrahydrofuran, Dimethylformamid, Essigsäureethylester oder Methylenchlorid eingesetzt.

Stellt man in einer bevorzugten Ausführungsform durch Zusatz von starken Deprotonierungsmitteln wie z.B. Natriumhydrid oder Kaliumtertiärbutylat das Enolatsalz der Verbindungen (I-1-a) bis (I-2-a) dar, kann auf den weiteren Zusatz von Säurebindemitteln verzichtet werden.

Als Basen können beim Verfahren (E) alle üblichen Protonenakzeptoren eingesetzt werden. Vorzugsweise verwendbar sind Alkalimetallhydride, Alkalimetallalkoholate, Alkali- oder Erdalkalimetallcarbonate oder -hydrogencarbonate oder Stickstoffbasen. Genannt seien beispielsweise Natriumhydrid, Natriummethanolat, Natriumhydroxid, Calciumhydroxid, Kaliumcarbonat, Natriumhydrogencarbonat, Triethylamin, Dibenzylamin, Diisopropylamin, Pyridin, Chinolin, Diazabicyclooctan (DABCO), Diazabicyclononen (DBN) und Diazabicycloundecen (DBU).

Die Reaktion kann bei Normaldruck oder unter erhöhtem Druck durchgeführt werden, vorzugsweise wird bei Normaldruck gearbeitet. Die Aufarbeitung geschieht nach üblichen Methoden.

Das erfindungsgemäße Verfahren (F) ist dadurch gekennzeichnet, dass man Verbindungen der Formeln (I-1-a) bis (I-2-a) jeweils mit Sulfonsäurechloriden der Formel (VIII) gegebenenfalls in Gegenwart eines Verdünnungsmittels und gegebenenfalls in Gegenwart eines Säurebindemittels umsetzt.

Beim Herstellungsverfahren (F) setzt man pro Mol Ausgangsverbindung der Formel (I-1-a) bis (I-2-a) ca. 1 Mol Sulfonsäurechlorid der Formel (VIII) bei -20 bis 150°C, vorzugsweise bei 0 bis 70°C um.

Das Verfahren (F) wird vorzugsweise in Gegenwart eines Verdünnungsmittels durchgeführt.

Als Verdünnungsmittel kommen alle inerten polaren organischen Lösungsmittel in Frage wie Ether, Amide, Ketone, Carbonsäureester, Nitrile, Sulfone, Sulfoxide oder halogenierte Kohlenwasserstoffe wie Methylenchlorid.

Vorzugsweise werden Dimethylsulfoxid, Tetrahydrofuran, Dimethylformamid, Essigsäureethylester, Methylenchlorid eingesetzt.

Stellt man in einer bevorzugten Ausführungsform durch Zusatz von starken Deprotonierungsmitteln (wie z.B. Natriumhydrid oder Kaliumtertiärbutylat) das Enolatsalz der Verbindungen (I-1-a) bis (I-2-a) dar, kann auf den weiteren Zusatz von Säurebindemitteln verzichtet werden.

Werden Säurebindemittel eingesetzt, so kommen übliche anorganische oder organische Basen in Frage, beispielhaft seien Natriumhydroxid, Natriumcarbonat, Kaliumcarbonat, Pyridin und Triethylamin aufgeführt.

Die Reaktion kann bei Normaldruck oder unter erhöhtem Druck durchgeführt werden, vorzugsweise wird bei Normaldruck gearbeitet. Die Aufarbeitung geschieht nach üblichen Methoden.

Das erfindungsgemäße Verfahren (G) ist dadurch gekennzeichnet, dass man Verbindungen der Formeln (I-1-a) bis (I-2-a) jeweils mit Phosphorverbindungen der Formel (IX) gegebenenfalls in Gegenwart eines Verdünnungsmittels und gegebenenfalls in Gegenwart eines Säurebindemittels umsetzt.

Beim Herstellungsverfahren (G) setzt man zum Erhalt von Verbindungen der Formeln (I-1-e) bis (I-2-e) auf 1 Mol der Verbindungen (I-1-a) bis (I-2-a), 1 bis 2, vorzugsweise 1 bis 1,3 Mol der Phosphorverbindung der Formel (IX) bei Temperaturen zwischen -40°C und 150°C, vorzugsweise zwischen -10 und 110°C um.

Das Verfahren (G) wird vorzugsweise in Gegenwart eines Verdünnungsmittels durchgeführt.

Als Verdünnungsmittel kommen alle inerten, polaren organischen Lösungsmittel in Frage wie Ether, Carbonsäureester, halogenierte Kohlenwasserstoffe, Ketone, Amide, Nitrile, Sulfone, Sulfoxide etc.

Vorzugsweise werden Acetonitril, Dimethylsulfoxid, Tetrahydrofuran, Dimethylformamid, Methylenchlorid eingesetzt.

Als gegebenenfalls zugesetzte Säurebindemittel kommen übliche anorganische oder organische Basen in Frage wie Hydroxide, Carbonate oder Amine. Beispielhaft seien Natriumhydroxid, Natriumcarbonat, Kaliumcarbonat, Pyridin und Triethylamin aufgeführt.

Die Umsetzung kann bei Normaldruck oder unter erhöhtem Druck durchgeführt werden, vorzugsweise wird bei Normaldruck gearbeitet. Die Aufarbeitung geschieht nach üblichen Methoden der Organischen Chemie. Die Endprodukte werden vorzugsweise durch Kristallisation, chromatographische Reinigung oder durch sogenanntes "Andestillieren", d.h. Entfernung der flüchtigen Bestandteile im Vakuum gereinigt.

Das Verfahren (H) ist dadurch gekennzeichnet, dass man Verbindungen der Formeln (I-1-a) bis (I-2-a) jeweils mit Metallhydroxiden bzw. Metallalkoxiden der Formel (X) oder Aminen der Formel (XI), gegebenenfalls in Gegenwart eines Verdünnungsmittels, umsetzt.

Als Verdünnungsmittel können bei dem erfindungsgemäßen Verfahren (H) vorzugsweise Ether wie Tetrahydrofuran, Dioxan, Diethylether oder aber Alkohole wie Methanol, Ethanol, Iso-propanol, aber auch Wasser eingesetzt werden. Das erfindungsgemäße Verfahren (H) wird im allgemeinen unter Normaldruck durchgeführt. Die Reaktionstemperatur liegt im allgemeinen zwischen -20°C und 100°C, vorzugsweise zwischen 0°C und 50°C.

Das erfindungsgemäße Verfahren (I) ist dadurch gekennzeichnet, dass man Verbindungen der Formeln (I-1-a) bis (I-2-a) jeweils mit (Iα) Verbindungen der Formel (XII) gegebenenfalls in Gegenwart eines Verdünnungsmittels und gegebenenfalls in Gegenwart eines Katalysators oder (Iβ) mit Verbindungen der Formel (XIII) gegebenenfalls in Gegenwart eines Verdünnungsmittels und gegebenenfalls in Gegenwart eines Säurebindemittels umsetzt.

Bei Herstellungsverfahren (Iα) setzt man pro Mol Ausgangsverbindung der Formeln (I-1-a) bis (I-2-a) ca. 1 Mol Isocyanat der Formel (XII) bei 0 bis 100°C, vorzugsweise bei 20 bis 50°C um.

Das Verfahren (Iα) wird vorzugsweise in Gegenwart eines Verdünnungsmittels durchgeführt.

Als Verdünnungsmittel kommen alle inerten organischen Lösungsmittel in Frage, wie aromatische Kohlenwasserstoffe, halogenierte Kohlenwasserstoffe, Ether, Amide, Nitrile, Sulfone oder Sulfoxide.

Gegebenenfalls können Katalysatoren zur Beschleunigung der Reaktion zugesetzt werden. Als Katalysatoren können sehr vorteilhaft zinnorganische Verbindungen, wie z.B. Dibutylzinndilaurat eingesetzt werden.

Es wird vorzugsweise bei Normaldruck gearbeitet.

Beim Herstellungsverfahren (Iβ) setzt man pro Mol Ausgangsverbindung der Formeln (I-1-a) bis (I-2-a) ca. 1 Mol Carbamidsäurechlorid der Formel (XIII) bei 0 bis 150°C, vorzugsweise bei 20 bis 70°C um.

Als gegebenenfalls zugesetzte Verdünnungsmittel kommen alle inerten polaren organischen Lösungsmittel in Frage wie Ether, Carbonsäureester, Nitrile, Ketone, Amide, Sulfone, Sulfoxide oder halogenierte Kohlenwasserstoffe.

Vorzugsweise werden Dimethylsulfoxid, Tetrahydrofuran, Dimethylformamid oder Methylenchlorid eingesetzt.

Stellt man in einer bevorzugten Ausführungsform durch Zusatz von starken Deprotonierungsmitteln (wie z.B. Natriumhydrid oder Kaliumtertiärbutylat) das Enolatsalz der Verbindung (I-1-a) bis (1-2-a) dar, kann auf den weiteren Zusatz von Säurebindemitteln verzichtet werden.

Werden Säurebindemittel eingesetzt, so kommen übliche anorganische oder organische Basen in Frage, beispielhaft seien Natriumhydroxid, Natriumcarbonat, Kaliumcarbonat, Triethylamin oder Pyridin genannt.

Die Reaktion kann bei Normaldruck oder unter erhöhtem Druck durchgeführt werden, vorzugsweise wird bei Normaldruck gearbeitet. Die Aufarbeitung geschieht nach üblichen Methoden.

Die Wirkstoffe eignen sich bei guter Pflanzenverträglichkeit und günstiger Warmblütertoxizität zur Bekämpfung von tierischen Schädlingen, insbesondere Insekten, Spinnentieren und Nematoden, die in der Landwirtschaft, in Forsten, im Vorrats- und Materialschutz sowie auf dem Hygienesektor vorkommen. Sie können vorzugsweise als Pflanzenschutzmittel eingesetzt werden. Sie sind gegen normal sensible und resistente Arten sowie gegen alle oder einzelne Entwicklungsstadien wirksam. Zu den oben erwähnten Schädlingen gehören:
Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophagus, Scutigera spp.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Acheta domesticus, Gryllotalpa spp., Locusta migratoria migratorioides, Melanoplus spp., Schistocerca gregaria.
Aus der Ordnung der Blattaria z.B. Blatta orientalis, Periplaneta americana, Leucophaea maderae, Blattella germanica. Aus der Ordnung der Dermaptera z.B. Forficula auricularia.
Aus der Ordnung der Isoptera z.B. Reticulitermes spp.
Aus der Ordnung der Phthiraptera z.B. Pediculus humanus corporis, Haematopinus spp., Linognathus spp., Trichodectes spp., Damalinia spp.
Aus der Ordnung der Thysanoptera z.B. Hercinothrips femoralis, Thrips tabaci, Thrips palmi, Frankliniella accidentalis.
Aus der Ordnung der Heteroptera z.B. Eurygaster spp., Dysdercus intermedius, Piesma quadrata, Cimex lectularius, Rhodnius prolixus, Triatoma spp.
Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Aphis pomi, Eriosoma lanigerum, Hyalopterus arundinis, Phylloxera vastatrix, Pemphigus spp., Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp., Psylla spp.
Aus der Ordnung der Lepidoptera z.B. Pectinophora gossypiella, Bupalus piniarius, Cheimatobia brumata, Lithocolletis blancardella, Hyponomeuta padella, Plutella xylostella, Malacosoma neustria, Euproctis chrysorrhoea, Lymantria spp., Bucculatrix thurberiella, Phyllocnistis citrella, Agrotis spp., Euxoa spp., Feltia spp., Earias insulana, Heliothis spp., Mamestra brassicae, Panolis flammea, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana, Cnaphalocerus spp., Oulema oryzae.
Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica, Lissorhoptrus oryzophilus.
Aus der Ordnung der Hymenoptera z.B. Diprion spp., Hoplocampa spp., Lasius spp., Monomorium pharaonis, Vespa spp.
Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa, Hylemyia spp., Liriomyza spp.
Aus der Ordnung der Siphonaptera z.B. Xenopsylla cheopis, Ceratophyllus spp.
Aus der Klasse der Arachnida z.B. Scorpio maurus, Latrodectus mactans, Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp., Hemitarsonemus spp., Brevipalpus spp.

Zu den pflanzenparasitären Nematoden gehören z.B. Pratylenchus spp., Radopholus similis, Ditylenchus dipsaci, Tylenchulus semipenetrans, Heterodera spp., Globodera spp., Meloidogyne spp., Aphelenchoides spp., Longidorus spp., Xiphinema spp., Trichodorus spp., Bursaphelenchus spp.

Die erfindungsgemäßen Verbindungen bzw. Wirkstoffkombinationen können gegebenenfalls in bestimmten Konzentrationen bzw. Aufwandmengen auch als Herbizide verwendet werden. Die Verbindungen lassen sich gegebenenfalls auch als Zwischen- oder Vorprodukte für die Synthese weiterer Wirkstoffe einsetzen.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Spross, Blatt, Blüte und Wurzel verstanden werden, wobei beispielhaft Blätter, Nadeln, Stängel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen.

Die erfindungsgemäße Behandlung der Pflanzen und Pflanzenteile mit den Wirkstoffen bzw. Wirkstoffkombinationen erfolgt direkt oder durch Einwirkung auf deren Umgebung, Lebensraum oder Lagerraum nach den üblichen Behandlungsmethoden, z.B. durch Tauchen, Sprühen, Spritzen, Verdampfen, Vernebeln, Streuen, Aufstreichen und bei Vermehrungsmaterial, insbesondere bei Samen, weiterhin durch ein- oder mehrschichtiges Umhüllen.

Die Wirkstoffe bzw. Wirkstoffkombinationen können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

### Als feste Trägerstoffe kommen in Frage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstängeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Einweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im Allgemeinen zwischen 0,1 und 95 Gew.-% Wirkstoff, vorzugsweise zwischen 0,5 und 90 %.

Die erfindungsgemäßen Wirkstoffe können als solche oder in ihren Formulierungen auch in Mischung mit bekannten Fungiziden, Bakteriziden, Akariziden, Nematiziden oder Insektiziden verwendet werden, um so z.B. das Wirkungsspektrum zu verbreitern oder Resistenzentwicklungen vorzubeugen. In vielen Fällen erhält man dabei synergistische Effekte, d.h. die Wirksamkeit der Mischung ist größer als die Wirksamkeit der Einzelkomponenten.

### Als Mischpartner kommen zum Beispiel folgende Verbindungen in Frage:

### Fungizide:

2-Phenylphenol; 8-Hydroxyquinoline sulfate; Acibenzolar-S-methyl; Aldimorph; Amidoflumet; Ampropylfos; Ampropylfos-potassium; Andoprim; Anilazine; Azaconazole; Azoxystrobin; Benalaxyl; Benodanil; Benomyl; Benthiavalicarb-isopropyl; Benzamacril; Benzamacril-isobutyl; Bilanafos; Binapacryl; Biphenyl; Bitertanol; Blasticidin-S; Bromuconazole; Bupirimate; Buthiobate; Butylamine; Calcium polysulfide; Capsimycin; Captafol; Captan; Carbendazim; Carboxin; Carpropamid; Carvone; Chinomethionat; Chlobenthiazone; Chlorfenazole; Chloroneb; Chlorothalonil; Chlozolinate; Clozylacon; Cyazofamid; Cyflufenamid; Cymoxanil; Cyproconazole; Cyprodinil; Cyprofuram; Dagger G; Debacarb; Dichlofluanid; Dichlone; Dichlorophen; Diclocymet; Diclomezine; Dicloran; Diethofencarb; Difenoconazole; Diflumetorim; Dimethirimol; Dimethomorph; Dimoxystrobin; Diniconazole; Diniconazole-M; Dinocap; Diphenylamine; Dipyrithione; Ditalimfos; Dithianon; Dodine; Drazoxolon; Edifenphos; Epoxiconazole; Ethaboxam; Ethirimol; Etridiazole; Famoxadone; Fenamidone; Fenapanil; Fenarimol; Fenbuconazole; Fenfuram; Fenhexamid; Fenitropan; Fenoxanil; Fenpiclonil; Fenpropidin; Fenpropimorph; Ferbam; Fluazinam; Flubenzimine; Fludioxonil; Flumetover; Flumorph; Fluoromide; Fluoxastrobin; Fluquinconazole; Flurprimidol; Flusilazole; Flusulfamide; Flutolanil; Flutriafol; Folpet; Fosetyl-Al; Fosetyl-sodium; Fuberidazole; Furalaxyl; Furametpyr; Furcarbanil; Furmecyclox; Guazatine; Hexachlorobenzene; Hexaconazole; Hymexazol; Imazalil; Imibenconazole; Iminoctadine triacetate; Iminoctadine tris(albesilate); Iodocarb; Ipconazole; Iprobenfos; Iprodione; Iprovalicarb; Irumamycin; Isoprothiolane; Isovaledione; Kasugamycin; Kresoxim-methyl; Mancozeb; Maneb; Meferimzone; Mepanipyrim; Mepronil; Metalaxyl; Metalaxyl-M; Metconazole; Methasulfocarb; Methfuroxam; Metiram; Metominostrobin; Metsulfovax; Mildiomycin; Myclobutanil; Myclozolin; Natamycin; Nicobifen; Nitrothal-isopropyl; Noviflumuron; Nuarimol; Ofurace; Orysastrobin; Oxadixyl; Oxolinic acid; Oxpoconazole; Oxycarboxin; Oxyfenthiin; Paclobutrazol; Pefurazoate; Penconazole; Pencycuron; Phosdiphen; Phthalide; Picoxystrobin; Piperalin; Polyoxins; Polyoxorim; Probenazole; Prochloraz; Procymidone; Propamocarb; Propanosine-sodium; Propiconazole; Propineb; Proquinazid; Prothioconazole; Pyraclostrobin; Pyrazophos; Pyrifenox; Pyrimethanil; Pyroquilon; Pyroxyfur; Pyrrolnitrine; Quinconazole; Quinoxyfen; Quintozene; Simeconazole; Spiroxamine; Sulfur; Tebuconazole; Tecloftalam; Tecnazene; Tetcyclacis; Tetraconazole; Thiabendazole; Thicyofen; Thifluzamide; Thiophanate-methyl; Thiram; Tioxymid; Tolclofos-methyl; Tolylfluanid; Triadimefon; Triadimenol; Triazbutil; Triazoxide; Tricyclamide; Tricyclazole; Tridemorph; Trifloxystrobin; Triflumizole; Triforine; Triticonazole; Uniconazole; Validamycin A; Vinclozolin; Zineb; Ziram; Zoxamide; (2S)-N-[2-[4-[[3-(4-chlorophenyl)-2-propynyl]oxy]-3-methoxyphenyl]-ethyl]-3-methyl- 2-[(methylsulfonyl)amino]-butanamide; 1-(1-naphthalenyl)-1H-pyrrole-2,5-dione; 2,3,5,6-tetrachloro-4-(methylsulfonyl)-pyridine; 2-amino-4-methyl-N-phenyl-5-thiazolecarboxamide; 2-chloro-N-(2,3-dihydro-1,1,3-trimethyl-1H-inden-4-yl)-3-pyridincarboxamide; 3,4,5-trichloro-2,6-pyridinedicarbonitrile; Actinovate; cis-1-(4-chlorophenyl)-2-(1H-1,2,4-triazole-1-yl)-cycloheptanol; methyl 1-(2,3-dihydro-2,2-dimethyl-1H-inden-1-yl)-1H-imidazole-5-carboxylate; monopotassium carbonate; N-(6-methoxy-3-pyridinyl)-cyclopropanecarboxamide; N-butyl-8-(1,1-dimethylethyl)-1-oxaspiro[4.5]decan-3-amine; Sodium tetrathiocarbonate;
sowie Kupfersalze und -zubereitungen, wie Bordeaux mixture; Copper hydroxide; Copper naphthenate; Copper oxychloride; Copper sulfate; Cufraneb; Cuprous oxide; Mancopper; Oxinecopper.

### Bakterizide:

Bronopol, Dichlorophen, Nitrapyrin, Nickel-Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide:

Abamectin, ABG-9008, Acephate, Acequinocyl, Acetamiprid, Acetoprole, Acrinathrin, AKD-1022, AKD-3059, AKD-3088, Alanycarb, Aldicarb, Aldoxycarb, Allethrin, Alpha-Cypermethrin (Alphamethrin), Amidoflumet, Aminocarb, Amitraz, Avermectin, AZ-60541, Azadirachtin, Azamethiphos, Azinphos-methyl, Azinphos-ethyl, Azocyclotin,
Bacillus popilliae, Bacillus sphaericus, Bacillus subtilis, Bacillus thuringiensis, Bacillus thuringiensis strain EG-2348, Bacillus thuringiensis strain GC-91, Bacillus thuringiensis strain NCTC-11821, Baculoviren, Beauveria bassiana, Beauveria tenella, Benclothiaz, Bendiocarb, Benfuracarb, Bensultap, Benzoximate, Beta-Cyfluthrin, Beta-Cypermethrin, Bifenazate, Bifenthrin, Binapacryl, Bioallethrin, Bioallethrin-S-cyclopentyl-isomer, Bioethanomethrin, Biopermethrin, Bioresmethrin, Bistrifluron, BPMC, Brofenprox, Bromophos-ethyl, Bromopropylate, Bromfenvinfos (-methyl), BTG-504, BTG-505, Bufencarb, Buprofezin, Butathiofos, Butocarboxim, Butoxycarboxim, Butylpyridaben,
Cadusafos, Camphechlor, Carbaryl, Carbofuran, Carbophenothion, Carbosulfan, Cartap, CGA-50439, Chinomethionat, Chlordane, Chlordimeform, Chloethocarb, Chlorethoxyfos, Chlorfenapyr, Chlorfenvinphos, Chlorfluazuron, Chlormephos, Chlorobenzilate, Chloropicrin, Chlorproxyfen, Chlorpyrifos-methyl, Chlorpyrifos (-ethyl), Chlovaporthrin, Chromafenozide, Cis-Cypermethrin, Cis-Resmethrin, Cis-Permethrin, Clocythrin, Cloethocarb, Clofentezine, Clothianidin, Clothiazoben, Codlemone, Coumaphos, Cyanofenphos, Cyanophos, Cycloprene, Cycloprothrin, Cydia pomonella, Cyfluthrin, Cyhalothrin, Cyhexatin, Cypermethrin, Cyphenothrin (1R-trans-isomer), Cyromazine,
DDT, Deltamethrin, Demeton-S-methyl, Demeton-S-methylsulphon, Diafenthiuron, Dialifos, Diazinon, Dichlofenthion, Dichlorvos, Dicofol, Dicrotophos, Dicyclanil, Diflubenzuron, Dimefluthrin, Dimethoate, Dimethylvinphos, Dinobuton, Dinocap, Dinotefuran, Diofenolan, Disulfoton, Docusat-sodium, Dofenapyn, DOWCO-439,
Eflusilanate, Emamectin, Emamectin-benzoate, Empenthrin (1R-isomer), Endosulfan, Entomopthora spp., EPN, Esfenvalerate, Ethiofencarb, Ethiprole, Ethion, Ethoprophos, Etofenprox, Etoxazole, Etrimfos,
Famphur, Fenamiphos, Fenazaquin, Fenbutatin oxide, Fenfluthrin, Fenitrothion, Fenobucarb, Fenothiocarb, Fenoxacrim, Fenoxycarb, Fenpropathrin, Fenpyrad, Fenpyrithrin, Fenpyroximate, Fensulfothion, Fenthion, Fentrifanil, Fenvalerate, Fipronil, Flonicamid, Fluacrypyrim, Fluazuron, Flubenzimine, Flubrocythrinate, Flucycloxuron, Flucythrinate, Flufenerim, Flufenoxuron, Flufenprox, Flumethrin, Flupyrazofos, Flutenzin (Flufenzine), Fluvalinate, Fonofos, Formetanate, Formothion, Fosmethilan, Fosthiazate, Fubfenprox (Fluproxyfen), Furathiocarb,
Gamma-Cyhalothrin, Gamma-HCH, Gossyplure, Grandlure, Granuloseviren,
Halfenprox, Halofenozide, HCH, HCN-801, Heptenophos, Hexaflumuron, Hexythiazox, Hydramethylnone, Hydroprene,
IKA-2002, Imidacloprid, Imiprothrin, Indoxacarb, Iodofenphos, Iprobenfos, Isazofos, Isofenphos, Isoprocarb, Isoxathion, Ivermectin,
Japonilure,
Kadethrin, Kernpolyederviren, Kinoprene,
Lambda-Cyhalothrin, Lindane, Lufenuron,
Malathion, Mecarbam, Mesulfenfos, Metaldehyd, Metam-sodium, Methacrifos, Methamidophos, Metharhizium anisopliae, Metharhizium flavoviride, Methidathion, Methiocarb, Methomyl, Methoprene, Methoxychlor, Methoxyfenozide, Metofluthrin, Metolcarb, Metoxadiazone, Mevinphos, Milbemectin, Milbemycin, MKI-245, MON-45700, Monocrotophos, Moxidectin, MTI-800,
Naled, NC-104, NC-170, NC-184, NC-194, NC-196, Niclosamide, Nicotine, Nitenpyram, Nithiazine, NNI-0001, NNI-0101, NNI-0250, NNI-9768, Novaluron, Noviflumuron,
OK-5101, OK-5201, OK-9601, OK-9602, OK-9701, OK-9802, Omethoate, Oxamyl, Oxydemetonmethyl,
Paecilomyces fumosoroseus, Parathion-methyl, Parathion (-ethyl), Permethrin (cis-, trans-), Petroleum, PH-6045, Phenothrin (1R-trans isomer), Phenthoate, Phorate, Phosalone, Phosmet, Phosphamidon, Phosphocarb, Phoxim, Piperonyl butoxide, Pirimicarb, Pirimiphos-methyl, Pirimiphos-ethyl, Potassium oleate, Prallethrin, Profenofos, Profluthrin, Promecarb, Propaphos, Propargite, Propetamphos, Propoxur, Prothiofos, Prothoate, Protrifenbute, Pymetrozine, Pyraclofos, Pyresmethrin, Pyrethrum, Pyridaben, Pyridalyl, Pyridaphenthion, Pyridathion, Pyrimidifen, Pyriproxyfen, Quinalphos,
Resmethrin, RH-5849, Ribavirin, RU-12457, RU-15525,
S-421, S-1833, Salithion, Sebufos, SI-0009, Silafluofen, Spinosad, Spirodiclofen, Spiromesifen, Sulfluramid, Sulfotep, Sulprofos, SZI-121,
Tau-Fluvalinate, Tebufenozide, Tebufenpyrad, Tebupirimfos, Teflubenzuron, Tefluthrin, Temephos, Temivinphos, Terbam, Terbufos, Tetrachlorvinphos, Tetradifon, Tetramethrin, Tetramethrin (1R-isomer), Tetrasul, Theta-Cypermethrin, Thiacloprid, Thiamethoxam, Thiapronil, Thiatriphos, Thiocyclam hydrogen oxalate, Thiodicarb, Thiofanox, Thiometon, Thiosultap-sodium, Thuringiensin, Tolfenpyrad, Tralocythrin, Tralomethrin, Transfluthrin, Triarathene, Triazamate, Triazophos, Triazuron, Trichlophenidine, Trichlorfon, Trichoderma atroviride, Triflumuron, Trimethacarb,
Vamidothion, Vaniliprole, Verbutin, Verticillium lecanii,
WL-108477, WL-40027,
YI-5201, YI-5301, YI-5302,
XMC, Xylylcarb,
ZA-3274, Zeta-Cypermethrin, Zolaprofos, ZXI-8901,
die Verbindung 3-Methyl-phenyl-propylcarbamat (Tsumacide Z),
die Verbindung 3-(5-Chlor-3-pyridinyl)-8-(2,2,2-trifluorethyl)-8-azabicyclo[3.2.1]octan-3-carbonitril (CAS-Reg.-Nr. 185982-80-3) und das entsprechende 3-endo-Isomere (CAS-Reg.-Nr. 185984-60-5) (vgl. WO-96/37494, WO-98/25923),
sowie Präparate, welche insektizid wirksame Pflanzenextrakte, Nematoden, Pilze oder Viren enthalten.

Auch eine Mischung mit anderen bekannten Wirkstoffen, wie Herbiziden oder mit Düngemitteln und Wachstumsregulatoren ist möglich.

Die erfindungsgemäßen Wirkstoffe können ferner beim Einsatz als Insektizide in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne dass der zugesetzte Synergist selbst aktiv wirksam sein muss.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnet sich der Wirkstoff bzw. Wirkstoffkombinationen durch eine hervorragende Residualwirkung auf Holz und Ton sowie durch eine gute Alkalistabilität auf gekälkten Unterlagen aus.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetic Modified Organisms) und deren Teile behandelt. Der Begriff "Teile" bzw. "Teile von Pflanzen" oder "Pflanzenteile" wurde oben erläutert.

Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Bio- und Genotypen sein.

Je nach Pflanzenarten bzw. Pflanzensorten, deren Standort und Wachstumsbedingungen (Böden, Klima, Vegetationsperiode, Ernährung) können durch die erfindungsgemäße Behandlung auch überadditive ("synergistische") Effekte auftreten. So sind beispielsweise erniedrigte Aufwandmengen und/oder Erweiterungen des Wirkungsspektrums und/oder eine Verstärkung der Wirkung der erfindungsgemäß verwendbaren Stoffe und Mittel, besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte möglich, die über die eigentlich zu erwartenden Effekte hinausgehen.

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehr der Pflanzen gegen tierische und mikrobielle Schädlinge, wie gegenüber Insekten, Milben, pflanzenpathogenen Pilzen, Bakterien und/oder Viren sowie eine erhöhte Toleranz der Pflanzen gegen bestimmte herbizide Wirkstoffe. Als Beispiele transgener Pflanzen werden die wichtigen Kulturpflanzen, wie Getreide (Weizen, Reis), Mais, Soja, Kartoffel, Baumwolle, Raps, Rüben, Zuckerrohr sowie Obstpflanzen (mit den Früchten Äpfel, Birnen, Zitrusfrüchten und Weintrauben) erwähnt, wobei Mais, Soja, Kartoffel, Baumwolle und Raps besonders hervorgehoben werden. Als Eigenschaften ("Traits") werden besonders hervorgehoben die erhöhte Abwehr der Pflanzen gegen Insekten durch in den Pflanzen entstehende Toxine, insbesondere solche, die durch das genetische Material aus Bacillus Thuringiensis (z.B. durch die Gene CryIA(a), CryIA(b), CryIA(c), CryIIA, CryIIIA, CryIIIB2, Cry9c Cry2Ab, Cry3Bb und CryIF sowie deren Kombinationen) in den Pflanzen erzeugt werden (im Folgenden "Bt Pflanzen"). Als Eigenschaften ("Traits") werden auch besonders hervorgehoben die erhöhte Abwehr von Pflanzen gegen Pilze, Bakterien und Viren durch Systemische Akquirierte Resistenz (SAR), Systemin, Phytoalexine, Elicitoren sowie Resistenzgene und entsprechend exprimierte Proteine und Toxine. Als Eigenschaften ("Traits") werden weiterhin besonders hervorgehoben die erhöhte Toleranz der Pflanzen gegenüber bestimmten herbiziden Wirkstoffen, beispielsweise Imidazolinonen, Sulfonylharnstoffen, Glyphosate oder Phosphinotricin (z.B. "PAT"-Gen). Die jeweils die gewünschten Eigenschaften ("Traits") verleihenden Gene können auch in Kombinationen miteinander in den transgenen Pflanzen vorkommen. Als Beispiele für "Bt Pflanzen" seien Maissorten, Baumwollsorten, Sojasorten und Kartoffelsorten genannt, die unter den Handelsbezeichnungen YIELD GARD® (z.B. Mais, Baumwolle, Soja), KnockOut® (z.B. Mais), StarLink® (z.B. Mais), Bollgard® (Baumwolle), Nucotn® (Baumwolle) und NewLeaf® (Kartoffel) vertrieben werden. Als Beispiele für Herbizid tolerante Pflanzen seien Maissorten, Baumwollsorten und Sojasorten genannt, die unter den Handelsbezeichnungen Roundup Ready® (Toleranz gegen Glyphosate z.B. Mais, Baumwolle, Soja), Liberty Link® (Toleranz gegen Phosphinotricin, z.B. Raps), IMI® (Toleranz gegen Imidazolinone) und STS® (Toleranz gegen Sulfonylharnstoffe z.B. Mais) vertrieben werden. Als Herbizid resistente (konventionell auf Herbizid-Toleranz gezüchtete) Pflanzen seien auch die unter der Bezeichnung Clearfield® vertriebenen Sorten (z.B. Mais) erwähnt. Selbstverständlich gelten diese Aussagen auch für in der Zukunft entwickelte bzw. zukünftig auf den Markt kommende Pflanzensorten mit diesen oder zukünftig entwickelten genetischen Eigenschaften ("Traits").

Die aufgeführten Pflanzen können besonders vorteilhaft mit den erfindungsgemäßen Verbindungen bzw. den erfindungsgemäßen Wirkstoffmischungen behandelt werden. Die bei den Wirkstoffen bzw. Mischungen oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen bzw. Mischungen.

Die erfindungsgemäßen Wirkstoffe bzw. Wirkstoffkombinationen wirken nicht nur gegen Pflanzen-, Hygiene- und Vorratsschädlinge, sondern auch auf dem veterinärmedizinischen Sektor gegen tierische Parasiten (Ektoparasiten) wie Schildzecken, Lederzecken, Räudemilben, Laufmilben, Fliegen (stechend und leckend), parasitierende Fliegenlarven, Läuse, Haarlinge, Federlinge und Flöhe. Zu diesen Parasiten gehören:
Aus der Ordnung der Anoplurida z.B. Haematopinus spp., Linognathus spp., Pediculus spp., Phtirus spp., Solenopotes spp..
Aus der Ordnung der Mallophagida und den Unterordnungen Amblycerina sowie Ischnocerina z.B. Trimenopon spp., Menopon spp., Trinoton spp., Bovicola spp., Werneckiella spp., Lepikentron spp., Damalina spp., Trichodectes spp., Felicola spp..
Aus der Ordnung Diptera und den Unterordnungen Nematocerina sowie Brachycerina z.B. Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp..
Aus der Ordnung der Siphonapterida z.B. Pulex spp., Ctenocephalides spp., Xenopsylla spp., Ceratophyllus spp..
Aus der Ordnung der Heteropterida z.B. Cimex spp., Triatoma spp., Rhodnius spp., Panstrongylus spp.. Aus der Ordnung der Blattarida z.B. Blatta orientalis, Periplaneta americana, Blattela germanica, Supella spp..
Aus der Unterklasse der Acaria (Acarida) und den Ordnungen der Meta- sowie Mesostigmata z.B. Argas spp., Ornithodorus spp., Otobius spp., Ixodes spp., Amblyomma spp., Boophilus spp., Dermacentor spp., Haemophysalis spp., Hyalomma spp., Rhipicephalus spp., Dermanyssus spp., Raillietia spp., Pneumonyssus spp., Sternostoma spp., Varroa spp..
Aus der Ordnung der Actinedida (Prostigmata) und Acaridida (Astigmata) z.B. Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp..

Die erfindungsgemäßen Wirkstoffe bzw. Wirkstoffkombinationen eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so dass durch den Einsatz der erfindungsgemäßen Wirkstoffe eine wirtschaftlichere und einfachere Tierhaltung möglich ist.

Die Anwendung der erfindungsgemäßen Wirkstoffe bzw. Wirkstoffkombinationen geschieht im Veterinärsektor in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändern, Halftern, Markierungsvorrichtungen usw.

Bei der Anwendung für Vieh, Geflügel, Haustiere etc. kann man die Wirkstoffe bzw. Wirkstoffkombinationen als Formulierungen (beispielsweise Pulver, Emulsionen, fließfähige Mittel), die die Wirkstoffe in einer Menge von 1 bis 80 Gew.-% enthalten, direkt oder nach 100 bis 10 000-facher Verdünnung anwenden oder sie als chemisches Bad verwenden.

Außerdem wurde gefunden, dass die erfindungsgemäßen Verbindungen bzw. Wirkstoffkombinationen eine hohe insektizide Wirkung gegen Insekten zeigen, die technische Materialien zerstören.

### Beispielhaft und vorzugsweise - ohne jedoch zu limitieren - seien die folgenden Insekten genannt:

### Käfer wie

Hylotrupes bajulus, Chlorophorus pilosis, Anobium punctatum, Xestobium rufovillosum, Ptilinus pecticornis, Dendrobium pertinex, Ernobius mollis, Priobium carpini, Lyctus brunneus, Lyctus africanus, Lyctus planicollis, Lyctus linearis, Lyctus pubescens, Trogoxylon aequale, Minthes rugicollis, Xyleborus spec. Tryptodendron spec. Apate monachus, Bostrychus capucins, Heterobostrychus brunneus, Sinoxylon spec. Dinoderus minutus.

Hautflügler wie Sirex juvencus, Urocerus gigas, Urocerus gigas taignus, Urocerus augur.

Termiten wie Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis, Coptotermes formosanus.

Borstenschwänze wie Lepisma saccharina.

Unter technischen Materialien sind im vorliegenden Zusammenhang nicht-lebende Materialien zu verstehen, wie vorzugsweise Kunststoffe, Klebstoffe, Leime, Papiere und Kartone, Leder, Holz, Holzverarbeitungsprodukte und Anstrichmittel. Ganz besonders bevorzugt handelt es sich bei dem vor Insektenbefall zu schützenden Material um Holz und Holzverarbeitungsprodukte.

Unter Holz und Holzverarbeitungsprodukten, welche durch das erfindungsgemäße Mittel bzw. dieses enthaltende Mischungen geschützt werden kann, ist beispielhaft zu verstehen:
Bauholz, Holzbalken, Eisenbahnschwellen, Brückenteile, Bootsstege, Holzfahrzeuge, Kisten, Paletten, Container, Telefonmasten, Holzverkleidungen, Holzfenster und -türen, Sperrholz, Spanplatten, Tischlerarbeiten oder Holzprodukte, die ganz allgemein beim Hausbau oder in der Bautischlerei Verwendung finden.

Die Wirkstoffe bzw. Wirkstoffkombinationen können als solche, in Form von Konzentraten oder allgemein üblichen Formulierungen wie Pulver, Granulate, Lösungen, Suspensionen, Emulsionen oder Pasten angewendet werden.

Die genannten Formulierungen können in an sich bekannter Weise hergestellt werden, z.B. durch Vermischen der Wirkstoffe mit mindestens einem Lösungs- bzw. Verdünnungsmittel, Emulgator, Dispergier- und/oder Binde- oder Fixiermittels, Wasser-Repellent, gegebenenfalls Sikkative und UV-Stabilisatoren und gegebenenfalls Farbstoffen und Pigmenten sowie weiteren Verarbeitungshilfsmitteln. Die zum Schutz von Holz und Holzwerkstoffen verwendeten insektiziden Mittel oder Konzentrate enthalten den erfindungsgemäßen Wirkstoff in einer Konzentration von 0,0001 bis 95 Gew.-%, insbesondere 0,001 bis 60 Gew.-%.

Die Menge der eingesetzten Mittel bzw. Konzentrate ist von der Art und dem Vorkommen der Insekten und von dem Medium abhängig. Die optimale Einsatzmenge kann bei der Anwendung jeweils durch Testreihen ermittelt werden. Im Allgemeinen ist es jedoch ausreichend 0,0001 bis 20 Gew.-%, vorzugsweise 0,001 bis 10 Gew.-%, des Wirkstoffs, bezogen auf das zu schützende Material, einzusetzen.

Als Lösungs- und/oder Verdünnungsmittel dient ein organisch-chemisches Lösungsmittel oder Lösungsmittelgemisch und/oder ein öliges oder ölartiges schwer flüchtiges organisch-chemisches Lösungsmittel oder Lösungsmittelgemisch und/oder ein polares organisch-chemisches Lösungsmittel oder Lösungsmittelgemisch und/oder Wasser und gegebenenfalls einen Emulgator und/oder Netzmittel.

Als organisch-chemische Lösungsmittel werden vorzugsweise ölige oder ölartige Lösungsmittel mit einer Verdunstungszahl über 35 und einem Flammpunkt oberhalb 30°C, vorzugsweise oberhalb 45°C, eingesetzt. Als derartige schwerflüchtige, wasserunlösliche, ölige und ölartige Lösungsmittel werden entsprechende Mineralöle oder deren Aromatenfraktionen oder mineralölhaltige Lösungsmittelgemische, vorzugsweise Testbenzin, Petroleum und/oder Alkylbenzol verwendet.

Vorteilhaft gelangen Mineralöle mit einem Siedebereich von 170 bis 220°C, Testbenzin mit einem Siedebereich von 170 bis 220°C, Spindelöl mit einem Siedebereich von 250 bis 350°C, Petroleum bzw. Aromaten vom Siedebereich von 160 bis 280°C, Terpentinöl und dgl. zum Einsatz.

In einer bevorzugten Ausführungsform werden flüssige aliphatische Kohlenwasserstoffe mit einem Siedebereich von 180 bis 210°C oder hochsiedende Gemische von aromatischen und aliphatischen Kohlenwasserstoffen mit einem Siedebereich von 180 bis 220°C und/oder Spindeöl und/oder Monochlornaphthalin, vorzugsweise α-Monochlornaphthalin, verwendet.

Die organischen schwerflüchtigen öligen oder ölartigen Lösungsmittel mit einer Verdunstungszahl über 35 und einem Flammpunkt oberhalb 30°C, vorzugsweise oberhalb 45°C, können teilweise durch leicht oder mittelflüchtige organisch-chemische Lösungsmittel ersetzt werden, mit der Maßgabe, dass das Lösungsmittelgemisch ebenfalls eine Verdunstungszahl über 35 und einen Flammpunkt oberhalb 30°C, vorzugsweise oberhalb 45°C, aufweist und dass das Insektizid-Fungizid-Gemisch in diesem Lösungsmittelgemisch löslich oder emulgierbar ist.

Nach einer bevorzugten Ausführungsform wird ein Teil des organisch-chemischen Lösungsmittel oder Lösungsmittelgemisches oder ein aliphatisches polares organisch-chemisches Lösungsmittel oder Lösungsmittelgemisch ersetzt. Vorzugsweise gelangen Hydroxyl- und/oder Ester- und/oder Ethergruppen enthaltende aliphatische organisch-chemische Lösungsmittel wie beispielsweise Glycolether, Ester oder dgl. zur Anwendung.

Als organisch-chemische Bindemittel werden im Rahmen der vorliegenden Erfindung die an sich bekannten wasserverdünnbaren und/oder in den eingesetzten organisch-chemischen Lösungsmitteln löslichen oder dispergier- bzw. emulgierbaren Kunstharze und/oder bindende trocknende Öle, insbesondere Bindemittel bestehend aus oder enthaltend ein Acrylatharz, ein Vinylharz, z.B. Polyvinylacetat, Polyesterharz, Polykondensations- oder Polyadditionsharz, Polyurethanharz, Alkydharz bzw. modifiziertes Alkydharz, Phenolharz, Kohlenwasserstoffharz wie Inden-Cumaronharz, Siliconharz, trocknende pflanzliche und/oder trocknende Öle und/oder physikalisch trocknende Bindemittel auf der Basis eines Natur- und/oder Kunstharzes verwendet.

Das als Bindemittel verwendete Kunstharz kann in Form einer Emulsion, Dispersion oder Lösung, eingesetzt werden. Als Bindemittel können auch Bitumen oder bituminöse Substanzen bis zu 10 Gew.-%, verwendet werden. Zusätzlich können an sich bekannte Farbstoffe, Pigmente, wasserabweisende Mittel, Geruchskorrigentien und Inhibitoren bzw. Korrosionsschutzmittel und dgl. eingesetzt werden.

Bevorzugt ist gemäß der Erfindung als organisch-chemische Bindemittel mindestens ein Alkydharz bzw. modifiziertes Alkydharz und/oder ein trocknendes pflanzliches Öl im Mittel oder im Konzentrat enthalten. Bevorzugt werden gemäß der Erfindung Alkydharze mit einem Ölgehalt von mehr als 45 Gew.-%, vorzugsweise 50 bis 68 Gew.-%, verwendet.

Das erwähnte Bindemittel kann ganz oder teilweise durch ein Fixierungsmittel(gemisch) oder ein Weichmacher(gemisch) ersetzt werden. Diese Zusätze sollen einer Verflüchtigung der Wirkstoffe sowie einer Kristallisation bzw. Ausfällem vorbeugen. Vorzugsweise ersetzen sie 0,01 bis 30 % des Bindemittels (bezogen auf 100 % des eingesetzten Bindemittels).

Die Weichmacher stammen aus den chemischen Klassen der Phthalsäureester wie Dibutyl-, Dioctyl- oder Benzylbutylphthalat, Phosphorsäureester wie Tributylphosphat, Adipinsäureester wie Di-(2-ethylhexyl)-adipat, Stearate wie Butylstearat oder Amylstearat, Oleate wie Butyloleat, Glycerinether oder höhermolekulare Glykolether, Glycerinester sowie p-Toluolsulfonsäureester.

Fixierungsmittel basieren chemisch auf Polyvinylalkylethern wie z.B. Polyvinylmethylether oder Ketonen wie Benzophenon, Ethylenbenzophenon.

Als Lösungs- bzw. Verdünnungsmittel kommt insbesondere auch Wasser in Frage, gegebenenfalls in Mischung mit einem oder mehreren der oben genannten organisch-chemischen Lösungs- bzw. Verdünnungsmittel, Emulgatoren und Dispergatoren.

Ein besonders effektiver Holzschutz wird durch großtechnische Imprägnierverfahren, z.B. Vakuum, Doppelvakuum oder Druckverfahren, erzielt.

Die anwendungsfertigen Mittel können gegebenenfalls noch weitere Insektizide und gegebenenfalls noch ein oder mehrere Fungizide enthalten.

Als zusätzliche Zumischpartner kommen vorzugsweise die in der WO 94/29 268 genannten Insektizide und Fungizide in Frage. Die in diesem Dokument genannten Verbindungen sind ausdrücklicher Bestandteil der vorliegenden Anmeldung.

Als ganz besonders bevorzugte Zumischpartner können Insektizide, wie Chlorpyriphos, Phoxim, Silafluofin, Alphamethrin, Cyfluthrin, Cypermethrin, Deltamethrin, Permethrin, Imidacloprid, NI-25, Flufenoxuron, Hexaflumuron, Transfluthrin, Thiacloprid, Methoxyphenoxid und Triflumuron,

sowie Fungizide wie Epoxyconazole, Hexaconazole, Azaconazole, Propiconazole, Tebuconazole, Cyproconazole, Metconazole, Imazalil, Dichlorfluanid, Tolylfluanid, 3-Iod-2-propinyl-butylcarbamat, N-Octyl-isothiazolin-3-on und 4,5-Dichlor-N-octylisothiazolin-3-on, sein.

Zugleich können die erfindungsgemäßen Verbindungen bzw. Wirkstoffkombinationen zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, eingesetzt werden.

Bewuchs durch sessile Oligochaeten, wie Kalkröhrenwürmer sowie durch Muscheln und Arten der Gruppe Ledamorpha (Entenmuscheln), wie verschiedene Lepas- und Scalpellum-Arten, oder durch Arten der Gruppe Balanomorpha (Seepocken), wie Balanus- oder Pollicipes-Species, erhöht den Reibungswiderstand von Schiffen und führt in der Folge durch erhöhten Energieverbrauch und darüber hinaus durch häufige Trockendockaufenthalte zu einer deutlichen Steigerung der Betriebskosten.

Neben dem Bewuchs durch Algen, beispielsweise Ectocarpus sp. und Ceramium sp., kommt insbesondere dem Bewuchs durch sessile Entomostraken-Gruppen, welche unter dem Namen Cirripedia (Rankenflusskrebse) zusammengefasst werden, besondere Bedeutung zu.

Es wurde nun überraschenderweise gefunden, dass die erfindungsgemäßen Verbindungen allein oder in Kombination mit anderen Wirkstoffen, eine hervorragende Antifouling (Antibewuchs)-Wirkung aufweisen.

Durch Einsatz von erfindungsgemäßen Verbindungen allein oder in Kombination mit anderen Wirkstoffen, kann auf den Einsatz von Schwermetallen wie z.B. in Bis(trialkylzinn)-sulfiden, Tri-n-butylzinnlaurat, Tri-n-butylzinnchlorid, Kupfer(I)-oxid, Triethylzinnchlorid, Tri-n-butyl(2-phenyl-4-chlorphenoxy)-zinn, Tributylzinnoxid, Molybdändisulfid, Antimonoxid, polymerem Butyltitanat, Phenyl-(bispyridin)-wismutchlorid, Tri-n-butylzinnfluorid, Manganethylenbisthiocarbamat, Zinkdimethyldithiocarbamat, Zinkethylenbisthiocarbamat, Zink- und Kupfersalze von 2-Pyridinthiol-1-oxid, Bisdimethyldithiocarbamoylzinkethylenbisthiocarbamat, Zinkoxid, Kupfer(I)-ethylen-bisdithiocarbamat, Kupferthiocyanat, Kupfernaphthenat und Tributylzinnhalogeniden verzichtet werden oder die Konzentration dieser Verbindungen entscheidend reduziert werden.

Die anwendungsfertigen Antifoulingfarben können gegebenenfalls noch andere Wirkstoffe, vorzugsweise Algizide, Fungizide, Herbizide, Molluskizide bzw. andere Antifouling-Wirkstoffe enthalten.

Als Kombinationspartner für die erfindungsgemäßen Antifouling-Mittel eignen sich vorzugsweise:
Algizide wie
   2-*tert*.-Butylarnino-4-cylopropylarnino-6-methylthio-1,3,5-triazin, Dichlorophen, Diuron, Endothal, Fentinacetat, Isoproturon, Methabenzthiazuron, Oxyfluorfen, Quinoclamine und Terbutryn;
Fungizide wie
   Benzo[*b*]thiophencarbonsäurecyclohexylamid-S,S-dioxid, Dichlofluanid, Fluorfolpet, 3-Iod-2-propinyl-butylcarbamat, Tolylfluanid und Azole wie
   Azaconazole, Cyproconazole, Epoxyconazole, Hexaconazole, Metconazole, Propiconazole und Tebuconazole;
Molluskizide wie
   Fentinacetat, Metaldehyd, Methiocarb, Niclosamid, Thiodicarb und Trimethacarb;
   oder herkömmliche Antifouling-Wirkstoffe wie
   4,5-Dichlor-2-octyl-4-isothiazolin-3-on, Diiodmethylparatrylsulfon, 2-(N,N-Dimethylthiocarbamoylthio)-5-nitrothiazyl, Kalium-, Kupfer-, Natrium- und Zinksalze von 2-Pyridinthiol-1-oxid, Pyridin-triphenylboran, Tetrabutyldistannoxan, 2,3,5,6-Tetrachlor-4-(methylsulfonyl)-pyridin, 2,4,5,6-Tetrachloroisophthalonitril, Tetramethylthiuramdisulfid und 2,4,6-Trichlorphenylmaleinimid.

Die verwendeten Antifouling-Mittel enthalten die erfindungsgemäßen Wirkstoff der erfindungsgemäßen Verbindungen in einer Konzentration von 0,001 bis 50 Gew.-%, insbesondere von 0,01 bis 20 Gew.-%.

Die erfindungsgemäßen Antifouling-Mittel enthalten desweiteren die üblichen Bestandteile wie z.B. in Ungerer, Chem. Ind. 1985, 37, 730-732 und Williams, Antifouling Marine Coatings, Noyes, Park Ridge, 1973 beschrieben.

Antifouling-Anstrichmittel enthalten neben den algiziden, fungiziden, molluskiziden und erfindungsgemäßen insektiziden Wirkstoffen insbesondere Bindemittel.

Beispiele für anerkannte Bindemittel sind Polyvinylchlorid in einem Lösungsmittelsystem, chlorierter Kautschuk in einem Lösungsmittelsystem, Acrylharze in einem Lösungsmittelsystem insbesondere in einem wässrigen System, Vinylchlorid/Vinylacetat-Copolymersysteme in Form wässriger Dispersionen oder in Form von organischen Lösungsmittelsystemen, Butadien/Styrol/Acrylnitril-Kautschuke, trocknende Öle, wie Leinsamenöl, Harzester oder modifizierte Hartharze in Kombination mit Teer oder Bitumina, Asphalt sowie Epoxyverbindungen, geringe Mengen Chlorkautschuk, chloriertes Polypropylen und Vinylharze.

Gegebenenfalls enthalten Anstrichmittel auch anorganische Pigmente, organische Pigmente oder Farbstoffe, welche vorzugsweise in Seewasser unlöslich sind. Ferner können Anstrichmittel Materialien, wie Kolophonium enthalten, um eine gesteuerte Freisetzung der Wirkstoffe zu ermöglichen. Die Anstriche können ferner Weichmacher, die rheologischen Eigenschaften beeinflussende Modifizierungsmittel sowie andere herkömmliche Bestandteile enthalten. Auch in Self-Polishing-Antifouling-Systemen können die erfindungsgemäßen Verbindungen oder die oben genannten Mischungen eingearbeitet werden.

Die Wirkstoffe bzw. Wirkstoffkombinationen eignen sich auch zur Bekämpfung von tierischen Schädlingen, insbesondere von Insekten, Spinnentieren und Milben, die in geschlossenen Räumen, wie beispielsweise Wohnungen, Fabrikhallen, Büros, Fahrzeugkabinen u.ä. vorkommen. Sie können zur Bekämpfung dieser Schädlinge allein oder in Kombination mit anderen Wirk- und Hilfsstoffen in Haushaltsinsektizid-Produkten verwendet werden. Sie sind gegen sensible und resistente Arten sowie gegen alle Entwicklungsstadien wirksam. Zu diesen Schädlingen gehören:
Aus der Ordnung der Scorpionidea z.B. Buthus occitanus.

Aus der Ordnung der Acarina z.B. Argas persicus, Argas reflexus, Bryobia ssp., Dermanyssus gallinae, Glyciphagus domesticus, Ornithodorus moubat, Rhipicephalus sanguineus, Trombicula alfreddugesi, Neutrombicula autumnalis, Dermatophagoides pteronissimus, Dermatophagoides forinae.

Aus der Ordnung der Araneae z.B. Aviculariidae, Araneidae.

Aus der Ordnung der Opiliones z.B. Pseudoscorpiones chelifer, Pseudoscorpiones cheiridium, Opiliones phalangium.

Aus der Ordnung der Isopoda z.B. Oniscus asellus, Porcellio scaber.

Aus der Ordnung der Diplopoda z.B. Blaniulus guttulatus, Polydesmus spp..

Aus der Ordnung der Chilopoda z.B. Geophilus spp..

Aus der Ordnung der Zygentoma z.B. Ctenolepisma spp., Lepisma saccharina, Lepismodes inquilinus.

Aus der Ordnung der Blattaria z.B. Blatta orientalies, Blattella germanica, Blattella asahinai, Leucophaea maderae, Panchlora spp., Parcoblatta spp., Periplaneta australasiae, Periplaneta americana, Periplaneta brunnea, Periplaneta fuliginosa, Supella longipalpa.

Aus der Ordnung der Saltatoria z.B. Acheta domesticus. Aus der Ordnung der Dermaptera z.B. Forficula auricularia. Aus der Ordnung der Isoptera z.B. Kalotermes spp., Reticulitermes spp.

Aus der Ordnung der Psocoptera z.B. Lepinatus spp., Liposcelis spp.

Aus der Ordnung der Coleptera z.B. Anthrenus spp., Attagenus spp., Dermestes spp., Latheticus oryzae, Necrobia spp., Ptinus spp., Rhizopertha dominica, Sitophilus granarius, Sitophilus oryzae, Sitophilus zeamais, Stegobium paniceum.

Aus der Ordnung der Diptera z.B. Aedes aegypti, Aedes albopictus, Aedes taeniorhynchus, Anopheles spp., Calliphora erythrocephala, Chrysozona pluvialis, Culex quinquefasciatus, Culex pipiens, Culex tarsalis, Drosophila spp., Fannia canicularis, Musca domestica, Phlebotomus spp., Sarcophaga carnaria, Simulium spp., Stomoxys calcitrans, Tipula paludosa.

Aus der Ordnung der Lepidoptera z.B. Achroia grisella, Galleria mellonella, Plodia interpunctella, Tinea cloacella, Tinea pellionella, Tineola bisselliella.

Aus der Ordnung der Siphonaptera z.B. Ctenocephalides canis, Ctenocephalides felis, Pulex irritans, Tunga penetrans, Xenopsylla cheopis.

Aus der Ordnung der Hymenoptera z.B. Camponotus herculeanus, Lasius fuliginosus, Lasius niger, Lasius umbratus, Monomorium pharaonis, Paravespula spp., Tetramorium caespitum.

Aus der Ordnung der Anoplura z.B. Pediculus humanus capitis, Pediculus humanus corporis, Phthirus pubis.

Aus der Ordnung der Heteroptera z.B. Cimex hemipterus, Cimex lectularius, Rhodinus prolixus, Triatoma infestans.

Die Anwendung im Bereich der Haushaltsinsektizide erfolgt allein oder in Kombination mit anderen geeigneten Wirkstoffen wie Phosphorsäureestern, Carbamaten, Pyrethroiden, Wachstumsregulatoren oder Wirkstoffen aus anderen bekannten Insektizidklassen.

Die Anwendung erfolgt in Aerosolen, drucklosen Sprühmitteln, z.B. Pump- und Zerstäubersprays, Nebelautomaten, Foggern, Schäumen, Gelen, Verdampferprodukten mit Verdampferplättchen aus Cellulose oder Kunststoff, Flüssigverdampfern, Gel- und Membranverdampfern, propellergetriebenen Verdampfern, energielosen bzw. passiven Verdampfungssystemen, Mottenpapieren, Mottensäckchen und Mottengelen, als Granulate oder Stäube, in Streuködern oder Köderstationen.

Die erfindungsgemäßen Wirkstoffe bzw. Wirkstoffkombinationen können auch als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe bzw. Wirkstoffkombinationen können z.B. bei den folgenden Pflanzen verwendet werden:
Dikotyle Unkräuter der Gattungen: Abutilon, Amaranthus, Ambrosia, Anoda, Anthemis, Aphanes, Atriplex, Bellis, Bidens, Capsella, Carduus, Cassia, Centaurea, Chenopodium, Cirsium, Convolvulus, Datura, Desmodium, Emex, Erysimum, Euphorbia, Galeopsis, Galinsoga, Galium, Hibiscus, Ipomoea, Kochia, Lamium, Lepidium, Lindernia, Matricaria, Mentha, Mercurialis, Mullugo, Myosotis, Papaver, Pharbitis, Plantago, Polygonum, Portulaca, Ranunculus, Raphanus, Rorippa, Rotala, Rumex, Salsola, Senecio, Sesbania, Sida, Sinapis, Solanum, Sonchus, Sphenoclea, Stellaria, Taraxacum, Thlaspi, Trifolium, Urtica, Veronica, Viola, Xanthium.
Dikotyle Kulturen der Gattungen: Arachis, Beta, Brassica, Cucumis, Cucurbita, Helianthus, Daucus, Glycine, Gossypium, Ipomoea, Lactuca, Linum, Lycopersicon, Nicotiana, Phaseolus, Pisum, Solanum, Vicia.
Monokotyle Unkräuter der Gattungen: Aegilops, Agropyron, Agrostis, Alopecurus, Apera, Avena, Brachiaria, Bromus, Cenchrus, Commelina, Cynodon, Cyperus, Dactyloctenium, Digitaria, Echinochloa, Eleocharis, Eleusine, Eragrostis, Eriochloa, Festuca, Fimbristylis, Heteranthera, Imperata, Ischaemum, Leptochloa, Lolium, Monochoria, Panicum, Paspalum, Phalaris, Phleum, Poa, Rottboellia, Sagittaria, Scirpus, Setaria, Sorghum.
Monokotyle Kulturen der Gattungen: Allium, Ananas, Asparagus, Avena, Hordeum, Oryza, Panicum, Saccharum, Secale, Sorghum, Triticale, Triticum, Zea.

Die Verwendung der erfindungsgemäßen Wirkstoffe bzw. Wirkstoffkombinationen ist jedoch keineswegs auf diese Gattungen beschränkt, sondern erstreckt sich in gleicher Weise auch auf andere Pflanzen.

Die erfindungsgemäßen Wirkstoffe bzw. Wirkstoffkombinationen eignen sich in Abhängigkeit von der Konzentration zur Totalunkrautbekämpfung, z.B. auf Industrie- und Gleisanlagen und auf Wegen und Plätzen mit und ohne Baumbewuchs. Ebenso können die erfindungsgemäßen Wirkstoffe zur Unkrautbekämpfung in Dauerkulturen, z.B. Forst, Ziergehölz-, Obst-, Wein-, Citrus-, Nuss-, Bananen-, Kaffee-, Tee-, Gummi-, Ölpalm-, Kakao-, Beerenfrucht- und Hopfenanlagen, auf Zier- und Sportrasen und Weideflächen sowie zur selektiven Unkrautbekämpfung in einjährigen Kulturen eingesetzt werden.

Die erfindungsgemäßen Verbindungen bzw. Wirkstoffkombinationen zeigen starke herbizide Wirksamkeit und ein breites Wirkungsspektrum bei Anwendung auf dem Boden und auf oberirdische Pflanzenteile. Sie eignen sich in gewissem Umfang auch zur selektiven Bekämpfung von monokotylen und dikotylen Unkräutern in monokotylen und dikotylen Kulturen, sowohl im Vorauflauf- als auch im Nachauflauf-Verfahren.

Die erfindungsgemäßen Wirkstoffe bzw. Wirkstoffkombinationen können in bestimmten Konzentrationen bzw. Aufwandmengen auch zur Bekämpfung von tierischen Schädlingen und pilzlichen oder bakteriellen Pflanzenkrankheiten verwendet werden. Sie lassen sich gegebenenfalls auch als Zwischen- oder Vorprodukte für die Synthese weiterer Wirkstoffe einsetzen.

Die Wirkstoffe bzw. Wirkstoffkombinationen können in die üblichen Formulierungen übergeführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage: z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im Allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden und/oder mit Stoffen, welche die Kulturpflanzen-Verträglichkeit verbessern ("Safenern") zur Unkrautbekämpfung verwendet werden, wobei Fertigformulierungen oder Tankmischungen möglich sind. Es sind also auch Mischungen mit Unkrautbekämpfungsmitteln möglich, welche ein oder mehrere bekannte Herbizide und einen Safener enthalten.

Für die Mischungen kommen bekannte Herbizide infrage, beispielsweise
Acetochlor, Acifluorfen (-sodium), Aclonifen, Alachlor, Alloxydim (-sodium), Ametryne, Amicarbazone, Amidochlor, Amidosulfuron, Aminopyralid, Anilofos, Asulam, Atrazine, Azafenidin, Azimsulfuron, Beflubutamid, Benazolin (-ethyl), Benzcarbazone, Benfuresate, Bensulfuron (-methyl), Bentazon, Benzfendizone, Benzobicyclon, Benzofenap, Benzoylprop (-ethyl), Bialaphos, Bifenox, Bispyribac (-sodium), Bromobutide, Bromofenoxim, Bromoxynil, Butachlor, Butafenacil (-allyl), Butroxydim, Butylate, Cafenstrole, Caloxydim, Carbetamide, Carfentrazone (-ethyl), Chlomethoxyfen, Chloramben, Chloridazon, Chlorimuron (-ethyl), Chlornitrofen, Chlorsulfuron, Chlortoluron, Cinidon (-ethyl), Cinmethylin, Cinosulfuron, Clefoxydim, Clethodim, Clodinafop (-propargyl), Clomazone, Clomeprop, Clopyralid, Clopyrasulfuron (-methyl), Cloransulam (-methyl), Cumyluron, Cyanazine, Cybutryne, Cycloate, Cyclosulfamuron, Cycloxydim, Cyhalofop (-butyl), 2,4-D, 2,4-DB, Desmedipham, Diallate, Dicamba, Dichlorprop (-P), Diclofop (-methyl), Diclosulam, Diethatyl (-ethyl), Difenzoquat, Diflufenican, Diflufenzopyr, Dimefuron, Dimepiperate, Dimethachlor, Dimethametryn, Dimethenamid, Dimexyflam, Dinitramine, Diphenamid, Diquat, Dithiopyr, Diuron, Dymron, Epropodan, EPTC, Esprocarb, Ethalfluralin, Ethametsulfuron (-methyl), Ethofumesate, Ethoxyfen, Ethoxysulfuron, Etobenzanid, Fenoxaprop (-P-ethyl), Fentrazamide, Flamprop (-isopropyl, -isopropyl-L, -methyl), Flazasulfuron, Florasulam, Fluazifop (-P-butyl), Fluazolate, Flucarbazone (-sodium), Flufenacet, Flumetsulam, Flumiclorac (-pentyl), Flumioxazin, Flumipropyn, Flumetsulam, Fluometuron, Fluorochloridone, Fluoroglycofen (-ethyl), Flupoxam, Flupropacil, Flurpyrsulfuron (-methyl, -sodium), Flurenol (-butyl), Fluridone, Fluroxypyr (-butoxypropyl, -meptyl), Flurprimidol, Flurtamone, Fluthiacet (-methyl), Fluthiamide, Fomesafen, Foramsulfuron, Glufosinate (-ammonium), Glyphosate (-isopropylammonium), Halosafen, Haloxyfop (-ethoxyethyl, -P-methyl), Hexazinone, HOK-201, Imazamethabenz (-methyl), Imazamethapyr, Imazamox, Imazapic, Imazapyr, Imazaquin, Imazethapyr, Imazosulfuron, Iodosulfuron (-methyl, -sodium), Ioxynil, Isopropalin, Isoproturon, Isouron, Isoxaben, Isoxachlortole, Isoxaflutole, Isoxapyrifop, KIH 485, Lactofen, Lenacil, Linuron, MCPA, Mecoprop, Mefenacet, Mesosulfurone, Mesotrione, Metamifop, Metamitron, Metazachlor, Methabenzthiazuron, Metobenzuron, Metobromuron, (alpha-) Metolachlor, Metosulam, Metoxuron, Metribuzin, Metsulfuron (-methyl), Molinate, Monolinuron, Naproanilide, Napropamide, Neburon, Nicosulfuron, Norflurazon, Orbencarb, Orthosulfamuron, Oryzalin, Oxadiargyl, Oxadiazon, Oxasulfuron, Oxaziclomefone, Oxyfluorfen, Paraquat, Pelargonsäure, Pendimethalin, Pendralin, Penoxsulam, Pentoxazone, Phenmedipham, Picolinafen, Pinoxaden, Piperophos, Pretilachlor, Primisulfuron (-methyl), Profluazol, Prometryn, Propachlor, Propanil, Propaquizafop, Propisochlor, Propoxycarbazone (-sodium), Propyzamide, Prosulfocarb, Prosulfuron, Pyraflufen (-ethyl), Pyrazogyl, Pyrazolate, Pyrazosulfuron (-ethyl), Pyrazoxyfen, Pyribenzoxim, Pyributicarb, Pyridate, Pyridatol, Pyriftalid, Pyriminobac (-methyl), Pyrithiobac (-sodium), Pyrimisulfan, Quinchlorac, Quinmerac, Quinoclamine, Quizalofop (-P-ethyl, -P-tefuryl), Rimsulfuron, Sethoxydim, Simazine, Simetryn, Sulcotrione, Sulfentrazone, Sulfometuron (-methyl), Sulfosate, Sulfosulfuron, Tebutam, Tebuthiuron, Tepraloxydim, Terbuthylazine, Terbutryn, Thenylchlor, Thiafluamide, Thiazopyr, Thidiazimin, Thifensulfuron (-methyl), Thiobencarb, Tiocarbazil, Topramezone, Tralkoxydim, Triallate, Triasulfuron, Tribenuron (-methyl), Triclopyr, Tridiphane, Trifluralin, Trifloxysulfuron, Triflusulfuron (-methyl), Tritosulfuron und

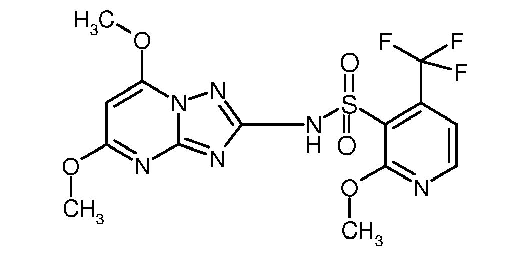

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe bzw. Wirkstoffkombinationen können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe bzw. Wirkstoffkombinationen können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im Allgemeinen liegen die Aufwandmengen zwischen 1 g und 10 kg Wirkstoff pro Hektar Bodenfläche, vorzugsweise zwischen 5 g und 5 kg pro ha.

Der vorteilhafte Effekt der Kulturpflanzen-Verträglichkeit der erfindungsgemäßen Wirkstoffkombinationen ist bei bestimmten Konzentrationsverhältnissen besonders stark ausgeprägt. Jedoch können die Gewichtsverhältnisse der Wirkstoffe in den Wirkstoffkombinationen in relativ großen Bereichen variiert werden. Im allgemeinen entfallen auf 1 Gewichtsteil Wirkstoff der Formel (I) Salzen 0,001 bis 1000 Gewichtsteile, vorzugsweise 0,01 bis 100 Gewichtsteile, besonders bevorzugt 0,05 bis 20 Gewichtsteile einer der oben unter (b') genannten, die Kulturpflanzen Verträglichkeit verbessernden Verbindungen (Antidots/Safener).

Die erfindungsgemäßen Wirkstoffkombinationen werden im allgemeinen in Form von Fertigformulierungen zur Anwendung gebracht. Die in den Wirkstoffkombinationen enthaltenen Wirkstoffe können aber auch in Einzelformulierungen bei der Anwendung gemischt, d.h. in Form von Tankmischungen zur Anwendung gebracht werden.

Für bestimmte Anwendungszwecke, insbesondere im Nachauflauf-Verfahren, kann es ferner vorteilhaft sein, in die Formulierungen als weitere Zusatzstoffe pflanzenverträgliche mineralische oder vegetabilische Öle (z.B. das Handelspräparat "Rako Binol") oder Ammoniumsalze wie z.B. Ammoniumsulfat oder Ammoniumrhodanid aufzunehmen.

Die neuen Wirkstoffkombinationen können als solche, in Form ihrer Formulierungen oder der daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Stäuben oder Streuen.

Die Aufwandmengen der erfindungsgemäßen Wirkstoffkombinationen können in einem gewissen Bereich variiert werden; sie hängen u.a. vom Wetter und von den Bodenfaktoren ab. Im allgemeinen liegen die Aufwandmengen zwischen 0,001 und 5 kg pro ha, vorzugsweise zwischen 0,005 und 2 kg pro ha, besonders bevorzugt zwischen 0,01 und 0,5 kg pro ha.

Die erfindungsgemäßen Wirkstoffkombinationen können vor und nach dem Auflaufen der Pflanzen appliziert werden, also im Vorauflauf und Nachauflauf-Verfahren.

Die erfindungsgemäß einzusetzenden Safener können je nach ihren Eigenschaften zur Vorbehandlung des Saatgutes der Kulturpflanze (Beizung der Samen) verwendet werden oder vor der Saat in die Saatfurchen eingebracht oder vor dem Herbizid separat angewendet werden oder zusammen mit dem Herbizid vor oder nach dem Ablaufen der Pflanzen angewendet werden.

Als Beispiele der Pflanzen werden die wichtigen Kulturpflanzen, wie Getreide (Weizen, Gerste, Reis), Mais, Soja, Kartoffel, Baumwolle, Raps, Rüben, Zuckerrohr sowie Obstpflanzen (mit den Früchten Äpfel, Birnen, Zitrusfrüchten und Weintrauben) erwähnt, wobei Getreide, Mais, Soja, Kartoffel, Baumwolle und Raps besonders hervorgehoben werden.

Fungizide lassen sich im Pflanzenschutz zur Bekämpfung von Plasmodiophoromycetes, Oomycetes, Chytridiomycetes, Zygomycetes, Ascomycetes, Basidiomycetes und Deuteromycetes einsetzen.

Bakterizide lassen sich im Pflanzenschutz zur Bekämpfung von Pseudomonadaceae, Rhizobiaceae, Enterobacteriaceae, Corynebacteriaceae und Streptomycetaceae einsetzen.

Beispielhaft aber nicht begrenzend seien einige Erreger von pilzlichen und bakteriellen Erkrankungen, die unter die oben aufgezählten Oberbegriffe fallen, genannt:
Xanthomonas-Arten, wie beispielsweise Xanthomonas campestris pv. oryzae;
Pseudomonas-Arten, wie beispielsweise Pseudomonas syringae pv. lachrymans;
Erwinia-Arten, wie beispielsweise Erwinia amylovora;
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder
Pseudoperonospora cubensis;
Plasmopara-Arten, wie beispielsweise Plasmopara viticola;
Bremia-Arten, wie beispielsweise Bremia lactucae;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea
(Konidienform: Drechslera, Syn: Helminthosporium);
Cochliobolus-Arten, wie beispielsweise Cochliobolus sativus
(Konidienform: Drechslera, Syn: Helminthosporium);
Uromyces-Arten, wie beispielsweise Uromyces appendiculatus;
Puccinia-Arten, wie beispielsweise Puccinia recondita;
Sclerotinia-Arten, wie beispielsweise Sclerotinia sclerotiorum;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die erfindungsgemäßen Wirkstoffe weisen auch eine starke stärkende Wirkung in Pflanzen auf. Sie eignen sich daher zur Mobilisierung pflanzeneigener Abwehrkräfte gegen Befall durch unerwünschte Mikroorganismen.

Unter pflanzenstärkenden (resistenzinduzierenden) Stoffen sind im vorliegenden Zusammenhang solche Substanzen zu verstehen, die in der Lage sind, das Abwehrsystem von Pflanzen so zu stimulieren, dass die behandelten Pflanzen bei nachfolgender Inokolation mit unerwünschten Mikroorgansimen weitgehende Resistenz gegen diese Mirkroorganismen entfalten.

Unter unerwünschten Mikroorganismen sind im vorliegenden Fall phytopathogene Pilze, Bakterien und Viren zu verstehen. Die erfindungsgemäßen Stoffe können also eingesetzt werden, um Pflanzen innerhalb eines gewissen Zeitraumes nach der Behandlung gegen den Befall durch die genannten Schaderreger zu schützen. Der Zeitraum, innerhalb dessen Schutz herbeigeführt wird, erstreckt sich im allgemeinen von 1 bis 10 Tage, vorzugsweise 1 bis 7 Tage nach der Behandlung der Pflanzen mit den Wirkstoffen.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Die erfindungsgemäßen Wirkstoffe eignen sich auch zur Steigerung des Ernteertrages. Sie sind außerdem mindertoxisch und weisen eine gute Pflanzenverträglichkeit auf.

Die erfindungsgemäßen Wirkstoffe können gegebenenfalls in bestimmten Konzentrationen und Aufwandmengen auch als Herbizide, zur Beeinflussung des Pflanzenwachstums, sowie zur Bekämpfung von tierischen Schädlingen verwendet werden. Sie lassen sich gegebenenfalls auch als Zwischen- und Vorprodukte für die Synthese weiterer Wirkstoffe einsetzen.

Im Materialschutz lassen sich die erfindungsgemäßen Stoffe zum Schutz von technischen Materialien gegen Befall und Zerstörung durch unerwünschte Mikroorganismen einsetzen.

Unter technischen Materialien sind im vorliegenden Zusammenhang nichtlebende Materialien zu verstehen, die für die Verwendung in der Technik zubereitet worden sind. Beispielsweise können technische Materialien, die durch erfindungsgemäße Wirkstoffe vor mikrobieller Veränderung oder Zerstörung geschützt werden sollen, Klebstoffe, Leime, Papier und Karton, Textilien, Leder, Holz, Anstrichmittel und Kunststoffartikel, Kühlschmierstoffe und andere Materialien sein, die von Mikroorganismen befallen oder zersetzt werden können. Im Rahmen der zu schützenden Materialien seien auch Teile von Produktionsanlagen, beispielsweise Kühlwasserkreisläufe, genannt, die durch Vermehrung von Mikroorganismen beeinträchtigt werden können. Im Rahmen der vorliegenden Erfindung seien als technische Materialien vorzugsweise Klebstoffe, Leime, Papiere und Kartone, Leder, Holz, Anstrichmittel, Kühlschmiermittel und Wärmeübertragungsflüssigkeiten genannt, besonders bevorzugt Holz.

Als Mikroorganismen, die einen Abbau oder eine Veränderung der technischen Materialien bewirken können, seien beispielsweise Bakterien, Pilze, Hefen, Algen und Schleimorganismen genannt. Vorzugsweise wirken die erfindungsgemäßen Wirkstoffe gegen Pilze, insbesondere Schimmelpilze, holzverfärbende und holzzerstörende Pilze (Basidiomyceten) sowie gegen Schleimorganismen und Algen.

Es seien beispielsweise Mikroorganismen der folgenden Gattungen genannt:
Alternaria, wie Alternaria tenuis,
Aspergillus, wie Aspergillus niger,
Chaetomium, wie Chaetomium globosum,
Coniophora, wie Coniophora puetana,
Lentinus, wie Lentinus tigrinus,
Penicillium, wie Penicillium glaucum,
Polyporus, wie Polyporus versicolor,
Aureobasidium, wie Aureobasidium pullulans,
Sclerophoma, wie Sclerophoma pityophila,
Trichoderma, wie Trichoderma viride,
Escherichia, wie Escherichia coli,
Pseudomonas, wie Pseudomonas aeruginosa,
Staphylococcus, wie Staphylococcus aureus.

Die Wirkstoffe können in Abhängigkeit von ihren jeweiligen physikalischen und/oder chemischen Eigenschaften in die üblichen Formulierungen überführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycolether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Fungiziden, Bakteriziden, Akariziden, Nematiziden oder Insektiziden verwendet werden, um so z.B. das Wirkungsspektrum zu verbreitern oder Resistenzentwicklungen vorzubeugen. In vielen Fällen erhält man dabei synergistische Effekte, d.h. die Wirksamkeit der Mischung ist größer als die Wirksamkeit der Einzelkomponenten.

Als Mischpartner kommen zum Beispiel folgende Verbindungen in Frage:

### Fungizide:

2-Phenylphenol; 8-Hydroxyquinoline sulfate; Acibenzolar-S-methyl; Aldimorph; Amidoflumet; Ampropylfos; Ampropylfos-potassium; Andoprim; Anilazine; Azaconazole; Azoxystrobin; Benalaxyl; Benodanil; Benomyl; Benthiavalicarb-isopropyl; Benzamacril; Benzamacril-isobutyl; Bilanafos; Binapacryl; Biphenyl; Bitertanol; Blasticidin-S; Bromuconazole; Bupirimate; Buthiobate; Butylamine; Calcium polysulfide; Capsimycin; Captafol; Captan; Carbendazim; Carboxin; Carpropamid; Carvone; Chinomethionat; Chlobenthiazone; Chlorfenazole; Chloroneb; Chlorothalonil; Chlozolinate; Clozylacon; Cyazofamid; Cyflufenamid; Cymoxanil; Cyproconazole; Cyprodinil; Cyprofuram; Dagger G; Debacarb; Dichlofluanid; Dichlone; Dichlorophen; Diclocymet; Diclomezine; Dicloran; Diethofencarb; Difenoconazole; Diflumetorim; Dimethirimol; Dimethomorph; Dimoxystrobin; Diniconazole; Diniconazole-M; Dinocap; Diphenylamine; Dipyrithione; Ditalimfos; Dithianon; Dodine; Drazoxolon; Edifenphos; Epoxiconazole; Ethaboxam; Ethirimol; Etridiazole; Famoxadone; Fenamidone; Fenapanil; Fenarimol; Fenbuconazole; Fenfuram; Fenhexamid; Fenitropan; Fenoxanil; Fenpiclonil; Fenpropidin; Fenpropimorph; Ferbam; Fluazinam; Flubenzimine; Fludioxonil; Flumetover; Flumorph; Fluoromide; Fluoxastrobin; Fluquinconazole; Flurprimidol; Flusilazole; Flusulfamide; Flutolanil; Flutriafol; Folpet; Fosetyl-Al; Fosetyl-sodium; Fuberidazole; Furalaxyl; Furametpyr; Furcarbanil; Furmecyclox; Guazatine; Hexachlorobenzene; Hexaconazole; Hymexazol; Imazalil; Imibenconazole; Iminoctadine triacetate; Iminoctadine tris(albesil; Iodocarb; Ipconazole; Iprobenfos; Iprodione; Iprovalicarb; Irumamycin; Isoprothiolane; Isovaledione; Kasugamycin; Kresoxim-methyl; Mancozeb; Maneb; Meferimzone; Mepanipyrim; Mepronil; Metalaxyl; Metalaxyl-M; Metconazole; Methasulfocarb; Methfuroxam; Metiram; Metominostrobin; Metsulfovax; Mildiomycin; Myclobutanil; Myclozolin; Natamycin; Nicobifen; Nitrothal-isopropyl; Noviflumuron; Nuarimol; Ofurace; Orysastrobin; Oxadixyl; Oxolinic acid; Oxpoconazole; Oxycarboxin; Oxyfenthiin; Paclobutrazol; Pefurazoate; Penconazole; Pencycuron; Phosdiphen; Phthalide; Picoxystrobin; Piperalin; Polyoxins; Polyoxorim; Probenazole; Prochloraz; Procymidone; Propamocarb; Propanosine-sodium; Propiconazole; Propineb; Proquinazid; Prothioconazole; Pyraclostrobin; Pyrazophos; Pyrifenox; Pyrimethanil; Pyroquilon; Pyroxyfur; Pyrrolnitrine; Quinconazole; Quinoxyfen; Quintozene; Simeconazole; Spiroxamine; Sulfur; Tebuconazole; Tecloftalam; Tecnazene; Tetcyclacis; Tetraconazole; Thiabendazole; Thicyofen; Thifluzamide; Thiophanate-methyl; Thiram; Tioxymid; Tolclofos-methyl; Tolylfluanid; Triadimefon; Triadimenol; Triazbutil; Triazoxide; Tricyclamide; Tricyclazole; Tridemorph; Trifloxystrobin; Triflumizole; Triforine; Triticonazole; Uniconazole; Validamycin A; Vinclozolin; Zineb; Ziram; Zoxamide; (2S)-N-[2-[4-[[3-(4-chlorophenyl)-2-propynyl]oxy]-3-methoxyphenyl]ethyl]-3-methyl- 2-[(methylsulfonyl)amino]-butanamide; 1-(1-naphthalenyl)-1H-pyrrole-2,5-dione; 2,3,5,6-tetrachloro-4-(methylsulfonyl)-pyridine; 2-amino-4-methyl-N-phenyl-5-thiazolecarboxamide; 2-chloro-N-(2,3-dihydro-1,1,3-trimethyl-1H-inden-4-yl)-3-pyridincarboxam ide; 3,4,5-trichloro-2,6-pyridinedicarbonitrile; Actinovate; cis-1-(4-chlorophenyl)-2-(1H-1,2,4-triazole-1-yl)-cycloheptanol; methyl 1-(2,3-dihydro-2,2-dimethyl-1H-inden-1-yl)-1H-imidazole-5-carboxylate; monopotassium carbonate; N-(6-methoxy-3-pyridinyl)-cyclopropanecarboxamide; N-butyl-8-(1,1-dimethylethyl)-1-oxaspiro[4.5]decan-3-amine; Sodium tetrathiocarbonate;
sowie Kupfersalze und -zubereitungen, wie Bordeaux mixture; Copper hydroxide; Copper naphthenate; Copper oxychloride; Copper sulfate; Cufraneb; Cuprous oxide; Mancopper; Oxinecopper.

### Bakterizide:

Bronopol, Dichlorophen, Nitrapyrin, Nickel-dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide:

Abamectin, ABG-9008, Acephate, Acequinocyl, Acetamiprid, Acetoprole, Acrinathrin, AKD-1022, AKD-3059, AKD-3088, Alanycarb, Aldicarb, Aldoxycarb, Allethrin, Allethrin 1R-isomers, Alpha-Cypermethrin (Alphamethrin), Amidoflumet, Aminocarb, Amitraz, Avermectin, AZ-60541, Azadirachtin, Azamethiphos, Azinphos-methyl, Azinphos-ethyl, Azocyclotin,
Bacillus popilliae, Bacillus sphaericus, Bacillus subtilis, Bacillus thuringiensis, Bacillus thuringiensis strain EG-2348, Bacillus thuringiensis strain GC-91, Bacillus thuringiensis strain NCTC-11821, Baculoviren, Beauveria bassiana, Beauveria tenella, Benclothiaz, Bendiocarb, Benfuracarb, Bensultap, Benzoximate, Beta-Cyfluthrin, Beta-Cypermethrin, Bifenazate, Bifenthrin, Binapacryl, Bioallethrin, Bioallethrin-S-cyclopentyl-isomer, Bioethanomethrin, Biopermethrin, Bioresmethrin, Bistrifluron, BPMC, Brofenprox, Bromophos-ethyl, Bromopropylate, Bromfenvinfos (-methyl), BTG-504, BTG-505, Bufencarb, Buprofezin, Butathiofos, Butocarboxim, Butoxycarboxim, Butylpyridaben,
Cadusafos, Camphechlor, Carbaryl, Carbofuran, Carbophenothion, Carbosulfan, Cartap, CGA-50439, Chinomethionat, Chlordane, Chlordimeform, Chloethocarb, Chlorethoxyfos, Chlorfenapyr, Chlorfenvinphos, Chlorfluazuron, Chlormephos, Chlorobenzilate, Chloropicrin, Chlorproxyfen, Chlorpyrifos-methyl, Chlorpyrifos (-ethyl), Chlovaporthrin, Chromafenozide, Cis-Cypermethrin, Cis-Resmethrin, Cis-Permethrin, Clocythrin, Cloethocarb, Clofentezine, Clothianidin, Clothiazoben, Codlemone, Coumaphos, Cyanofenphos, Cyanophos, Cycloprene, Cycloprothrin, Cydia pomonella, Cyfluthrin, Cyhalothrin, Cyhexatin, Cypermethrin, Cyphenothrin (1R-trans-isomer), Cyromazine,
DDT, Deltamethrin, Demeton-S-methyl, Demeton-S-methylsulphon, Diafenthiuron, Dialifos, Diazinon, Dichlofenthion, Dichlorvos, Dicofol, Dicrotophos, Dicyclanil, Diflubenzuron, Dimeflurhrin, Dimethoate, Dimethylvinphos, Dinobuton, Dinocap, Dinotefuran, Diofenolan, Disulfoton, Docusat-sodium, Dofenapyn, DOWCO-439,
Eflusilanate, Emamectin, Emamectin-benzoate, Empenthrin (1R-isomer), Endosulfan, Entomopthora spp., EPN, Esfenvalerate, Ethiofencarb, Ethiprole, Ethion, Ethoprophos, Etofenprox, Etoxazole, Etrimfos,
Famphur, Fenamiphos, Fenazaquin, Fenbutatin oxide, Fenfluthrin, Fenitrothion, Fenobucarb, Fenothiocarb, Fenoxacrim, Fenoxycarb, Fenpropathrin, Fenpyrad, Fenpyrithrin, Fenpyroximate, Fensulfothion, Fenthion, Fentrifanil, Fenvalerate, Fipronil, Flonicamid, Fluacrypyrim, Fluazuron, Flubenzimine, Flubrocythrinate, Flucycloxuron, Flucythrinate, Flufenerim, Flufenoxuron, Flufenprox, Flumethrin, Flupyrazofos, Flutenzin (Flufenzine), Fluvalinate, Fonofos, Formetanate, Formothion, Fosmethilan, Fosthiazate, Fubfenprox (Fluproxyfen), Furathiocarb,
Gamma-cyhalothrin, Gamma-HCH, Gossyplure, Grandlure, Granuloseviren,
Halfenprox, Halofenozide, HCH, HCN-801, Heptenophos, Hexaflumuron, Hexythiazox, Hydramethylnone, Hydroprene,
IKA-2002, Imidacloprid, Imiprothrin, Indoxacarb, Iodofenphos, Iprobenfos, Isazofos, Isofenphos, Isoprocarb, Isoxathion, Ivermectin,
Japonilure,
Kadethrin, Kernpolyederviren, Kinoprene,
Lambda-Cyhalothrin, Lindane, Lufenuron,

Malathion, Mecarbam, Mesulfenfos, Metaldehyd, Metam-sodium, Methacrifos, Methamidophos, Metharhizium anisopliae, Metharhizium flavoviride, Methidathion, Methiocarb, Methomyl, Methoprene, Methoxychlor, Methoxyfenozide, Metofluthrin, Metolcarb, Metoxadiazone, Mevinphos, Milbemectin, Milbemycin, MKI-245, MON-45700, Monocrotophos, Moxidectin, MTI-800,
Naled, NC-104, NC-170, NC-184, NC-194, NC-196, Niclosamide, Nicotine, Nitenpyram, Nithiazine, NNI-0001, NNI-0101, NNI-0250, NNI-9768, Novaluron, Noviflumuron,
OK-5101, OK-5201, OK-9601, OK-9602, OK-9701, OK-9802, Omethoate, Oxamyl, Oxydemetonmethyl,
Paecilomyces fumosoroseus, Parathion-methyl, Parathion (-ethyl), Permethrin (cis-, trans-), Petroleum, PH-6045, Phenothrin (1R-trans isomer), Phenthoate, Phorate, Phosalone, Phosmet, Phosphamidon, Phosphocarb, Phoxim, Piperonyl butoxide, Pirimicarb, Pirimiphos-methyl, Pirimiphos-ethyl, Potassium oleate, Prallethrin, Profenofos, Profluthrin, Promecarb, Propaphos, Propargite, Propetamphos, Propoxur, Prothiofos, Prothoate, Protrifenbute, Pymetrozine, Pyraclofos, Pyresmethrin, Pyrethrum, Pyridaben, Pyridalyl, Pyridaphenthion, Pyridathion, Pyrimidifen, Pyriproxyfen,
Quinalphos,
Resmethrin, RH-5849, Ribavirin, RU-12457, RU-15525,
S-421, S-1833, Salithion, Sebufos, SI-0009, Silafluofen, Spinosad, Spirodiclofen, Spiromesifen, Sulfluramid, Sulfotep, Sulprofos, SZI-121,
Tau-Fluvalinate, Tebufenozide, Tebufenpyrad, Tebupirimfos, Teflubenzuron, Tefluthrin, Temephos, Temivinphos, Terbam, Terbufos, Tetrachlorvinphos, Tetradifon, Tetramethrin, Tetramethrin (1R-isomer), Tetrasul, Theta-Cypermethrin, Thiacloprid, Thiamethoxam, Thiapronil, Thiatriphos, Thiocyclam hydrogen oxalate, Thiodicarb, Thiofanox, Thiometon, Thiosultap-sodium, Thuringiensin, Tolfenpyrad, Tralocythrin, Tralomethrin, Transfluthrin, Triarathene, Triazamate, Triazophos, Triazuron, Trichlophenidine, Trichlorfon, Trichoderma atroviride, Triflumuron, Trimethacarb,
Vamidothion, Vaniliprole, Verbutin, Verticillium lecanii,
WL-108477, WL-40027,
YI-5201, YI-5301, YI-5302,
XMC, Xylylcarb,
ZA-3274, Zeta-Cypermethrin, Zolaprofos, ZXI-8901,
die Verbindung 3-Methyl-phenyl-propylcarbamat (Tsumacide Z),
die Verbindung 3-(5-Chlor-3-pyridinyl)-8-(2,2,2-trifluorethyl)-8-azabicyclo[3.2.1]octan-3-carbonitril (CAS-Reg.-Nr. 185982-80-3) und das entsprechende 3-endo-Isomere (CAS-Reg.-Nr. 185984-60-5) (vgl. WO-96/37494, WO-98/25923),
sowie Präparate, welche insektizid wirksame Pflanzenextrakte, Nematoden, Pilze oder Viren enthalten.

Auch eine Mischung mit anderen bekannten Wirkstoffen, wie Herbiziden oder mit Düngemitteln und Wachstumsregulatoren ist möglich.

Darüber hinaus weisen die erfindungsgemäßen Verbindungen der Formel (I) auch sehr gute antimykotische Wirkungen auf. Sie besitzen ein sehr breites antimykotisches Wirkungsspektrum, insbesondere gegen Dermatophyten und Sproßpilze, Schimmel und diphasische Pilze (z.B. gegen Candida-Spezies wie Candida albicans, Candida glabrata) sowie Epidermophyton floccosum, Aspergillus-Spezies wie Aspergillus niger und Aspergillus fumigatus, Trichophyton-Spezies wie Trichophyton mentagrophytes,

Microsporon-Spezies wie Microsporon canis und audouinii. Die Aufzählung dieser Pilze stellt keinesfalls eine Beschränkung des erfaßbaren mykotischen Spektrums dar, sondern hat nur erläuternden Charakter.

Die Wirkstoffe können als solche, in Form ihrer Formulierungen oder den daraus bereiteten Anwendungsformen, wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Beim Einsatz der erfindungsgemäßen Wirkstoffe als Fungizide können die Aufwandmengen je nach Applikationsart innerhalb eines größeren Bereiches variiert werden. Bei der Behandlung von Pflanzenteilen liegen die Aufwandmengen an Wirkstoff im allgemeinen zwischen 0,1 und 10.000 g/ha, vorzugsweise zwischen 10 und 1.000 g/ha. Bei der Saatgutbehandlung liegen die Aufwandmengen an Wirkstoff im allgemeinen zwischen 0,001 und 50 g pro Kilogramm Saatgut, vorzugsweise zwischen 0,01 und 10 g pro Kilogramm Saatgut. Bei der Behandlung des Bodens liegen die Aufwandmengen an Wirkstoff im allgemeinen zwischen 0,1 und 10.000 g/ha, vorzugsweise zwischen 1 und 5.000 g/ha.

Die Herstellung und die Verwendung der erfindungsgemäßen Wirkstoffe geht aus den nachfolgenden Beispielen hervor.

### Beispiele

### Beispiel I-1-a-1

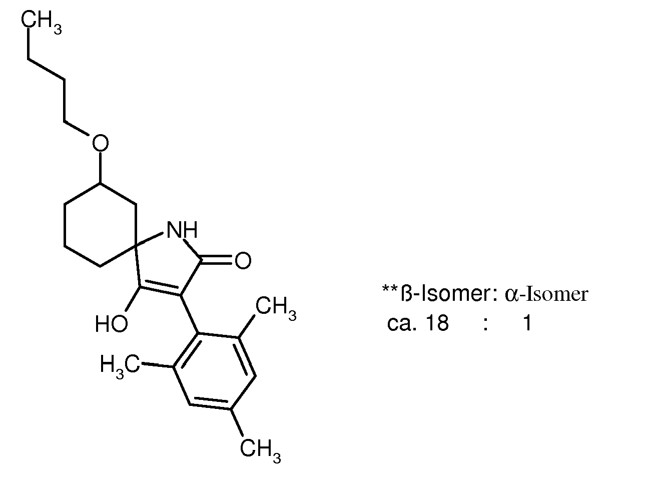

In einem 100 ml Dreihalskolben mit Thermometer und Rückflusskühler werden unter Argon 2,2 Äq. = 1,33 g Kalium-tert.-butylat-95 %ig-(11,3 mMol) in 5 ml Dimethylacetamid vorgelegt. Bei 80°C tropft man 2 g der Verbindung gemäß Beispiel II-1 (5,13 mMol) in 5 ml Dimethylacetamid zu. Man rührt 1 Stunde bei 80°C.

Das Reaktionsgemisch wird in 100 ml Eiswasser eingerührt; mit konz. HCl auf pH 2 gestellt und der Niederschlag abgesaugt.

Es erfolgt säulenchromatographische Reinigung an Kieselgel (Dichlormethan:Essigsäureethylester, 5:3).

Ausbeute: 1,8 g (94 % der Theorie) 72°C.
** Angereichertes Isomeres nach Reinigung durch Säulenchromatographie an Kieselgel

In Analogie zu Beispiel (I-1-a-1) und gemäß den allgemeinen Angaben zur Herstellung erhält man folgende Verbindungen der Formel (I-1-a)

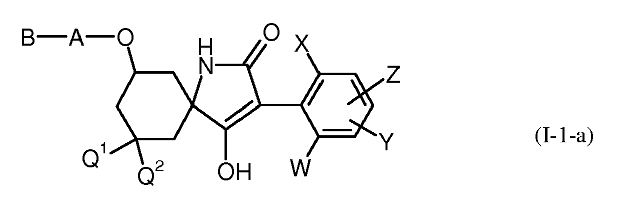

| **Bsp.-Nr.** | **W** | **X** | **Y** | **Z** | **A** | **B** | **Q¹** | **Q²** | **Fp.°C** | **Isomer** |
|---|---|---|---|---|---|---|---|---|---|---|
| I-1-a-2 | CH₃ | CH₃ | 4-CH₃ | H | CH₂ | H | H | H | Öl * 3.4-3.48, 3.69 - 3.7 (m, 1H, O-CH) 6.83, (s, 2H, ArH) | α:β 1:1 |
| I-1-a-3 | CH₃ | CH₃ | 4-CH₃ | H | CH₂ | H | CH₃ | CH₃ | Öl * 0.97, 1.07 (2s, 6H, C(CH₃)₂) | α** |
| I-1-a-4 | CH₃ | CH₃ | 4-CH₃ | H | CH₂ | i-C₃H₇ | CH₃ | CH₃ | Wachs * 3.20 (dd, 2H, OCH₂) 6.84 (s, 2-H, ArH) | α** |
| I-1-a-5 | H | CH₃ | 5-(4-Cl-Ph) | H | CH₂ | C₃H₇ | H | H | 139 | α** |
| I-1-a-6 | H | CH₃ | 5-(4-Cl-Ph) | H | CH₂ | C₃H₇ | H | H | 155 | β** |
| I-1-a-7 | Br | C₂H₅ | 4-CH₃ | H | CH₂ | CH₃ | H | H | 258 | α** |
| I-1-a-8 | Cl | C₂H₅ | 4-Br | H | CH₂ | C₃H₇ | H | H | 110 | β** |
| I-1-a-9 | Cl | C₂H₅ | 4-Br | H | CH₂ | C₃H₇ | H | H | 128 | α** |
| I-1-a-10 | Br | C₂H₅ | 4-CH₃ | H | CH₂ | C₂H₅ | H | H | 235 | α** |
| I-1-a-11 | Br | C₂H₅ | 4-CH₃ | H | CH₂ | C₃H₇ | H | H | 228 | α** |
| I-1-a-12 | Br | C₂H₅ | 4-CH₃ | H | CH₂ | C₂H₅ | H | H | 174 | β** |
| I-1-a-13 | H | CH₃ | H | 5-CH₃ | CH₂ | CH₃ | H | H | 208 | α** |
| I-1-a-14 | Br | C₂H₅ | 4-CH₃ | H | CH₂ | H | H | H | 200 | β** |
| I-1-a-15 | H | CH₃ | H | 5-CH₃ | CH₂ | CH₃ | H | H | Öl *2.09, 2.25 (2s, 6H, Ar-CH₃) 3.46 (q, 2H, O-CH₂-CH₃) | β** |
| I-1-a-16 | Br | C₂H₅ | 4-CH₃ | H | CH₂ | H | H | H | 228 | ** |
| I-1-a-17 | Br | C₂H₅ | 4-CH₃ | H | CH₂ | C₃H₇ | H | H | 228 | β** |
| I-1-a-18 | Br | C₂H₅ | 4-CH₃ | H | CH₂ | CH₃ | H | H | 194 | β** |
| I-1-a19 | CH₃ | CH₃ | 4-CH₃ | H | CH₂ | C₂H₅ | H | H | 194 | β** |
| I-1-a-20 | CH₃ | CH₃ | 4-CH₃ | H | CH₂ | C₂H₅ | H | H | 253 | α** |
| I-1-a-21 | H | CH₃ | 4-CH₃ | 5-CH₃ | CH₂ | H | H | H | 219 | β** |
| I-1-a-22 | CH₃ | CH₃ | 4-CH₃ | 3-CH₃ | CH₂ | C₂H₅ | H | H | 237 | β** |
| I-1-a-23 | CH₃ | CH₃ | 4-CH₃ | 3-CH₃ | CH₂ | C₂H₅ | H | H | 226 | α** |
| I-1-a-24 | H | CH₃ | H | 5-CH₃ | CH₂ | H | H | H | 96 | β** |
| I-1-a-25 | H | CH₃ | H | 5-CH₃ | CH₂ | H | H | H | 199 | α** |
| I-1-a-26 | CH₃ | CH₃ | 4-CH₃ | 3-CH₃ | CH₂ | CH₃ | H | H | 229 | β** |
| I-1-a-27 | CH₃ | CH₃ | 4-CH₃ | 3-CH₃ | CH₂ | CH₃ | H | H | 265 | α** |
| I-1-a-28 | H | CH₃ | 4-CH₃ | 5-CH₃ | CH₂ | CH₃ | H | H | 182 | β** |
| I-1-a-29 | H | CH₃ | 4-CH₃ | 5-CH₃ | CH₂ | CH₃ | H | H | 199 | α** |
| I-1-a-30 | H | CH₃ | H | 5-CH₃ | CH₂ | C₂H₅ | H | H | Wachs | β** |
| I-1-a-31 | H | CH₃ | H | 5-CH₃ | CH₂ | C₂H₅ | H | H | 192 | α** |
| I-1-a-32 | C₂H₅ | Cl | 4-Cl | H | CH₂ | C₃H₇ | H | H | 78 | β** |
| I-1-a-33 | C₂H₅ | Cl | 4-Cl | H | CH₂ | C₃H₇ | H | H | 139 | α** |
| I-1-a-34 | C₂H₅ | Br | 4-CH₃ | H | -(CH₂)₂- | OCH₃ | H | H | 213 | α** |
| I-1-a-35 | CH₃ | CH₃ | 4-CH₃ | 3-CH₃ | CH₂ | H | H | H | 254 | β** |
| I-1-a-36 | CH₃ | CH₃ | 4-CH₃ | H | CH₂ | CH₃ | H | H | 130 | β** |
| I-1-a-37 | C₂H₅ | Br | 4-CH₃ | H | CH₂ | H | CH₃ | CH₃ | 247 | β** |
| I-1-a-38 | CH₃ | CH₃ | 4-CH₃ | H | CH₂ | CH₃ | H | H | 248 | α** |
| I-1-a-39 | CH₃ | CH₃ | 4-CH₃ | 3-CH₃ | CH₂ | H | H | H | 253 | α** |
| I-1-a-40 | C₂H₅ | Br | 4-CH₃ | H | -(CH₂)₂- | OCH₃ | H | H | 129 | β** |
| I-1-a-41 | CH₃ | CH₃ | 4-CH₃ | H | CH₂ | C₃H₇ | CH₃ | CH₃ | Wachs | β** |
| I-1-a-42 | CH₃ | CH₃ | 4-CH₃ | H | CH₂ | C₃H₇ | CH₃ | CH₃ | 255 | α** |
| I-1-a-43 | CH₃ | CH₃ | 4-CH₃ | H | CH₂ | C₃H₇ | H | H | 211 | β** |
| I-1-a-44 | H | CH₃ | 4-CH₃ | 5-CH₃ | CH₂ | C₂H₅ | H | H | 87 | β** |
| I-1-a-45 | CH₃ | CH₃ | 4-CH₃ | H | -(CH₂)₂- | OCH₃ | H | H | Öl | β** |
| I-1-a-46 | CH₃ | CH₃ | 4-CH₃ | H | -(CH₂)₂- | OCH₃ | H | H | 175 | α** |
| I-1-a-47 | C₂H₅ | C₂H₅ | 4-CH₃ | H | CH₂ | C₃H₇ | H | H | 133 | β** |
| I-1-a-48 | C₂H₅ | C₂H₅ | 4-CH₃ | H | CH₂ | C₃H₇ | H | H | 219 | α** |
| I-1-a-49 | C₂H₅ | Br | 4-Cl | H | CH₂ | C₃H₇ | H | H | 114 | β** |
| I-1-a-50 | C₂H₅ | Br | 4-Cl | H | CH₂ | C₃H₇ | H | H | 185 | α** |
| I-1-a-51 | C₂H₅ | Br | 4-Cl | H | CH₂ | CH₃ | H | H | 69 | β** |
| I-1-a-52 | C₂H₅ | Br | 4-Cl | H | CH₂ | H | H | H | 208 | β** |
| I-1-a-53 | CH₃ | CH₃ | 4-CH₃ | H | CH₂ | H | CH₃ | CH₃ | 274 | β** |
| I-1-a-54 | C₂H₅ | Br | 4-Cl | H | CH₂ | CH₃ | H | H | 223 | α** |
| I-1-a-55 | C₂H₅ | Br | 4-Cl | H | CH₂ | H | H | H | 215 | α** |
| I-1-a-56 | C₂H₅ | Br | 4-Cl | H | CH₂ | C₂H₅ | H | H | 96 | β** |
| I-1-a-57 | C₂H₅ | Br | 4-Cl | H | CH₂ | C₂H₅ | H | H | 80 | α** |
| I-1-a-58 | CH₃ | CH₃ | 4-CH₃ | H | CH₂ | H | H | H | 216 | β** |
| I-1-a-59 | CH₃ | CH₃ | 4-CH₃ | H | CH₂ | H | H | H | 267 | α** |
| I-1-a-60 | C₂H₅ | Br | 4-CH₃ | H | CH₂ | C₃H₇ | CH₃ | CH₃ | 225 | β** |
| I-1-a-61 | C₂H₅ | Br | 4-CH₃ | H | CH₂ | C₃H₇ | CH₃ | CH₃ | 270 | α** |
| I-1-a-62 | C₂H₅ | Br | 4-CH₃ | H | CH₂ | i-C₃H₇ | CH₃ | CH₃ | 207 | β** |
| I-1-a-63 | CH₃ | C₂H₅ | 4-CH₃ | H | CH₂ | C₃H₇ | H | H | 198 | β** |
| I-1-a-64 | C₂H₅ | OCH₃ | 4-Cl | H | CH₂ | C₃H₇ | H | H | 111-115 | β** |
| I-1-a-65 | C₂H₅ | OC₂H₅ | 4-Cl | H | CH₂ | C₃H₇ | H | H | *3.55(m, 1H, OCH) 3.97(m, 2H, OCH₂) | β** |
| I-1-a-66 | C₂H₅ | OCH₃ | 4-Cl | H | CH₂ | H | H | H | 175-180 | β** |
| I-1-a-67 | C₂H₅ | OCH₃ | 4-Cl | H | CH₂ | CH₃ | H | H | 87-97 | β** |
| I-1-a-68 | C₂H₅ | OCH₃ | 4-Cl | H | CH₂ | C₂H₅ | H | H | 182-184 | β** |
| I-1-a-69 | CH₃ | C₂H₅ | 4-CH₃ | H | CH₂ | C₃H₇ | H | H | 199 | α** |
| I-1-a-70 | CH₃ | C₂H₅ | 4-CH₃ | H | CH₂ | H | H | H | 221 | α** |
| I-1-a-71 | CH₃ | C₂H₅ | 4-CH₃ | H | CH₂ | H | H | H | 88 | β** |
| I-1-a-72 | CH₃ | C₂H₅ | 4-Br | H | CH₂ | C₂H₅ | H | H | 209 | α** |
| I-1-a-73 | CH₃ | C₂H₅ | 4-Br | H | CH₂ | C₂H₅ | H | H | 175 | β** |
| I-1-a-74 | CH₃ | C₂H₅ | 4-Br | H | CH₂ | C₃H₇ | H | H | 194 | α** |
| I-1-a-75 | CH₃ | C₂H₅ | 4-Br | H | CH₂ | C₃H₇ | H | H | 163 | β** |
| I-1-a-76 | CH₃ | C₂H₅ | 4-Br | H | CH₂ | CH₃ | H | H | 218 | α** |
| I-1-a-77 | CH₃ | C₂H₅ | 4-Br | H | CH₂ | CH₃ | H | H | 92 | β** |
| I-1-a-78 | CH₃ | C₂H₅ | 4-Br | H | CH₂ | H | H | H | 206 | α** |
| I-1-a-79 | CH₃ | C₂H₅ | 4-Br | H | CH₂ | H | H | H | 89 | β** |
| I-1-a-80 | H | CH₃ | 4-CH₃ | 5-CH₃ | CH₂ | C₂H₅ | H | H | 198 | α** |
| I-1-a-81 | Cl | H₃CO-(CH₂)₂-O- | 4-CH₃ | H | CH₂ | C₃H₇ | H | H | Wachs | β** |
| I-1-a-82 | Cl | Cl | 4-Cl | H | -(CH₂)₂- | OCH₃ | H | H | 209 | α** |
| I-1-a-83 | Cl | Cl | 4-Cl | H | CH₂ | C₃H₇ | H | H | 99 | β** |
| I-1-a-84 | Cl | Cl | 4-Cl | H | -(CH₂)₂- | OCH₃ | H | H | 162 | β** |
| I-1-a-85 | H | C₂H₅ | 4-Cl | H | -(CH₂)₂- | OCH₃ | H | H | 117 | β** |
| I-1-a-86 | H | C₂H₅ | 4-Cl | H | -(CH₂)₂- | OCH₃ | H | H | Wachs | α** |
| I-1-a-87 | C₂H₅ | Cl | 4-CH₃ | H | CH₂ | C₃H₇ | H | H | 1) | β** |
| I-1-a-88 | H | CH₃ | H | 5-CH₃ | CH₂ | 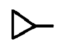 | H | H | 197 | α** |
| I-1-a-89 | CH₃ | CH₃ | 4-CH₃ | H | CH₂ | 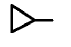 | H | H | 113 | α** |
| I-1-a-90 | CH₃ | CH₃ | 4-CH₃ | H | CH₂ | 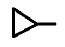 | H | H | 110 | β** |
| I-1-a-91 | C₂H₅ | OCH₃ | 4-Cl | H | -(CH₂)₂- | OCH₃ | H | H | 165 | α** |
| I-1-a-92 | C₂H₅ | OCH₃ | 4-Cl | H | -(CH₂)₂- | OCH₃ | H | H | Öl | β** |
| I-1-a-93 | H | CH₃ | H | 5-CH₃ | CH₂ | 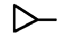 | H | H | 129 | β** |
| I-1-a-94 | C₂H₅ | Br | 4-CH₃ | H | CH₂ | 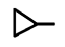 | H | H | 191 | β** |
| I-1-a-95 | C₂H₅ | Br | 4-CH₃ | H | CH₂ | 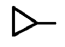 | H | H | 238 | α** |

| | | | | | | | | | | |
|---|---|---|---|---|---|---|---|---|---|---|
| * ¹H-NMR (400 MHz, d₆-DMSO): Verschiebung δ in ppm ** Angereichertes Isomeres nach Reinigung durch Säulenchromatographie an Kieselgel 1) ¹H-NMR (400 MHz, d₆-DMSO): δ = 3.55 (m, 1H, CHO), 7.00 (s, 1H, Ar-H) | | | | | | | | | | |

### Beispiel I-1-b-1

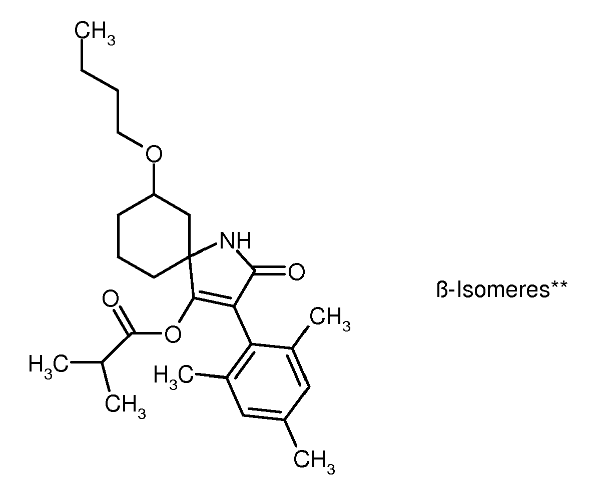

In einem 100 ml Dreihalskolben mit Thermometer und Rückflusskühler werden unter Argon 0,25 g der Verbindung gemäß Beispiel I-1-a-1 in 30 ml wasserfreiem Essigsäureethylester und 0,1 ml Triethylamin (0,7 mMol) vorgelegt. Man katalysiert mit 10 mg Steglich-Base und versetzt unter Rückfluss mit 0,08 ml Isobuttersäurechlorid in 2 ml wasserfreiem Essigsäureethylester. Man rührt 1 Stunde. Es erfolgt Reaktionsverfolgung durch Dünnschichtchromatographie. Das Reaktionsgemisch wird im Vakuum eingeengt. Anschließend erfolgt eine säulenchromatographische Reinigung des Rückstandes an Kieselgel (n-Hexan: Essigsäureethylester 8:2).

Ausbeute: 0,2 g (62,2 % der Theorie) Fp. 153°C.
** angereichertes Isomeres nach Reinigung durch Säulenchromatographie an Kieselgel

In Analogie zu Beispiel (I-1-b-1) und gemäß den allgemeinen Aufgaben zur Herstellung erhält man folgende Verbindungen der Formel (I-1-b)

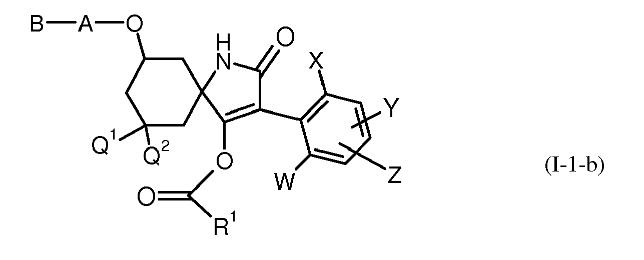

| **Bsp.-Nr.** | **W** | **X** | **Y** | **Z** | **A** | **B** | **Q¹** | **Q²** | **R¹** | **Fp.°C** | **Isomeres**** |
|---|---|---|---|---|---|---|---|---|---|---|---|
| I-1-b-2 | Br | C₂H₅ | 4-CH₃ | H | CH₂ | C₃H₇ | H | H | H₃CO-CH₂- | *2,26 (s, 3H, Ar-4-CH₃) 4,07 (q, 2H, CO-CH₂O-) | α |
| I-1-b-3 | Br | C₂H₅ | 4-CH₃ | H | CH₂ | C₃H₇ | H | H | i-C₃H₇ | *2,26 (s, 3H, Ar-4-CH₃) 3,81 (m, 1H, CH-O) | α |
| I-1-b-4 | Br | C₂H₅ | 4-CH₃ | H | CH₂ | C₂H₅ | H | H | H₃CO-CH₂- | 169-172 | β |
| I-1-b-5 | Br | C₂H₅ | 4-CH₃ | H | CH₂ | C₂H₅ | H | H | i-C₃H₇ | 184-187 | β |
| I-1-b-6 | C₂H₅ | Cl | 4-Br | H | CH₂ | C₃H₇ | H | H | H₃CO-CH₂- | *3,83 (m, br, 1H, O-CH) 7.33 (d, 1H, Ar-H) | α |
| I-1-b-7 | C₂H₅ | Cl | 4-Br | H | CH₂ | C₃H₇ | H | H | i-C₃H₇ | *1,06 (m, 6H, CH(CH₃) 7,32 (d, 1H, Ar-H) | α |
| I-1-b-8 | C₂H₅ | Cl | 4-Br | H | CH₂ | C₃H₇ | H | H | H₃CO-CH₂- | *3,42 (m, 3H, CH-O, CH₂O), 4.08 (q, 2H, COCH₂O) | β |
| I-1-b-9 | C₂H₅ | Cl | 4-Br | H | CH₂ | C₃H₇ | H | H | i-C₃H₇ | *1,06 (m, 6H, CH(CH₃)₂), 7,33 (d, 1H, Ar-H) | β |
| I-1-b-10 | Br | C₂H₅ | 4-CH₃ | H | CH₂ | CH₃ | H | H | i-C₃H₇ | *1,06 (m, 6H, CH(CH₃)₂) 2,29 (s, 3H, Ar-CH₃) | β |
| I-1-b-11 | Br | C₂H₅ | 4-CH₃ | H | CH₂ | H | H | H | i-C₃H₇ | *1,06 (m, 6H, CH(CH₃)₂) 3,34 (s, 3H, OCH₃) | β |
| I-1-b-12 | Br | C₂H₅ | 4-CH₃ | H | CH₂ | H | H | H | H₃CO-CH₂- | 188-189 | β |
| I-1-b-13 | Br | C₂H₅ | 4-CH₃ | H | CH₂ | C₃H₇ | H | H | i-C₃H₇ | 166-167 | β |
| I-1-b-14 | Br | C₂H₅ | 4-CH₃ | H | CH₂ | C₃H₇ | H | H | H₃CO-CH₂- | *2,29 (s, 3H, Ar-CH₃) 4,07 (q, 2H, CO-CH₂O) | β |
| I-1-b-15 | Br | C₂H₅ | 4-CH₃ | H | CH₂ | CH₃ | H | H | H₃CO-CH₂- | 202-205 | β |
| I-1-b-16 | CH₃ | CH₃ | 4-CH₃ | H | CH₂ | C₃H₇ | H | H | i-C₃H₇ | *2,52 (m, 1H, CH(CH₃)₂) 6,81 (s, 2H, ArH) | β |
| I-1-b-17 | CH₃ | CH₃ | 4-CH₃ | H | CH₂ | C₂H₅ | H | H | i-C₃H₇ | 178 | β |
| I-1-b-18 | C₂H₅ | Cl | 4-Cl | H | CH₂ | C₃H₇ | H | H | i-C₃H₇ | 176 | β |
| I-1-b-19 | C₂H₅ | Cl | 4-Cl | H | CH₂ | C₃H₇ | H | H | H₅C₂O-CH₂- | 157-159 | β |
| I-1-b-20 | C₂H₅ | Cl | 4-Cl | H | CH₂ | C₃H₇ | H | H | H₃CO-CH₂- | *2,64 (m, 2H, Ar-CH₂) 3.30 (s, 3H, OCH₃) | β |
| I-1-b-21 | CH₃ | CH₃ | 4-CH₃ | H | (CH₂)₂ | OCH₃ | H | H | H₃CO-CH₂- | *2,25 (s, 3H, ArCH₃) 3,97 (s, 2H, O-CH₂)) | β |
| I-1-b-22 | CH₃ | CH₃ | 4-CH₃ | H | CH₂ | C₃H₇ | H | H | H₃CO-CH₂- | *2,25 (s, 3H, ArCH₃) 3,16 (s, 3H, OCH₃) | β |
| I-1-b-23 | C₂H₅ | Br | 4-Cl | H | CH₂ | C₃H₇ | H | H | H₃CO-CH₂- | 157 | β |
| I-1-b-24 | C₂H₅ | Br | 4-CH₃ | H | CH₂ | H | CH₃ | CH₃ | H₃CO-CH₂- | 218 | β |
| I-1-b-25 | C₂H₅ | C₂H₅ | 4-CH₃ | H | CH₂ | C₃H₇ | H | H | H₃CO-CH₂- | 156 | β |
| I-1-b-26 | CH₃ | CH₃ | 4-CH₃ | H | CH₂ | C₃H₇ | H | H | H₅C₂O-CH₂- | *2,24 (s, 3H, ArCH₃) 3,28 (q, 2H, OCH₂-CH₃) | β |
| I-1-b-27 | C₂H₅ | C₂H₅ | 4-CH₃ | H | CH₂ | C₃H₇ | H | H | H₅C₂O-CH₂- | 139-141 | β |
| I-1-b-28 | CH₃ | CH₃ | 4-CH₃ | H | CH₂ | C₃H₇ | H | H | t-C₄H₉ | 190 | β |
| I-1-b-29 | C₂H₅ | Br | 4-CH₃ | H | CH₂ | i-C₃H₇ | CH₃ | CH₃ | i-C₃H₇ | 180-183 | β |
| I-1-b-30 | C₂H₅ | Br | 4-CH₃ | H | CH₂ | H | CH₃ | CH₃ | i-C₃H₇ | 185-198 | β |
| I-1-b-31 | C₂H₅ | Br | 4-Cl | H | CH₂ | C₃H₇ | H | H | 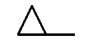 | 224-226 | β |
| I-1-b-32 | CH₃ | CH₃ | 4-CH₃ | H | CH₂ | C₃H₇ | H | H | 2-Cl-C₆H₄- | 192-194 | β |
| I-1-b-33 | C₂H₅ | Br | 4-Cl | H | CH₂ | C₃H₇ | H | H | 2-Cl-C₆H₄- | 216 | β |
| I-1-b-34 | CH₃ | CH₃ | 4-CH₃ | H | CH₂ | C₃H₇ | H | H | 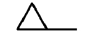 | 199 | β |
| I-1-b-35 | CH₃ | CH₃ | 4-CH₃ | H | CH₂ | C₃H₇ | H | H | 4-Cl-C₆H₄-O-CH₂- | 168 | β |
| I-1-b-36 | CH₃ | CH₃ | 4-CH₃ | H | CH₂ | C₂H₅ | H | H | H₃CO-CH₂- | 142-143 | β |
| I-1-b-37 | CH₃ | C₂H₅ | 4-CH₃ | H | CH₂ | H | H | H | H₃CO-CH₂- | 195-197 | β |
| I-1-b-38 | CH₃ | C₂H₅ | 4-CH₃ | H | CH₂ | C₃H₇ | H | H | H₃CO-CH₂- | 150-152 | β |
| I-1-b-39 | CH₃ | C₂H₅ | 4-Br | H | CH₂ | H | H | H | H₃CO-CH₂- | 163-165 | β |
| I-1-b-40 | CH₃ | C₂H₅ | 4-Br | H | CH₂ | CH₃ | H | H | H₃CO-CH₂- | 187-189 | β |
| I-1-b-41 | CH₃ | C₂H₅ | 4-Br | H | CH₂ | C₂H₅ | H | H | H₃CO-CH₂- | *2.50(m, 2H, ArCH₂) 3,25 (s, 3H, OCH₃) | β |
| I-1-b-42 | CH₃ | C₂H₅ | 4-Br | H | CH₂ | C₃H₇ | H | H | H₃CO-CH₂- | 150-152 | β |
| I-1-b-43 | CH₃ | CH₃ | 4-CH₃ | H | CH₂ | C₃H₇ | H | H | 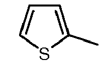 | 219-222 | β |
| I-1-b-44 | C₂H₅ | Br | 4-Cl | H | CH₂ | C₃H₇ | H | H | 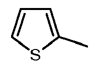 | *2.68(m, 2H, Ar-CH₂) 7,66 (dd, 1H, Thienyl-H₅) | β |
| I-1-b-45 | CH₃ | CH₃ | 4-CH₃ | H | CH₂ | C₃H₇ | H | H | Cl-CH2- | 177-180 | β |
| I-1-b-46 | CH₃ | CH₃ | 4-CH₃ | H | CH₂ | C₃H₇ | H | H | 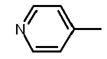 | 252-254 | β |
| I-1-b-47 | CH₃ | C₂H₅ | 4-Br | H | CH₂ | H | H | H | i-C₃H₇ | *1.01(dt, 6H, CH(CH₃)₂) 3,34 (s, 3H, OCH₃) | β |
| I-1-b-48 | CH₃ | C₂H₅ | 4-Br | H | CH₂ | CH₃ | H | H | i-C₃H₇ | *1.01(dt, 6H, CH(CH₂)₂) 2,21 (d, 3H, ArCH₃) | β |
| I-1-b-49 | CH₃ | C₂H₅ | 4-Br | H | CH₂ | C₂H₅ | H | H | i-C₃H₇ | *2.21(d, 3H, ArCH₃) 3,48 (m, 3H, OCH und OCH₃) | β |
| I-1-b-50 | CH₃ | C₂H₅ | 4-Br | H | CH₂ | C₃H₇ | H | H | i-C₃H₇ | *1.01(dt, 6H, CH(CH₃)₂) 3,44 (m, 3H, OCH und OCH₂) | β |
| I-1-b-51 | H | C₂H₅ | 4-Cl | H | (CH₂)₂ | OCH₃ | H | H | i-C₃H₇ | *1.04 (d, 6H, CH(CH₃)₂) 3.6 (m, 2H, OCH₂) | β** |
| I-1-b-52 | C₂H₅ | Cl | 4-CH₃ | H | CH₂ | C₃H₇ | H | H | i-C₃H₇ | erstarrter Schaum | β** |
| I-1-b-53 | Cl | Cl | 4-Cl | H | (CH₂)₂ | OCH₃ | H | H | i-C₃H₇ | *1.14(d, 6H, CH(CH₃)₂) 3.59 (m, 4H, 2 x O-CH₂) | β** |

| | | | | | | | | | | | |
|---|---|---|---|---|---|---|---|---|---|---|---|
| * ¹H-NMR (300 MHz, CDCl₃): Verschiebungen δ in ppm. ** Angereichertes Isomeres nach Reinigung durch Säulenchromatographie oder Umkristallisation. | | | | | | | | | | | |

### Beispiel I-1-c-1

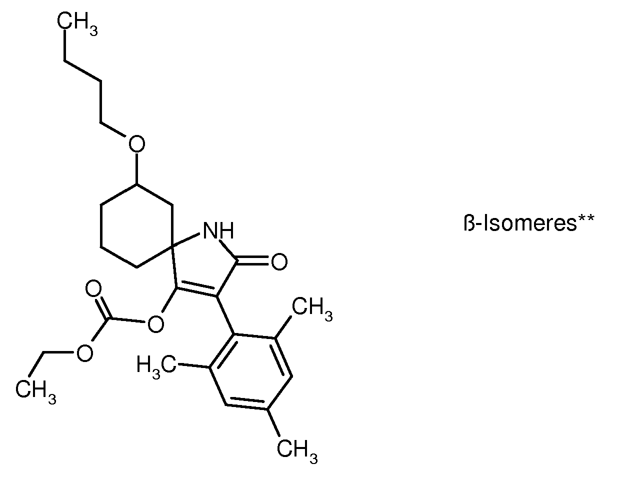

In einem 100 ml Dreihalskolben mit Thermometer und Rückflusskühler werden unter Argon 0,715 g der Verbindung gemäß Beispiel I-1-a-1 in 30 ml wasserfreiem Dichlormethan und 0,28 ml Triethylamin vorgelegt und bei 20°C mit 0,22 g (0.002 Mol) Chlorameisensäureethylester in 2 ml wasserfreiem Dichlormethan versetzt. Man rührt 1 Stunde. Das Reaktionsgemisch wird im Vakuum eingeengt.

Anschließend erfolgt eine säulenchromatographische Reinigung des Rückstands an Kieselgel (n-Hexan : Essigsäureethylester 8:2)
Ausbeute: 0,38 g (44 % der Theorie), Fp. 181°C.
** Angereichertes Isomeres nach Reinigung durch Säulenchromatographie an Kieselgel.

In Analogie zu Beispiel (I-1-c-1) und gemäß den allgemeinen Angaben zur Herstellung erhält man folgende Verbindungen der Formel (I-1-c)

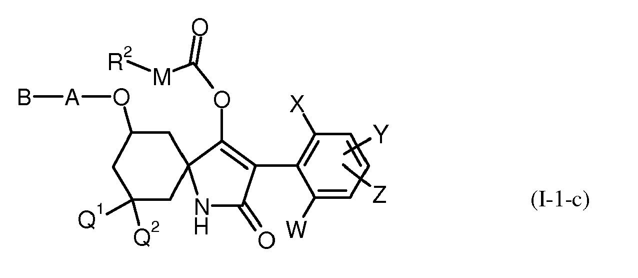

| **Bsp.-Nr.** | **W** | **X** | **Y** | **Z** | **A** | **B** | **Q¹** | **Q²** | **M** | **R²** | **Fp.°C** | **Isomer**** |
|---|---|---|---|---|---|---|---|---|---|---|---|---|
| I-1-c-2 | CH₃ | CH₃ | 4-CH₃ | H | CH₂ | H | H | H | O | C₂H₅ | Öl * 3.73 (m, 1H, O-CH) 3.98 (q, 2H, O-CH₂) | α |
| I-1-c-3 | CH₃ | CH₃ | 4-CH₃ | H | CH₂ | H | H | H | O | C₂H₅ | 179 | β |
| I-1-c-4 | CH₃ | CH₃ | 4-CH₃ | H | CH₂ | C₃H₇ | H | H | O | C₂H₅ | *2,24(s, 3H, Ar-4-CH₃) 4,00 (q, 2H, O-CH₂CH₃) | α |
| I-1-c-5 | Br | C₂H₅ | 4-CH₃ | H | CH₂ | C₃H₇ | H | H | O | C₂H₅ | *2,31 (s, 3H, Ar-CH₃) 3,83 (m, 1H, CH O) | α |
| I-1-c-6 | Br | C₂H₅ | 4-CH₃ | H | CH₂ | C₂H₅ | H | H | O | C₂H₅ | 188-191 | β |
| I-1-c-7 | C₂H₅ | Cl | 4-Br | H | CH₂ | C₃H₇ | H | H | O | C₂H₅ | *3,83 (m, 1H, CHO) 4,08 (q, 2H, O-CH₂CH₃) | α |
| I-1-c-8 | C₂H₅ | Cl | 4-Br | H | CH₂ | C₃H₇ | H | H | O | C₂H₅ | 188 | β |
| I-1-c-9 | Br | C₂H₅ | 4-CH₃ | H | CH₂ | CH₃ | H | H | O | C₂H₅ | *2,31 (s, 3H, Ar-4-CH₃), 3,47 (m, 1H, CH-O), 4,07 (q, C(O)-O-CH₂) | β |
| I-1-c-10 | Br | C₂H₅ | 4-CH₃ | H | CH₂ | H | H | H | O | C₂H₅ | 182-185 | β |
| I-1-c-11 | Br | C₂H₅ | 4-CH₃ | H | CH₂ | C₃H₇ | H | H | O | C₂H₅ | 182-183 | β |
| I-1-c-12 | CH₃ | CH₃ | 4-CH₃ | 3-CH₃ | CH₂ | CH₃ | H | H | O | C₂H₅ | 192 | β |
| I-1-c-13 | H | CH₃ | H | 5-CH₃ | CH₂ | C₂H₅ | H | H | O | C₂H₅ | Wachs | β |
| I-1-c-14 | CH₃ | CH₃ | 4-CH₃ | H | CH₂ | H | CH₃ | CH₃ | O | C₂H₅ | Wachs | β |
| I-1-c-15 | C₂H₅ | Cl | 4-Cl | H | CH₂ | C₃H₇ | H | H | O | C₂H₅ | 123 | β |
| I-1-c-16 | CH₃ | CH₃ | 4-CH₃ | H | CH₂ | C₃H₇ | H | H | O | C₂H₅ | 190-193 | β |
| I-1-c-17 | CH₃ | CH₃ | 4-CH₃ | H | CH₂ | C₂H₅ | H | H | O | C₂H₅ | 185-188 | β |
| I-1-c-18 | CH₃ | CH₃ | 4-CH₃ | H | (CH₂)₂ | OCH₃ | H | H | O | C₂H₅ | 165-170 | β |
| I-1-c-19 | CH₃ | CH₃ | 4-CH₃ | H | CH₂ | C₃H₇ | H | H | O | C₂H₅ | 150-154 | β |
| I-1-c-20 | C₂H₅ | Br | 4-CH₃ | H | CH₂ | H | CH₃ | CH₃ | O | C₂H₅ | 206-208 | β |
| I-1-c-21 | C₂H₅ | C₂H₅ | 4-CH₃ | H | CH₂ | C₃H₇ | H | H | O | C₂H₅ | 194-197 | β |
| I-1-c-22 | C₂H₅ | Br | 4-Cl | H | CH₂ | C₃H₇ | H | H | O | C₂H₅ | 202-204 | β |
| I-1-c-23 | C₂H₅ | OC₂H₅ | 4-Cl | H | CH₂ | C₃H₇ | H | H | O | C₂H₅ | 168-172 | β |
| I-1-c-24 | C₂H₅ | OCH₃ | 4-Cl | H | CH₂ | C₂H₅ | H | H | O | C₂H₅ | 148-156 | β |
| I-1-c-25 | C₂H₅ | OCH₃ | 4-Cl | H | CH₂ | CH₃ | H | H | O | C₂H₅ | *2.55 (m, 2H, ArCH₂) 4.03 (q, 2H, O-CH₂) | β |
| I-1-c-26 | C₂H₅ | OCH₃ | 4-Cl | H | CH₂ | C₃H₇ | H | H | O | C₂H₅ | 155-163 | β |
| I-1-c-27 | C₂H₅ | OCH₃ | 4-Cl | H | CH₂ | H | H | H | O | C₂H₅ | 181-187 | β |
| I-1-c-28 | C₂H₅ | Br | 4-CH₃ | H | CH₂ | C₃H₇ | H | H | O | C₂H₅ | 156-158 | β |
| I-1-c-29 | C₂H₅ | Br | 4-CH₃ | H | CH₂ | i-C₃H₇ | CH₃ | CH₃ | O | C₂H₅ | *2.29 (s, 3H, ArCH₃) 4.05 (q, 2H, O-CH₂) | β |
| I-1-c-30 | CH₃ | C₂H₅ | 4-CH₃ | H | CH₂ | H | H | H | O | C₂H₅ | 165-167 | β |
| I-1-c-31 | CH₃ | C₂H₅ | 4-CH₃ | H | CH₂ | C₃H₇ | H | H | O | C₂H₅ | 185-188 | β |
| I-1-c-32 | C₂H₅ | Br | 4-Cl | H | CH₂ | H | H | H | O | C₂H₅ | *2.62 (m, 2H, ArCH₂) 4.07 (q, 2H, OCH₂) | β |
| I-1-c-33 | CH₃ | C₂H₅ | 4-Br | H | CH₂ | H | H | H | O | C₂H₅ | *2.21 (s, 3H, ArCH₃) 4.04 (q, 2H, OCH₂) | β |
| I-1-c-34 | CH₃ | C₂H₅ | 4-Br | H | CH₂ | CH₃ | H | H | O | C₂H₅ | 200-202 | β |
| I-1-c-35 | CH₃ | C₂H₅ | 4-Br | H | CH₂ | C₂H₅ | H | H | O | C₂H₅ | *2.52 (m, 2H, ArCH₂) 4.04 (q, 2H, OCH₂) | β |
| I-1-c-36 | CH₃ | C₂H₅ | 4-Br | H | CH₂ | C₃H₇ | H | H | O | C₂H₅ | 192-193 | β |
| I-1-c-37 | C₂H₅ | Br | 4-Cl | H | CH₂ | C₃H₇ | H | H | O | CH₂=CH-CH₂- | 171-180 | β |
| I-1-c-38 | CH₃ | CH₃ | 4-CH₃ | H | CH₂ | C₃H₇ | H | H | O | t-C₄H₉-CH₂- | 212-214 | β |
| I-1-c-39 | C₂H₅ | Br | 4-Cl | H | CH₂ | C₃H₇ | H | H | O | t-C₄H₉-CH₂- | 240-243 | β |
| I-1-c-40 | CH₃ | CH₃ | 4-CH₃ | H | CH₂ | C₃H₇ | H | H | O | CH₂=CH-CH₂- | 159-167 | β |
| I-1-c-41 | CH₃ | CH₃ | 4-CH₃ | H | CH₂ | C₃H₇ | H | H | O | C₆H₅-CH₂- | *2.29 (s, 3H, ArCH₃) 4.96 (s, 2H, O-CH₂Ar) | β |
| I-1-c-42 | C₂H₅ | Br | 4-Cl | H | CH₂ | C₃H₇ | H | H | O | C₆H₅-CH₂- | 171-174 | β |
| I-1-c-43 | H | C₂H₅ | 4-Cl | H | -(CH₂)₂- | OCH₃ | H | H | O | C₂H₅ | *3.6 (m, 2H, O-CH₂) 4.05 (q, 2H, CO-O-CH₂) | β** |
| I-1-c-44 | C₂H₅ | Cl | 4-CH₃ | H | CH₂ | C₃H₇ | H | H | O | C₂H₅ | 186-190 | β** |
| I-1-c-45 | C₂H₅ | OCH₃ | 4-CH3 | H | CH₂ | C₃H₇ | H | H | O | C₂H₅ | 178-181 | β** |

| | | | | | | | | | | | | |
|---|---|---|---|---|---|---|---|---|---|---|---|---|
| * ¹H-NMR (400 MHz, CD₃CN): Verschiebung δ in ppm ** Angereichertes Isomeres nach Reinigung durch Säulenchromatographie oder durch Umkristallisation | | | | | | | | | | | | |

### Beispiel I-1-c-46

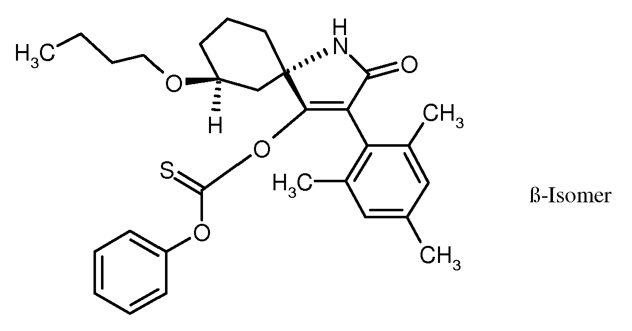

0.214 g der Verbindung gemäß Beispiel I-1-a-43 (0.6 mmol) werden in 10 ml Dichlormethan vorgelegt und mit 0,10 ml Triethylamin (0.72 mmol, 1.2 eq) versetzt. Man gibt 0.09 ml Chlorthioameisensäure-O-phenylester (0.66 mmol, 1.1 eq) hinzu und rührt übers Wochenende bei Raumtemperatur. Es wird mit 2.5 % Natriumcarbonatlösung versetzt, anschließend extrahiert, mit Natriumsulfat getrocknet und säulenchromatographisch mit Essigsäureethylester / n-Heptan (3:7 nach 100:0) gereinigt. Man erhält 0.084 g (28.4 %) eines Feststoffs (Fp: 192-94°C).

In Analogie zu Beispiel I-1-c-46 erhält man Beispiel I-1-c-47 vom Fp. 166-168°C

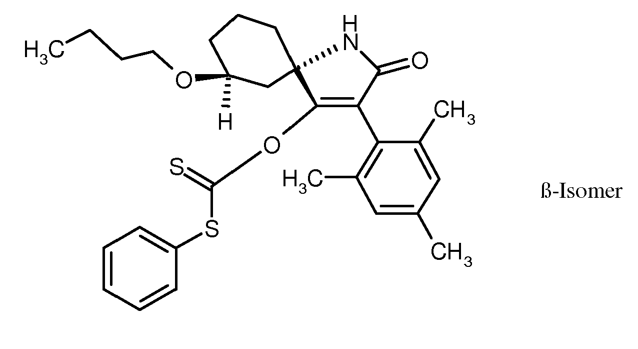

### Beispiel I-1-d-1

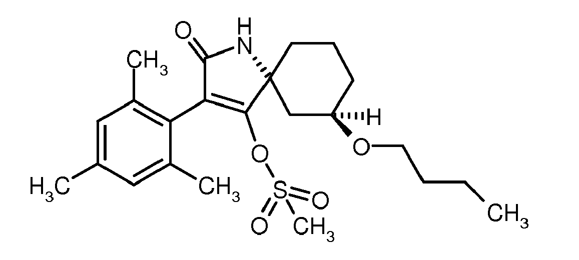

0,063 g (0,176 mmol) der Verbindung gemäß Bsp.-Nr. I-1-a-43 wird in 10 ml Dichlormethan vorgelegt und mit 0,03 ml Triethylamin versetzt. Man gibt 0,02 ml Methansulfonsäurechlorid portionsweise zu und lässt 24 h bei Raumtemperatur rühren. Es wird mit 5 %iger NaHCO₃-Lösung versetzt, die organischen Phase abgetrennt und die wässrige Phase mit Dichlormethan extrahiert. Nach Trockenen der vereinigten organischen Phasen mit Natriumsulfat wird im Vakuum zur Trockne eingeengt und der erhaltene Rückstand chromatographisch mit Essigsäureethylester/n-Heptan (Gradient 1/4 nach 2/1 ) gereinigt.
Ausbeute: 0,05 g (68% d. Theorie), Fp. 183-184°C

In Analogie zu Beispiel (I-1-d-1) und gemäß den allgemeinen Angaben zur Herstellung erhält man folgende Verbindungen der Formel (I-1-d)

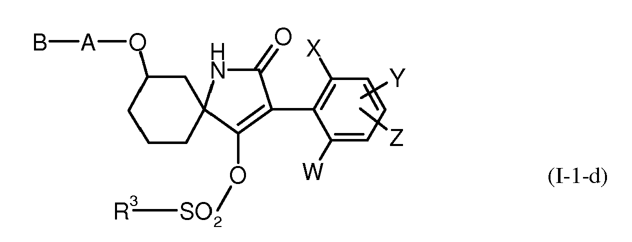

| **Bsp.-Nr.** | **W** | **X** | **Y** | **Z** | **A** | **B** | **Q¹** | **Q²** | **R³** | **Fp.°C** | **Isomer**** |
|---|---|---|---|---|---|---|---|---|---|---|---|
| I-1-d-2 | CH₃ | CH₃ | 4-CH₃ | H | CH₂ | C₃H₇ | H | H | 4-CH₃-C₆H₄ | 237 | β |
| I-1-d-3 | C₂H₅ | Br | 4-Cl | H | CH₂ | C₃H₇ | H | H | 4-CH₃-C₆H₄ | 231-234 | β |

| | | | | | | | | | | | |
|---|---|---|---|---|---|---|---|---|---|---|---|
| ** Angereichertes Isomeres nach Reinigung durch Säulenchromatographie oder durch Umkristallisation. | | | | | | | | | | | |

### Beispiel I-1-f-1

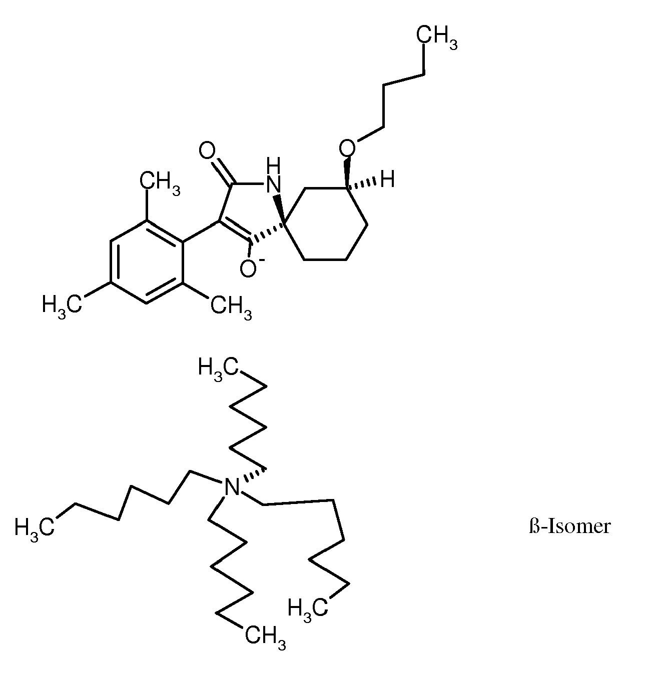

0.143 g der Verbindung gemäß Beispiel I-1-a-43 (0.4 mmol) werden in 8 ml Methanol gelöst und mit 0,39 ml einer 40 %igen methanolischen Lösung von Tetrabutylammoniumhydroxid (1 eq) versetzt. Nach 4 h bei Raumtemperatur wird eingeengt und der entstandene Rückstand noch dreimal mit Methanol ausgekreist. Man erhält so 0,3 g eines hochviskosen Öls als Produkt in quantitativer Ausbeute.
¹H-NMR (CDCl₃): 3.44 ppm (q, 2H, OCH₂), 2.88 ppm (pseudo-t, 8H, NCH₂)

### Beispiel I-1-g-1

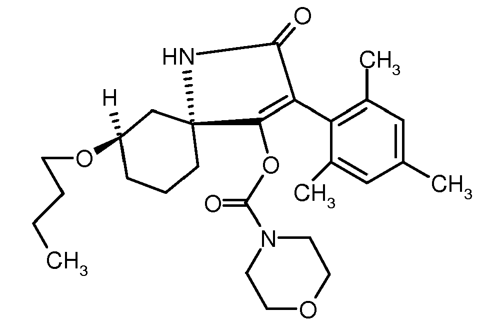

0.179 g (0.5 mmol) der Verbindung gemäß Beispiel I-1-a-43 werden in 5 ml Chloroform gelöst und bei Raumtemperatur mit 0.09 g (1.2 eq) des Morpholino-N-carbonsäurechlorids sowie 0.1 ml Triethylamin versetzt. Man erhitzt 24 h unter Rückfluss zum Sieden, gibt anschließend auf gesättigte Natriumchloridlösung. Die organische Phase wird abgetrennt und anschließend mit Natriumsulfat getrocknet. Nach chromatographischer Reinigung an Kieselgel mit einem n-Heptan/Essigsäureethylacetat Gradienten (4:1 nach 1:4) erhält man 140 mg eines Feststoffs (Ausbeute 59 %).
Fp°C: 189-196°C

### Beispiel II-1

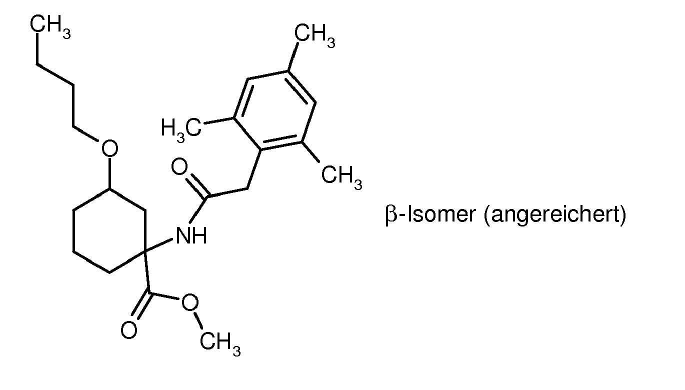

In einem 100 ml Dreihalskolben mit Thermometer und Rückflusskühler werden unter Argon 3,8 g der Verbindung gemäß Beispiel XIV-1 (0.015 Mol) in 50 ml wasserfreiem Tetrahydrofuran und 4,6 ml Triethylamin vorgelegt und bei 0-10°C mit 2,95 g (0.01 Mol) Mesitylenessigsäurechlorid in 5 ml wasserfreiem Tetrahydrofuran versetzt. Man rührt 1 Stunde. Das Reaktionsgemisch wird im Vakuum eingeengt.

Anschließend erfolgt säulenchromatographische Reinigung des Rückstands an Kieselgel (Hexan : Essigsäureethylester 8:2).
Ausbeute: 2,1 g (35 % der Theorie) Fp. 98°C.

In Analogie zu Beispiel (II-1) und gemäß den allgemeinen Angaben zur Herstellung erhält man folgende Verbindungen der Formel (II)

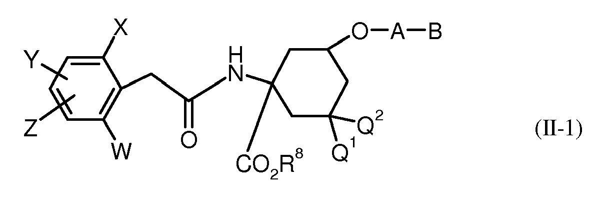

| **Bsp.-Nr.** | **W** | **X** | **Y** | Z | **A** | **B** | **Q¹** | **Q²** | **R⁸** | **Fp.°C** | Isomer |
|---|---|---|---|---|---|---|---|---|---|---|---|
| II-2 | CH₃ | CH₃ | 4-CH₃ | H | CH₂ | H | H | H | CH₃ | Öl * 2.89. 3.24 (2s, 3H, OCH₃) 3.57, 3.58 (2s, 3H, CO₂CH₃) | α : β ca. 1:1 |
| II-3 | CH₃ | CH₃ | 4-CH₃ | H | CH₂ | H | CH₃ | CH₃ | CH₃ | 148 | α |
| II-4 | CH₃ | CH₃ | 4-CH₃ | H | CH₂ | i-C₃H₇ | CH₃ | CH₃ | CH₃ | Öl * 3.18-3.21 (m, 2H, O- CH₂) 0.86, 0,88 (2s, 6H, (C(CH₃)₂) | α : β ca. 2:1 |
| II-5 | H | CH₃ | 5-(4-Cl-Ph) | H | CH₂ | C₃H₇ | H | H | CH₃ | Öl * 0.79, 0.87 (2t, 3H, CH₂- CH₃) 3.32, 2.33 (2s, 3H, ArCH₃) | α : β ca. 3:1 |
| II-6 | H | CH₃ | H | 5-CH₃ | CH₂ | H | H | H | CH₃ | *2,96, 3,23 (2s, 3H, OCH₃), 3,59 (2s, 3H, CO₂CH₃ | α : β** ca. 1:1 |
| II-7 | H | CH₃ | 4-CH₃ | 5-CH₃ | CH₂ | H | H | H | CH₃ | 125 | α : β** ca. 1:2 |
| II-8 | CH₃ | CH₃ | 4-CH₃ | 3-CH₃ | CH₂ | H | H | H | CH₃ | 136 | α : β** ca. 1:8 |
| II-9 | Br | C₂H₅ | 4-CH₃ | H | CH₂ | H | H | H | CH₃ | 114 | α : β** ca. 1:3 |
| II-10 | H | CH₃ | H | 5-CH₃ | CH₂ | CH₃ | H | H | CH₃ | Öl | α : β** ca. 3:2 |
| II-11 | H | CH₃ | 4-CH₃ | 5-CH₃ | CH₂ | CH₃ | H | H | CH₃ | 117 | α : β** ca. 1:35 |
| II-12 | CH₃ | CH₃ | 4-CH₃ | 3-CH₃ | CH₂ | CH₃ | H | H | CH₃ | 143 | α : β** ca. 1:28 |
| II-13 | Br | C₂H₅ | 4-CH₃ | H | CH₂ | CH₃ | H | H | CH₃ | 128 | α : β** ca. 1:2 |
| II-14 | CH₃ | CH₃ | 4-CH₃ | H | CH₂ | CH₃ | H | H | CH₃ | 129 | α : β** ca. 1:59 |
| II-15 | Br | C₂H₅ | 4-CH₃ | H | CH₂ | C₂H₅ | H | H | CH₃ | 120 | α : β** ca. 1:83 |
| II-16 | Br | C₂H₅ | 4-CH₃ | H | CH₂ | C₂H₅ | H | H | CH₃ | Öl *3,57 (s, 3H, CO₂CH₃) | α : β** ca. 3,7:1 |
| II-17 | H | CH₃ | H | 5-CH₃ | CH₂ | C₂H₅ | H | H | CH₃ | Öl log P 3.87, 3.21 | α : β ca. 1:1 |
| II-18 | H | CH₃ | 4-CH₃ | 5-CH₃ | CH₂ | C₂H₅ | H | H | CH₃ | Öl, log P 4.2, 3.51 *6,94-6,94 (m, 2H, ArH) | α : β ca. 5:4 |
| II-19 | CH₃ | CH₃ | 4-CH₃ | 3-CH₃ | CH₂ | C₂H₅ | H | H | CH₃ | Öl *6,85, 6,88 (2s, 1H, ArH) | α : β ca. 1:1 |
| II-20 | CH₃ | CH₃ | 4-CH₃ | H | CH₂ | C₂H₅ | H | H | CH₃ | 125 | α : β ca. 1:54 |
| II-21 | C₂H₅ | Cl | 4-Br | H | CH₂ | C₃H₇ | H | H | CH₃ | Öl *2,65-2,68 (m, 2H, Ar- CH₂-CH₃) | α : β ca. 1:1 |
| II-22 | C₂H₅ | Cl | 4-Cl | H | CH₂ | C₃H₇ | H | H | CH₃ | Öl, log P 5.28, 4.46 *2,65-2,69 (m, 2H, Ar- CH₂CH₃) | α : β ca. 1:1 |
| II-23 | Br | C₂H₅ | 4-CH₃ | H | CH₂ | C₃H₇ | H | H | CH₃ | Öl *2,63-2,66 (m, 2H, Ar- CH₂CH₃) | α : β ca. 3:2 |
| II-24 | Br | C₂H₅ | 4-CH₃ | H | CH₂ | CH₃ | H | H | CH₃ | Öl *3,57 (s, 3H, CO₂CH₃), 7,09 (s, 1H, Ar-H) | α** : β ca. 4,7:1 |
| II-25 | H | CH₃ | 4-CH₃ | 5-CH₃ | CH₂ | CH₃ | H | H | CH₃ | Öl log P 3.7, 3.04 | α + β** 2:1 |
| II-26 | Cl | Cl | 4-Cl | H | (CH₂)₂ | OCH₃ | H | H | CH₃ | 106 | α** |
| II-27 | Cl | H₃CO-(CH₂)₂-O- | 4-CH₃ | H | CH₂ | C₃H₇ | H | H | CH₃ | <60 | β** |
| II-28 | C₂H₅ | Br | 4-CH₃ | H | (CH₂)₂ | OCH₃ | H | H | CH₃ | Öl log P 3.64 | α** |
| II-29 | C₂H₅ | Br | 4-CH₃ | H | CH₂ | H | CH₃ | CH₃ | CH₃ | 133 | α + β** ca. 1:12 |
| II-30 | CH₃ | CH₃ | 4-CH₃ | H | CH₂ | CH₃ | H | H | CH₃ | Öl log P 3.74, 3.14 | α + β** ca. 3.5:1 |
| II-31 | CH₃ | CH₃ | 4-CH₃ | 3-CH₃ | CH₂ | H | H | H | CH₃ | Öl log P 3.48, 2.99 | α + β** ca. 5.4:1 |
| II-32 | C₂H₅ | Br | 4-CH₃ | H | (CH₂)₂ | OCH₃ | H | H | CH₃ | Öl log P 3.64, 3.18 | α + β** ca. 1:5.7 |
| II-33 | CH₃ | CH₃ | 4-CH₃ | H | CH₂ | C₃H₇ | CH₃ | CH₃ | CH₃ | Öl log P 5.26, 4.96 | α + β** ca. 1:2.8 |
| II-34 | C₂H₅ | OCH₃ | 4-Cl | H | CH₂ | C₃H₇ | H | H | CH₃ | Öl log P 4.85, 4.2 | α + β ca. 2:6.5 |
| II-35 | CH₃ | CH₃ | 4-CH₃ | H | (CH₂)₂ | OCH₃ | H | H | CH₃ | Öl log P 3.17, 2.76 | α + β ca. 1:1 |
| II-36 | C₂H₅ | C₂H₅ | 4-CH₃ | H | CH₂ | C₃H₇ | H | H | CH₃ | Öl log P 5.43, 4.70 | α + β** ca. 1:2 |
| II-37 | C₂H₅ | Br | 4-Cl | H | CH₂ | C₃H₇ | H | H | CH₃ | Öl log P 5.4, 4.56 | α + β** ca. 1:2,4 |
| II-38 | C₂H₅ | Br | 4-Cl | H | CH₂ | CH₃ | H | H | CH₃ | Öl log P 4.41, 3.61 | α + β ca. 1:1 |
| II-39 | C₂H₅ | Br | 4-Cl | H | CH₂ | H | H | H | CH₃ | Öl log P 3.96, 3.31 | α + β ca. 1:1.3 |
| II-40 | CH₃ | CH₃ | 4-CH₃ | H | CH₂ | H | CH₃ | CH₃ | CH₃ | 159 | α + β** ca. 1:19,7 |
| II-41 | C₂H₅ | Br | 4-Cl | H | CH₂ | C₂H₅ | H | H | CH₃ | Öl log P 4.95, 4.15 | α + β ca. 1:1 |
| II-42 | C₂H₅ | Br | 4-CH₃ | H | CH₂ | C₃H₇ | CH₃ | CH₃ | CH₃ | Öl log P 5.62, 5.32 | α + β** ca. 1:2.7 |
| II-43 | C₂H₅ | Br | 4-CH₃ | H | CH₂ | i-C₃H₇ | CH₃ | CH₃ | CH₃ | Öl log P 5.69, 5.41 | α + β** ca. 1:6.9 |
| II-44 | CH₃ | C₂H₅ | 4-CH₃ | H | CH₂ | C₃H₇ | H | H | CH₃ | Öl log P 5.1, 4.6 | α + β** ca. 1:3 |
| II-45 | CH₃ | C₂H₅ | 4-Br | H | CH₂ | C₂H₅ | H | H | CH₃ | Öl log P 4.84, 4.09 | α + β** ca. 1:2 |
| II-46 | CH₃ | C₂H₅ | 4-Br | H | CH₂ | C₃H₇ | H | H | CH₃ | Öl log P 5.26, 4.49 | α + β** ca. 1:3 |
| II-47 | CH₃ | C₂H₅ | 4-Br | H | CH₂ | CH₃ | H | H | CH₃ | Öl log P 4.33, 3.61 | α + β ca. 4:5 |
| II-48 | CH₃ | C₂H₅ | 4-Br | H | CH₂ | H | H | H | CH₃ | Öl log P 3.85, 3.24 | α + β** ca. 1:2 |
| II-49 | Cl | Cl | 4-Cl | H | CH₂ | C₃H₇ | H | H | CH₃ | 147 | β** |
| II-50 | Cl | Cl | 4-Cl | H | -(CH₂)₂- | OCH₃ | H | H | CH₃ | 164 | β** |
| II-51 | H | C₂H₅ | 4-Cl | H | CH₂ | C₃H₇ | H | H | CH₃ | 126 | β** |
| II-52 | OCH₃ | Cl | 4-CH₃ | H | CH₂ | C₃H₇ | H | H | CH₃ | 100 | β** |
| II-53 | OC₂H₅ | Cl | 4-CH₃ | H | CH₂ | C₃H₇ | H | H | CH₃ | 106 | β** |
| II-54 | C₂H₅ | OCH₃ | 4-Cl | H | -(CH₂)₂- | OCH₃ | H | H | CH₃ | Öl log P 3.29, 2.97 | α + β** ca. 1:1 |
| II-55 | H | C₂H₅ | 4-Cl | H | -(CH₂)₂- | OCH₃ | H | H | CH₃ | 120 | α + β** ca. 1:20 |
| II-56 | H | C₂H₅ | 4-Cl | H | -(CH₂)₂- | OC_{H3} | H | H | CH₃ | Öl log P 3.35, 2.85 | α + β** ca. 5:1 |
| II-57 | H | CH₃ | 5-CH₃ | H | CH₂ | 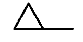 | H | H | CH₃ | Öl log P 3.76, 3.11 | α + β ca. 1:1 |
| II-58 | CH₃ | CH₃ | 4-CH₃ | H | CH₂ | 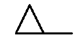 | H | H | CH₃ | Öl log P 4.14, 3.51 | α + β ca. 3:4 |
| II-59 | C₂H₅ | Br | 4-CH₃ | H | CH₂ | 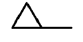 | H | H | CH₃ | Öl log P 4.58, 3.90 | α + β ca. 3:4 |
| II-60 | C₂H₅ | Br | 4-Br | H | CH₂ | C₃H₇ | H | H | CH₃ | 115 | β** |
| II-61 | C₂H₅ | Cl | 4-CH₃ | H | CH₂ | C₃H₇ | H | H | CH₃ | 105 | β** |
| II-62 | C₂H₅ | OCH₃ | 4-CH₃ | H | CH₂ | C₃H₇ | H | H | CH₃ | | β** |

| | | | | | | | | | | | |
|---|---|---|---|---|---|---|---|---|---|---|---|
| * ¹H-NMR (400 MHz, CD₃CN): Verschiebung δ in ppm ** Angereichertes Isomeres nach Reinigung durch Säulenchromatographie an Kieselgel | | | | | | | | | | | |

### Beispiel XIV-1

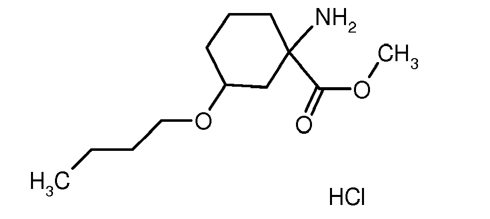

In einem 3000 ml Dreihalskolben mit Thermometer und Rückflusskühler werden unter Argon 120 g (1 Eq.) der Verbindung gemäß Beispiel XVII-1 (enthält Kalium-Salze) in 1200 ml Methanol bei 0 - 5°C vorgelegt und 52 ml Thionylchlorid zugetropft. Man rührt 30 Min. bei 0°C und dann 1 Tag bei 40°C. Man kühlt auf 5°C ab, saugt das Salz ab und engt das Filtrat im Vakuum ein.

Ausbeute: 108 g (72 % der Theorie) zäher Sirup über zwei Stufen ausgehend vom Hydantoin der Formel (XXI).
¹H-NMR (400 MHz, d₆-DMSO): δ = 0.85 - 0.90 (m, 3H, CH₂C-H₃), 3.73, 3.76 (2s, 3H, OCH₃) ppm.

In Analogie zu Beispiel (XIV-1) und gemäß den allgemeinen Angaben zur Herstellung erhält man folgende Verbindungen der Formel (XIV) in Form ihrer Hydrochloride

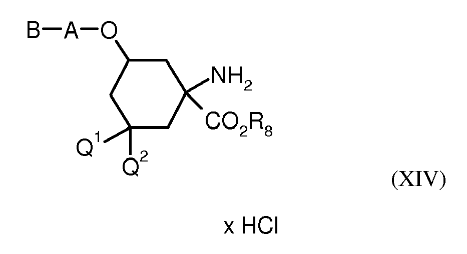

| **Bsp.-Nr.** | **A** | **B** | **Q**¹ | **Q²** | **R⁸** | **¹H-NMR (400 MHz, d₆-DMSO)** |
|---|---|---|---|---|---|---|
| | | | | | | **Verschiebungen** δ **in ppm** |
| XIV-2 | CH₂ | H | H | H | CH₃ | 3.19, 3.24 (2s, 3H, OCH₃) |
| | | | | | | 3.73, 3.76 (2s, 3H, CO₂CH₃) |
| XIV-3 | CH₂ | CH₃ | H | H | CH₃ | 1.06, 1.11 (2t, 3H, CH₂-CH₃) |
| | | | | | | 3.73, 3.76 (2s, 3H, OCH₃) |
| XIV-4 | CH₂ | C₂H₅ | H | H | CH₃ | 0.82- 0.88 (m, 3H, CH₂-CH₃) |
| | | | | | | 3.73, 3.76 (2s, 3H, OCH₃ |
| XIV-5 | CH₂ | H | CH₃ | CH₃ | CH₃ | 3.17, 3.35 (2s, 3H, OCH₃) |
| | | | | | | 3.75, 3.77 (2s, 3H, CO₂CH₂ |
| XIV-6 | CH₂ | i-C₃H₇ | CH₃ | CH₃ | CH₃ | 0.84, 0.85 (2s, 6H, C(CH₃)₂)) |
| | | | | | | 3.14-3.22 (m, 2H, OCH₂) |
| XIV-7 | CH₂ | 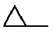 | H | H | CH₃ | -0.15-0.02, 0.27-0.31 (2m, 4H, 2Cyclopr. -CH₂) 3.59 (2s, 3H, CO₂CH₃) |
| XIV-8 | CH₂ | 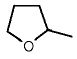 | H | H | CH₃ | 3.58, 3.60, 3.61, 3.63 (4s, 3H, CO₂CH₃) |

### Beispiel XVII-1

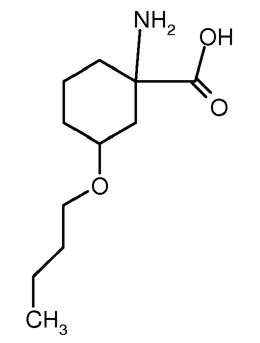

In einem 3000 ml Dreihalskolben mit Thermometer und Rückflusskühler werden unter Argon 135 g der Verbindung gemäß Beispiel XXI-1 in 600 ml 20 %-ige KOH suspendiert. Man rührt unter Rückfluss unter Stickstoffatmosphäre. Es erfolgt dünnschichtchromatographischer Kontrolle. Man rotiert mit ca. 25 % des Volumens ein und stellt bei 0 - 10°C mit HCl-konz. auf pH 4-5. Die verbleibende Lösung wird im Vakuum eingeengt und der Rückstand getrocknet.

Die Gesamtmenge wurde ohne weitere Charakterisierung in die Synthese von Beispiel XIV-1 eingesetzt. Die Hydantoine der Formel (XXI) sind neu und lassen sich nach dem nachfolgenden Verfahren (J) herstellen.

In Analogie zu Beispiel (XVII-1) und gemäß den allgemeinen Angaben zur Herstellung erhält man folgende Verbindungen der Formel (XVII)

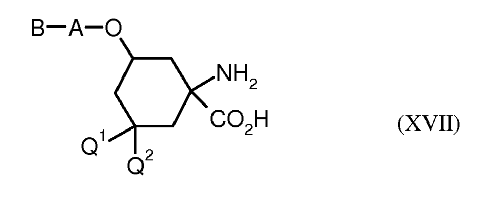

| **Bsp.-Nr.** | **A** | **B** | **Q¹** | **Q²** |
|---|---|---|---|---|
| XVII-2 | CH₂ | H | H | H |
| XVII-3 | CH₂ | CH₃ | H | H |
| XVII-4 | CH₂ | C₂H₅ | H | H |
| XVII-5 | CH₂ | H | CH₃ | CH₃ |
| XVII-6 | CH₂ | i-C₃H₇ | CH₃ | CH₃ |
| XVII-7 | CH₂ | 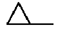 | H | H |
| XVII-8 | CH₂ | 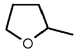 | H | H |

### Beispiel (I-2-a-1)

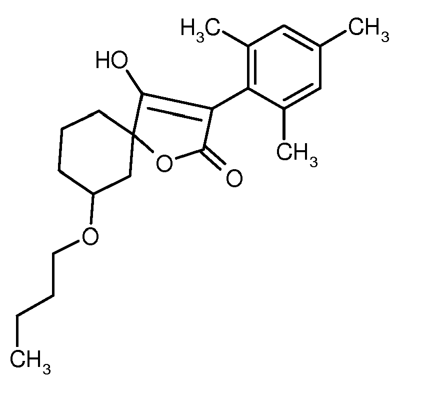

0,48 g (2 mmol) Mesitylessigsäurechlorid und 0,39 g (2 mmol) 1-Hydroxy-3-n-butoxy-cyclohexyl-carbonsäure-ethylester werden 10 h auf 140°C erhitzt. Nach dem Abkühlen gibt man 5 ml DMF zu und tropft 2,4 ml 1M Kalium-t-butylatlösung (2,4 mmol) zu. Es wird 10 h bei Raumtemperatur gerührt. Anschließend wird das Lösungsmittel einrotiert. Der Rückstand wird verteilt zwischen Wasser und Essigsäureethylester, die wässrige Phase mit 2 N HCl angesäuert und das Produkt mit Essigsäureethylester extrahiert. Die organische Phase wird getrocknet und einrotiert.
Ausbeute: 0,10 g (13 % d.Th.)
logP 3.57

In Analogie zu Beispiel (I-2-a-1) und gemäß den allgemeinen Angaben zur Herstellung erhält man folgende Verbindungen der Formel (I-2-a)

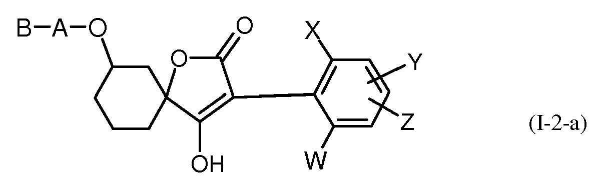

| **Bsp.-Nr.** | **W** | **X** | **Y** | **Z** | **A** | **B** | **logP** |
|---|---|---|---|---|---|---|---|
| I-2-a-2 | H | CH₃ | 5-(4-Cl-Ph) | 4-CH₃ | CH₂ | H | 3.4 |
| I-2-a-3 | C₂H₅ | C₂H₅ | 4-Br | H | CH₂ | H | 2.98 |
| I-2-a-4 | CH₃ | Br | 4-Cl | H | CH₂ | H | 2.46 |
| I-2-a-5 | Cl | Br | 4-CH₃ | H | CH₂ | H | 2.40 |
| I-2-a-6 | H | CH₃ | 4-Cl | H | CH₂ | H | 2.19 |
| I-2-a-7 | H | CH₃ | H | H | CH₂ | H | 1.81 |
| I-2-a-8 | H | CH₃ | 4-Cl | H | CH₂ | H | 2.11 |
| I-2-a-9 | H | Br | 4-CH₃ | 5-CH₃ | CH₂ | H | 2.35 |
| I-2-a-10 | CH₃ | CH₃ | 4-Br | H | CH₂ | H | 2.40 |
| I-2-a-11 | H | CH₃ | 4-CH₃ | 5-CH₃ | CH₂ | H | 2.21 |
| I-2-a-12 | CH₃ | CH₃ | 4-CH₃ | 3-CH₃ | CH₂ | H | 2.50 |
| I-2-a-13 | CH₃ | C₂H₅ | 4-Br | H | CH₂ | H | 2.74 |
| I-2-a-14 | CH₃ | CH₃ | 4-CH₃ | H | CH₂ | H | 2.31¹⁾ |
| I-2-a-15 | Cl | Cl | H | H | CH₂ | H | 1.93 |
| I-2-a-16 | CH₃ | OCH₃ | 4-CH₃ | H | CH₂ | H | 2.10¹⁾ |
| I-2-a-17 | CH₃ | C₂H₅ | 4-(4-Cl-Ph) | H | CH₂ | CH₃ | 4.19 |
| I-2-a-18 | H | Br | 4-Br | 5-CH₃ | CH₂ | H | 2.58 |
| I-2-a-19 | Br | Br | 4-CH₃ | H | CH₂ | H | 2.51 |
| I-2-a-20 | CH₃ | Cl | 4-Cl | H | CH₂ | H | 2.41 |
| I-2-a-21 | Cl | Cl | 4-CH₃ | H | CH₂ | H | 2.27 |
| I-2-a-22 | H | Cl | 4-CH₃ | H | CH₂ | H | 2.13 |
| I-2-a-23 | H | CH₃ | 5-Br | H | CH₂ | H | 2.30 |
| I-2-a-24 | H | 2-CF₃ | 4-Cl | H | CH₂ | H | 2.37 |
| I-2-a-25 | CH₃ | Cl | H | H | CH₂ | H | 1.92 |
| I-2-a-26 | C₂H₅ | Br | 4-Br | H | CH₂ | H | 2.77 |
| I-2-a-27 | CH₃ | Cl | 4-CH₃ | H | CH₂ | H | 2.31 |
| I-2-a-28 | CH₃ | Br | 4-CH₃ | H | CH₂ | H | 2.36 |
| I-2-a-29 | Cl | Br | 4-C₂H₅ | H | CH₂ | H | 2.62 |
| I-2-a-30 | CH₃ | CH₃ | 5-(4-Cl-Ph) | 4-CH₃ | CH₂ | H | 3.60 |
| I-2-a-31 | H | Cl | 5-(4-Cl-Ph) | H | CH₂ | H | 3.14 |
| I-2-a-32 | H | CH₃ | 5-(4-Cl-Ph) | H | CH₂ | H | 3.19¹⁾ |
| I-2-a-33 | H | CH₃ | 5-(3-Cl-Ph) | H | CH₂ | H | 3.16 |
| I-2-a-34 | H | Cl | 5-(3-Cl-Ph) | H | CH₂ | H | 3.11 |
| I-2-a-35 | H | CH₃ | H | 5-CH₃ | CH₂ | H | 2.15 |
| I-2-a-36 | H | Br | 4-Cl | H | CH₂ | H | 2.25 |
| I-2-a-37 | H | Cl | 4-Br | H | CH₂ | H | 2.31 |
| I-2-a-38 | H | Cl | H | H | CH₂ | H | 1.86 |
| I-2-a-39 | H | Cl | 4-Cl | H | CH₂ | H | 2.21 |
| I-2-a-40 | H | Cl | 4-Br | 5-Cl | CH₂ | H | 2.53 |
| I-2-a-41 | CH₃ | CH₃ | 4-CH₃ | 3-Br | CH₂ | H | 2.70 |
| I-2-a-42 | CH₃ | CH₃ | 5-(4-Cl-Ph) | H | CH₂ | H | 3.29 |
| I-2-a-43 | H | CH₃ | 4-Cl | 5-CH₃ | CH₂ | H | 2.47 |
| I-2-a-44 | H | Br | 4-CH₃ | 5-F | CH₂ | H | 2.25 |
| I-2-a-45 | H | CH₃ | 4-CH₃ | 5-F | CH₂ | H | 2.25 |
| I-2-a-46 | CH₃ | CH₃ | H | 3-Cl | CH₂ | H | 2.42 |
| I-2-a-47 | CH₃ | Br | 4-Br | 3-CH₃ | CH₂ | H | 2.78 |
| I-2-a-48 | H | Cl | 4-CH₃ | 5-Cl | CH₂ | H | 2.47 |
| I-2-a-49 | H | Br | H | 5-CH₃ | CH₂ | H | 2.16 |
| I-2-a-50 | H | CH₃ | 4-OCH₃ | H | CH₂ | H | 2.21 |
| I-2-a-51 | H | Br | H | 5-Br | CH₂ | H | 2.27 |
| I-2-a-52 | Cl | Cl | H | 3-CH₃ | CH₂ | H | 2.23 |
| I-2-a-53 | Cl | Cl | 4-Br | 3-CH₃ | CH₂ | H | 2.70 |
| I-2-a-54 | CH₃ | CH₃ | 4-CH₃ | 3-F | CH₂ | H | 2.45 |
| I-2-a-55 | C₂H₅ | C₂H₅ | 4-Br | H | CH₂ | C₃H₇ | 4.24 |
| I-2-a-56 | H | Cl | 4-CH₃ | H | CH₂ | C₃H₇ | 3.32 |
| I-2-a-57 | H | CH₃ | H | H | CH₂ | C₃H₇ | 3.03 |
| I-2-a-58 | H | CH₃ | 4-CH₃ | H | CH₂ | C₃H₇ | 3.32 |
| I-2-a-59 | H | Br | 4-CH₃ | 5-CH₃ | CH₂ | C₃H₇ | 3.57 |
| I-2-a-60 | CH₃ | Cl | 4-CH₃ | H | CH₂ | C₃H₇ | 3.49 |
| I-2-a-61 | CH₃ | CH₃ | 4-CH₃ | 3-CH₃ | CH₂ | C₃H₇ | 3.76 |
| I-2-a-62 | H | CH₃ | H | 5-CH₃ | CH₂ | C₃H₇ | 3.36 |
| I-2-a-63 | CH₃ | C₂H₅ | 4-Br | H | CH₂ | C₃H₇ | 3.99 |
| I-2-a-1 | CH₃ | CH₃ | 4-CH₃ | H | CH₂ | C₃H₇ | 3.57 |

Die Verbindungen der Formel (I-2-a) fallen in der Regel als Isomerengemische an und wurden in den mit )¹ gekennnzeichneten Fällen durch Säulenchromatographie in die cis- bzw. trans-Diasteromeren aufgetrennt.

Ph =

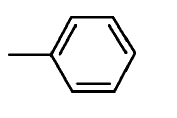

### Beispiel (I-2-b-1)

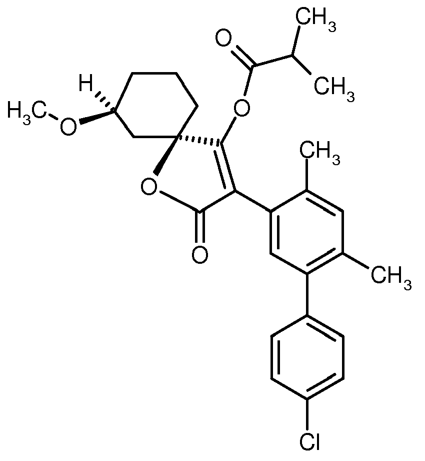

0,20 g (0,48 mmol) der Verbindung gemäß Beispiel I-2-a-32 (cis-Isomer) und 0,06 g (0,58 mmol) Triethylamin werden in 20 ml Dichlormethan vorgelegt, tropfenweise mit 0,58 ml (0,58 mmol) einer 1M Lösung von Isobuttersäurechlorid in Tetrahydrofuran versetzt und 12 h bei Raumtemperatur gerührt.

Zur Aufarbeitung wäscht man mit 10 % Citronensäure, 10 % Natronlauge, Wasser, trocknet und rotiert ein.

Die Reinigung erfolgt mittels Säulenchromatographie (Kieselgel, Dichlormethan/Aceton 95:5).

Ausbeute: 0,23 g (98 % d.Th.)
logP 5.37
¹H-NMR (400 MHz, CD₃CN): δ = 1.02 (d, 6H, CH(CH₃)₂), 3.31 (s, 3H, OCH₃), 3.50
(m, 1H, 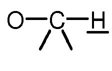 ) ppm

In Analogie zu Beispiel (I-2-b-1) und gemäß den allgemeinen Angaben zur Herstellung erhält man folgende Verbindungen der Formel (I-2-b)

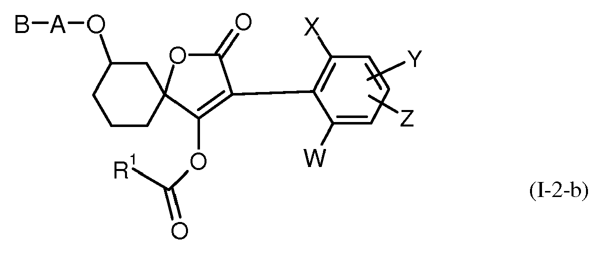

| **Bsp.-Nr.** | **W** | **X** | **Y** | **Z** | **A** | **B** | **R¹** | **logP** | **Isomer** |
|---|---|---|---|---|---|---|---|---|---|
| I-2-b-2 | H | CH₃ | 5-(4-Cl-Ph) | 4-CH₃ | CH₂ | H | i-C₃H₇ | 5.25 | trans |
| I-2-b-3 | CH₃ | CH₃ | 4-CH₃ | H | CH₂ | H | i-C₃H₇ | 4.32 | cis |
| I-2-b-4 | CH₃ | CH₃ | 4-CH₃ | H | CH₂ | H | i-C₃H₇ | 4.15 | trans |
| I-2-b-5 | CH₃ | OCH₃ | 4-CH₃ | H | CH₂ | H | i-C₃H₇ | 3.90 | cis |
| I-2-b-6 | CH₃ | OCH₃ | 4-CH₃ | H | CH₂ | H | i-C₃H₇ | 3.75 | trans |
| I-2-b-7 | H | CH₃ | 4-Cl | H | CH₂ | H | i-C₃H₇ | 4.19,3.98 | α + β ca. 1:1 |
| I-2-b-8 | CH₃ | Cl | 4-Cl | H | CH₂ | H | i-C₃H₇ | 4.49,4.34 | α + β ca. 1:1 |

| | | | | | | | | | |
|---|---|---|---|---|---|---|---|---|---|
| Ph = 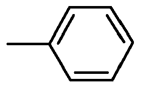 | | | | | | | | | |

### Beispiel (I-2-c-1)

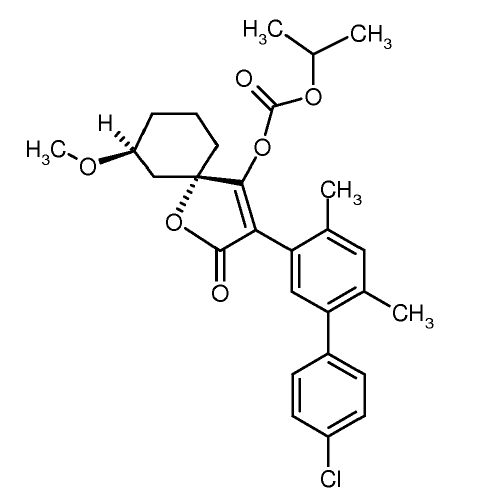

0,20 g (0,48 mmol) der Verbindung gemäß Beispiel I-2-a-32 (trans-Isomer) und 0,06 g (0,58 mmol) Triethylamin werden in 20 ml Dichlormethan vorgelegt, tropfenweise mit 0,58 ml (0,58 mmol) einer 1M Lösung von Isobuttersäurechlorid in Tetrahydrofuran versetzt und 12 h bei Raumtemperatur gerührt.

Zur Aufarbeitung wäscht man mit 10 % Citronensäure, 10 % Natronlauge, Wasser, trocknet und rotiert ein.

Die weitere Reinigung wird durchgeführt mittels Säulenchromatographie (Kieselgel, Dichlormethan/Aceton 95:5).
Ausbeute: 0,20 g (75 % d.Th.)
logP 5.21
¹H-NMR (400 MHz, CD₃CN): δ = 1.04 (d, 6H, CH(CH₃)₂), 3.28 (s, 3H, OCH₃), 3.51
(m, 1H, 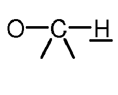 ) ppm

In Analogie zu Beispiel (I-2-c-1) und gemäß den allgemeinen Angaben zur Herstellung erhält man folgende Verbindungen der Formel (I-2-c)

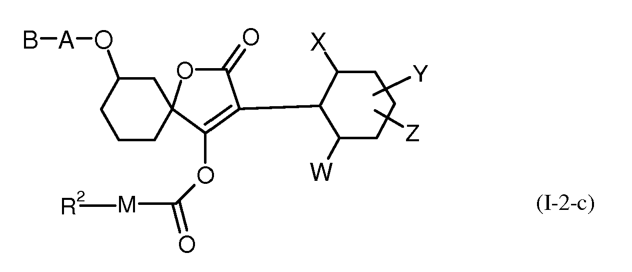

| **Bsp.-Nr.** | **W** | **X** | **Y** | **Z** | **A** | **B** | **M** | **R²** | **logP** | **Isomer** |
|---|---|---|---|---|---|---|---|---|---|---|
| I-2-c-2 | H | CH₃ | 5-(4-Cl-Ph) | 4-CH₃ | CH₂ | H | O | i-C₃H₇ | 5.32 | cis |
| I-2-c-3 | CH₃ | CH₃ | 4-CH₃ | H | CH₂ | H | O | i-C₃H₇ | 4.17 | trans |
| I-2-c-4 | CH₃ | CH₃ | 4-CH₃ | H | CH₂ | H | O | i-C₃H₇ | 4.34 | cis |
| I-2-c-5 | CH₃ | OCH₃ | 4-CH₃ | H | CH₂ | H | O | i-C₃H₇ | 3.75 | trans |
| I-2-c-6 | CH₃ | OCH₃ | 4-CH₃ | H | CH₂ | H | O | i-C₃H₇ | 3.90 | cis |

Ph =

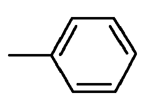

### Verfahren (J)

### Synthese von 7-Alkoxy-1,3-diazaspiro-[4,5]-decan-2,4-dionen der Formel (XXI) als Vorstufen von 1-Amino-3-alkoxycyclohexancarbonsäuren der Formel (XVII)

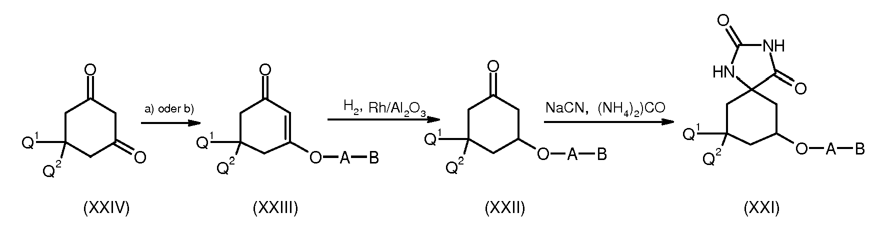

Darstellung von 3-Methoxycyclohex-2-enon (XXIII-1)Variante a)

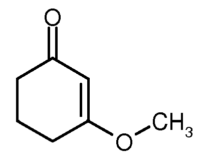

In einem 2L-Dreihalskolben werden 100 g (0,89 mol) Cyclohexan-1,3-dion vorgelegt, in 300 ml Methanol, 1000 ml Toluol und 97,6 ml (0,89 mol) Trimethylorthoformiat gelöst, mit 5 g p-Toluolsulfonsäure-Dihydrat versetzt und 2 h unter Rückfluss erhitzt. Nach Abkühlen wird 4 x mit je 200 ml 10 %iger NaOH gewaschen und die organische Phase über Natriumsulfat getrocknet und am Rotationsdampfer konzentriert. Man erhält 73,4 g eines hellbraunen Öls, welches ohne weitere Reinigung im Folgeschritt eingesetzt wird.

Darstellung von 3-Propoxycyclohex-2-enon (XXIII-2) Variante b)

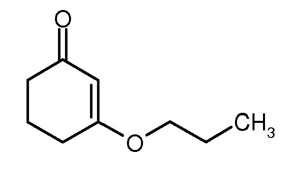

In einem 2L-Dreihalskolben werden 100 g (0,89 mol) Cyclohexan-1,3-dion vorgelegt, in 166,6 ml (2,23 mol) n-Propanol und 600 ml Toluol und 97,6 ml (892 mmol) Trimethylorthoformiat gelöst, mit 5 g p-Toluolsulfonsäure-Dihydrat versetzt und 5 h unter Rückfluss am Wasserabscheider gerührt, bis kein Wasser mehr abgeschieden wird. Anschließend wird die Lösung unter vermindertem Druck am Rotationsdampfer konzentriert, der Rückstand in 400 ml MTBE aufgenommen und dreimal mit 100 ml 10 %iger NaOH und gesättigter NaCl-Lösung gewaschen. Die organische Phase wird über Natriumsulfat getrocknet und am Rotationsdampfer konzentriert. Man erhält 124,8 g Ausbeute eines gelben Öls, welches ohne weitere Reinigung im Folgeschritt eingesetzt wird.

In Analogie zu den Beispielen (XXIII-1) und (XXIII-2) und den gemäß in der Literatur beschriebenen weiteren Verfahren erhält man folgende Verbindungen der Formel (XXIII)

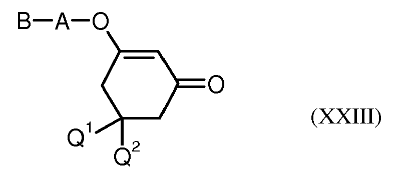

| **Bsp.-Nr.** | **A** | **B** | **Q¹** | **Q²** |
|---|---|---|---|---|
| XXIII-3 | CH₂ | CH₃ | H | H |
| XXIII-4 | CH₂ | C₃H₇ | H | H |
| XXIII-5 | CH₂ | H | CH₃ | CH₃ |
| XXIII-6 | CH₂ | i-C₃H₇ | CH₃ | CH₃ |
| XXIII-7 | CH₂ | 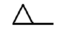 | H | H |
| XXIII-8 | CH₂ | 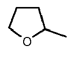 | H | H |

### Beispiel XXII-1

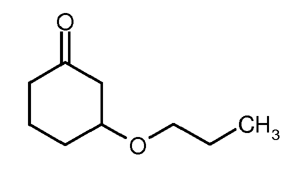

122 g (0,791 mol) 3-Propoxycyclohex-2-enon (XXIII-2) werden in 1200 ml Ethylacetat gelöst, mit 12,2 g Rh/Al₂O₃ (5 % Rh) versetzt und im Autoklaven unter 6,5 bar Wasserstoffdruck 9 h bei Raumtemperatur hydriert. Der Katalysator wird abfiltriert und mit Ethylacetat gewaschen und die Lösung am Rotationsdampfer konzentriert. Das so erhaltene braune Öl wird im Hochvakuum destilliert. Man erhält 2 Fraktionen von je 47 g (99 % rein) bzw. 47,7 g (78 % rein; enthält 21 % 3-Propoxycyclohexan-1-ol als einzige Verunreinigung) entsprechend einer Gesamtausbeute von 68 %.

In Analogie zu Beispiel (XXII-1) und gemäß den literaturbekannten Angaben zur Hydrierung von Verbindungen der Formel (XXIII) erhält man folgende Verbindungen der Formel (XXII)

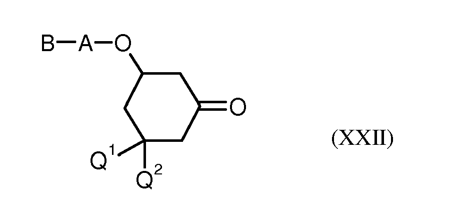

| **Bsp.-Nr.** | **A** | **B** | **Q¹** | **Q²** |
|---|---|---|---|---|
| XXII-2 | CH₂ | H | H | H |
| XXII-3 | CH₂ | CH₃ | H | H |
| XXII-4 | CH₂ | C₃H₇ | H | H |
| XXII-5 | CH₂ | H | CH₃ | CH₃ |
| XXII-6 | CH₂ | i-C₃H₇ | CH₃ | CH₃ |
| XXII-7 | CH₂ | 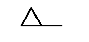 | H | H |
| XXII-8 | CH₂ | 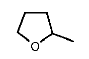 | H | H |

### Beispiel XXI-1

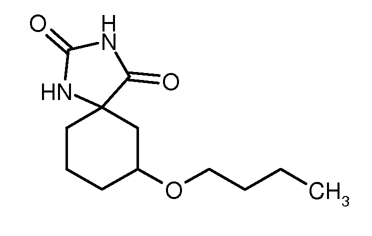

Es werden 18,4 g (1.1 Eq.) NaCN und 154,2 g (4,7 Eq.) Ammoniumcarbonat in 612 ml Wasser vorgelegt. Bei Raumtemperatur wird 61,2 g (1 Eq.) der Verbindung gemäß Beispiel (XXII-4) gelöst in 612 ml Ethanol langsam zugetropft. Nach 16 h bei 55-60°C wird auf Raumtemperatur abgekühlt und am Rotationsverdampfer bis zur Trockne einrotiert.

Der Feststoff wird in 300 ml Ethanol 30 min. ausgerührt, die Lösung abdekantiert und das Ausrühren wiederholt. Vereinigte Ethanolphasen über MgSO₄ trocknen, abnutschen, einrotieren.
Ausbeute: 61,4 g (88 % der Theorie)
¹H-NMR (400 MHz, DMSO): 7.70 (bs, 1H); 6.66 (bs, 1H); 3.70-3.76 (m, 0,5H); 3.31-3.43 (m, 2.5H); 1.91-1.99 (m, 0.5H); 1.82-1.88 (m, 0.5H); 1,26-1.75 (bm, 11H); 0.86-0.92 (m, 3H)

In Analogie zu Beispiel (XXI-1) und gemäß den in der Literatur beschriebenen Verfahren zur Herstellung (z.B. L. Munday, J.Chem. Soc. 4372 (1961)) erhält man folgende Verbindungen der Formel (XXI)

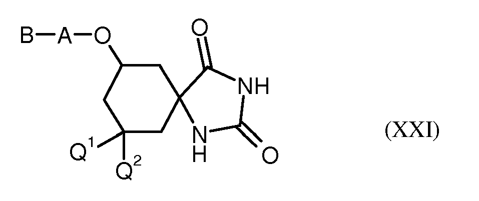

| **Bsp.-Nr.** | **A** | **B** | **Q¹** | **Q²** | ¹H-NMR (400 MHz, DMSO): Verschiebung δ in ppm |
|---|---|---|---|---|---|
| XXI-2 | CH₂ | H | H | H | 7.67 (bs, 1H); 6.80 (bs, 1H); 3,59-3.66 (m, 0.5H); 3.25-3.35 (m, 0.5H) 3.22 (s, 1.5H); 3.18 (s, 1.5H); 1.95-2.01 (m, 0.5H); 1.80-1.87 (dd, 0.5H); 1,27-1,75 (bm, 7H) |
| XXI-3 | CH₂ | CH₃ | H | H | 7.97 (bs, 1H); 7.06 (bs, 1H); 3.70-3.76 (m, 0.5H); 3.34-3.45 (bm, 2.5H); 1.91-1.98 (m, 0.5H); 1.82-1.89 (dd, 0.5H); 1,58-1.77 (bm, 3H); 1.32-1.58 (bm, 4H); 1.02-1.12 (m, 3H) |
| XXI-4 | CH₂ | C₃H₇ | H | H | 7.98 (bs, 1H); 6.98 (bs, 1H); 3.70-3.76 (m, 0.5H); 3.3-3.43 (m, 2.5H); 1.92-2.00 (m, 0.5H); 1.85-1.91 (dd, 0.5H); 1.58-1.80 (bm, 3H); 1.35-1.56 (bm, 6H); 0.82-0.9 (m, 3H) |
| XXI-5 | CH₂ | H | CH₃ | CH₃ | 0.9, 0.99 (2s, 6H, 2 x CH₃); 3.21 (s, 3H, OCH₃) |
| XXI-6 | CH₂ | i-C₃H₇ | CH₃ | CH₃ | 0.82, (d, 6H, CH(CH₃)₂); 0.90, 0.99 (2s, 6H, 2 x CH₃) |
| XXI-7 | CH₂ | 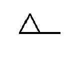 | H | H | 0.01, 0.30 (2m, 4H, 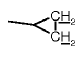 ); 3.08 (m, 2H,-O-CH₂ 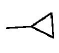 ); 3.32, 3.62 (2m, 1H, CH-O) |
| XXI-8 | CH₂ | 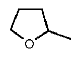 | H | H | 2.30-2.90 (mehrere Multipletts, 12H, CH₂); 3.30-4.0 (mehrere Multipletts, 6H, 2 x O-CH, 2 x OCH₂); 8.26 (bs, 1H, NH) |

| | | | | | |
|---|---|---|---|---|---|
| * Die Bestimmung der in den voran stehenden Tabellen und Herstellungsbeispielen angegebenen logP-Werte erfolgt gemäß EEC-Directive 79/831 Annex V.A8 durch HPLC (High Performance Liquid Chromatography) an einer Phasenumkehrsäule (C 18). Temperatur: 43°C. | | | | | |

Die Bestimmung erfolgt im sauren Bereich bei pH 2.3 mit 0,1 % wässriger Phosphorsäure und Acetonitril als Eluenten; linearer Gradient von 10 % Acetonitril bis 95 % Acetonitril.

Die Bestimmung mit der LC-MS im sauren Bereich erfolgt bei pH 2,7 mit 0,1 % wässriger Ameisensäure und Acetonitril (enthält 0,1 % Ameisensäure) als Eluenten; linearer Gradient von 10 % Acetonitril bis 95 % Acetonitril.

Die Bestimmung mit der LC-MS im neutralen Bereich erfolgt bei pH 7.8 mit 0,001 molarer wässriger Ammoniumhydrogencarbonat-Lösung und Acetonitril als Eluenten; linearer Gradient von 10 % Acetonitril bis 95 % Acetonitril.

Die Eichung erfolgt mit unverzweigtem Alkan-2-onen (mit 3 bis 16 Kohlenstoffatomen), deren logP-Werte bekannt sind (Bestimung der logP-Werte anhand der Retentionszeiten durch lineare Interpolation zwischen zwei aufeinander folgenden Alkanonen).

Die lambda-max-Werte wurde an Hand der UV-Spektren von 200 nm bis 400 nm in den Maxima der chromatographischen Signale ermittelt.

### Beispiel A

### Myzus-Test (Spritzbehandlung)

| | |
|---|---|
| Lösungsmittel: | 78 Gewichtsteile Aceton |
| | 1,5 Gewichtsteile Dimethylformamid |
| Emulgator: | 0,5 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Chinakohlblattscheiben *(Brassica pekinensis),* die von allen Stadien der Grünen Pfirsichblattlaus *(Myzus persicae)* befallen sind, werden mit einer Wirkstoffzubereitung der gewünschten Konzentration gespritzt.

Nach der gewünschten Zeit wird die Wirkung in % bestimmt. Dabei bedeutet 100 %, dass alle Blattläuse abgetötet wurden; 0 % bedeutet, dass keine Blattläuse abgetötet wurden.

Bei diesem Test zeigen die Verbindungen der Herstellungsbeispiele I-1-a-1, I-1-a-2, I-1-a-6, I-1-a-7, I-1-a-8, I-1-a-9, I-1-a-10, I-1-a-12, I-1-a-14, I-1-a-15, I-1-a-16, I-1-a-17, I-1-a-18, I-1-a-19, I-1-a-20, I-1-a-21, I-1-a-22, I-1-a-24, I-1-a-25, I-1-a-26, I-1-a-27, I-1-a-28, I-1-a-29, I-1-a-30, I-1-a-32, I-1-a-35, I-1-a-36, I-1-a-40, I-1-a-51, I-1-a-52, I-2-a-1, I-2-a-2, I-2-a-4, I-2-a-5, I-2-a-6, I-2-a-7, I-2-a-8, I-2-a-9, I-2-a-10, I-2-a-11, I-2-a-12, I-2-a-13, I-2-a-14, I-2-a-15, I-2-a-16, I-2-a-18, I-2-a-20, I-2-a-21, I-2-a-22, I-2-a-26, I-2-a-27, I-2-a-28, I-2-a-29, I-2-a-31, I-2-a-32, I-2-a-33, I-2-a-34, I-2-a-35, I-2-a-36, I-2-a-37, I-2-a-39, I-2-a-42, I-2-a-45, I-2-a-46, I-2-a-47, I-2-a-49, I-2-a-50, I-2-a-52, I-2-a-54, I-2-b-1, I-2-b-2, I-2-b-3, I-2-b-4, I-2-b-5, I-2-b-6, I-1-c-2, I-1-c-3, I-2-c-1, I-2-c-2, I-2-c-3, I-2-c-4, 1-2-c-5 und I-2-c-6 mit Wirkstoffkonzentrationen von 500 g/ha eine Abtötung gegen Myzus persicae von ≥90 %.

### Beispiel B

### Phaedon-Test (Spritzbehandlung)

| | |
|---|---|
| Lösungsmittel: | 78 Gewichtsteile Aceton |
| | 1,5 Gewichtsteile Dimethylformamid |
| Emulgator: | 0,5 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Chinakohlblattscheiben *(Brassica pekinensis)* werden mit einer Wirkstoffzubereitung der gewünschten Konzentration gespritzt und nach dem Abtrocknen mit Larven des Meerrettichblattkäfers *(Phaedon cochleariae)* besetzt.

Nach der gewünschten Zeit wird die Wirkung in % bestimmt. Dabei bedeutet 100 %, dass alle Käferlarven abgetötet wurden; 0 % bedeutet, dass keine Käferlarven abgetötet wurden.

Bei diesem Test zeigen Verbindungen der Herstellungsbeispiele I-1-a-1, I-1-a-2, I-1-b-1, I-1-a-5, I-I-a-6, I-1-a-8, I-1-a-14, I-1-a-15, I-1-a-17, I-1-a-19, I-1-a-20, I-1-a-21, I-1-a-22, I-1-a-24, I-1-a-26, I-I-a-28, I-1-a-29, I-1-a-30, I-1-a-31, I-1-a-36, I-1-a-38, I-1-a-44, I-1-a-46, I-1-a-51, I-2-a-1, I-2-a-2, I-2-a-12, I-2-a-14, I-2-a-16, I-2-a-17, I-2-a-31, I-2-a-33, I-2-a-42 und I-2-b-1 mit Wirkstoffkonzentrationen von 500 g/ha eine Abtötung gegen Phaedon cochleariae von ≥90 %.

### Beispiel C

### Spodoptera frugiperda-Test (Spritzbehandlung)

| | |
|---|---|
| Lösungsmittel: | 78 Gewichtsteile Aceton |
| | 1,5 Gewichtsteile Dimethylformamid |
| Emulgator: | 0,5 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Maisblattscheiben (Zea mays) werden mit einer Wirkstoffzubereitung der gewünschten Konzentration gespritzt und nach dem Abtrocknen mit Raupen des Heerwurms (Spodoptera frugiperda) besetzt.

Nach der gewünschten Zeit wird die Wirkung in % bestimmt. Dabei bedeutet 100 %, dass alle Raupen abgetötet wurden; 0 % bedeutet, dass keine Raupe abgetötet wurde.

Bei diesem Test zeigen die Verbindungen der Herstellungsbeispiele I-1-c-3, I-1-a-6, I-1-a-36, I-2-a-8, I-2-a-11, I-2-a-14, I-2-a-31, I-2-a-32, I-2-b-3 und I-2-c-4 mit Wirkstoffkonzentrationen von 500 g/ha eine Abtötung gegen Spodoptera frugiperda von ≥80 %.

### Beispiel D

### Tetranychus-Test (OP-resistent/Spritzbehandlung)

| | |
|---|---|
| Lösungsmittel: | 78 Gewichtsteile Aceton |
| | 1,5 Gewichtsteile Dimethylformamid |
| Emulgator: | 0,5 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Bohnenblattscheiben *(Phaseolus vulgaris),* die von allen Stadien der gemeinen Spinnmilbe *(Tetranychus urticae)* befallen sind, werden mit einer Wirkstoffzubereitung der gewünschten Konzentration gespritzt.

Nach der gewünschten Zeit wird die Wirkung in % bestimmt. Dabei bedeutet 100 %, dass alle Spinnmilben abgetötet wurden; 0 % bedeutet, dass keine Spinnmilben abgetötet wurden.

Bei diesem Test zeigen die Verbindungen der Herstellungsbeispiele I-1-a-1, I-1-a-6, I-1-a-7, I-1-a-10, I-1-a-14, I-1-a-15, I-1-a-18, I-1-a-26, I-1-a-28, I-1-a-29, I-1-a-30, I-1-a-32, I-1-a-33, I-1-a-35, I-1-a-36, I-1-a-40, I-1-a-42, I-1-a-45, I-1-a-46, I-1-a-51, I-1-a-52, I-2-a-1, I-2-a-2, I-2-a-16, I-2-a-42, I-2-b-1, I-2-b-3, I-2-b-4, I-2-b-5, I-2-b-6, I-2-c-1, I-2-c-2, I-2-c-3, I-2-c-4, I-2-c-5, I-2-c-6, I-1-c-2, I-1-c-1, I-1-c-3, I-1-a-5 und I-1-a-4 mit Wirkstoffkonzentrationen von 100 g/ha eine abtötende Wirkung gegen Tetranychus urticae von ≥70 %.

### Beispiel E

### Grenzkonzentrations-Test / Bodeninsekten-Behandlung transgener Pflanzen

| | |
|---|---|
| Testinsekt: | **Diabrotica balteata - Larven im Boden** |
| Lösungsmittel: | 7 Gewichtsteile Aceton |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird auf den Boden gegossen. Dabei spielt die Konzentration des Wirkstoffs in der Zubereitung praktisch keine Rolle, entscheidend ist allein die Wirkstoffgewichtsmenge pro Volumeneinheit Boden, welche in ppm (mg/l) angegeben wird. Man füllt den Boden in 0,25 1 Töpfe und lässt diese bei 20°C stehen.

Sofort nach dem Ansatz werden je Topf 5 vorgekeimte Maiskörner der Sorte YIELD GUARD (Warenzeichen von Monsanto Domp., USA) gelegt. Nach 2 Tagen werden in den behandelten Boden die entsprechenden Testinsekten gesetzt. Nach weiteren 7 Tagen wird der Wirkungsgrad des Wirkstoffs durch Auszählen der aufgelaufenen Maispflanzen bestimmt (1 Pflanze = 20 % Wirkung).

### Beispiel F

### Heliothis virescens - Test - Behandlung transgener Pflanzen

| | |
|---|---|
| Lösungsmittel: | 7 Gewichtsteile Aceton |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Sojatriebe (Glycine max) der Sorte Roundup Ready (Warenzeichen der Monsanto Comp. USA) werden durch Tauchen in die Wirkstoffzubereitung der gewünschten Konzentration behandelt und mit der Tabakknospenraupe Heliothis virescens besetzt, solange die Blätter noch feucht sind.

Nach der gewünschten Zeit wird die Abtötung der Insekten bestimmt.

### Beispiele G

### Herbizide Wirkung im Vorauflauf

Samen von mono- bzw. dikotylen Unkraut- bzw. Kulturpflanzen werden in Holzfasertöpfen in sandiger Lehmerde ausgelegt und mit Erde abgedeckt. Die in Form von benetzbaren Pulvern (WP) oder als Emulsionskonzentrate (EC) formulierten Testverbindungen werden dann als wässrige Suspension mit einer Wasseraufwandmenge von umgerechnet 800 l/ha unter Zusatz von 0,2 % Netzmittel auf die Oberfläche der Abdeckerde appliziert.

Nach Behandlung werden die Töpfe im Gewächshaus aufgestellt und unter guten Wachstumsbedingungen für die Testpflanzen gehalten. Die visuelle Bonitur der Schäden an den Versuchspflanzen erfolgt nach einer Versuchszeit von 3 Wochen im Vergleich zu unbehandelten Kontrollen (herbizide Wirkung in Prozent (%): 100 % Wirkung = Pflanzen sind abgestorben, 0 % Wirkung = wie Kontrollpflanzen).

### Herbizide Wirkung im Nachauflauf

Samen von mono- bzw. dikotylen Unkraut- bzw. Kulturpflanzen werden in Holzfasertöpfen in sandigem Lehmboden ausgelegt, mit Erde abgedeckt und im Gewächshaus unter guten Wachstumsbedingungen angezogen. 2 bis 3 Wochen nach der Aussaat werden die Versuchspflanzen im Einblattstadium behandelt. Die in Form von benetzbaren Pulvern (WP) oder als Emulsionskonzentrate (EC) formulierten Testverbindungen werden dann als wässrige Suspension mit einer Wasseraufwandmenge von umgerechnet 800 l/ha unter Zusatz von 0,2 % Netzmittel auf die grünen Pflanzenteile gesprüht. Nach ca. 3 Wochen Standzeit der Versuchspflanzen im Gewächshaus unter optimalen Wachstumsbedingungen wird die Wirkung der Präparate visuell im Vergleich zu behandelten Kontrollen bonitiert (herbizide Wirkung in Prozent (%): 100 % Wirkung = Pflanzen sind abgestorben, 0 % Wirkung = wie Kontrollpflanzen).

Folgende Verbindungen zeigen im Vorauflauf mit 320 g/ha a.i. gegen Avena sativa, Lolium multiflorum und Setaria viridis eine Wirkung von ≥ 80 %:
I-1-a-12, I-1-a-17, I-1-a-36, I-1-a-63, I-1-a-71.

Folgende Verbindungen zeigen im Nachauflauf mit 320 g/ha a.i. gegen Avena sativa, Lolium multiflorum und Setaria viridis und Echinochloa eine Wirkung von ≥ 70 %:
I-1-a-1, I-1-a-12, I-1-a-14, I-1-a-17, I-1-a-18, I-1-a-36, I-1-a-40, I-1-a-47, I-1-a-66, I-1-a-67, I-1-a-63, I-1-a-71, I-1-a-49, I-1-a-64, I-1-a-79, I-1-b-1, I-1-b-26, I-1-c-1, I-1-c-2, I-1-c-3, I-1-c-18, I-2-a-3, I-2-a-13.

### Beispiele H

### Herbizide Wirkung im Nachauflauf

Samen von mono- bzw. dikotylen Unkraut- bzw. Kulturpflanzen werden in Holzfasertöpfen oder in Plastiktöpfen in sandigem Lehmerde ausgelegt, mit Erde abgedeckt und im Gewächshaus, während der Vegetationsperiode auch im Freien ausserhalb des Gewächshauses, unter guten Wachstumsbedingungen angezogen. 2- 3 Wochen nach der Aussaat werden die Versuchspflanzen im Ein- bis Drei-Blattstadium behandelt. Die als Spritzpulver (WP) oder Flüssigkeit (EC) formulierten Testverbindungen werden in verschiedenen Dosierungen mit einer Wasseraufwandmenge von umgerechnet 300 l/ha unter Zusatz von Netzmittel (0,2 bis 0,3 %) auf die Pflanzen und die Bodenoberfläche gespritzt. 3 bis 4 Wochen nach Behandlung der Versuchspflanzen wird die Wirkung der Präparate visuell im Vergleich zu unbehandelten Kontrollen bonitiert (herbizide Wirkung in Prozent (%): 100 % Wirkung = Pflanzen sind abgestorben, 0 % Wirkung = wie Kontrollpflanzen).

### Verwendung von Safenern

Soll zusätzlich getestet werden, ob Safener die Pflanzenverträglichkeit von Testsubstanzen bei den Kulturpflanzen verbessern können, werden folgende Möglichkeiten für die Anwendung des Safeners verwendet:
- Samen der Kulturpflanzen werden vor der Aussaat mit der Safenersubstanz gebeizt (Angabe der Safenermenge in Prozent bezogen auf das Samengewicht)
- Kulturpflanzen werden vor der Anwendung der Testsubstanzen mit dem Safener mit einer bestimmten Hektaraufwandmenge gespritzt (üblicherweise 1 Tag vor Anwendung der Prüfsubstanzen)
- der Safener wird zusammen mit der Testsubstanz als Tankmischung appliziert (Angabe der Safenermenge in g/ha oder als Verhältnis zum Herbizid).

Durch Vergleich der Wirkung von Testsubstanzen auf Kulturpflanzen, welche ohne und mit Safener behandelt wurden, kann die Wirkung der Safenersubstanz beurteilt werden.

### Gefäßversuche mit Getreide im Gewächshaus

### Mefenpyr 1 Tag vor Herbizidapplikation

**Tabelle**

| | Aufwandmenge | Sommerweizen |
|---|---|---|
| | g a.i./ha | beobachtet (%) |
| Bsp.- I-1-a-2 | 100 | 60 |
| Bsp.- I-1-a-2 | 100 + 100 | 15 |
| + Mefenpyr | | |

**Tabelle**

| | Aufwandmenge | Sommergerste | Sommerweizen |
|---|---|---|---|
| | g a.i./ha | beobachtet (%) | beobachtet (%) |
| Bsp.- I-1-a-12 | 12,5 | 99 | 97 |
| | 6,25 | 97 | 10 |
| | 3,125 | 30 | |
| Bsp.- I-1-a-12 | 12,5 + 100 | 30 | 20 |
| + Mefenpyr | 6,25 + 100 | 5 | 5 |
| | 3,125 + 100 | 0 | |

**Tabelle**

| | Aufwandmenge | Sommergerste | Sommerweizen |
|---|---|---|---|
| | g a.i./ha | beobachtet (%) | beobachtet (%) |
| Bsp.- I-1-a-14 | 25 | 93 | 93 |
| | 12,5 | 30 | 30 |
| Bsp.- I-1-a-14 | 25 + 100 | 30 | 50 |
| + Mefenpyr | 12,5 + 100 | 10 | 15 |

**Tabelle**

| | Aufwandmenge | Sommerweizen |
|---|---|---|
| | g a.i./ha | beobachtet (%) |
| Bsp.- I-1-a-17 | 25 | 70 |
| | 12,5 | 60 |
| | 6,25 | 10 |
| Bsp.- I-1-a-17 | 25 + 100 | 30 |
| + Mefenpyr | 12,5 + 100 | 10 |
| | 6,25 + 100 | 0 |

**Tabelle**

| | Aufwandmenge | Sommergerste | Sommerweizen |
|---|---|---|---|
| | g a.i./ha | beobachtet (%) | beobachtet (%) |
| Bsp.- I-1-a-18 | 12,5 | 93 | 95 |
| | 6,25 | 50 | 40 |
| | 3,125 | | 30 |
| Bsp.- I-1-a-18 | 12,5 + 100 | 40 | 40 |
| + Mefenpyr | 6,25 + 100 | 5 | 5 |
| | 3,125 + 100 | | 0 |

**Tabelle**

| | Aufwandmenge | Sommergerste |
|---|---|---|
| | g a.i./ha | beobachtet (%) |
| Bsp.- I-1-a-19 | 100 | 30 |
| | 50 | 25 |
| Bsp.- I-1-a-19 | 100 + 100 | 10 |
| + Mefenpyr | 50 + 100 | 0 |

**Tabelle**

| | Aufwandmenge | Sommergerste | Sommerweizen |
|---|---|---|---|
| | g a.i./ha | beobachtet (%) | beobachtet (%) |
| Bsp.- I-1-b-8 | 50 | 70 | 50 |
| | 25 | 30 | 30 |
| Bsp.- I-1-b-8 | 50 + 100 | 20 | 20 |
| + Mefenpyr | 25 + 100 | 10 | 10 |

**Tabelle**

| | Aufwandmenge | Sommergerste | Sommerweizen |
|---|---|---|---|
| | g a.i./ha | beobachtet (%) | beobachtet (%) |
| Bsp.- I-1-b-9 | 50 | 90 | 90 |
| | 25 | 30 | 20 |
| Bsp.- I-1-b-9 | 50 + 100 | 20 | 10 |
| + Mefenpyr | 25 + 100 | 10 | 0 |

**Tabelle**

| | Aufwandmenge | Sommerweizen |
|---|---|---|
| | g a.i./ha | beobachtet (%) |
| Bsp.- I-1-c-9 | 100 | 60 |
| | 50 | 30 |
| Bsp.- I-1-c-9 | 100 + 100 | 20 |
| + Mefenpyr | 50 + 100 | 10 |

**Tabelle**

| | Aufwandmenge | Sommerweizen |
|---|---|---|
| | g a.i./ha | beobachtet (%) |
| Bsp.- I-1-c-10 | 12,5 | 60 |
| Bsp.- I-1-c-10 | 12,5 + 100 | 10 |
| + Mefenpyr | | |

**Tabelle**

| | Aufwandmenge | Sommerweizen |
|---|---|---|
| | g a.i./ha | beobachtet (%) |
| Bsp.- I-1-b-11 | 50 | 70 |
| | 25 | 10 |
| Bsp.- I-1-b-11 | 50 + 100 | 10 |
| + Mefenpyr | 25 + 100 | 5 |

**Tabelle**

| | Aufwandmenge | Sommerweizen |
|---|---|---|
| | g a.i./ha | beobachtet (%) |
| Bsp.- I-1-b-14 | 6,25 | 30 |
| | 3,125 | 20 |
| Bsp.- I-1-b-14 | 6,25 + 100 | 0 |
| + Mefenpyr | 3.125 + 100 | 0 |

### Gefäßversuche mit Mais im Gewächshaus

### Isoxadifen 1 Tag vor Herbizidapplikation

**Tabelle**

| | Aufwandmenge | Cecilia Mais |
|---|---|---|
| | g a.i./ha | beobachtet (%) |
| Bsp.- I-1-c-10 | 12,5 | 40 |
| Bsp.- I-1-c-10 | 12,5 + 100 | 15 |
| + Isoxadifen | | |

### Gefäßversuche mit Getreide im Freiland

### Mefenpyr 1 Tag vor Herbizidapplikation

**Tabelle**

| | Aufwandmenge | Sommergerste | Sommerweizen |
|---|---|---|---|
| | g a.i./ha | beobachtet (%) | beobachtet (%) |
| Bsp.- I-1-a-43 | 25 | 70 | 60 |
| | 12,5 | 10 | 30 |
| Bsp.- I-1-a-43 | 25 + 100 | 5 | 0 |
| + Mefenpyr | 12,5 + 100 | 0 | 0 |

**Tabelle**

| | Aufwandmenge | Sommergerste | Sommerweizen |
|---|---|---|---|
| | g a.i./ha | beobachtet (%) | beobachtet (%) |
| Bsp.- I-1-a-43 | 100 | 30 | 30 |
| | 50 | 20 | 15 |
| Bsp.- I-1-a-43 | 100 + 100 | 0 | 0 |
| + Mefenpyr | 50 + 100 | 0 | 0 |

### Gefäßversuche mit Getreide im Freiland

### Mefenpyr in Tankmischung 50 g/ha

**Tabelle**

| | Aufwandmenge | Sommergerste | Sommerweizen |
|---|---|---|---|
| | g a.i./ha | beobachtet (%) | beobachtet (%) |
| Bsp.- I-1-a-1 | 100 | 70 | 35 |
| Bsp.- I-1-a-1 | 100 + 50 | 20 | 15 |
| + Mefenpyr | | | |

**Tabelle**

| | Aufwandmenge | Sommergerste | Sommerweizen |
|---|---|---|---|
| | g a.i./ha | beobachtet (%) | beobachtet (%) |
| Bsp.- I-1-a-43 | 100 | 60 | 60 |
| | 50 | 50 | 50 |
| | 25 | 40 | 40 |
| Bsp.- I-1-a-43 | 100 + 50 | 25 | 10 |
| + Mefenpyr | 50 + 50 | 25 | 5 |
| | 25 + 50 | 5 | 0 |

### Methode: Tankmix Post-emergence

**Tabelle**

| | Aufwandmenge | Sommergerste | Wintergerste |
|---|---|---|---|
| | g a.i./ha | (Baronesse) | beobachtet (%) |
| | | beobachtet (%) | |
| Bsp.- I-1-a-43 | 80 | 30 | 30 |
| Bsp.- I-1-a-43 | 80 + 100 | 0 | 5 |
| + Cloquintocet | | | |

**Tabelle**

| | Aufwandmenge | Sommergerste | Wintergerste |
|---|---|---|---|
| | g a.i./ha | (Scarlett) | beobachtet (%) |
| | | beobachtet (%) | |
| Bsp.- I-1-a-43 | 20 | 30 | 20 |
| Bsp.- I-1-a-43 | 20 + 100 | 10 | 10 |
| + Isoxadifen | | | |

## Patentansprüche

1. Verbindungen der Formel (I) 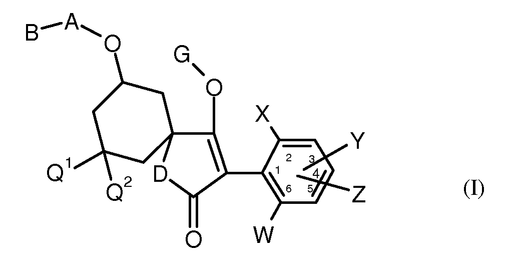 in welcher
W für Wasserstoff, Alkyl, Halogen, Alkoxy, Halogenalkyl, Halogenalkoxy oder Cyano steht,
X für Halogen, Alkyl, Alkoxy, Halogenalkyl, Halogenalkoxy oder Cyano steht,
Y in der 4-Position für Wasserstoff, Halogen, Alkoxy, Cyano, Halogenalkyl oder Halogenalkoxy steht,
Z für Wasserstoff steht,
W auch für Wasserstoff, Halogen oder Alkyl steht,
X auch für Halogen, Alkyl, Alkoxy, Halogenalkyl, Halogenalkoxy oder Cyano steht,
Y in der 4-Position auch für gegebenenfalls substituiertes Phenyl oder Hetaryl steht,
Z auch für Wasserstoff steht,
W ebenfalls für Wasserstoff, Halogen oder Alkyl steht,
X ebenfalls für Halogen, Alkyl, Alkoxy, Halogenalkyl, Halogenalkoxy oder Cyano steht,
Y ebenfalls in der 5-Position für gegebenenfalls substituiertes Phenyl oder Hetaryl steht,
Z in der 4-Position ebenfalls für Wasserstoff, Alkyl oder Halogen steht,
W außerdem für Wasserstoff, Methyl, Propyl, Isopropyl, Halogen, Cyano oder Trifluormethyl steht,
X außerdem für Halogen, Alkyl, Alkoxy, Halogenalkyl, Halogenalkoxy oder Cyano steht,
Y in der 4-Position außerdem für Alkyl steht,
Z außerdem für Wasserstoff steht,
W weiterhin für Wasserstoff, Halogen, Alkyl oder Alkoxy steht,
X weiterhin für Halogen, Alkyl, Alkoxy, Halogenalkyl, Halogenalkoxy oder Cyano steht,
Y in der 4-Position weiterhin für Wasserstoff, Halogen, Alkyl, Halogenalkyl oder Halogenalkoxy steht,
Z in der 3- oder 5-Position weiterhin für Halogen, Alkyl, Halogenalkyl, Cyano, Alkoxy oder Halogenalkox y steht,
A für eine gegebenenfalls substituierte Alkandiylgruppe oder für gegebenenfalls substituiertes und/oder gegebenenfalls durch ein Heteroatom unterbrochenes Cycloalkyl steht,
B für Wasserstoff oder jeweils gegebenenfalls substituiertes Alkyl, Alkenyl, Alkoxy, Alkoxyalkyloxy, Phenyl, Hetaryl oder für gegebenenfalls substituiertes und/oder gegebenenfalls durch Heteroatome und/oder C=O unterbrochenes Cycloalkyl steht,
D für Sauerstoff steht,
Q¹ für Wasserstoff, für jeweils gegebenenfalls substituiertes Alkyl, Alkoxy, Alkoxyalkyl oder Alkylthioalkyl, für gegebenenfalls substituiertes Cycloalkyl, worin gegebenenfalls eine Methylengruppe durch Heteroatome ersetzt ist oder für gegebenenfalls substituiertes Phenyl, Hetaryl, Phenylalkyl oder Hetarylalkyl steht,
Q² für Wasserstoff oder Alkyl steht,
Q¹ und Q² gemeinsam mit dem Kohlenstoff, an das sie gebunden sind für einen gegebenenfalls substituierten C₃-C₆-Ring stehen, der gegebenenfalls durch ein Heteroatom unterbrochen sein kann,
G für Wasserstoff (a) oder für eine der Gruppen 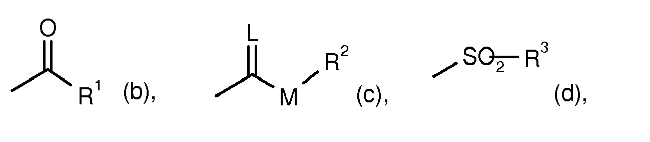 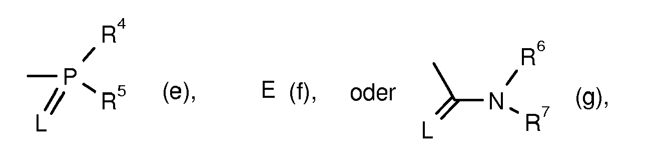 steht,
worin
E für ein Metallion oder ein Ammoniumion steht,
L für Sauerstoff oder Schwefel steht,
M für Sauerstoff oder Schwefel steht,
R¹ für jeweils gegebenenfalls durch Halogen oder Cyano substituiertes Alkyl, Alkenyl, Alkoxyalkyl, Alkylthioalkyl oder Polyalkoxyalkyl oder für jeweils gegebenenfalls durch Halogen, Alkyl oder Alkoxy substituiertes Cycloalkyl oder Heterocyclyl oder für jeweils gegebenenfalls substituiertes Phenyl, Phenylalkyl, Hetaryl, Phenoxyalkyl oder Hetaryloxyalkyl steht,
R² für jeweils gegebenenfalls durch Halogen oder Cyano substituiertes Alkyl, Alkenyl, Alkoxyalkyl oder Polyalkoxyalkyl oder für jeweils gegebenenfalls substituiertes Cycloalkyl, Phenyl oder Benzyl steht,
R³, R⁴ und R⁵ unabhängig voneinander für jeweils gegebenenfalls durch Halogen substituiertes Alkyl, Alkoxy, Alkylamino, Dialkylamino, Alkylthio, Alkenylthio oder Cycloalkylthio oder für jeweils gegebenenfalls substituiertes Phenyl, Benzyl, Phenoxy oder Phenylthio stehen,
R⁶ und R⁷ unabhängig voneinander für Wasserstoff, für jeweils gegebenenfalls durch Halogen oder Cyano substituiertes Alkyl, Cycloalkyl, Alkenyl, Alkoxy, Alkoxyalkyl, für jeweils gegebenenfalls substituiertes Phenyl oder Benzyl stehen, oder gemeinsam mit dem N-Atom, an das sie gebunden sind, einen gegebenenfalls Sauerstoff oder Schwefel enthaltenden und gegebenenfalls substituierten Cyclus bilden.

2. Verbindungen der Formel (I) gemäß Anspruch 1, in welcher
W für Wasserstoff, C₁-C₆-Alkyl, C₂-C₆-Alkenyl, C₂-C₆-Alkinyl, Halogen, C₁-C₆-Alkoxy, C₁-C₄-Halogenalkyl, C₁-C₄-Halogenalkoxy oder Cyano steht,
X für Halogen, C₁-C₆-Alkyl, C₂-C₆-Alkenyl, C₂-C₆-Alkinyl, C₁-C₆-Alkoxy, C₁-C₆-Alkoxy-C₁-C₄-alkoxy, C₁-C₄-Halogenalkyl, C₁-C₄-Halogenalkoxy oder Cyano steht,
Y in der 4-Position für Wasserstoff, Halogen, C₁-C₆-Alkoxy, Cyano, C₁-C₄-Halogenalkyl oder C₁-C₄-Halogenalkoxy steht,
Z für Wasserstoff steht,
W auch für Wasserstoff, Halogen oder C₁-C₆-Alkyl steht,
X auch für Halogen, C₁-C₆-Alkyl, C₁-C₆-Alkoxy, C₁-C₄-Halogenalkyl, C₁-C₄-Halogenalkoxy oder Cyano steht,
Y auch in der 4-Position für durch V¹ und V² substituiertes Phenyl oder Pyridyl steht,
Z auch für Wasserstoff steht,
V¹ auch für Halogen, C₁-C₁₂-Alkyl, C₁-C₆-Alkoxy, C₁-C₄-Halogenalkyl oder C₁-C₄-Halogenalkoxy steht,
V² auch für Wasserstoff, Halogen, C₁-C₆-Alkyl, C₁-C₆-Alkoxy oder C₁-C₄-Halogenalkyl steht,
V¹ und V² gemeinsam auch für C₃-C₄-Alkandiyl stehen, welches gegebenenfalls durch Halogen und/oder C₁-C₂-Alkyl substituiert sein kann und welches gegebenenfalls durch ein oder zwei Sauerstoffatome unterbrochen sein kann,
W ebenfalls für Wasserstoff, Halogen oder C₁-C₆-Alkyl steht,
X ebenfalls für Halogen, C₁-C₆-Alkyl, C₁-C₆-Alkoxy, C₁-C₄-Halogenalkyl, C₁-C₄-Halogenalkoxy oder Cyano steht,
Y ebenfalls in der 5-Position für durch V¹ und V² substituiertes Phenyl oder Pyridyl steht,
Z ebenfalls in der 4-Position für Wasserstoff, C₁-C₆-Alkyl oder Halogen steht,
V¹ ebenfalls für Halogen, C₁-C₁₂-Alkyl, C₁-C₆-Alkoxy, C₁-C₄-Halogenalkyl oder C₁-C₄-Halogenalkoxy steht,
V² ebenfalls für Wasserstoff, Halogen, C₁-C₆-Alkyl, C₁-C₆-Alkoxy oder C₁-C₄-Halogenalkyl steht,
V¹ und V² gemeinsam ebenfalls für C₃-C₄-Alkandiyl stehen, welches gegebenenfalls durch Halogen und/oder C₁-C₂-Alkyl substituiert sein kann und welches gegebenenfalls durch ein oder zwei Sauerstoffatome unterbrochen sein kann,
W außerdem für Wasserstoff, C₁-C₆-Alkyl, C₂-C₆-Alkenyl, C₂-C₆-Alkinyl, C₁-C₆-Alkoxy, Halogen, Trifluormethyl oder Cyano steht,
X außerdem für Halogen, C₁-C₆-Alkyl, C₂-C₆-Alkenyl, C₂-C₆-Alkinyl, C₁-C₆-Alkoxy, C₁-C₆-Alkoxy-C₁-C₄-alkoxy, C₁-C₄-Halogenalkyl, C₁-C₄-Halogenalkoxy oder Cyano steht,
Y außerdem in der 4-Position für C₁-C₆-Alkyl steht,
Z außerdem für Wasserstoff steht,
W weiterhin für Wasserstoff, Halogen, C₁-C₆-Alkyl oder C₁-C₆-Alkoxy steht,
X weiterhin für Halogen, C₁-C₆-Alkyl, C₁-C₆-Alkoxy, C₁-C₄-Halogenalkyl, C₁-C₄-Halogenalkoxy oder Cyano steht,
Y weiterhin in der 4-Position für Wasserstoff, Halogen oder C₁-C₆-Alkyl, C₁-C₄-Halogenalkyl oder C₁-C₄-Halogenalkoxy steht,
Z weiterhin in der 3- oder 5-Position für Halogen, C₁-C₆-Alkyl, C₁-C₄-Halogenalkyl, Cyano, C₁-C₆-Alkoxy oder C₁-C₄-Halogenalkoxy steht,
A für eine gegebenenfalls durch C₁-C₄-Alkyl substituierte C₁-C₄-Alkandiylgruppe oder für gegebenenfalls durch C₁-C₄-Alkyl substituiertes C₅-C₈-Cycloalkyl steht, in welchem gegebenenfalls eine Methylengruppe durch Sauerstoff ersetzt ist,
B für Wasserstoff oder für jeweils gegebenenfalls durch Halogen substituiertes C₁-C₈-Alkyl, C₂-C₈-Alkenyl, C₁-C₆-Alkoxy, C₁-C₆-Alkoxy-C₁-C₄-alkoxy, C₁-C₄-Alkoxy-bis-C₁-C₄-alkoxy, für gegebenenfalls durch Halogen, C₁-C₆-Alkyl, C₁-C₆-Alkoxy, C₁-C₄-Halogenalkyl, C₁-C₄-Halogenalkoxy, Cyano oder Nitro substituiertes Phenyl, für gegebenenfalls durch Halogen, C₁-C₄-Alkyl oder C₁-C₂-Halogenalkyl substituiertes Pyridyl, Pyrimidyl, Thiazolyl oder Thienyl oder für gegebenenfalls durch Halogen, C₁-C₄-Alkyl, C₁-C₄-Alkoxy oder C₁-C₂-Halogenalkyl substituiertes C₃-C₈-Cycloalkyl steht, in welchem gegebenenfalls eine oder zwei nicht direkt benachbarte Methylengruppen durch Sauerstoff ersetzt, zwei Methylengruppen durch den Rest -O-CO- oder drei Methylengruppen durch den Rest -O-CO-O- ersetzt sind,
D für Sauerstoff steht,
Q¹ für Wasserstoff oder für jeweils gegebenenfalls durch Halogen substituiertes C₁-C₆-Alkyl, C₁-C₆-Alkoxy, C₁-C₄-Alkoxy-C₁-C₄-alkyl, C₁-C₄-Alkylthio-C₁-C₄alkyl oder für gegebenenfalls durch Halogen, C₁-C₄-Alkyl oder C₁-C₄-Alkoxy substituiertes C₃-C₆-Cycloalkyl worin gegebenenfalls eine Methylengruppe durch Sauerstoff ersetzt ist oder für jeweils gegebenenfalls einfach bis zweifach durch Halogen, C₁-C₄-Alkyl, C₁-C₄-Alkoxy, C₁-C₄-Halogenalkyl oder C₁-C₄Halogenalkoxy substituiertes Phenyl, Phenyl-C₁-C₂-alkyl oder Hetaryl steht,
Q² für Wasserstoff oder C₁-C₆-Alkyl steht, oder
Q¹ und Q² gemeinsam mit dem Kohlenstoff, an das sie gebunden sind für einen gegebenenfalls einfach bis zweifach durch Fluor, Chlor, C₁-C₄-Alkyl, C₁-C₄-Alkoxy oder Trifluormethyl substituierten C₃-C₆-Ring stehen, worin gegebenenfalls eine Methylengruppe durch Sauerstoff ersetzt sein kann,
G für Wasserstoff (a) oder für eine der Gruppen 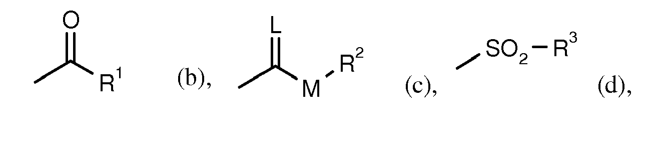 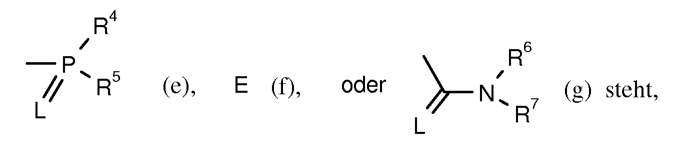 in welchen
E für ein Metallion oder ein Ammoniumion steht,
L für Sauerstoff oder Schwefel steht und
M für Sauerstoff oder Schwefel steht,
R¹ für jeweils gegebenenfalls durch Halogen oder Cyano substituiertes C₁-C₂₀-Alkyl, C₂-C₂₀-Alkenyl, C₁-C₈-Alkoxy-C₁-C₈-alkyl, C₁-C₈-Alkylthio-C₁-C₈-alkyl oder Poly-C₁-C₈-alkoxy-C₁-C₈-alkyl oder für gegebenenfalls durch Halogen, C₁-C₆-Alkyl oder C₁-C₆-Alkoxy substituiertes C₃-C₈-Cycloalkyl, in welchem gegebenenfalls eine oder zwei nicht direkt benachbarte Methylengruppen durch Sauerstoff und/oder Schwefel ersetzt sind,
für gegebenenfalls durch Halogen, Cyano, Nitro, C₁-C₆-Alkyl, C₁-C₆-Alkoxy, C₁-C₆-Halogenalkyl, C₁-C₆-Halogenalkoxy, C₁-C₆-Alkylthio oder C₁-C₆-Alkylsulfonyl substituiertes Phenyl,
für gegebenenfalls durch Halogen, Nitro, Cyano, C₁-C₆-Alkyl, C₁-C₆-Alkoxy, C₁-C₆-Halogenalkyl oder C₁-C₆-Halogenalkoxy substituiertes Phenyl-C₁-C₆-alkyl,
für gegebenenfalls durch Halogen oder C₁-C₆-Alkyl substituiertes 5- oder 6-gliedriges Hetaryl mit ein oder zwei Heteroatomen aus der Reihe Sauerstoff, Schwefel und Stickstoff,
für gegebenenfalls durch Halogen oder C₁-C₆-Alkyl substituiertes Phenoxy-C₁-C₆-alkyl oder
für gegebenenfalls durch Halogen, Amino oder C₁-C₆-Alkyl substituiertes 5- oder 6-gliedriges Hetaryloxy-C₁-C₆-alkyl steht mit ein oder zwei Heteroatomen aus der Reihe Sauerstoff, Schwefel und Stickstoff,
R² für jeweils gegebenenfalls durch Halogen oder Cyano substituiertes C₁-C₂₀-Alkyl, C₂-C₂₀-Alkenyl, C₁-C₈-Alkoxy-C₂-C₈-alkyl oder Poly-C₁-C₈-alkoxy-C₂-C₈-alkyl,
für gegebenenfalls durch Halogen, C₁-C₆-Alkyl oder C₁-C₆-Alkoxy substituiertes C₃-C₈-Cycloalkyl oder
für jeweils gegebenenfalls durch Halogen, Cyano, Nitro, C₁-C₆-Alkyl, C₁-C₆-Alkoxy, C₁-C₆-Halogenalkyl oder C₁-C₆-Halogenalkoxy substituiertes Phenyl oder Benzyl steht,
R³ für gegebenenfalls durch Halogen substituiertes C₁-C₈-Alkyl oder jeweils gegebenenfalls durch Halogen, C₁-C₆-Alkyl, C₁-C₆-Alkoxy, C₁-C₄-Halogenalkyl, C₁-C₄-Halogenalkoxy, Cyano oder Nitro substituiertes Phenyl oder Benzyl steht,
R⁴ und R⁵ unabhängig voneinander für jeweils gegebenenfalls durch Halogen substituiertes C₁-C₈-Alkyl, C₁-C₈-Alkoxy, C₁-C₈-Alkylamino, Di-(C₁-C₈-alkyl)amino, C₁-C₈-Alkylthio oder C₃-C₈-Alkenylthio oder für jeweils gegebenenfalls durch Halogen, Nitro, Cyano, C₁-C₄-Alkoxy, C₁-C₄-Halogenalkoxy, C₁-C₄-Alkylthio, C₁-C₄-Halogenalkylthio, C₁-C₄-Alkyl oder C₁-C₄-Halogenalkyl substituiertes Phenyl, Phenoxy oder Phenylthio stehen,
R⁶ und R⁷ unabhängig voneinander für Wasserstoff, für jeweils gegebenenfalls durch Halogen oder Cyano substituiertes C₁-C₈-Alkyl, C₃-C₈-Cycloalkyl, C₁-C₈-Alkoxy, C₃-C₈-Alkenyl oder C₁-C₈-Alkoxy-C₂-C₈-alkyl, für jeweils gegebenenfalls durch Halogen, C₁-C₈-Alkyl, C₁-C₈-Halogenalkyl oder C₁-C₈-Alkoxy substituiertes Phenyl oder Benzyl oder zusammen für einen gegebenenfalls durch C₁-C₆-Alkyl substituierten C₃-C₆-Alkylenrest stehen, in welchem gegebenenfalls eine Methylengruppe durch Sauerstoff oder Schwefel ersetzt ist.

3. Verbindungen der Formel (I) gemäß Anspruch 1, in welcher
W für Wasserstoff, Chlor, Brom, C₁-C₄-Alkyl, C₂-C₄-Alkenyl, C₂-C₄-Alkinyl, C₁-C₄-Alkoxy, C₁-C₂-Halogenalkyl oder C₁-C₂-Halogenalkoxy steht,
X für Chlor, Brom, C₁-C₄-Alkyl, C₂-C₄-Alkenyl, C₂-C₄-Alkinyl, C₁-C₄-Alkoxy, C₁-C₄-Alkoxy-C₁-C₃-alkoxy, C₁-C₂-Halogenalkyl, C₁-C₂-Halogenalkoxy oder Cyano steht,
Y in der 4-Position für Wasserstoff, Fluor, Chlor, Brom, Methoxy, Ethoxy, Cyano, Trifluormethyl, Difluormethoxy oder Trifluormethoxy steht,
Z für Wasserstoff steht,
W auch für Wasserstoff, Chlor, Brom oder C₁-C₄-Alkyl steht,
X auch für Chlor, Brom, C₁-C₄-Alkyl, C₁-C₄-Alkoxy, C₁-C₂-Halogenalkyl, C₁-C₂-Halogenalkoxy oder Cyano steht,
Y auch in der 4-Position für den Rest 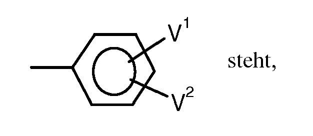
Z auch für Wasserstoff steht,
V¹ auch für Fluor, Chlor, C₁-C₄-Alkyl, C₁-C₄-Alkoxy, C₁-C₂-Halogenalkyl oder C₁-C₂-Halogenalkoxy steht,
V² auch für Wasserstoff, Fluor, Chlor, C₁-C₄-Alkyl, C₁-C₄-Alkoxy oder C₁-C₂-Halogenalkyl steht,
V¹ und V² gemeinsam auch für -O-CH₂-O- oder -O-CF₂-O- steht,
W ebenfalls für Wasserstoff, Chlor, Brom oder C₁-C₄-Alkyl steht,
X ebenfalls für Chlor, Brom, C₁-C₄-Alkyl oder C₁-C₂-Halogenalkyl steht,
Y ebenfalls in der 5-Position für den Rest 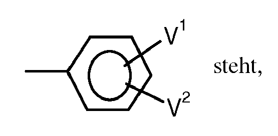
Z ebenfalls in der 4-Position für Wasserstoff, C₁-C₄-Alkyl oder Chlor steht,
V¹ ebenfalls für Fluor, Chlor, C₁-C₄-Alkyl, C₁-C₄-Alkoxy, C₁-C₂-Halogenalkyl oder C₁-C₂-Halogenalkoxy steht,
V² ebenfalls für Wasserstoff, Fluor, Chlor, C₁-C₄-Alkyl, C₁-C₄-Alkoxy oder C₁-C₂-Halogenalkyl steht,
V¹ und V² gemeinsam ebenfalls für -O-CH₂-O- oder -O-CF₂-O- stehen,
W außerdem für Wasserstoff, C₁-C₄-Alkyl, C₂-C₄-Alkenyl, C₂-C₄-Alkinyl, C₁-C₄-Alkoxy, Chlor, Brom oder Trifluormethyl steht,
X außerdem für Chlor, Brom, C₁-C₄-Alkyl, C₂-C₄-Alkenyl, C₂-C₄-Alkinyl, C₁-C₄-Alkoxy, C₁-C₄-Alkoxy-C₁-C₃-alkoxy, C₁-C₂-Halogenalkyl, C₁-C₂-Halogenalkoxy oder Cyano steht,
Y außerdem in der 4-Position für C₁-C₄-Alkyl steht,
Z außerdem für Wasserstoff steht,
W weiterhin für Wasserstoff, Chlor, Brom, C₁-C₄-Alkyl oder C₁-C₄-Alkoxy steht,
X weiterhin für Chlor, Brom, C₁-C₄-Alkyl, C₁-C₄-Alkoxy, C₁-C₂-Halogenalkyl, C₁-C₂-Halogenalkoxy oder Cyano steht,
Y weiterhin in der 4-Position für Wasserstoff, Chlor, Brom, C₁-C₄-Alkyl, C₁-C₂-Halogenalkyl oder C₁-C₂-Halogenalkoxy steht,
Z weiterhin in der 3- oder 5-Position für Fluor, Chlor, Brom, C₁-C₄-Alkyl, C₁-C₂-Halogenalkyl, C₁-C₄-Alkoxy oder C₁-C₂-Halogenalkoxy steht,
A für eine gegebenenfalls durch C₁-C₂-Alkyl substituierte C₁-C₃-Alkandiylgruppe oder für C₅-C₆-Cycloalkyl steht, in welchen gegebenenfalls eine Methylengruppe durch Sauerstoff ersetzt ist,
B für Wasserstoff oder jeweils gegebenenfalls einfach bis dreifach durch Fluor oder Chlor substituiertes C₁-C₆-Alkyl, C₂-C₆-Alkenyl, C₁-C₄-Alkoxy, C₁-C₄-Alkoxy-C₁-C₃-alkoxy, C₁-C₄-Alkoxy-bis-C₁-C₃-alkoxy, für gegebenenfalls einfach bis dreifach durch Fluor, Chlor, Brom, C₁-C₄-Alkyl, C₁-C₄-Alkoxy, C₁-C₂-Halogenalkyl, C₁-C₂-Halogenalkoxy, Cyano oder Nitro substituiertes Phenyl, für jeweils gegebenenfalls einfach bis zweifach durch Fluor, Chlor, Brom, Methyl, Ethyl oder Trifluormethyl substituiertes Pyridyl, Pyrimidyl, Thiazolyl oder Thienyl oder für gegebenenfalls einfach bis zweifach durch Fluor, Chlor, Methyl, Methoxy oder Trifluormethyl substituiertes C₃-C₆-Cycloalkyl steht, in welchem gegebenenfalls zwei nicht direkt benachbarte Methylengruppen durch Sauerstoff ersetzt sind,
D für Sauerstoff steht,
Q¹ für Wasserstoff, für jeweils gegebenenfalls einfach bis dreifach durch Fluor substituiertes C₁-C₄-Alkyl, C₁-C₄-Alkoxy, C₁-C₂-Alkoxy-C₁-C₂-alkyl, oder für gegebenenfalls einfach bis zweifach durch Fluor, Chlor, Methyl, Ethyl oder Methoxy substituiertes C₃-C₆-Cycloalkyl steht, worin gegebenenfalls eine Methylengruppe durch Sauerstoff ersetzt sein kann,
Q² für Wasserstoff oder C₁-C₄-Alkyl steht,
Q¹ und Q² gemeinsam mit dem Kohlenstoffatom, an das sie gebunden sind, für einen gegebenenfalls einfach durch Fluor, Methyl, Methoxy oder Trifluormethyl substituierten C₃-C₆-Ring stehen, worin gegebenenfalls eine Methylengruppe durch Sauerstoff ersetzt sein kann,
G für Wasserstoff (a) oder für eine der Gruppen 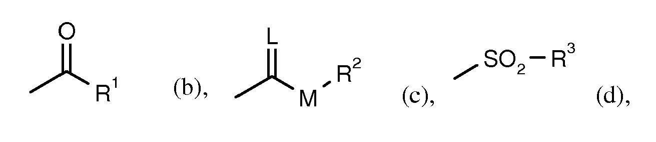 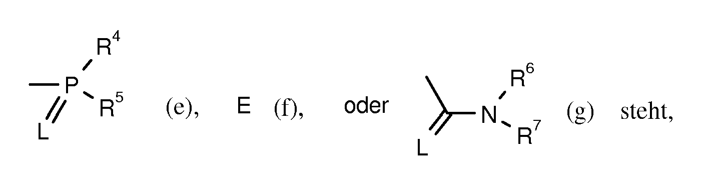 in welchen
E für ein Metallion oder ein Ammoniumion steht,
L für Sauerstoff oder Schwefel steht und
M für Sauerstoff oder Schwefel steht,
R¹ für jeweils gegebenenfalls einfach bis dreifach durch Fluor oder Chlor substituiertes C₁-C₁₆-Alkyl, C₂-C₁₆-Alkenyl, C₁-C₆-Alkoxy-C₁-C₄-alkyl, C₁-C₆-Alkylthio-C₁-C₄-alkyl oder Poly-C₁-C₆-alkoxy-C₁-C₄-alkyl oder für gegebenenfalls einfach bis zweifach durch Fluor, Chlor, C₁-C₅-Alkyl oder C₁-C₅-Alkoxy substituiertes C₃-C₇-Cycloalkyl, in welchem gegebenenfalls eine oder zwei nicht direkt benachbarte Methylengruppen durch Sauerstoff und/oder Schwefel ersetzt sind,
für gegebenenfalls einfach bis dreifach durch Fluor, Chlor, Brom, Cyano, Nitro, C₁-C₄-Alkyl, C₁-C₄-Alkoxy, C₁-C₃-Halogenalkyl, C₁-C₃-Halogenalkoxy, C₁-C₄-Alkylthio oder C₁-C₄-Alkylsulfonyl substituiertes Phenyl,
für gegebenenfalls einfach bis zweifach durch Fluor, Chlor, Brom, C₁-C₄-Alkyl, C₁-C₄-Alkoxy, C₁-C₃-Halogenalkyl oder C₁-C₃-Halogenalkoxy substituiertes Phenyl-C₁-C₄-alkyl,
für jeweils gegebenenfalls einfach bis zweifach durch Fluor, Chlor, Brom oder C₁-C₄-Alkyl substituiertes Pyrazolyl, Thiazolyl, Pyridyl, Pyrimidyl, Furanyl oder Thienyl,
für gegebenenfalls einfach bis zweifach durch Fluor, Chlor, Brom oder C₁-C₄-Alkyl substituiertes Phenoxy-C₁-C₅-alkyl oder
für jeweils gegebenenfalls einfach bis zweifach durch Fluor, Chlor, Brom, Amino oder C₁-C₄-Alkyl substituiertes Pyridyloxy-C₁-C₅-alkyl, Pyrimidyloxy-C₁-C₅-alkyl oder Thiazolyloxy-C₁-C₅-alkyl steht,
R² für jeweils gegebenenfalls einfach bis dreifach durch Fluor oder Chlor substituiertes C₁-C₁₆-Alkyl, C₂-C₁₆-Alkenyl, C₁-C₆-Alkoxy-C₂-C₆-alkyl oder Poly-C₁-C₆-alkoxy-C₂-C₆-alkyl,
für gegebenenfalls einfach bis zweifach durch Fluor, Chlor, C₁-C₄-Alkyl oder C₁-C₄-Alkoxy substituiertes C₃-C₇-Cycloalkyl oder
für jeweils gegebenenfalls einfach bis dreifach durch Fluor, Chlor, Brom, Cyano, Nitro, C₁-C₄-Alkyl, C₁-C₃-Alkoxy, C₁-C₃-Halogenalkyl oder C₁-C₃-Halogenalkoxy substituiertes Phenyl oder Benzyl steht,
R³ für gegebenenfalls einfach bis dreifach durch Fluor oder Chlor substituiertes C₁-C₆-Alkyl oder jeweils gegebenenfalls einfach bis zweifach durch Fluor, Chlor, Brom, C₁-C₄-Alkyl, C₁-C₄-Alkoxy, C₁-C₂-Halogenalkoxy, C₁-C₂-Halogenalkyl, Cyano oder Nitro substituiertes Phenyl oder Benzyl steht,
R⁴ und R⁵ unabhängig voneinander für jeweils gegebenenfalls einfach bis dreifach durch Fluor oder Chlor substituiertes C₁-C₆-Alkyl, C₁-C₆-Alkoxy, C₁-C₆-Alkylamino, Di-(C₁-C₆-alkyl)amino, C₁-C₆-Alkylthio oder C₃-C₄-Alkenylthio oder für jeweils gegebenenfalls einfach bis zweifach durch Fluor, Chlor, Brom, Nitro, Cyano, C₁-C₃-Alkoxy, C₁-C₃-Halogenalkoxy, C₁-C₃-Alkylthio, C₁-C₃-Halogenalkylthio, C₁-C₃-Alkyl oder C₁-C₃-Halogenalkyl substituiertes Phenyl, Phenoxy oder Phenylthio stehen,
R⁶ und R⁷ unabhängig voneinander für Wasserstoff, für jeweils gegebenenfalls einfach bis dreifach durch Fluor oder Chlor substituiertes C₁-C₆-Alkyl, C₃-C₆-Cycloalkyl, C₁-C₆-Alkoxy, C₃-C₆-Alkenyl oder C₁-C₆-Alkoxy-C₂-C₆-alkyl, für jeweils gegebenenfalls einfach bis dreifach durch Fluor, Chlor, Brom, C₁-C₅-Halogenalkyl, C₁-C₅-Alkyl oder C₁-C₅-Alkoxy substituiertes Phenyl oder Benzyl, oder zusammen für einen gegebenenfalls durch C₁-C₄-Alkyl substituierten C₃-C₆-Alkylenrest stehen, in welchem gegebenenfalls eine Methylengruppe durch Sauerstoff oder Schwefel ersetzt ist.

4. Verbindungen der Formel (I) gemäß Anspruch 1, in welcher
W für Wasserstoff, Methyl, Chlor, Brom, Ethyl, Methoxy, Ethoxy oder Trifluormethyl steht,
X für Chlor, Brom, Methyl, Ethyl, Propyl, Methoxy, Ethoxy, Propoxy, Methoxyethoxy, Ethoxy-ethoxy, Trifluormethyl, Difluormethoxy, Trifluormethoxy oder Cyano steht,
Y in der 4-Position für Wasserstoff, Chlor, Brom, Trifluormethyl oder Trifluormethoxy steht,
Z für Wasserstoff steht,
W auch für Wasserstoff, Chlor, Brom, Methyl oder Ethyl steht,
X auch für Chlor, Brom, Methyl, Ethyl, Propyl, Methoxy, Trifluormethyl, Difluormethoxy oder Cyano steht,
Y auch in der 4-Position für den Rest 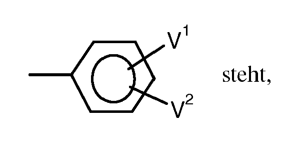
Z auch für Wasserstoff steht,
V¹ auch für Fluor, Chlor, Methyl, Methoxy, Trifluormethyl oder Trifluormethoxy steht,
V² auch für Wasserstoff, Fluor, Chlor, Methyl, Methoxy oder Trifluormethyl steht,
W ebenfalls für Wasserstoff, Chlor oder Methyl steht,
X ebenfalls für Chlor, Methyl oder Trifluormethyl steht,
Y ebenfalls in der 5-Position für den Rest 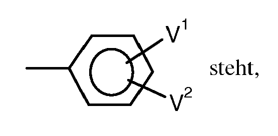
Z ebenfalls in der 4-Position für Wasserstoff oder Methyl steht,
V¹ ebenfalls für Fluor, Chlor, Methyl, Methoxy, Trifluormethyl oder Trifluormethoxy steht,
V² ebenfalls für Wasserstoff, Fluor, Chlor, Methyl, Methoxy oder Trifluormethyl steht,
W außerdem für Wasserstoff, Methyl, Ethyl, Methoxy, Ethoxy, Chlor oder Brom steht,
X außerdem für Chlor, Brom, Methyl, Ethyl, Propyl, Methoxy, Ethoxy, Propoxy, Methoxy-ethoxy, Ethoxy-ethoxy, Trifluormethyl, Difluormethoxy, Trifluormethoxy oder Cyano steht,
Y außerdem in der 4-Position für Methyl oder Ethyl steht,
Z außerdem für Wasserstoff steht,
W weiterhin für Wasserstoff, Chlor, Brom, Methyl oder Ethyl steht,
X weiterhin für Chlor, Brom, Methyl, Ethyl, Methoxy, Trifluormethyl, Difluormethoxy oder Trifluormethoxy steht,
Y weiterhin in der 4-Position für Wasserstoff, Chlor, Brom, Methyl oder Ethyl steht,
Z weiterhin in der 3- oder 5-Position für Fluor, Chlor, Brom, Methyl, Ethyl, Trifluormethyl oder Trifluormethoxy steht,
A für -CH₂-, -CHCH₃-, -CH₂-CH₂-, -CH₂-CHCH₃-, -CH₂-CH₂-CH₂- steht,
B für Wasserstoff, Methyl, Ethyl, Propyl, iso-Propyl, Butyl, iso-Butyl, C₂-C₄-Alkenyl, Methoxy, Ethoxy, Propoxy, Isopropoxy, Butoxy, Isobutoxy, Methoxyethoxy, Ethoxy-ethoxy, für gegebenenfalls einfach bis zweifach durch Fluor, Chlor, Brom, Methyl, Methoxy, Trifluormethyl, Trifluormethoxy, Cyano oder Nitro substituiertes Phenyl, für Cyclopropyl, für Cyclopentyl oder Cyclohexyl steht, in welchem gegebenenfalls eine Methylengruppe durch Sauerstoff ersetzt ist,
D für Sauerstoff steht,
Q¹ für Wasserstoff, Methyl, Ethyl, Propyl, iso-Propyl, Cyclopropyl, Cyclopentyl oder Cyclohexyl steht,
Q² für Wasserstoff, Methyl oder Ethyl steht,
Q¹ und Q² gemeinsam mit dem Kohlenstoffatom an das sie gebunden sind für Cyclopropyl, Cyclopentyl oder Cyclohexyl stehen,
G für Wasserstoff (a) oder für eine der Gruppen 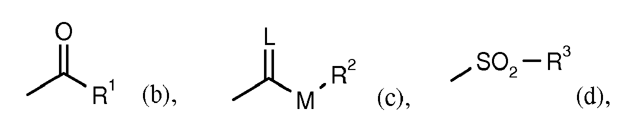 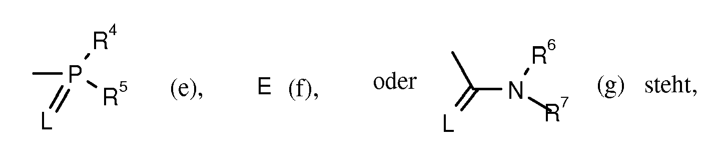 in welchen
E für ein Metallion oder ein Ammoniumion steht,
L für Sauerstoff oder Schwefel steht und
M für Sauerstoff oder Schwefel steht,
R¹ für jeweils gegebenenfalls einfach bis dreifach durch Fluor oder Chlor substituiertes C₁-C₁₀-Alkyl, C₂-C₁₀-Alkenyl, C₁-C₄-Alkoxy-C₁-C₂-alkyl, C₁-C₄-Alkylthio-C₁-C₂-alkyl oder für gegebenenfalls einfach durch Fluor, Chlor, Methyl, Ethyl oder Methoxy substituiertes C₃-C₆-Cycloalkyl,
für gegebenenfalls einfach bis zweifach durch Fluor, Chlor, Brom, Cyano, Nitro, Methyl, Ethyl, n-Propyl, i-Propyl, Methoxy, Ethoxy, Trifluormethyl oder Trifluormethoxy substituiertes Phenyl,
für jeweils gegebenenfalls einfach durch Chlor, Brom oder Methyl substituiertes Furanyl, Thienyl oder Pyridyl steht,
R² für jeweils gegebenenfalls einfach bis dreifach durch Fluor oder Chlor substituiertes C₁-C₁₀-Alkyl, C₂-C₁₀-Alkenyl oder C₁-C₄-Alkoxy-C₂-C₄-alkyl,
für Cyclopentyl oder Cyclohexyl
oder für jeweils gegebenenfalls einfach bis zweifach durch Fluor, Chlor, Cyano, Nitro, Methyl, Ethyl, Methoxy, Trifluormethyl oder Trifluormethoxy substituiertes Phenyl oder Benzyl steht,
R³ für jeweils gegebenenfalls einfach bis dreifach durch Fluor oder Chlor substituiertes Methyl, Ethyl, Propyl oder iso-Propyl, oder gegebenenfalls einfach durch Fluor, Chlor, Brom, Methyl, Ethyl, iso-Propyl, tert.-Butyl, Methoxy, Ethoxy, iso-Propoxy, Trifluormethyl, Trifluormethoxy, Cyano oder Nitro substituiertes Phenyl steht,
R⁴ und R⁵ unabhängig voneinander für C₁-C₄-Alkoxy oder C₁-C₄-Alkylthio oder für jeweils gegebenenfalls einfach durch Fluor, Chlor, Brom, Nitro, Cyano, Methyl, Methoxy, Trifluormethyl oder Trifluormethoxy substituiertes Phenyl, Phenoxy oder Phenylthio stehen,
R⁶ und R⁷ unabhängig voneinander für Wasserstoff, für C₁-C₄-Alkyl, C₃-C₆-Cycloalkyl, C₁-C₄-Alkoxy, C₃-C₄-Alkenyl oder C₁-C₄-Alkoxy-C₂-C₄-alkyl, für gegebenenfalls einfach bis zweifach durch Fluor, Chlor, Brom, Methyl, Methoxy oder Trifluormethyl substituiertes Phenyl, oder zusammen für einen C₅-C₆-Alkylenrest stehen, in welchem gegebenenfalls eine Methylengruppe durch Sauerstoff oder Schwefel ersetzt ist.

5. Verbindungen der Formel (I) gemäß Anspruch 1, in welcher
W für Wasserstoff, Methyl, Ethyl, Chlor oder Brom steht,
X für Chlor, Brom, Methyl, Ethyl, Propyl, Methoxy, Ethoxy, Propoxy, Trifluormethyl, Difluormethoxy oder Cyano steht,
Y in der 4-Position für Wasserstoff, Chlor, Brom, Methoxy, Trifluormethyl oder Trifluormethoxy steht,
Z für Wasserstoff steht,
A für -CH₂- steht,
B für Wasserstoff, Methyl, Ethyl, Propyl, iso-Propyl, C₂-C₄-Alkenyl, Methoxy, Ethoxy, Propoxy, Isopropoxy, Butoxy, Isobutoxy, für gegebenenfalls einfach bis zweifach durch Fluor, Chlor, Brom, Methyl, Methoxy, Trifluormethyl, Trifluormethoxy, Cyano oder Nitro substituiertes Phenyl, für Cyclopropyl, Cyclopentyl oder Cyclohexyl steht,
D für Sauerstoff steht,
Q¹ für Wasserstoff oder Methyl steht,
Q² für Wasserstoff oder Methyl steht,
G für Wasserstoff (a) oder für eine der Gruppen 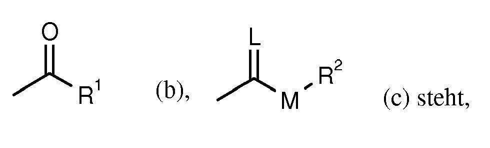 in welchen
L für Sauerstoff steht und
M für Sauerstoff oder Schwefel steht,
R¹ für jeweils gegebenenfalls einfach bis dreifach durch Fluor oder Chlor substituiertes C₁-C₆-Alkyl, C₂-C₆-Alkenyl, C₁-C₂-Alkoxy-C₁-C₂-alkyl, C₁-C₂-Alkylthio-C₁-C₂-alkyl oder für Cyclopropyl, Cyclopentyl oder Cyclohexyl,
für gegebenenfalls einfach durch Fluor, Chlor, Brom, Cyano, Nitro, Methyl, Methoxy, Trifluormethyl oder Trifluormethoxy substituiertes Phenyl,
für jeweils gegebenenfalls einfach durch Chlor oder Methyl substituiertes Furanyl, Thienyl oder Pyridyl steht,
R² für C₁-C₈-Alkyl, C₂-C₆-Alkenyl oder C₁-C₂-Alkoxy-C₂-C₃-alkyl,
für Cyclopentyl oder Cyclohexyl,
oder für jeweils gegebenenfalls einfach durch Fluor, Chlor, Cyano, Nitro, Methyl, Methoxy, Trifluormethyl oder Trifluormethoxy substituiertes Phenyl oder Benzyl steht.

6. Verbindungen der Formel (I) gemäß Anspruch 1, in welcher
W für Wasserstoff, Chlor, Brom, Methyl oder Ethyl steht,
X für Chlor, Brom, Methyl, Ethyl, Propyl, Methoxy, Trifluormethyl, Difluormethoxy oder Cyano steht,
Y in der 4-Position für den Rest 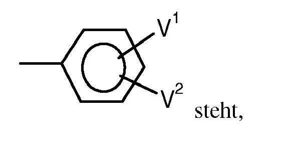
Z für Wasserstoff steht,
V¹ für Fluor, Chlor, Methyl, Methoxy, Trifluormethyl oder Trifluormethoxy steht,
V² für Wasserstoff, Fluor, Chlor, Methyl, Methoxy oder Trifluormethyl steht,
Y für 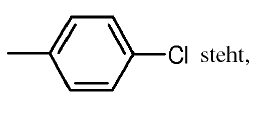
A für -CH₂- steht,
B für Wasserstoff, Methyl, Ethyl, Propyl, iso-Propyl, C₂-C₄-Alkenyl, Methoxy, Ethoxy, Propoxy, Isopropoxy, Butoxy, Isobutoxy, für gegebenenfalls einfach bis zweifach durch Fluor, Chlor, Brom, Methyl, Methoxy, Trifluormethyl, Trifluormethoxy, Cyano oder Nitro substituiertes Phenyl steht,
D für Sauerstoff steht,
Q¹ für Wasserstoff oder Methyl steht,
Q² für Wasserstoff oder Methyl steht,
G für Wasserstoff (a) oder für eine der Gruppen 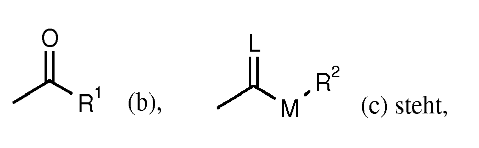 in welchen
L für Sauerstoff steht und
M für Sauerstoff oder Schwefel steht,
R¹ für jeweils gegebenenfalls einfach bis dreifach durch Fluor oder Chlor substituiertes C₁-C₆-Alkyl, C₂-C₆-Alkenyl, C₁-C₂-Alkoxy-C₁-C₂-alkyl, C₁-C₂-Alkylthio-C₁-C₂-alkyl oder für Cyclpropyl, Cyclopentyl oder Cyclohexyl,
für gegebenenfalls einfach durch Fluor, Chlor, Brom, Cyano, Nitro, Methyl, Methoxy, Trifluormethyl oder Trifluormethoxy substituiertes Phenyl,
für jeweils gegebenenfalls einfach durch Chlor oder Methyl substituiertes Furanyl, Thienyl oder Pyridyl steht,
R² für C₁-C₈-Alkyl, C₂-C₆-Alkenyl oder C₁-C₂-Alkoxy-C₂-C₃-alkyl,
für Cyclopentyl oder Cyclohexyl,
oder für jeweils gegebenenfalls einfach durch Fluor, Chlor, Cyano, Nitro, Methyl, Methoxy, Trifluormethyl oder Trifluormethoxy substituiertes Phenyl oder Benzyl steht.

7. Verbindungen der Formel (I) gemäß Anspruch 1, in welcher
W für Wasserstoff oder Methyl steht,
X für Chlor oder Methyl steht,
Y in der 5-Position für den Rest 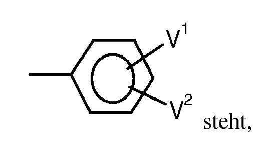
Z in der 4-Position für Wasserstoff oder Methyl steht,
V¹ für Fluor, Chlor, Methyl, Methoxy, Trifluormethyl oder Trifluormethoxy steht,
V² für Wasserstoff, Fluor, Chlor, Methyl, Methoxy oder Trifluormethyl steht,
Y für 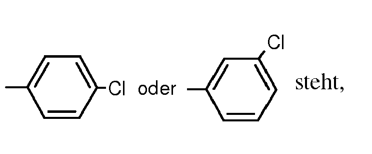
A für -CH₂- steht,
B für Wasserstoff, Methyl, Ethyl, Propyl, iso-Propyl, C₂-C₄-Alkenyl, Methoxy, Ethoxy, Propoxy, Isopropoxy, Butoxy, Isobutoxy, für gegebenenfalls einfach bis zweifach durch Fluor, Chlor, Brom, Methyl, Methoxy, Trifluormethyl, Trifluormethoxy, Cyano oder Nitro substituiertes Phenyl steht,
D für Sauerstoff steht,
Q¹ für Wasserstoff oder Methyl steht,
Q² für Wasserstoff oder Methyl steht,
G für Wasserstoff (a) oder für eine der Gruppen 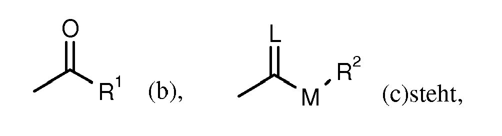 in welchen
L für Sauerstoff steht und
M für Sauerstoff oder Schwefel steht,
R¹ für jeweils gegebenenfalls einfach bis dreifach durch Fluor oder Chlor substituiertes C₁-C₆-Alkyl, C₂-C₆-Alkenyl, C₁-C₂-Alkoxy-C₁-C₂-alkyl, C₁-C₂-Alkylthio-C₁-C₂-alkyl oder für Cyclopropyl, Cyclopentyl oder Cyclohexyl,
für gegebenenfalls einfach durch Fluor, Chlor, Brom, Cyano, Nitro, Methyl, Methoxy, Trifluormethyl oder Trifluormethoxy substituiertes Phenyl,
für jeweils gegebenenfalls einfach durch Chlor oder Methyl substituiertes Furanyl, Thienyl oder Pyridyl steht,
R² für C₁-C₈-Alkyl, C₂-C₆-Alkenyl oder C₁-C₂-Alkoxy-C₂-C₃-alkyl,
für Cyclopentyl oder Cyclohexyl,
oder für jeweils gegebenenfalls einfach durch Fluor, Chlor, Cyano, Nitro, Methyl, Methoxy, Trifluormethyl oder Trifluormethoxy substituiertes Phenyl oder Benzyl steht.

8. Verbindungen der Formel (I) gemäß Anspruch 1, in welcher
W für Wasserstoff, Methyl, Chlor oder Brom steht,
X für Chlor, Brom, Methyl, Ethyl, Propyl, Methoxy, Ethoxy, Propoxy, Trifluormethyl, Difluormethoxy oder Cyano steht,
Y in der 4-Position für Methyl oder Ethyl steht,
Z für Wasserstoff steht,
A für -CH₂- steht,
B für Wasserstoff, Methyl, Ethyl, Propyl, iso-Propyl, C₂-C₄-Alkenyl, Methoxy, Ethoxy, Propoxy, Isopropoxy, Butoxy, Isobutoxy, für gegebenenfalls einfach bis zweifach durch Fluor, Chlor, Brom, Methyl, Methoxy, Trifluormethyl, Trifluormethoxy, Cyano oder Nitro substituiertes Phenyl, für Cyclopropyl, für Cyclopentyl oder Cyclohexyl steht, in welchem gegebenenfalls eine Methylengruppe durch Sauerstoff ersetzt ist,
Q¹ für Wasserstoff oder Methyl steht,
Q² für Wasserstoff oder Methyl steht,
D für Sauerstoff steht,
G für Wasserstoff (a) oder für eine der Gruppen 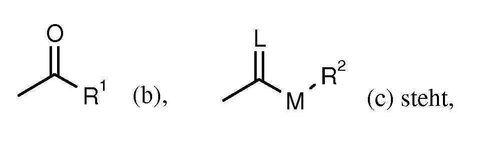 in welchen
L für Sauerstoff steht und
M für Sauerstoff oder Schwefel steht,
R¹ für jeweils gegebenenfalls einfach bis dreifach durch Fluor oder Chlor substituiertes C₁-C₆-Alkyl, C₂-C₆-Alkenyl, C₁-C₂-Alkoxy-C₁-C₂-alkyl, C₁-C₂-Alkylthio-C₁-C₂-alkyl oder für Cyclopropyl, Cyclopentyl oder Cyclohexyl,
für gegebenenfalls einfach durch Fluor, Chlor, Brom, Cyano, Nitro, Methyl, Methoxy, Trifluormethyl oder Trifluormethoxy substituiertes Phenyl,
für jeweils gegebenenfalls einfach durch Chlor oder Methyl substituiertes Furanyl, Thienyl oder Pyridyl steht,
R² für C₁-C₁₀-Alkyl, C₂-C₁₀-Alkenyl oder C₁-C₂-Alkoxy-C₂-C₄-alkyl,
für Cyclopentyl oder Cyclohexyl,
oder für jeweils gegebenenfalls einfach durch Fluor, Chlor, Cyano, Nitro, Methyl, Methoxy, Trifluormethyl oder Trifluormethoxy substituiertes Phenyl oder Benzyl steht.

9. Verbindungen der Formel (I) gemäß Anspruch 1, in welcher
W für Wasserstoff, Methyl, Chlor oder Brom steht,
X für Chlor, Brom, Methyl, Methoxy oder Trifluormethyl steht,
Y in der 4-Position für Wasserstoff, Chlor, Brom oder Methyl steht,
Z in der 3- oder 5-Position für Chlor, Fluor, Brom, Methyl, Ethyl, Trifluormethyl oder Trifluormethoxy steht,
A für -CH₂- steht,
B für Wasserstoff, Methyl, Ethyl, Propyl, iso-Propyl, C₂-C₄-Alkenyl, Methoxy, Ethoxy, Propoxy, Isopropoxy, Butoxy, Isobutoxy, für gegebenenfalls einfach bis zweifach durch Fluor, Chlor, Brom, Methyl, Methoxy, Trifluormethyl, Trifluormethoxy, Cyano oder Nitro substituiertes Phenyl, für Cyclopropyl, für Cyclopentyl oder Cyclohexyl steht, in welchem gegebenenfalls eine Methylengruppe durch Sauerstoff ersetzt ist,
Q¹ für Wasserstoff oder Methyl steht,
Q² für Wasserstoff oder Methyl steht,
D für Sauerstoff steht,
G für Wasserstoff (a) oder für eine der Gruppen 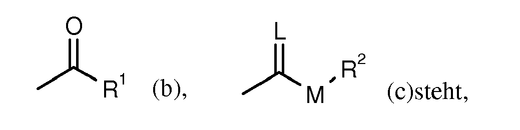 in welchen
L für Sauerstoff steht und
M für Sauerstoff oder Schwefel steht,
R¹ für jeweils gegebenenfalls einfach bis dreifach durch Fluor oder Chlor substituiertes C₁-C₆-Alkyl, C₂-C₆-Alkenyl, C₁-C₂-Alkoxy-C₁-C₂-alkyl, C₁-C₂-Alkylthio-C₁-C₂-alkyl oder für Cyclopropyl, Cyclopentyl oder Cyclohexyl,
für gegebenenfalls einfach durch Fluor, Chlor, Brom, Cyano, Nitro, Methyl, Methoxy, Trifluormethyl oder Trifluormethoxy substituiertes Phenyl,
für jeweils gegebenenfalls einfach durch Chlor oder Methyl substituiertes Furanyl, Thienyl oder Pyridyl steht,
R² für C₁-C₁₀-Alkyl, C₂-C₁₀-Alkenyl oder C₁-C₂-Alkoxy-C₂-C₄-alkyl,
für Cyclopentyl oder Cyclohexyl,
oder für jeweils gegebenenfalls einfach durch Fluor, Chlor, Cyano, Nitro, Methyl, Methoxy, Trifluormethyl oder Trifluormethoxy substituiertes Phenyl oder Benzyl steht.

10. Verfahren zur Herstellung von Verbindungen der Formel (I) gemäß Anspruch 1, **dadurch gekennzeichnet, dass** man zum Erhalt von
(B) Verbindungen der Formel (I-2-a)
in welcher
A, B, Q¹, Q², W, X, Y und Z die oben angegebenen Bedeutungen haben,
Verbindungen der Formel (III) 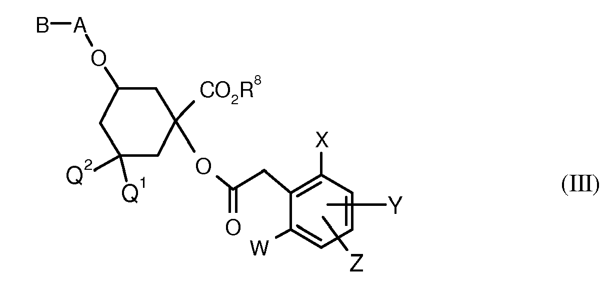 in welcher
A, B, Q¹, Q², W, X, Y, Z und R⁸ die oben angegebenen Bedeutungen haben,
in Gegenwart eines Verdünnungsmittels und in Gegenwart einer Base intramolekular kondensiert,
(C) Verbindungen der oben gezeigten Formel (I-2-b), in welchen R¹, A, B, Q¹, Q², W, X, Y und Z die oben angegebenen Bedeutungen haben, Verbindungen der oben gezeigten Formel (I-2-a), in welchen A, B, Q¹, Q², W, X, Y und Z die oben angegebenen Bedeutungen haben, jeweils
α) mit Verbindungen der Formel (IV) 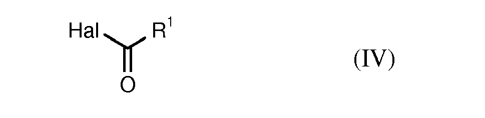 in welcher
R¹ die oben angegebene Bedeutung hat und
Hal für Halogen steht
oder
β) mit Carbonsäureanhydriden der Formel (V)
R¹-CO-O-CO-R¹ (V)
in welcher
R¹ die oben angegebene Bedeutung hat,
gegebenenfalls in Gegenwart eines Verdünnungsmittels und gegebenenfalls in Gegenwart eines Säurebindemittels umsetzt;
(D) Verbindungen der oben gezeigten Formel (I-2-c), in welchen R², A, B, Q¹, Q², W, M, X, Y und Z die oben angegebenen Bedeutungen haben und L für Sauerstoff steht, Verbindungen der oben gezeigten Formel (I-2-a), in welchen A, B, Q¹, Q², W, X, Y und Z die oben angegebenen Bedeutungen haben, jeweils
mit Chlorameisensäureestern oder Chlorameisensäurethioestern der Formel (VI)
R²-M-CO-Cl (VI)
in welcher
R² und M die oben angegebenen Bedeutungen haben,
gegebenenfalls in Gegenwart eines Verdünnungsmittels und gegebenenfalls in Gegenwart eines Säurebindemittels umsetzt;
(E) Verbindungen der oben gezeigten Formel (I-2-c), in welchen R², A, B, Q¹, Q², W, M, X, Y und Z die oben angegebenen Bedeutungen haben und L für Schwefel steht, Verbindungen der oben gezeigten Formel (I-2-a), in welchen A, B, Q¹, Q², W, X, Y und Z die oben angegebenen Bedeutungen haben, jeweils mit Chlormonothioameisensäureestern oder Chlordithioameisensäureestern der Formel (VII) 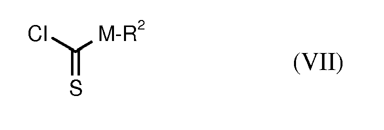 in welcher
M und R² die oben angegebenen Bedeutungen haben,
gegebenenfalls in Gegenwart eines Verdünnungsmittels und gegebenenfalls in Gegenwart eines Säurebindemittels umsetzt,
(F) Verbindungen der oben gezeigten Formel (I-2-d), in welchen R³, A, B, W, Q¹, Q², X, Y und Z die oben angegebenen Bedeutungen haben, Verbindungen der oben gezeigten Formel (I-2-a), in welchen A, B, Q¹, Q², W, X, Y und Z die oben angegebenen Bedeutungen haben, jeweils
mit Sulfonsäurechloriden der Formel (VIII)
R³-SO₂-Cl (VIII)
in welcher
R³ die oben angegebene Bedeutung hat,
gegebenenfalls in Gegenwart eines Verdünnungsmittels und gegebenenfalls in Gegenwart eines Säurebindemittels umsetzt,
(G) Verbindungen der oben gezeigten Formel (I-2-e), in welchen L, R⁴, R⁵, A, B, Q¹, Q², W, X, Y und Z die oben angegebenen Bedeutungen haben, Verbindungen der oben gezeigten Formel (I-2-a), in welchen A, B, Q¹, Q², W, X, Y und Z die oben angegebenen Bedeutungen haben, jeweils
mit Phosphorverbindungen der Formel (IX) 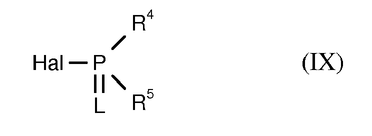 in welcher
L, R⁴ und R⁵ die oben angegebenen Bedeutungen haben und
Hal für Halogen steht,
gegebenenfalls in Gegenwart eines Verdünnungsmittels und gegebenenfalls in Gegenwart eines Säurebindemittels umsetzt,
(H) Verbindungen der oben gezeigten Formel (I-2-f), in welchen E, A, B, Q¹, Q², W, X, Y und Z die oben angegebenen Bedeutungen haben, Verbindungen der Formel (I-2-a), in welchen A, B, Q¹, Q², W, X, Y und Z die oben angegebenen Bedeutungen haben, jeweils
mit Metallverbindungen oder Aminen der Formeln (X) oder (XI)
Me(OR¹⁰)ₜ (X) 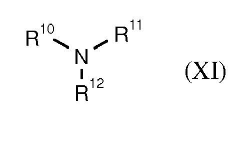
in welchen
Me für ein ein- oder zweiwertiges Metall,
t für die Zahl 1 oder 2 und
R¹⁰, R¹¹, R¹² unabhängig voneinander für Wasserstoff oder Alkyl stehen, gegebenenfalls in Gegenwart eines Verdünnungsmittels umsetzt,
(I) Verbindungen der oben gezeigten Formel (I-2-g), in welchen L, R⁶, R⁷, A, B, Q¹, Q², W, X, Y und Z die oben angegebenen Bedeutungen haben, Verbindungen der oben gezeigten Formel (I-2-a), in welchen A, B, Q¹, Q², W, X, Y und Z die oben angegebenen Bedeutungen haben, jeweils
α) mit Isocyanaten oder Isothiocyanaten der Formel (XII)
R⁶-N=C=L (XII)
in welcher
R⁶ und L die oben angegebenen Bedeutungen haben,
gegebenenfalls in Gegenwart eines Verdünnungsmittels und gegebenenfalls in Gegenwart eines Katalysators umsetzt oder
β) mit Carbamidsäurechloriden oder Thiocarbamidsäurechloriden der Formel (XIII) 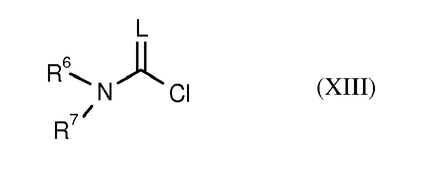 in welcher
L, R⁶ und R⁷ die oben angegebenen Bedeutungen haben,
gegebenenfalls in Gegenwart eines Verdünnungsmittels und gegebenenfalls in Gegenwart eines Säurebindemittels, umsetzt.

11. Mittel enthaltend einen wirksamen Gehalt an einer Wirkstoffkombination umfassend als Komponenten
(a') mindestens ein substituiertes, cyclisches Ketoenol der Formel (I), gemäß Anspruch 1
und
(b') zumindest eine die Kulturpflanzen-Verträglichkeit verbessernde Verbindung aus der folgenden Gruppe von Verbindungen:
4-Dichloracetyl-1-oxa-4-aza-spiro[4.5]-decan (AD-67, MON-4660), 1-Dichloracetyl-hexahydro-3,3,8a-trimethylpyrrolo[1,2-a]-pyrimidin-6(2H)-on (Dicyclonon, BAS-145138), 4-Dichloracetyl-3,4-dihydro-3-methyl-2H-1,4-benzoxazin (Benoxacor), 5-Chlor-chinolin-8-oxy-essigsäure-(1-methyl-hexylester) (Cloquintocetmexyl - vgl. auch verwandte Verbindungen in EP-A-86750, EP-A-94349, EP-A-191736, EP-A-492366), 3-(2-Chlor-benzyl)-1-(1-methyl-1-phenyl-ethyl)-harnstoff (Cumyluron), a-(Cyanomethoximino)-phenylacetonitril (Cyometrinil), 2,4-Dichlor-phenoxyessigsäure (2,4-D), 4-(2,4-Dichlor-phenoxy)-buttersäure (2,4-DB), 1-(1-Methyl-1-phenyl-ethyl)-3-(4-methyl-phenyl)-harnstoff (Daimuron, Dymron), 3,6-Dichlor-2-methoxy-benzoesäure (Dicamba), Piperidin-1-thiocarbonsäure-S-1-methyl-1-phenyl-ethylester (Dimepiperate), 2,2-Dichlor-N-(2-oxo-2-(2-propenylamino)-ethyl)-N-(2-propenyl)-acetamid (DKA-24), 2,2-Dichlor-N,N-di-2-propenyl-acetamid (Dichlormid), 4,6-Dichlor-2-phenyl-pyrimidin (Fenclorim), 1-(2,4-Dichlor-phenyl)-5-trichlormethyl-1H-1,2,4-triazol-3-carbonsäure-ethylester (Fenchlorazole-ethyl - vgl. auch verwandte Verbindungen in EP-A-174562 und EP-A-346620), 2-Chlor-4-trifluormethyl-thiazol-5-carbonsäure-phenylmethylester (Flurazole), 4-Chlor-N-(1,3-dioxolan-2-yl-methoxy)-α-trifluor-acetophenonoxim (Fluxofenim), 3-Dichloracetyl-5-(2-furanyl)-2,2-dimethyl-oxazolidin (Furilazole, MON-13900), Ethyl-4,5-dihydro-5,5-diphenyl-3-isoxazolcarboxylat (Isoxadifenethyl - vgl. auch verwandte Verbindungen in WO-A-95/07897), 1-(Ethoxycarbonyl)-ethyl-3,6-dichlor-2-methoxybenzoat (Lactidichlor), (4-Chlor-o-tolyl-oxy)-essigsäure (MCPA), 2-(4-Chlor-o-tolyloxy)-propionsäure (Mecoprop), Diethyl-1-(2,4-dichlor-phenyl)-4,5-dihydro-5-methyl-1H-pyrazol-3,5-dicarboxylat (Mefenpyr-diethyl - vgl. auch verwandte Verbindungen in WO-A-91/07874) 2-Dichlormethyl-2-methyl-1,3-dioxolan (MG-191), 2-Propenyl-1-oxa-4-azaspiro-[4.5]decane-4-carbodithioate (MG-838), 1,8-Naphthalsäureanhydrid, α-(1,3-Dioxolan-2-yl-methoximino)-phenylacetonitril (Oxabetrinil), 2,2-Dichlor-N-(1,3-dioxolan-2-yl-methyl)-N-(2-propenyl)-acetamid (PPG-1292), 3-Dichloracetyl-2,2-dimethyl-oxazolidin (R-28725), 3-Dichloracetyl-2,2,5-trimethyl-oxazolidin (R-29148), 4-(4-Chlor-o-tolyl)-buttersäure, 4-(4-Chlor-phenoxy)-buttersäure, Diphenylmethoxyessigsäure, Diphenylmethoxyessigsäure-methylester, Diphenylmethoxyessigsäure-ethylester, 1-(2-Chlor-phenyl)-5-phenyl-1H-pyrazol-3-carbonsäure-methylester, 1-(2,4-Dichlor-phenyl)-5-methyl-1H-pyrazol-3-carbonsäure-ethylester, 1-(2,4-Dichlor-phenyl)-5-isopropyl-1H-pyrazol-3-carbonsäure-ethylester, 1-(2,4-Dichlor-phenyl)-5-(1,1-dimethyl-ethyl)-1H-pyrazol-3-carbonsäure-ethylester, 1-(2,4-Dichlor-phenyl)-5-phenyl-1H-pyrazol-3-carbonsäure-ethylester (vgl. auch verwandte Verbindungen in EP-A-269806 und EP-A-333131), 5-(2,4-Dichlor-benzyl)-2-isoxazolin-3-carbonsäure-ethylester, 5-Phenyl-2-isoxazolin-3-carbonsäure-ethylester, 5-(4-Fluor-phenyl)-5-phenyl-2-isoxazolin-3-carbonsäure-ethylester (vgl. auch verwandte Verbindungen in WO-A-91/08202), 5-Chlor-chinolin-8-oxy-essigsäure-(1,3-dimethyl-but-1-yl)-ester, 5-Chlor-chinolin-8-oxy-essigsäure-4-allyloxy-butylester, 5-Chlor-chinolin-8-oxy-essigsäure-1-allyloxy-prop-2-yl-ester, 5-Chlor-chinoxalin-8-oxy-essigsäure-methylester, 5-Chlor-chinolin-8-oxy-essigsäure-ethylester, 5-Chlor-chinoxalin-8-oxy-essigsäure-allylester, 5-Chlor-chinolin-8-oxy-essigsäure-2-oxo-prop-1-yl-ester, 5-Chlor-chinolin-8-oxy-malonsäure-diethylester, 5-Chlor-chinoxalin-8-oxy-malonsäure-diallylester, 5-Chlor-chinolin-8-oxy-malonsäure-diethylester (vgl. auch verwandte Verbindungen in EP-A-582198), 4-Carboxy-chroman-4-yl-essigsäure (AC-304415, vgl. EP-A-613618), 4-Chlor-phenoxy-essigsäure, 3,3'-Dimethyl-4-methoxy-benzophenon, 1-Brom-4-chlormethylsulfonyl-benzol, 1-[4-(N-2-Methoxybenzoylsulfamoyl)-phenyl]-3-methyl-harnstoff (alias N-(2-Methoxy-benzoyl)-4-[(methylamino-carbonyl)-amino]-benzolsulfonamid), 1-[4-(N-2-Methoxybenzoylsulfamoyl)-phenyl]-3,3-dimethyl-harnstoff, 1-[4-(N-4,5-Dimethylbenzoylsulfamoyl)-phenyl]-3-methyl-harnstoff, 1-[4-(N-Naphthylsulfamoyl)-phenyl]-3,3-dimethyl-harnstoff, N-(2-Methoxy-5-methyl-benzoyl)-4-(cyclopropylaminocarbonyl)-benzolsulfonamid,
und/oder eine der folgenden durch allgemeine Formeln definierten Verbindungen der allgemeinen Formel (IIa) 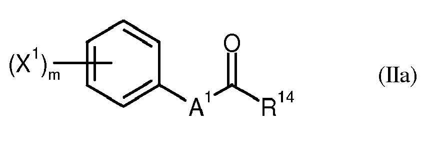 oder der allgemeinen Formel (IIb) 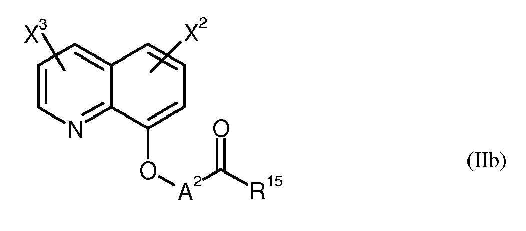 oder der Formel (IIc) 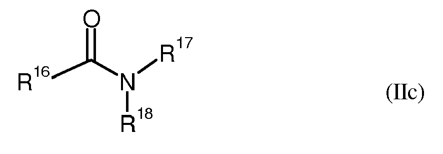 wobei
m für eine Zahl 0, 1, 2, 3, 4 oder 5 steht,
A¹ für eine der nachstehend skizzierten divalenten heterocyclischen Gruppierungen steht, 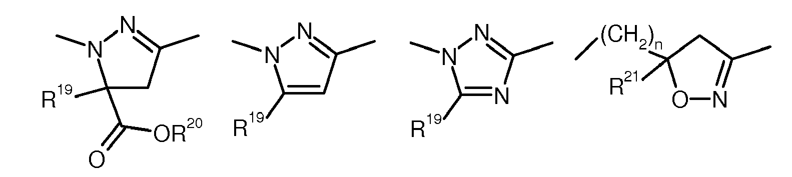
n für eine Zahl 0, 1, 2, 3, 4 oder 5 steht,
A² für gegebenenfalls durch C₁-C₄-Alkyl und/oder C₁-C₄-Alkoxy-carbonyl und /oder C₁-C₄-Alkenyloxy-carbonyl substituiertes Alkandiyl mit 1 oder 2 Kohlenstoffatomen steht,
R¹⁴ für Hydroxy, Mercapto, Amino, C₁-C₆-Alkoxy, C₁-C₆-Alkylthio, C₁-C₆-Alkylamino oder Di-(C₁-C₄-alkyl)-amino steht,
R¹⁵ für Hydroxy, Mercapto, Amino, C₁-C₇-Alkoxy, C₁-C₆-Alkenyloxy, C₁-C₆-Alkenyloxy-C₁-C₆-alkoxy, C₁-C₆-Alkylthio, C₁-C₆-Alkylamino oder Di-(C₁-C₄-alkyl)-amino steht,
R¹⁶ für gegebenenfalls durch Fluor, Chlor und/oder Brom substituiertes C₁-C₄-Alkyl steht,
R¹⁷ für Wasserstoff, jeweils gegebenenfalls durch Fluor, Chlor und/oder Brom substituiertes C₁-C₆-Alkyl, C₂-C₆-Alkenyl oder C₂-C₆-Alkinyl, C₁-C₄-Alkoxy-C₁-C₄-alkyl, Dioxolanyl-C₁-C₄-alkyl, Furyl, Furyl-C₁-C₄-alkyl, Thienyl, Thiazolyl, Piperidinyl, oder gegebenenfalls durch Fluor, Chlor und/oder Brom oder C₁-C₄-Alkyl substituiertes Phenyl steht,
R¹⁸ für Wasserstoff, jeweils gegebenenfalls durch Fluor, Chlor und/oder Brom substituiertes C₁-C₆-Alkyl, C₂-C₆-Alkenyl oder C₂-C₆-Alkinyl, C₁-C₄-Alkoxy-C₁-C₄-alkyl, Dioxolanyl-C₁-C₄-alkyl, Furyl, Furyl-C₁-C₄-alkyl, Thienyl, Thiazolyl, Piperidinyl, oder gegebenenfalls durch Fluor, Chlor und/oder Brom oder C₁-C₄-Alkyl substituiertes Phenyl steht, oder R¹⁷ und R¹⁸ auch gemeinsam für jeweils gegebenenfalls durch C₁-C₄-Alkyl, Phenyl, Furyl, einen annellierten Benzolring oder durch zwei Substituenten, die gemeinsam mit dem C-Atom, an das sie gebunden sind, einen 5-oder 6-gliedrigen Carboxyclus bilden, substituiertes C₃-C₆-Alkandiyl oder C₂-C₅-Oxaalkandiyl steht,
R¹⁹ für Wasserstoff, Cyano, Halogen, oder für jeweils gegebenenfalls durch Fluor, Chlor und/oder Brom substituiertes C₁-C₄-Alkyl, C₃-C₆-Cycloalkyl oder Phenyl steht,
R²⁰ für Wasserstoff, jeweils gegebenenfalls durch Hydroxy, Cyano, Halogen oder C₁-C₄-Alkoxy substituiertes C₁-C₆-Alkyl, C₃-C₆-Cycloalkyl oder Tri-(C₁-C₄-alkyl)-silyl steht,
R²¹ für Wasserstoff, Cyano, Halogen, oder für jeweils gegebenenfalls durch Fluor, Chlor und/oder Brom substituiertes C₁-C₄-Alkyl, C₃-C₆-Cycloalkyl oder Phenyl steht,
X¹ für Nitro, Cyano, Halogen, C₁-C₄-Alkyl, C₁-C₄-Halogenalkyl, C₁-C₄-Alkoxy oder C₁-C₄-Halogenalkoxy steht,
X² für Wasserstoff, Cyano, Nitro, Halogen, C₁-C₄-Alkyl, C₁-C₄-Halogenalkyl, C₁-C₄-Alkoxy oder C₁-C₄-Halogenalkoxy steht,
X³ für Wasserstoff, Cyano, Nitro, Halogen, C₁-C₄-Alkyl, C₁-C₄-Halogenalkyl, C₁-C₄-Alkoxy oder C₁-C₄-Halogenalkoxy steht,
und/oder die folgenden durch allgemeine Formeln definierten Verbindungen
der allgemeinen Formel (IId) 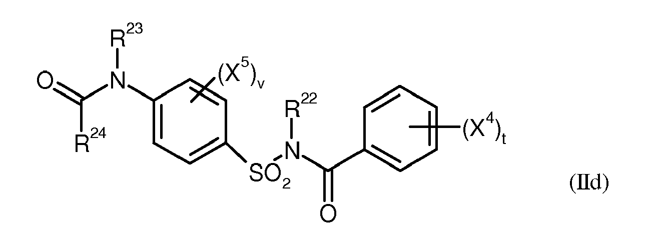
oder der allgemeinen Formel (IIe) 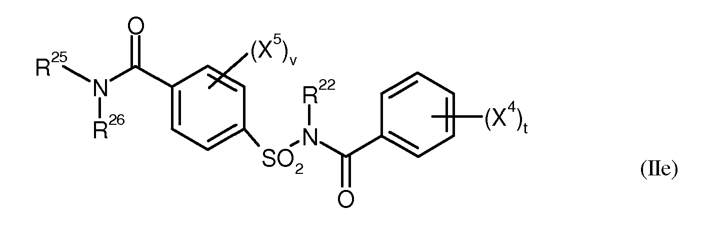 wobei t für eine Zahl 0, 1, 2, 3, 4 oder 5 steht,
v für eine Zahl 0, 1, 2, 3, 4 oder 5 steht,
R²² für Wasserstoff oder C₁-C₄-Alkyl steht,
R²³ für Wasserstoff oder C₁-C₄-Alkyl steht,
R²⁴ für Wasserstoff, jeweils gegebenenfalls durch Cyano, Halogen oder C₁-C₄-Alkoxy substituiertes C₁-C₆-Alkyl, C₁-C₆-Alkoxy, C₁-C₆-Alkylthio, C₁-C₆-Alkylamino oder Di-(C₁-C₄-alkyl)-amino, oder jeweils gegebenenfalls durch Cyano, Halogen oder C₁- C₄-Alkyl substituiertes C₃-C₆-Cycloalkyl, C₃-C₆-Cycloalkyloxy, C₃-C₆-Cycloalkyl- thio oder C₃-C₆-Cycloalkylamino steht,
R²⁵ für Wasserstoff, gegebenenfalls durch Cyano, Hydroxy, Halogen oder C₁-C₄-Alkoxy substituiertes C₁-C₆-Alkyl, jeweils gegebenenfalls durch Cyano oder Halogen sub- stituiertes C₃-C₆-Alkenyl oder C₃-C₆-Alkinyl, oder gegebenenfalls durch Cyano, Halogen oder C₁-C₄-Alkyl substituiertes C₃-C₆-Cycloalkyl steht,
R²⁶ für Wasserstoff, gegebenenfalls durch Cyano, Hydroxy, Halogen oder C₁-C₄-Alkoxy substituiertes C₁-C₆-Alkyl, jeweils gegebenenfalls durch Cyano oder Halogen sub- stituiertes C₃-C₆-Alkenyl oder C₃-C₆-Alkinyl, gegebenenfalls durch Cyano, Halogen oder C₁-C₄-Alkyl substituiertes C₃-C₆-Cycloalkyl, oder gegebenenfalls durch Nitro, Cyano, Halogen, C₁-C₄-Alkyl, C₁-C₄-Halogenalkyl, C₁-C₄-Alkoxy oder C₁-C₄- Halogenalkoxy substituiertes Phenyl steht, oder zusammen mit R²⁵ für jeweils gege- benenfalls durch C₁-C₄-Alkyl substituiertes C₂-C₆-Alkandiyl oder C₂-C₅-Oxa- alkandiyl steht,
X⁴ für Nitro, Cyano, Carboxy, Carbamoyl, Formyl, Sulfamoyl, Hydroxy, Amino, Halogen, C₁-C₄-Alkyl, C₁-C₄-Halogenalkyl, C₁-C₄-Alkoxy oder C₁-C₄-Halogenalkoxy steht, und
X⁵ für Nitro, Cyano, Carboxy, Carbamoyl, Formyl, Sulfamoyl, Hydroxy, Amino, Halogen, C₁-C₄-Alkyl, C₁-C₄-Halogenalkyl, C₁-C₄-Alkoxy oder C₁- C₄-Halogenalkoxy steht.

12. Verbindungen der Formel (III) 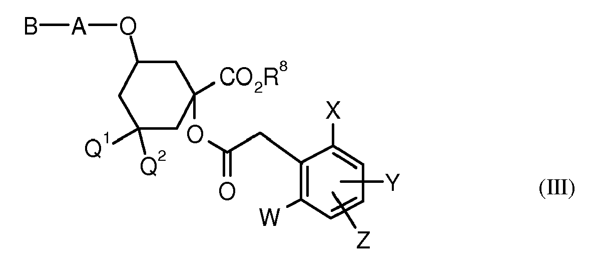 in welcher
A, B, Q¹, Q², W, X, Y, Z und R⁸ die oben angegebenen Bedeutungen haben.

13. Verbindungen der Formel (XX) 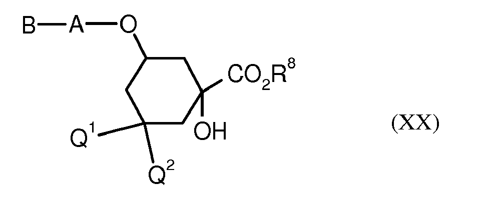 in welcher
A, B, Q¹, Q² und R⁸ die oben angegebenen Bedeutungen haben.

14. Schädlingsbekämpfungsmittel und/oder Herbizide und/oder Fungizide, **gekennzeichnet durch** einen Gehalt an mindestens einer Verbindung der Formel (I) gemäß Anspruch 1.

15. Verfahren zur Bekämpfung von tierischen Schädlingen und/oder unerwünschten Pflanzenbewuchs und/oder Pilzen, **dadurch gekennzeichnet, dass** man Verbindungen der Formel (I) gemäß Anspruch 1 auf Schädlinge und/oder ihren Lebensraum einwirken lässt.

16. Verwendung von Verbindungen der Formel (I) gemäß Anspruch 1 zur Bekämpfung von tierischen Schädlingen und/oder unerwünschtem Pflanzenbewuchs und/oder Pilzen.

17. Verfahren zur Herstellung von Schädlingsbekämpfungsmitteln und/oder Herbiziden und/oder Fungiziden, **dadurch gekennzeichnet, dass** man Verbindungen der Formel (I) gemäß Anspruch 1 mit Streckmitteln und/oder oberflächenaktiven Stoffen vermischt.

18. Verwendung von Verbindung der Formel (I) gemäß Anspruch 1 zur Herstellung von Schädlingsbekämpfungsmitteln und/oder Herbiziden und/oder Fungiziden.

19. Mittel nach Anspruch 11, bei dem die die Kulturpflanzen-Verträglichkeit verbessernde Verbindung aus der folgenden Gruppe von Verbindungen ausgewählt ist:
Cloquintocet-mexyl, Fenchlorazole-ethyl, Isoxadifen-ethyl, Mefenpyr-diethyl, Furilazole, Fenclorim, Cumyluron, Dymron oder die Verbindungen IIe-5 oder IIe-11.

20. Verfahren zum Bekämpfen von unerwünschtem Pflanzenwuchs, **dadurch gekennzeichnet, dass** man ein Mittel gemäß Anspruch 11 auf die Pflanzen oder ihre Umgebung einwirken lässt.

21. Verwendung eines Mittels gemäß Anspruch 11 zur Bekämpfung von unerwünschten Pflanzenwuchs.

22. Verfahren zur Bekämpfung von unerwünschten Pflanzenbewuchs, **dadurch gekennzeichnet, dass** man eine Verbindung der Formel (I) gemäß Anspruch 1 und eine die Kulturpflanzenverträglichkeit verbessernde Verbindung gemäß Anspruch 11 in zeitlich naher Abfolge getrennt oder in Mischung auf die Pflanzen oder ihre Umgebung einwirken lässt.
